# EUROPEAN PATENT SPECIFICATION

(11) **EP 2 449 084 B1**
(45) Date of publication and mention of the grant of the patent: **28.10.2020**
(21) Application number: 10794826.7
(22) Date of filing: 02.07.2010
(51) Int. Cl.: C12M 1/00, C12M 1/34, C12M 1/04, C12M 1/107, C12P 5/02

(54) **METHOD FOR PRODUCING METHANE FROM CARBON DIOXIDE**
VERFAHREN ZUR HERSTELLUNG VON METHAN AUS KOHLENDIOXID
PROCÉDÉ POUR LA PRODUCTION DE MÉTHANE À PARTIR DE DIOXIDE DE CARBONE

(30) Priority: 02.07.2009 US 222621 P
(43) Date of publication of application: 09.05.2012
(73) Proprietor: The University of Chicago, Chicago, IL 60637 (US)
(72) Inventor: METS, Laurens, Chicago IL 60654 (US)
(74) Representative: Heselberger, Johannes
(86) International application number: PCT/US2010/040944
(87) International publication number: WO 2011/003081

(56) References cited:
- WO-A1-02/075022
- WO-A1-2008/094282
- WO-A2-2009/155587
- US-A- 4 608 133
- US-A- 5 922 204
- US-A1- 2006 011 491
- US-A1- 2008 286 624
- CHENG ET AL.: 'Direct Biological Conversion of Electrical Current into Methane by Electromethanogenesis' ENVIRON. SCI. TECHNOL., [Online] vol. 43, no. 10, 26 March 2009, pages 3953 - 3958, XP008150151 Retrieved from the Internet: <URL:http://pubs.acs.org/doi/abs/10.1021/es 803531g> [retrieved on 2010-08-13]
- WALDRON ET AL.: ''Differential Expression of Methanogenesis Genes of Methanothermobacter thermoautotrophicus (Formerly Methanobacterium thermoautotrophicum) in Pure Culture and in Cocultures with Fatty Acid-Oxidizing Syntrophs' APPLIED AND ENVIRONMENTAL MICROBIOLOGY vol. 68, no. 3, March 2002, pages 1173 - 1179, XP002551248
- LUO ET AL.: 'Salinity Constraints on Subsurface Archaeal Diversity and Methanogenesis in Sedimentary Rock Rich in Organic Matter' APPL ENVIRON MICROBIOL. vol. 73, no. 13, July 2007, pages 4171 - 4179, XP008150152
- 'GreenShift Industrial Design Corporation: GreenShift Acquires Rights to Patented Carbon Dioxide Reduction Technology' NEW STRAIN OF THERMOPHILIC CYANOBACTERIA CONVERTS EXHAUST CARBON DIOXIDE INTO PURE OXYGEN AND CLEAN WATER., [Online] 12 December 2005, page 2, XP008150154 Retrieved from the Internet: <URL:http://www.businesswire.com/portal/sit e/home/permalink/?ndmViewld=news_view8news Id=20051212005469&newsLang=en> [retrieved on 2010-08-13]

## Description

### Background

This patent relates to conversion of carbon dioxide into methane using electrical energy, which conversion may also create or generate byproducts, such as carbon credits or oxygen, for example.

The US annually consumes about 90 ExaJoules (EJ) of carbon-based fuels, 88% of its total energy consumption in 2008. The use of these fuels is supported by heavily capitalized processing, distribution and utilization industries.

The sustainability of these systems is questionable on two counts. First, the US imports 25% of the energy it uses, a proportion that is projected to increase substantially. Imported energy is obtained from sources that are under pressure to serve increasing demand from growing economies in other parts of the world. Second, more than 96% of the carbon-based fuels are obtained from fossil reserves, which are finite. Useful energy is obtained from carbon-based fuels by oxidizing reduced states of carbon to carbon dioxide. For fossil fuels, this process is basically open-loop, producing CO₂ with no compensating carbon reduction process to close the cycle. The consequent gradual accumulation of atmospheric CO₂ is beginning to cause changes in the global climate that threaten many aspects of our way of life. Therefore, a process that can close this carbon energy cycle for the total energy economy is needed.

An annual flux of 58,000 EJ of solar energy strikes US soil, making it our most abundant carbon-free energy resource - 500 times current consumption. Solar energy has the unique advantage of being a domestic resource not just in the US, but everywhere that people live. Its widespread use as a primary resource would secure energy independence throughout the world. Nevertheless, today solar energy is only a marginal component of the energy economy, providing less than 0.1% of marketed US energy consumption. Exploitation of solar energy is limited principally because it is intermittent and cannot be relied upon to provide the base-load energy that must be available whenever needed. What is lacking is a method for storing solar energy in a stable form that can be tapped whenever needed. Ideally, such a storage form should fit smoothly into the existing energy infrastructure so that it can be quickly deployed once developed.

There is a need in the energy industry for systems to convert one form of energy into another. In particular, there is a need for systems to convert electricity into a form of energy that can be stored inexpensively on industrial scales. Many sources of electricity generation cannot be adjusted to match changing demand. For example, coal power plants run most efficiently when maintained at a constant rate and cannot be adjusted as easily as natural gas (methane) fired power plants. Likewise, wind turbines generate electricity when the wind is blowing which may not necessarily happen when electricity demand is highest.

There is also a need to convert electricity into a form that can be transported long distances without significant losses. Many opportunities for wind farms, geothermal, hydroelectric or solar based power generation facilities are not located close to major population centers, but electric power losses over hundreds of miles add significant cost to such distant power facilities.

Methane is one of the most versatile forms of energy and can be stored easily. There already exists much infrastructure for transporting and distributing methane as well as infrastructure for converting methane into electricity and for powering vehicles. Methane also has the highest energy density per carbon atom of all fossil fuels, and therefore of all fossil fuels, methane releases the least carbon dioxide per unit energy when burned. Hence, systems for converting electricity into methane would be highly useful and valuable in all energy generation and utilization industries.

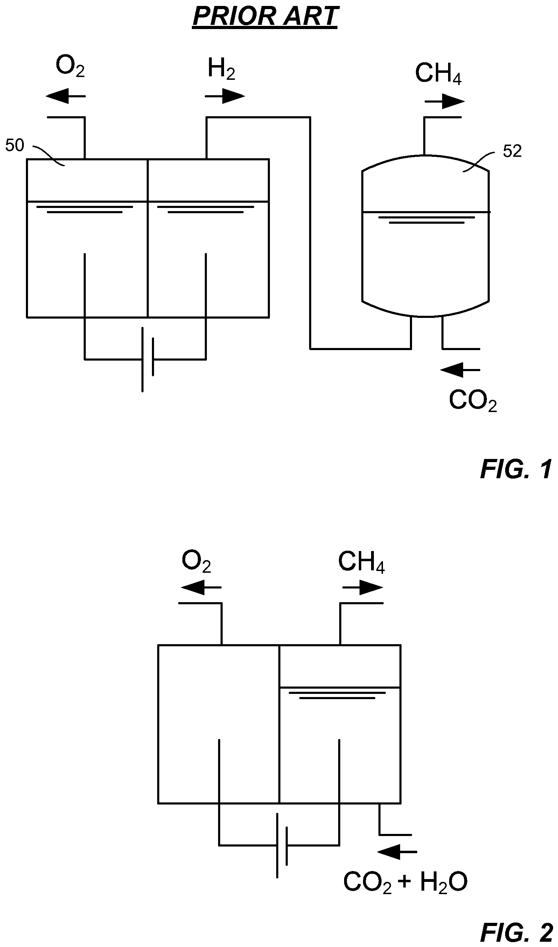

In principle, it would be possible to produce methane from electric power in a two-step process, such as outlined schematically in Fig. 1. The first step would use the electric power to make hydrogen gas from water in a standard water electrolysis system 50. In a second step, the hydrogen gas could then be pumped into a methanogenic reaction chamber 52 such as a highly specific biological reactor of methanogenic microbes. One such biological reactor is described in U.S. patent application 12/333,932 by Laurens Mets. WO 2002075022 relates to methods using neutral red to mediate the interconversion of chemical and electrical energy.

### Summary

The present disclosure describes a system to convert electric power into methane including a biological reactor having at least a first chamber containing at least a cathode, a culture comprising methanogenic microorganisms, and water, and a second chamber containing at least an anode. The biological reactor has an operating state wherein the culture is maintained at a temperature above 50 °C. The system also includes a source of electricity coupled to the anode and the cathode, a supply of carbon dioxide coupled to the first chamber, and an outlet to receive methane from the first chamber.

The present invention is directed to a method for producing methane, the method comprising:
(a) supplying electricity to an anode and a porous electrically conductive cathode of a biological reactor, wherein the biological reactor
   (i) is coupled to a source of electricity and a source of carbon dioxide,
   (ii) has at least a first chamber containing at least the cathode, a culture comprising methanogenic microorganisms, and water, a second chamber containing at least the anode, and a proton permeable, gas impermeable barrier separating the anode from the cathode, and
   (iii) has a current collector coupled to each of the anode and the cathode, wherein the cathode is impregnated with the methanogenic microorganisms and with an aqueous electrolytic medium,
      wherein the methanogenic microorganisms are in a passage formed between the barrier and the current collector coupled to the cathode and located between an inlet for carbon dioxide and an outlet for methane,
      wherein the culture is maintained in the aqueous electrolytic medium in the passage of the first chamber at a temperature above 50 °C;
(b) supplying carbon dioxide to the first chamber through the inlet of the first chamber, whereupon the carbon dioxide is dissolved into the aqueous electrolytic medium;
(c) circulating the aqueous electrolytic medium comprising the dissolved carbon dioxide through the cathode; and
(d) collecting methane from the outlet of the first chamber.
   According to another aspect of the present disclosure, a method of converting electricity into methane includes supplying electricity to an anode and a cathode of biological reactor having at least a first chamber containing at least the cathode, a culture comprising methanogenic microorganisms, and water, and a second chamber containing at least the anode, the biological reactor having an operating state, wherein the culture is maintained at a temperature above 50 °C. The method also includes supplying carbon dioxide to the first chamber, and collecting methane from the first chamber.

According to a further aspect of the present disclosure, a method of creating carbon credits includes supplying electricity to an anode and a cathode of biological reactor having at least a first chamber containing at least the cathode, a culture comprising methanogenic microorganisms, and water, and a second chamber containing at least the anode, the biological reactor having an operating state wherein the culture is maintained at a temperature about 50 °C. The method also includes supplying carbon dioxide to the first chamber, and receiving carbon credits for the carbon dioxide converted in the biological reactor into methane.

### Brief Description of the Drawings

It is believed that the disclosure will be more fully understood from the following description taken in conjunction with the accompanying drawings. Some of the figures may have been simplified by the omission of selected elements for the purpose of more clearly showing other elements. Such omissions of elements in some figures are not necessarily indicative of the presence or absence of particular elements in any of the exemplary embodiments, except as may be explicitly delineated in the corresponding written description. None of the drawings is necessarily to scale.
Fig. 1 is a schematic view of a system for converting carbon dioxide into methane according to the prior art;
Fig. 2 is a schematic view of a system for converting carbon dioxide into methane according to the present disclosure;
Fig. 3 is a cross-sectional view of a reference example of a biological reactor for converting carbon dioxide into methane;
Fig. 4 is a cross-sectional view of an embodiment of a biological reactor for converting carbon dioxide into methane;
Fig. 5 is a cross-sectional view of yet another embodiment of a biological reactor for converting carbon dioxide into methane;
Fig. 6 is a cross-sectional view of a further embodiment of a biological reactor for converting carbon dioxide into methane;
Fig. 7 is a schematic view of an embodiment of a biological reactor with a plurality anodes and cathodes;
Fig. 8 is a cross-sectional view of the system of Fig. 7 taken along line 8-8;
Fig. 9 is a cross-sectional view of one of the plurality of biological reactors of Fig. 8 taken along line 9-9;
Fig. 10 is a cross-sectional view of a variant biological reactor for use in the system of Fig. 7;
Fig. 11 is a schematic view of a series arrangement of biological reactors according to the present disclosure;
Fig. 12 is a schematic view of a parallel arrangement of biological reactors according to the present disclosure;
Fig. 13 is a schematic view of a stand-alone system according to the present disclosure;
Fig. 14 is a schematic view of an integrated system according to the present disclosure; and
Fig. 15 is a graph of methane production over time with varying voltage applied across the anode and cathode of a biological reactor according to Fig. 3.

### Detailed Description of Various Embodiments

Although the following text sets forth a detailed description of numerous different embodiments and reference examples, it should be understood that the legal scope of the invention is defined by the words of the claims set forth at the end of this patent.

It should also be understood that, unless a term is expressly defined in this patent using the sentence "As used herein, the term '_________' is hereby defined to mean..." or a similar sentence, there is no intent to limit the meaning of that term, either expressly or by implication, beyond its plain or ordinary meaning, and such term should not be interpreted to be limited in scope based on any statement made in any section of this patent (other than the language of the claims). To the extent that any term recited in the claims at the end of this patent is referred to in this patent in a manner consistent with a single meaning, that is done for sake of clarity only so as to not confuse the reader, and it is not intended that such claim term be limited, by implication or otherwise, to that single meaning.

The present disclosure addresses the processing or conversion of carbon dioxide into methane using an electro-biological apparatus. The apparatus may be referred to herein as a biological reactor, bioreactor, processor, converter or generator. It will be recognized that this designation is not intended to limit the role that the converter may perform within a system including one or more converters.

For example, the apparatus provides a non-fossil carbon-based energy resource. In this regard, the apparatus is being used to generate an energy resource that may be substituted for fossil-based carbon fuels, to reduce reliance on fossil-based carbon fuels, for example. Additionally, the apparatus converts or processes carbon dioxide to generate this energy resource. In this regard, the apparatus removes carbon dioxide from the environment, which may be a beneficial activity in and of itself. Such removal of carbon dioxide from the environment may happen by removing carbon dioxide directly from the atmosphere or by utilizing carbon dioxide from another industrial process and thereby preventing such carbon dioxide from being released into the atmosphere or into a storage system or into another process. Further, the apparatus converts or processes carbon dioxide into methane using electricity to convert electricity into another energy resource when demand for electricity may be such that the electricity would otherwise be wasted or even sold at a loss to the electricity producer, for example. In this regard, the apparatus may be viewed as part of an energy storage system. In the operation of a power grid, or an individual power plant or other power source on the grid, or as part of a facility not associated with a power grid, or in the operation of a biological reactor, available power output may be used by one or more biological reactors to consume as an input carbon dioxide, water or electrical power and to produce methane or oxygen when business conditions are favorable to provide an incentive greater than for other use of such inputs. Such conditions may exist when certain regulatory policies, power purchase agreements, carbon credits, futures trading opportunities, storage capacity, electrical demand, taxes, tax credits or abatements, contracts, customer preferences, transmission capacity, pricing conditions, or other market incentives can provide sufficient value for operation of the biological reactor to produce methane or oxygen or to consume carbon dioxide, water or electrical power. In addition to the above and other uses, the apparatus converts electrical energy or power into methane which may be transmitted via natural gas transmission pipes which on a per unit energy basis are less expensive than electrical transmission lines and in some locales the electrical transmission lines may not have as much spare transmission capacity as the natural gas transmission lines. In this regard, the apparatus may be viewed as part of an energy transmission system. All of these roles may be performed by an apparatus according to the present disclosure.

As illustrated in Fig. 2, the biological reactor according to the present disclosure may include a container that is divided into at least a first chamber and a second chamber. At least one cathode is disposed in the first chamber, and at least one anode is disposed in the second chamber. The first chamber has inlets that are connected to a source of carbon dioxide gas and a source of water, and an outlet that is connected, for example, to a storage device used to store methane produced in the first chamber. The first and second chambers are separated by a divider that is permeable to ions (protons) to permit them to move from the second chamber to the first chamber. This membrane is impermeable to the gaseous products and by-products of the conversion process to limit or prevent them from moving between the first chamber and the second chamber.

### I. Description of System and Biological Reactor

Methanogenic microorganisms may be cultured, for example, in shake or stirred tank bioreactors, hollow fiber bioreactors, or fluidized bed bioreactors, and operated in a batch, fed batch, continuous, semi-continuous, or perfusion mode. In batch mode (single batch), an initial amount of medium containing nutrients necessary for growth is added to the biological reactor, and the biological reactor is operated until the number of viable cells rises to a steady-state maximum, or stationary condition. In fed-batch mode, concentrated media or selected amounts of single nutrients are added at fixed intervals to the culture. Methanogenic microorganisms can survive for years under fed batch conditions, provided that any waste products are effectively minimized or removed to prevent loss of efficiency of methane production over time. Any inhibitory waste products may be removed by continuous perfusion production processes, well known in the art. Perfusion processes may involve simple dilution by continuous feeding of fresh medium into the culture, while the same volume is continuously withdrawn from the reactor. Perfusion processes may also involve continuous, selective removal of medium by centrifugation while cells are retained in the culture or by selective removal of toxic components by dialysis, adsorption, electrophoresis, or other methods. Continuously perfused cultures may be maintained for weeks, months or years.

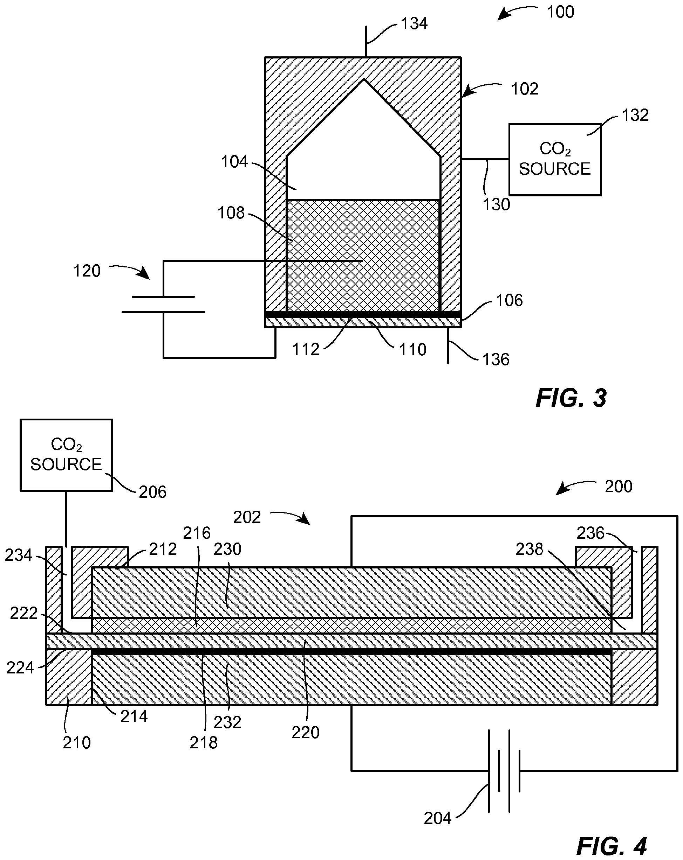

Fig. 3 illustrates a reference example of a system 100 that may be used, for example, to convert electric power into methane. The system 100 includes a biological reactor 102 having at least a first chamber 104 and a second chamber 106. The first chamber 104 may contain at least a cathode 108, a culture comprising living methanogenic microorganisms, and water. In particular, the culture may comprise autotrophic and/or hydrogenotrophic methanogenic archaea, and the water may be part of an aqueous electrolytic medium compatible with the living microorganisms. The second chamber may contain at least an anode 110.

The biological reactor 102 may also include a selectively permeable barrier 112, which may be a proton permeable barrier, separating the anode 110 from the cathode 108. The barrier 112 may be at least gas semipermeable (e.g., certain gases may pass through, while others are limited), although according to certain reference examples, the barrier 112 is impermeable to gases. According to certain reference examples, the barrier 112 may prevent gases produced on each side of the barrier from mixing.

According to certain reference examples, the barrier 112 may be a solid polymer electrolyte membrane (PEM), such as is available under tradename Nafion from E. I. du Pont de Nemours and Company. For optimum energy conversion in the reactor according to certain reference examples, it is believed that the permeability of the barrier to hydronium ions should preferably be a minimum of two orders of magnitude greater on a molar basis than permeability of the barrier to oxygen under conditions of operation of the reactor. Other suitable PEM membranes that meet these criteria, such as sulfonated polyarylene block copolymers (see, e.g., Bae, B., K Miyatake, and M. Watanabe. Macromolecules 43:2684-2691 (2010)) and PTFE-supported Nafion (see, e.g., G.-B. Jung, et al, J Fuel Cell Technol 4:248-255(2007)), are under active development in numerous laboratories. Suitable commercial PEM membranes, in addition to Nafion, include Gore-Select (PRIMEA), Flemion (Asahi), 3M Fluoropolymer ionomer, SPEEK (Polyfuel), Kynar blended membrane (Arkema), Fumapem (FuMA-Tech), and Solupor (Lydall).

In the biological reactor 102, water acts as a primary net electron donor for the methanogenic microorganisms (e.g, methanogenic archaea) in the biological reactor. Accordingly, it is also believed that the barrier 112 should be permeable for hydronium ions (H₃O⁺) (i.e., enable hydronium ions to cross the barrier 112 from the anode 110 to the cathode 108 and complete the electrical circuit).

The cathode 108 may be of a high surface to volume electrically conductive material. For example, the cathode 108 may be made of a porous electrically conductive material. In particular, the cathode 108 may be made from a reticulated vitreous carbon foam according to certain reference examples. As explained in greater detail below, other materials may be used. According to certain reference examples, the pores of the cathode may be large enough (e.g., greater than 1-2 micrometers in minimum dimension) to accommodate living methanogenic microorganisms within the pores. The electrical conductivity of the cathode matrix is preferably at least two orders of magnitude greater than the ion conductivity of the aqueous electrolytic medium contained within its pores.

It will be recognized that the role of the cathode 108 is to supply electrons to the microorganisms while minimizing side-reactions and minimizing energy loss. Additionally, it is advantageous for the cathode to be inexpensive. At the present time, it is believed that certain materials may be more or less suitable for inclusion in the reactor.

For instance, platinum cathodes may be less suitable for inclusion in the reactor. In this regard, the platinum provides a surface highly active for catalyzing hydrogen gas production from the combination of protons or hydronium ions with electrons provided by the cathode. The activity of platinum cathode catalysts for hydrogen formation in aqueous solutions is so high that the hydrogen concentration in the vicinity of the catalyst quickly rises above its solubility limit and hydrogen gas bubbles emerge. Despite the fact that the methanogenic microorganisms are evolved to consume hydrogen in the process of methane formation, hydrogen in bubbles re-dissolves only slowly in the medium and is largely unavailable to the microorganisms. Consequently, much of the energy consumed in hydrogen formation at a platinum catalyst does not contribute to methane formation. Additionally, the binding energy of hydrogen is higher than the binding energy per bond of methane. This difference results in an energetic loss when hydrogen gas is produced as an intermediate step.

On the other hand, a solid carbon cathode is an example of an inexpensive, electrically conductive material that has low activity for hydrogen formation and that can provide electrons to microorganisms. However, it will be recognized that electron transfer between microorganisms and an external electron source or sink, such as an electrode, requires some level of proximity between the microorganisms and the electrode and the total rate of electron transfer is related to the area of electrode in close contact with microorganisms. Since a porous electrode that allows the microorganisms to enter the pores has a much larger surface area in proximity to the microorganisms than a planar electrode of equivalent dimensions, the porous electrode is expected to provide superior volumetric current density.

A suitable porous cathode material may be provided by reticulated vitreous carbon foam, as demonstrated in Example 1. It is inexpensive and conductive. Its porous structure provides for electrical connections to a large number of the microorganisms allowing for a high volumetric productivity. Additionally, the vitreous nature of the carbon provides low activity for hydrogen production, which increases both energetic and Faradaic efficiency. It will also be recognized that vitreous carbon is also very resistant to corrosion.

Other suitable porous electrode materials may include, but are not limited to graphite foam (see, e.g., US Patent 6033506), woven carbon and graphite materials, carbon, graphite or carbon nanotube impregnated paper (see, e.g., Hu, L., et al. Proc Nat Acad Sci USA 106: 21490-4 (2009)), and metal foams, or woven or non-woven mesh comprised of materials, such as titanium, that are non-reactive under the conditions of the reaction and that present a high surface to volume ratio.

Further enhancement of electron transfer between the cathode and the microorganisms may be achieved with conductive fibers. Suitable conductive fibers may consist of conductive pili generated by the microorganisms as described in more detail below. Alternatively or additionally, nanowires, such as carbon nanotubes (Iijima, S. Nature 354:56 (1991)), may be attached directly to the cathode. Wang, J. et al, J. Am. Chem. Soc. 125:2408-2409 (2003) and references therein, , provide techniques for modifying glassy carbon electrodes with carbon nanotubes. Additionally, conductive organic polymers may be used for this purpose (see, e.g., Jiang, P. Angewandte Chemie 43:4471-4475 (2004)). Non-conductive materials that bind the microorganisms to the surface of the electrode may also enhance electron transfer. Suitable non-conductive binders include but are not limited to poly-cationic polymers such as poly-lysine or poly(beta -aminosulfonamides). The living methanogenic microorganisms may also produce biological materials, such as those that support biofilm formation, that effectively bind them to the surface of the electrode.

The anode 110 may comprise a Pt-carbon catalytic layer or other materials known to provide low overpotential for the oxidation of water to oxygen.

As illustrated in Fig. 3, a source of electricity 120 is coupled to the anode 110 and the cathode 108. As mentioned above, the source 120 may be generated from carbon-free, renewable sources. In particular, the source 120 may be generated from carbon-free, renewable sources such as solar sources (e.g., photovoltaic cell arrays) and wind sources (e.g., wind turbines). However, according to other reference examples, the source 120 may be a coal power plant, a fuel cell, a nuclear power plant. According to still further reference examples, the source 120 may be a connector to an electrical transmission grid. Further details are provided below.

Based upon dynamic computational models of porous electrodes containing aqueous electrolyte, the optimal conductivity of the cathode electrolyte is believed to be preferably in the range of 0.01 to 0.25 S/cm or higher in the operating state of the reactor. Higher conductivity of the electrolyte may reduce ohmic losses in the reactor and hence may increase energy conversion efficiency. Computational models further suggest that the optimal thickness of the porous cathode (perpendicular to the planes of the reactor layers) may be between 0.2 cm and 0.01 cm, or less. Thinner cathode layers may have lower ohmic resistance under a given set of operating conditions and hence may have an increased energy conversion efficiency.

The biological reactor 102 may operate at an electrical current density above 6 mA/cm². For example, the biological reactor 102 may operate at an electrical current density of between 6 and 10 mA/cm². According to certain reference examples, the biological reactor 102 may operate at electrical current densities at least one order of magnitude higher (e.g., 60-100 mA/cm²). The current may be supplied as direct current, or may be supplied as pulsed current such as from rectified alternating current. The frequency of such pulsed current is not constrained by the properties of the reactor. The frequency of the pulsed current may be variable, such as that generated by variable speed turbines, for example wind turbines lacking constant-speed gearing

The living methanogenic microorganisms (e.g., autotrophic and/or hydrogenotrophic methanogenic archaea) may be impregnated into the cathode 108. While various reference examples and variants of the microorganisms are described in greater detail in the following section, it is noted that the microorganisms may be a strain of archaea adapted to nearly stationary growth conditions according to certain reference examples. In addition, according to certain reference examples, the microorganisms may be Archaea of the subkingdom *Euryarchaeota*, in particular, the microorganisms may consist essentially of *Methanothermobacter thermautotrophicus.*

As explained in greater detail below, the biological reactor 102 may have an operating state wherein the culture is maintained at a temperature above 50° C, although certain reference examples may have an operating state in the range of between approximately 60° C and 100° C. The biological reactor 102 may also have a dormant state wherein electricity and/or carbon dioxide is not supplied to the reactor 102. According to such a dormant state, the production of methane may be significantly reduced relative to the operating state, such that the production may be several orders of magnitude less than the operating state, and likewise the requirement for input electrical power and for input carbon dioxide may be several orders of magnitude less than the operating state. According to certain reference examples, the biological reactor 102 may change between the operating state and the dormant state or between dormant state and operating state without addition of microorganisms to the reactor 102. Additionally, according to certain reference examples, the reactor 102 may change between dormant and operating state rapidly, and the temperature of the reactor 102 may be maintained during the dormant state to facilitate the rapid change.

The biological reactor 102 may have an inlet 130 connected to the first chamber for receiving gaseous carbon dioxide. The inlet 130 may be coupled to a supply of carbon dioxide 132 to couple the supply of carbon dioxide to the first chamber 104. The biological reactor 102 may also have an outlet 134 to receive methane from the first chamber.

The biological reactor 102 may also have an outlet 136 connected to the second chamber 106 for receiving byproducts. For example, gaseous oxygen may be generated in the second chamber 106 as a byproduct of the production of methane in the first chamber 104. According to certain reference examples, oxygen may be the only gaseous byproduct of the biological reactor 102. In either event, the gaseous oxygen may be received by the outlet 134 connected to the second chamber 106.

In keeping with the reference example of Fig. 3, a method of the present disclosure may include supplying electricity to the anode 110 and the cathode 108 of the biological reactor 102 having at least the first chamber 104 containing at least the cathode 108, a culture comprising living methanogenic microorganisms (e.g., autotrophic and/or hydrogenotrophic methanogenic archaea), and water (e.g., as part of an aqueous electrolytic medium compatible with the living microorganisms), and the second chamber 106 containing at least the anode 110, wherein the culture is maintained at a temperature above 50 °C. Further, the method may include generating electricity from carbon-free, renewable sources, such as from solar and wind sources, as noted above. According to certain reference examples, electricity may be supplied during a non-peak demand period. Further details are provided in section III, below.

The method may also include supplying carbon dioxide to the first chamber 104. As noted above, the method may include recycling carbon dioxide from at least a concentrated industrial source or atmospheric carbon dioxide, which carbon dioxide is supplied to the first chamber 104.

The method may further include collecting methane from the first chamber 104. The method may further include storing and transporting the methane. The method may also include collecting other gaseous products or byproducts of the biological reactor; for example, the method may include collecting oxygen from the second chamber 106.

It will be recognized that while the system of Fig. 3 may be viewed as operating to convert electricity into methane, it is also possible to view the system of Fig. 3 as operating to create or earn carbon credits, as an alternative to carbon sequestration for example. According to such a method, the method would include supplying electricity to the anode 110 and the cathode 108 of biological reactor 102 having at least the first chamber 104 containing at least the cathode 108, methanogenic microorganisms (e.g., methanogenic archaea), and water (e.g., as part of an aqueous electrolytic medium compatible with the living microorganisms), and a second chamber containing at least the anode, wherein the culture is maintained at a temperature above 50 °C. The method would also include supplying carbon dioxide to the first chamber 104. Finally, the method would include receiving carbon credits for the carbon dioxide converted in the biological reactor 102 into methane. According to such a method, the carbon dioxide may be recycled from a concentrated industrial source.

It will be recognized that the system 100 is only one such reference example of a system. Additional embodiments and variants of the system 100 are illustrated in Figs. 4-10, and will be described in the following section. While these embodiments are generally shown in cross-section, assuming a generally cylindrical shape for the reactor and disc-like shapes for the anode and cathode, which may be arranged parallel to one another as illustrated, it will be appreciated that other geometries may be used instead.

Fig. 4 illustrates a system 200 that includes a biological reactor 202, a source of electricity 204 and a source of carbon dioxide 206. As illustrated, the source of electricity 204 and the source of carbon dioxide 206 are both coupled to the biological reactor 202. The biological reactor 202 uses a circulating liquid/gas media, as explained in greater detail below.

The biological reactor 202 includes a housing 210 that defines, in part, first and second chambers 212, 214. The reactor 202 also includes a cathode 216 disposed in the first chamber 212, and an anode 218 disposed in the second chamber 214. The first and second chambers 212, 214 are separated by proton permeable, gas impermeable barrier 220, the barrier 220 having surfaces 222, 224 which also define in part the first and second chambers 212, 214.

The biological reactor 202 also includes current collectors 230, 232, one each for the cathode 216 and the anode 218. The current collector 230 for the cathode 216 may be made as a solid disc of material, so as to maintain a sealed condition within the chamber 212 between an inlet 234 for the carbon dioxide and an outlet 236 for the methane (and potentially byproducts). The inlet 234 and the outlet 236 may be defined in the housing 210. The current collector 232 for the anode 218 may also define a porous gas diffusion layer, on which the anode catalyst layer is disposed. It will be recognized that a porous gas diffusion layer should be provided so as to permit gaseous byproducts to exit the second chamber 214, because the barrier 220 prevents their exit through the outlet 236 via the first chamber 212.

In keeping with the disclosure above, the cathode 216 is made of a porous material, such as a reticulated carbon foam. The cathode 216 is impregnated with the methanogenic microorganisms and with the aqueous electrolytic medium. The methanogenic microorganisms (e.g., archaea) are thus in a passage 238 formed between the barrier 220 and the current collector 230 between the inlet 234 and the outlet 236.

In operation, carbon dioxide is dissolved into the aqueous electrolytic medium and is circulated through the cathode 216. The methanogenic microorganisms may reside within the circulating electrolytic medium or may be bound to the porous cathode 216. In the presence of an electric current, the methanogenic microorganisms process the carbon dioxide to generate methane. The methane is carried by the electrolytic medium out of the reactor 202 via the outlet 236. According to such an embodiment, post-processing equipment, such as a liquid/gas separator, may be connected to the outlet to extract the methane from the solution.

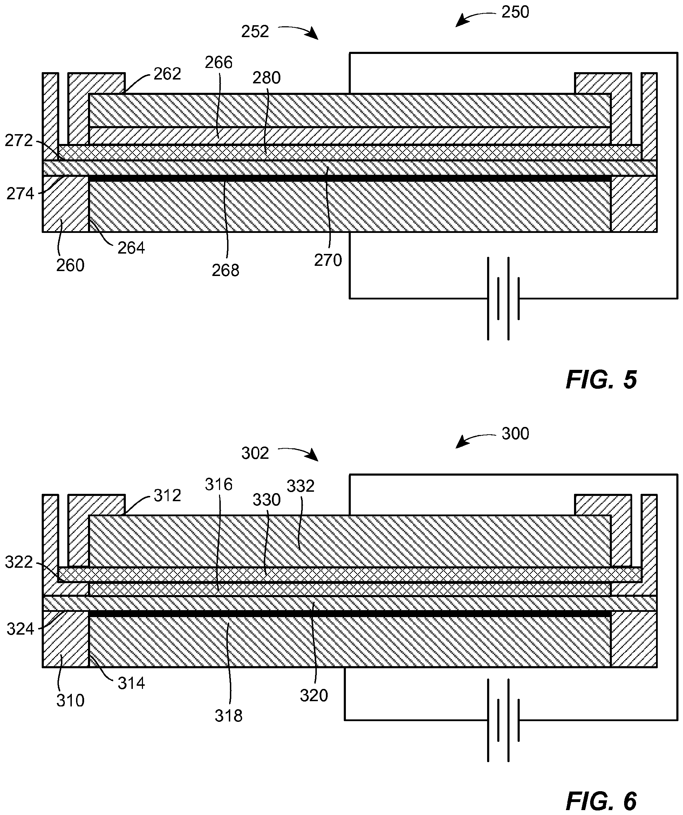

Fig. 5 illustrates a system 250 including a reactor 252 that is a variant of that illustrated in Fig. 4. Similar to the reactor 202, the reactor 252 includes a housing 260 that defines, in part, first and second chambers 262, 264. The reactor 252 also includes a cathode 266 disposed in the first chamber 262, and an anode 268 disposed in the second chamber 264. The first and second chambers 262, 264 are separated by proton permeable, gas impermeable barrier 270, the barrier 270 having surfaces 272, 274 that also define in part the first and second chambers 262, 264.

Unlike the embodiment illustrated in Fig. 4, the embodiment illustrated in Fig. 5 also includes a porous, proton conducting gas diffusion layer 280. The gas diffusion layer 280 is disposed between the cathode 266 and the barrier 270. Using this gas diffusion layer 280, gaseous carbon dioxide may enter the first chamber 212 through the gas diffusion layer 280 and then diffuse into the cathode 266, while gaseous methane produced by the microorganisms may diffuse from the cathode 266 into the layer 280 and then out of the first chamber 212. Proton-conducting gas diffusion layers suitable for use as layer 280 may be produced by coating porous materials with proton-conducting ionomer, by incorporating ionomer directly into the porous matrix, or by chemical derivitization of porous matrix materials with sulfate, phosphate, or other groups that promote proton-conduction, for example.

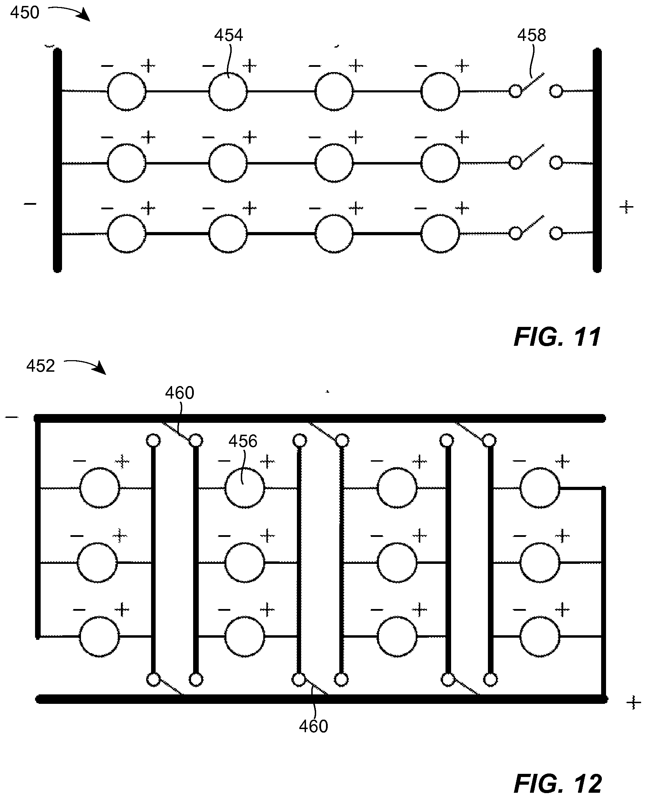

It will thus be recognized that the carbon dioxide and the methane are not carried by a circulating liquid media according to the embodiment of Fig. 5. Instead, the culture and the media are contained in the first chamber 262, while only the gaseous carbon dioxide and the methane circulate between inlet and outlet. Such an embodiment may present certain advantages relative to the reactor 202 of Fig. 4, in that the handling of the methane post-processing or generation may be simplified. Further, the absence of a circulating liquid media in the reactor 202 may simplify the serial connection between multiple reactors, as illustrated in Fig. 11. However, while the circulating media in the embodiment of Fig. 4 provided any water required by the culture, it may be necessary to couple equipment to the reactor to provide water vapor to the culture, in addition to the gaseous carbon dioxide. The electrolytic medium and microorganisms may be retained within the pores of the cathode 266 by surface tension or alternatively by including materials within the electrolyte that increase its viscosity or form a gel.

Fig. 6 illustrates a system 300 including a reactor 302 that is a variant of that illustrated in Fig. 5. Similar to the reactors 202 and 252, the reactor 302 includes a housing 310 that defines, in part, first and second chambers 312, 314. The reactor 302 also includes a cathode 316 disposed in the first chamber 312, and an anode 318 disposed in the second chamber 314. The first and second chambers 312, 314 are separated by proton permeable, gas impermeable barrier 320, the barrier 320 having surfaces 322, 324 that also define in part the first and second chambers 312, 314.

Moreover, similar to the embodiment illustrated in Fig. 5, the embodiment illustrated in Fig. 6 also includes a porous, proton conducting gas diffusion layer 330. However, the gas diffusion layer 330 is not disposed between the cathode 316 and the barrier 320, but instead is disposed between the cathode 316 and the current collector 332. In this arrangement, the gas diffusion layer 330 is current-conducting rather than proton-conduction like the gas diffusion layer 280 in reactor 252. Current would pass through the layer 330 into the cathode 316. As in the reactor 252, the carbon dioxide still would enter the first chamber 312 passes through the gas diffusion layer 330 and diffuse into the cathode 316, while methane produced by the microorganisms would diffuse from the cathode 316 through the layer 330.

As a result, the embodiment of Fig. 6 illustrates a reactor wherein the gaseous carbon dioxide enters the cathode from a side or along a path opposite that of the protons. By comparison, the embodiment of Fig. 5 illustrates a reactor wherein the gaseous carbon dioxide and the protons enter the cathode from the same side or along a similar path. The counter-diffusion of the embodiment of Fig. 6 may provide slower production than that of Fig. 5, but may provide acceptable production levels. As to the material used for the barrier 320 according to such an embodiment, it is believed that a porous carbon foam impregnated with Nafion particles may be suitable.

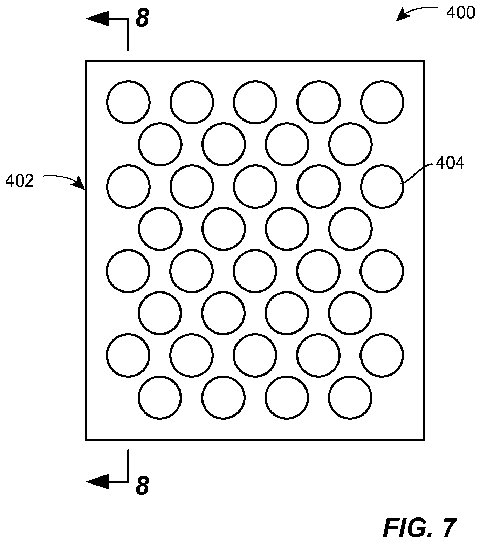

Figs. 7-10 illustrate a system 400 including a biological reactor 402 that highlights several aspects of the present disclosure over and above those illustrated in Figs. 2-6. In particular, while the general nature of the reactor (with first and second chambers, anode, cathode, barrier, microorganisms, and aqueous electrolytic medium) has much in common with the systems illustrated in Figs. 2-6, the reactor 402 illustrates new geometries, as well as a reactor in which a plurality of anodes and a plurality of cathodes are present.

In particular, as illustrated in Fig. 7, the reactor 402 includes a number of tubular reactor subunits 404 that may be arranged in a matrix format. It will be recognized that the particular arrangement of the subunits 404 utilizes an offset relative to the arrangement of adjacent rows of subunits 404, so as to increase the number of subunits 404 within a volume. It will also be recognized that adjacent rows of subunits 404 may be aligned with each other instead. It will also be recognized that while four rows of five subunits 404 each and four rows of four subunits 404 each have been illustrated, this should not be taken as limiting the reactor 402 thereby.

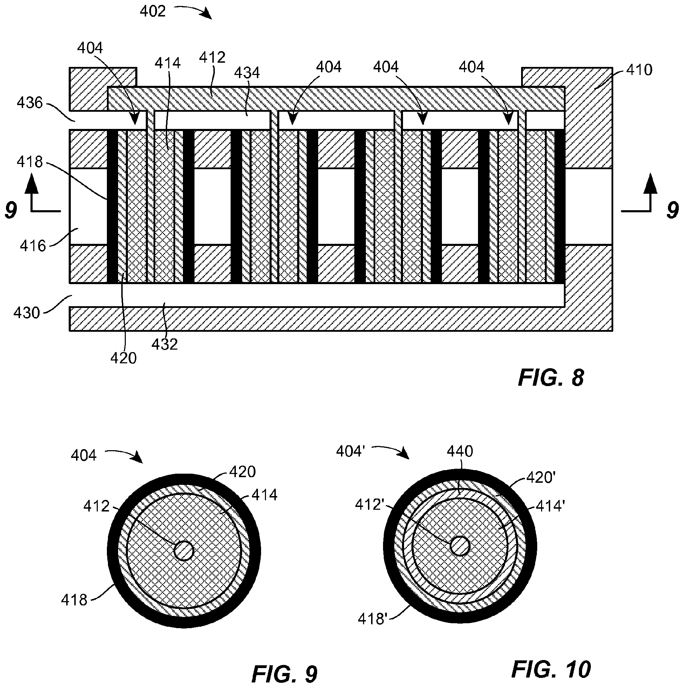

Fig. 8 illustrates a plurality of subunits in cross-section, so as to appreciate the similarities and differences with the systems illustrated in Figs. 2-6 above. While it need not be the case for all embodiments, each of the subunits 404 illustrated in Fig. 8 is identical, such that discussion of any one of the subunits 404 would be inclusive of remarks that may be made relative to the other subunits 404 as well.

As seen in Fig. 8, the reactor 402 includes a housing 410, in which the subunits 404 are disposed. It will be recognized that the housing 410 is sealed against leakage of products and byproducts as explained in greater detail below. Disposed at one end of the housing 410 is a common current collector 412 that is connected to a generally tubular cathode 414 of each of the subunits 404. In a similar fashion, the reactor 402 includes a porous gas diffusion layer/current collector 416 that is connected to a generally tubular anode 418 of each subunit 404. Disposed between the cathode 414 and the anode 418 is a generally tubular proton-permeable, gas impermeable barrier 420, as is discussed in greater detail above. This arrangement is also illustrated in Fig. 9.

According to this embodiment, the carbon dioxide enters the reactor 402 via an inlet 430 and moves along a passage 432. The carbon dioxide then passes along the porous cathode 414, which is impregnated with methanogenic microorganisms and aqueous electrolytic medium. The methane produced in the cathode 414 then is collected in a space 434 that is connected to the outlet 436.

Fig. 10 illustrates a variant to the subunit 404 illustrated relative to the system 400 in Figs. 7 and 8. Given the similarities between the subunit 404 and its variant, the common structures will be designated with a prime.

As illustrated in Fig. 10, the subunit 404' includes a tubular cathode 414', a tubular anode 418' and a tubular barrier 420'. As in the subunit 404, the tubular cathode 414' is disposed centrally of the subunit 404', with the anode 418' disposed radially outward of the cathode 416' and the barrier 420' disposed therebetween. However, similar to the variants described in Fig. 5, the subunit 404' includes a porous, proton-conducting gas diffusion layer 440. This layer 440 may be in communication with the passage 432 and the space 434 in a reactor 402, instead of the cathode 414'. As such, carbon dioxide would pass from the inlet 430 through the layer 440 to the cathode 414', while methane would pass from the cathode 414' through the layer 440 to the outlet 436. An arrangement similar to Fig. 10, but with an electrically conductive gas diffusion layer arranged as in Fig 6 between the cathode 414' and the current collector 412' is also possible.

Figs. 11 and 12 illustrate two different power management options that may be used with any of the reactors described above. In this regard, it will be recognized that each of the systems 450, 452 illustrated in Figs. 11 and 12 may include a plurality of individual reactors 454, 456.

In Fig. 11, the individual reactors 454 are connected in series to match a fixed or constant voltage. The system 450 accommodates a variable current by providing a plurality of switches 458 to permit additional series chains of reactors 454 to be switched into the circuit to match variable current. In Fig. 12, the individual reactors 456 are connected in parallel to match a fixed or constant current. The system 452 accommodates a variable voltage by providing pairs of switches 460 to permit additional parallel planes of reactors 456 to be switched into the circuit to match variable voltage. It will be recognized that it may also be possible to address variable current and variable voltage applications with addressable switching so as to build dynamic parallel reactor planes and to adjust the lengths of series chains as needed.

### II. Cultures comprising methanogenic microorganisms

### Cultures

With regard to the present invention, the reactor (also referred to herein as the electromethanogenic reactor, the electrobiological methanogenesis reactor, the biological reactor, the bioreactor, etc.) comprises a culture comprising methanogenic microorganisms (a term used interchangeably with "methanogens"). The term "culture" as used herein refers to a population of live microorganisms in or on culture medium. When part of the reactor, the culture medium also serves as the electrolytic medium facilitating electrical conduction within the reactor.

### Monocultures, Substantially Pure Cultures

In some embodiments, the culture is a monoculture and/or is a substantially-pure culture. As used herein the term "monoculture" refers to a population of microorganisms derived or descended from a single species (which may encompass multiple strains) or a single strain of microorganism. The monoculture in some aspects is "pure," i.e., nearly homogeneous, except for (a) naturally-occurring mutations that may occur in progeny and (b) natural contamination by non-methanogenic microorganisms resulting from exposure to non-sterile conditions. Organisms in monocultures can be grown, selected, adapted, manipulated, modified, mutated, or transformed, e.g. by selection or adaptation under specific conditions, irradiation, or recombinant DNA techniques, without losing their monoculture nature.

As used herein, a "substantially-pure culture" refers to a culture that substantially lacks microorganisms other than the desired species or strain(s) of microorganism. In other words, a substantially-pure culture of a strain of microorganism is substantially free of other contaminants, which can include microbial contaminants (e.g., organisms of different species or strain). In some embodiments, the substantially-pure culture is a culture in which greater than or about 70%, greater than or about 75%, greater than or about 80%, greater than or about 85%, greater than or about 90%, greater than or about 91%, greater than or about 92%, greater than or about 93%, greater than or about 94%, greater than or about 95%, greater than or about 96%, greater than or about 97%, greater than or about 98%, greater than or about 99% of the total population of the microorganisms of the culture is a single, species or strain of methanogenic microorganism. By way of example, in some embodiments, the substantially-pure culture is a culture in which greater than 70%, 75%, 80%, 85%, 90%, 91%, 92%, 93%, 94%, 95%, 96%, 97%, 98%, 99% or more of the total population of microorganisms of the culture is a single methanogenic microorganism species, e.g., *Methanothermobacter thermautotrophicus.*

When initially set up, the biological reactor is inoculated with a pure or substantially pure monoculture. As the biological reactor is exposed to non-sterile conditions during operation, the biological reactor may be contaminated by other non-methanogenic microorganisms in the environment without significant impact on operating efficiency over a period of 1, 2, 3, 4, 5, 6, 7, 8, 9, 10, 11, 12 months, or 1.5 or 2 years.

### Mixed Cultures

In other embodiments, the culture comprises a plurality of (e.g., a mixture or combination of two or more) different species of methanogenic microorganisms. In some aspects, the culture comprises two, three, four, five, six, seven, eight, nine, ten, or more different species of methanogenic microorganisms. In some aspects, the culture comprises a plurality of different species of methanogenic microorganisms, but the culture is substantially free of any non-methanogenic microorganism.

In yet other embodiments, the culture comprises a plurality of microorganisms of different species, in which at least one is a methanogenic microorganism. In some aspects of this embodiment, the culture comprises at least one species of methanogenic microorganism and further comprises at least one selected non-methanogenic microorganism. In some aspects, the culture comprises two or more different species of methanogens, and, optionally comprises at least one selected non-methanogenic microorganism.

Suitable cultures of mixtures of two or more microbes are also readily isolated from the specified environmental sources (Bryant et al. Archiv Microbiol 59:20-31 (1967) "Methanobacillus omelianskii, a symbiotic association of two species of bacteria). Suitable mixtures may be consortia in which cells of two or more species are physically associated or they may be syntrophic mixtures in which two or more species cooperate metabolically without physical association. Also, suitable mixtures may be consortia in which cells of two or more species are physically associated or they may be syntrophic mixtures in which two or more species cooperate metabolically with physical association. Mixed cultures may have useful properties beyond those available from pure cultures of known hydrogenotrophic methanogens. These properties may include, for instance, resistance to contaminants in the gas feed stream, such as oxygen, ethanol, or other trace components, or aggregated growth, which may increase the culture density and volumetric gas processing capacity of the culture. Another contaminant in the gas feed stream may be carbon monoxide.

Suitable cultures of mixed organisms may also be obtained by combining cultures isolated from two or more sources. One or more of the species in a suitable mixed culture should be an Archaeal methanogen. Any non-Archael species may be bacterial or eukaryotic.

Mixed cultures have been described in the art. See, for example, Cheng et al., U.S. 2009/0317882, and Zeikus US 2007/7250288.

### Reactor States and Growth Phases

As described herein, the reactor may be in a dormant (e.g., off) state or in an operating (e.g., on) state with regard to the production of methane, and, consequently, the reactor may be turned "on" or "off as desired in accordance with the need or desire for methane production. In some embodiments, the methanogenic microorganisms of the culture are in a state which accords with the state of the reactor. Therefore, in some embodiments, the methanogenic microorganisms are in a dormant state in which the methanogenic microorganisms are not producing methane (e.g., not producing methane at a detectable level). In alternative embodiments, the methanogenic microorganisms are in an operating state in which the methanogenic microorganisms are producing methane (e.g., producing methane at a detectable level).

When the methanogenic microorganisms are in the operating state, the methanogenic microorganisms may be in one of a variety of growth phases, which differ with regard to the growth rate of the microorganisms (which can be expressed, e.g., as doubling time of microorganism number or cell mass). The phases of growth typically observed include a lag phase, an active growth phase (also known as exponential or logarithmic phase when microorganisms multiply rapidly), a stationary phase, and a death phase (exponential or logarithmic decline in cell numbers). In some aspects, the methanogenic microorganisms of the biological reactor are in a lag phase, an active growth phase, a stationary phase, or a nearly stationary phase .

### Active growth phase

In some embodiments, the methanogenic microorganisms are in an active growth phase in which the methanogenic microorganisms are actively multiplying at a rapid rate.

In some aspects, during operation of the biological reactor, the doubling time of the microorganisms may be rapid or similar to that observed during the growth phase in its natural environment or in a nutrient-rich environment. For example, the doubling time of many methanogenic microorganisms in the active growth phase is about 15 minutes, about 20 minutes, about 30 minutes, about 45 minutes, about 60 minutes, about 75 minutes, about 80 minutes, about 90 minutes, or about 2 hours.

### Stationary growth phase, nearly stationary growth phase

Stationary phase represents a growth phase in which, after the logarithmic or active growth phase, the rate of cell division and the rate of cell death are in equilibrium or near equilibrium, and thus a relatively constant concentration of microorganisms is maintained in the reactor. (See, Eugene W. Nester, Denise G. Anderson, C. Evans Roberts Jr., Nancy N. Pearsall, Martha T. Nester; Microbiology: A Human Perspective, 2004, Fourth Edition, Chapter 4).

In other embodiments, the methanogenic microorganisms are in an stationary growth phase or nearly stationary growth phase in which the methanogenic microorganisms are not rapidly growing or have a substantially reduced growth rate. In some aspects, the doubling time of the methanogenic microorganisms is about 1 week or greater, including about 2, 3, 4 weeks or greater, or 1, 2, 3, 4, 5, 6, 7, 8, 9, 10, 11 or 12 months or greater.

In some embodiments, the reactor comprises a culture comprising methanogenic microorganisms, which microorganisms are initially in an active growth phase, and subsequently in a stationary or nearly stationary phase. In some embodiments, the reactor comprises a culture comprising methanogenic microorganisms which cycle between a dormant and an operating state.

### Methanogenesis

As used herein, the term "methanogenic" refers to microorganisms that produce methane as a metabolic byproduct. In some embodiments, the reactor (also referenced herein interchangeably as electromethanogenic reactor, biological reactor or bioreactor, etc.) comprises a culture comprising hydrogenotrophic methanogenic microorganisms. As used herein, the term "hydrogenotrophic" refers to a microorganism capable of converting hydrogen to another compound as part of its metabolism. Hydrogenotrophic methanogenic microorganisms are capable of utilizing hydrogen in the production of methane. In some embodiments, the reactor comprises a culture comprising autotrophic methanogenic microorganisms. As used herein, the term "autotrophic" refers to a microorganism capable of using carbon dioxide and a source of reducing power to provide all carbon and energy necessary for growth and maintenance of the cell (e.g., microorganism). Suitable sources of reducing power may include but are not limited to hydrogen, hydrogen sulfide, sulfur, formic acid, carbon monoxide, reduced metals, sugars (e.g., glucose, fructose), acetate, photons, or cathodic electrodes or a combination thereof. In some aspects, the methanogenic microorganisms produce methane from carbon dioxide, electricity, and water, a process referred to as electrobiological methanogenesis.

The methanogenic microorganisms produce substantial amounts of methane in the operating state, as described herein. In some aspects, the methanogenic microorganisms produce methane in an active growth phase or stationary growth phase or nearly stationary growth phase.

The efficiency of methane production per molecule of carbon dioxide (CO₂) by the methanogenic microorganisms may be any efficiency suitable for the purposes herein. It has been reported that naturally-occurring methanogenic microorganisms in the active growth phase produce methane at a ratio of about 8 CO₂ molecules converted to methane per molecule of CO₂ converted to cellular material, ranging up to a ratio of about 20 CO₂ molecules converted to methane per molecule of CO₂ converted to cellular material. In some embodiments, the methanogenic microorganisms of the biological reactor of the present invention demonstrate an increased efficiency, particularly when adapted to stationary phase growth conditions. Accordingly, in some aspects, the ratio of the number of CO₂ molecules converted to methane to the number of CO₂ molecules converted to cellular material is higher than the ratio of naturally-occurring methanogenic microorganisms in the active growth phase. In exemplary embodiments, the ratio of the number of CO₂ molecules converted to methane to the number of CO₂ molecules converted to cellular material is N:1, wherein N is a number greater than 20, e.g. about 25, 30, 35, 40, 45, 50, 55, 60, 65, 70, 75, 80, or higher. In some aspects, N is less than 500, less than 400, less than 300, or less than 200. In some aspects, N ranges from about 40 to about 150.

### Archaea

### Naturally-Occurring Archaea

In some embodiments, the methanogenic microorganisms, e.g., the autotrophic methanogenic microorganisms, are archaea. The term "Archaea" refers to a categorization of organisms of the division *Mendosicutes,* typically found in unusual environments and distinguished from the rest of the prokaryotes by several criteria, including ether-linked membrane lipids and lack of muramic acid in cell walls. On the basis of ssrRNA analysis, the Archaea consist of two phylogenetically-distinct groups: *Crenarchaeota* and *Euryarchaeota.* On the basis of their physiology, the Archaea can be organized into three partially overlapping groupings: methanogens (prokaryotes that produce methane); extreme halophiles (prokaryotes that live at very high concentrations of salt (NaCl); and extreme (hyper) thermophiles (prokaryotes that live at very high temperatures - e.g., 50-122°C). Besides the unifying archaeal features that distinguish them from bacteria (i.e., no murein in cell wall, ether-linked membrane lipids, etc.), these prokaryotes exhibit unique structural or biochemical attributes which adapt them to their particular habitats. The *Crenarchaeota* consist mainly of hyperthermophilic sulfur-dependent prokaryotes and the *Euryarchaeota* contain the methanogens and extreme halophiles.

Methanogens (or methanobacteria) suitable for practice of the invention are readily obtainable from public collections of organisms or can be isolated from a variety of environmental sources. Such environmental sources include anaerobic soils and sands, bogs, swamps, marshes, estuaries, dense algal mats, both terrestrial and marine mud and sediments, deep ocean and deep well sites, sewage and organic waste sites and treatment facilities, and animal intestinal tracts and feces. Examples of suitable organisms have been classified into four different genera within the Methanobacteria class (e.g. *Methanobacterium alcaliphilum, Methanobacterium bryantii, Methanobacterium congolense, Methanobacterium defluvii, Methanobacterium espanolae, Methanobacterium formicicum, Methanobacterium ivanovii, Methanobacterium palustre, Methanobacterium thermaggregans, Methanobacterium uliginosum, Methanobrevibacter acididurans, Methanobrevibacter arboriphilicus, Methanobrevibacter gottschalkii, Methanobrevibacter olleyae, Methanobrevibacter rum inantium, Methanobrevibacter smithii, Methanobrevibacter woesei, Methanobrevibacter wolinii, Methanothermobacter marburgensis, Methanothermobacter thermautotrophicum* (also known as *Methanothermobacter thermoautotroiphicus*), *Methanothermobacter thermoflexus, Methanothermobacter thermophilus, Methanothermobacter wolfeii, Methanothermus sociabilis),* 5 different genera within the Methanomicrobia class (e.g. *Methanocorpusculum bavaricum, Methanocorpusculum parvum, Methanoculleus chikuoensis, Methanoculleus submarinus, Methanogenium frigidum, Methanogenium liminatans, Methanogenium marinum, Methanosarcina acetivorans, Methanosarcina barkeri, Methanosarcina mazei, Methanosarcina thermophila, Methanomicrobium mobile*), 7 different genera within the Methanococci class (e.g. *Methanocaldococcusjannaschii, Methanococcus aeolicus, Methanococcus maripaludis, Methanococcus vannielii, Methanococcus voltaei, Methanothermococcus thermolithotrophicus, Methanocaldococcus fervens, Methanocaldococcus indicus, Methanocaldococcus infernus, Methanocaldococcus vulcanius*), and one genus within the Methanopyri class (e.g. *Methanopyrus kandleri*)*.* Suitable cultures are available from public culture collections (e.g. the American Type Culture Collection, the Deutsche Sammlung von Mikroorganismen und Zellkulturen GmbH, and the Oregon Collection of Methanogens). In some embodiments, the methanogen is selected from the group consisting of *Methanosarcinia barkeri, Methanococcus maripaludis,* and *Methanothermobacter thermoautotrophicus.*

Additional species of methanogens suitable for purposes of the present invention include, but are not limited to, *Methanobacterium formicum, Methanobrevibacter ruminantium, Methanocalculus chunghsingensis, Methanococcoides burtonii, Methanococcus deltae, Methanocorpusculum labreanum, Methanoculleus bourgensis (Methanogenium olentangyi, Methanogenium bourgense*), *Methanoculleus marisnigri, Methangenium cariaci, Methanogenium organophilum, Methanopyrus kandleri, Methanoregula boonei.* In some embodiments, the biological reactor comprises a culture (e.g. monoculture or substantially pure culture) of thermophilic or hyperthermophilic microorganisms, which may also be halophiles. In some embodiments, the methanogenic microorganism is from the phylum *Euryarchaeota.* Examples of species of thermophilic or hyperthermophilic autotrophic methanogens suitable for the purposes of the present invention include *Methanocaldococcus fervens, Methanocaldococcus indicus, Methanocaldococcus infernos, Methanocaldococcus jannaschii, Methanocaldococcus vulcanius, Methanopyrus kandleri, Methanothermobacter defluvii, Methanothermobacter marburgensis, Methanothermobacter thermautotrophicus, Methanothermobacter thermoflexus, Methanothermobacter thermophilus, Methanothermobacter wolfeii, Methanothermococcus okinawensis, Methanothermococcus thermolithotrophicus, Methanothermusfervidus, Methanothermus sociabilis, Methanotorris formicicus,* and *Methanotorris igneus.*

In accordance with the foregoing, in some embodiments, the methanogenic microorganisms are of the superkingdom Archaea, formerly called Archaebacteria. In certain aspects, the archaea are of the phylum: *Crenarchaeota, Euryarchaeota*, *Korarchaeota, Nanoarchaeota*, or *Thaumarchaeota.* In some aspects, the *Crenarchaeota* are of the class *Thermoprotei.* In some aspects, the *Euryarchaeota* are of the class: *archaeoglobi*, *halobacteria, methanobacteria, methanococci, methanomicrobia, methanopyri, thermococci, thermoplasmata.* In some embodiments, the *Korarchaeota* are of the class: *Candidatus Korarchaeum* or korarchaeote SRI-306. In some aspects, the *Nanoarchaeota* are of the class nanoarchaeum. In some aspects, the *Thaumarchaeota* is of the class *Cenarchaeales* or marine archaeal group 1.

In some embodiments, the methanogenic microorganisms are of the order: *Candidatus Korarchaeum, Nanoarchaeum, Caldisphaerales, Desulfurococcales, Fervidicoccales, Sulfolobales, Thermoproteales, Archaeoglobales, Halobacteriales, Methanobacteriales, Methanococcales, Methanocellales, Methanomicrobiales, Methanosarcinales, Methanopyrales, Thermococcales, Thermoplasmatales, Cenarchaeales,* or *Nitrosopumilales.*

In some embodiments, the culture comprises a classified species of the Archaea phylum *Euryarchaeota*, including, but not limited to, any of those set forth in Table 1. In some embodiments, the culture comprises an unclassified species of *Euryarchaeota*, including, but not limited to, any of those set forth in Table 2. In some embodiments, the culture comprises an unclassified species of Archaea, including, but not limited to, any of those set forth in Table 3.

In some embodiments, the culture comprises a classified species of the Archaea phylum *Crenarchaeota,* including but not limited to any of those set forth in Table 4. In some embodiments, the culture comprises an unclassified species of the Archaea phylum *Crenarchaeota,* including, but not limited to, any of those set forth in Table 5.

The archaea listed in Tables 1-5 are known in the art. See, for example, the entries for "Archaea" in the Taxonomy Browser of the National Center for Biotechnological Information (NCBI) website.

### Modified Archaea

Any of the above naturally-occurring methanogenic microorganisms may be modified. Accordingly, in some embodiments, the culture of the reactor comprises methanogenic microorganisms that have been modified (e.g., adapted in culture, genetically modified) to exhibit or comprise certain characteristics or features, which, optionally, may be specific to a given growth phase (active growth phase, stationary growth phase, nearly stationary growth phase) or reactor state (e.g., dormant state, operating state). For example, in some embodiments, the culture of the reactor comprises a methanogenic microorganism that has been modified to survive and/or grow in a desired culture condition which is different from a prior culture condition in which the methanogenic microorganism survived and/or grew, e.g., the natural environment from which the microorganism was isolated, or a culture condition previously reported in literature. The desired culture conditions may differ from the prior environment in temperature, pH, pressure, cell density, volume, humidity, salt content, conductivity, carbon content, nitrogen content, vitamin-content, amino acid content, mineral-content, or a combination thereof. In some embodiments, the culture of the biological reactor comprises a methanogenic microorganism, which, before adaptation in culture or genetic modification, is one that is not a halophile and/or not a thermophile or hyperthermophile, but, through adaptation in culture or genetic modification, has become a halophile and/or thermophile or hyperthermophile. Also, for example, in some embodiments, the methanogenic microorganism before genetic modification is one which does not express a protein, but through genetic modification has become a methanogenic microorganism which expresses the protein. Further, for example, in some embodiments, the methanogenic microorganism before adaptation in culture or genetic modification, is one which survives and/or grows in the presence of a particular carbon source, nitrogen source, amino acid, mineral, salt, vitamin, or combination thereof but through adaptation in culture or genetic modification, has become a methanogenic microorganism which survives and/or grows in the substantial absence thereof. Alternatively or additionally, in some embodiments, the methanogenic microorganism before adaptation in culture or genetic modification, is one which survives and/or grows in the presence of a particular amount or concentration of carbon source, nitrogen source, amino acid, mineral, salt, vitamin, but through adaptation in culture or genetic modification, has become a methanogenic microorganism which survives and/or grows in a different amount or concentration thereof.

In some embodiments, the methanogenic microorganisms are adapted to a particular growth phase or reactor state. Furthermore, for example, the methanogenic microorganism in some embodiments is one which, before adaptation in culture or genetic modification, is one which survives and/or grows in a given pH range, but through adaptation in culture becomes a methanogenic microorganism that survives and/or grows in different pH range. In some embodiments, the methanogenic microorganisms (e.g., archaea) are adapted in culture to a nearly stationary growth phase in a pH range of about 3.5 to about 10 (e.g., about 5.0 to about 8.0, about 6.0 to about 7.5). Accordingly, in some aspects, the methanogenic microorganisms are adapted in culture to a nearly stationary growth phase at a pH of about 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, or 10.0. In some embodiments, the methanogenic microorganisms (e.g., archaea) are adapted in culture to an active growth phase in a pH range of about 6.5 to about 7.5 (e.g., about 6.8 to about 7.3). Accordingly, in some aspects, the methanogenic microorganisms are adapted in culture to a nearly stationary growth phase at a pH of about 6.5, 6.6, 6.7, 6.8, 6.9, 7.0, 7.1, 7.2, 7.3, 7.4, or 7.5.

As used herein, the term "adaptation in culture" refers to a process in which microorganisms (e.g., naturally-occurring archaea) are cultured under a set of desired culture conditions (e.g., high salinity, high temperature, substantial absence of any carbon source, low pH, etc.), which differs from prior culture conditions. The culturing under the desired conditions occurs for a period of time which is sufficient to yield modified microorganisms (progeny of the parental line (i.e. the unadapted microorganisms)) which survive and/or grow (and/or produce methane) under the desired condition(s). The period of time of adaptation in some aspects is 1 day, 2 days, 3 days, 4 days, 5 days, 6 days, 7 days, 8 days, 9 days, 10 days, 11 days, 12 days, 13 days, 2 weeks, 3 weeks 4 weeks, 5 weeks, 6 weeks 1 month, 2 months, 3 months, 4 months, 5 months 6 months, 7 months, 8 months, 9 months, 10 months, 12 months, 1 year, 2 years. The process of adapting in culture selects for microorganisms that can survive and/or grow and/or produce methane in the desired culture conditions; these selected microorganisms remain in the culture, whereas the other microorganisms that cannot survive and/or grow and/or produce methane in the desired culture conditions eventually die in the culture. In some embodiments, as a result of the adaptation in culture, the methanogenic microorganisms produce methane at a higher efficiency, e.g., at a ratio of the number of carbon dioxide molecules converted to methane to the number of carbon dioxide molecules converted to cellular materials which is higher than N:1, wherein N is a number greater than 20, as further described herein.

In some embodiments, the adaptation process occurs before the microorganisms are placed in the reactor. In some embodiments, the adaptation process occurs after the microorganisms are placed in the reactor. In some embodiments, the microorganisms are adapted to a first set of conditions and then placed in the reactor, and, after placement into the biological reactor, the microorganisms are adapted to another set of conditions.

For purposes of the present invention, in some embodiments, the culture of the reactor comprises a methanogenic microorganism (e.g., archaea) which has been adapted in culture to survive and/or grow in a high salt and/or high conductivity culture medium. For example, the culture of the biological reactor comprises a methanogenic microorganism (e.g., archaea) which has been adapted in culture to survive and/or grow in a culture medium having a conductivity of about 1 to about 25 S/m.

In alternative or additional embodiments, the culture of the reactor comprises a methanogenic microorganism (e.g., archaea) which has been adapted in culture to survive and/or grow at higher temperature (e.g., a temperature which is between about 1 and about 15 degrees C greater than the temperature that the microorganisms survives and/or grows before adaptation). In exemplary embodiments, the methanogenic microorganisms are adapted to survive and/or grow in a temperature which is greater than 50 °C, e.g., greater than 55 °C, greater than 60 °C, greater than 65 °C, greater than 70 °C, greater than 75 °C, greater than 80 °C, greater than 85 °C, greater than 90°C, greater than 95 °C, greater than 100 °C, greater than 105 °C, greater than 110 °C, greater than 115 °C, greater than 120 °C.

In some embodiments, the culture comprises a methanogenic microorganism (e.g., archaea) which has been adapted in culture to grow and/or survive in conditions which are low in or substantially absent of any vitamins. In some aspects, the culture comprises a methanogenic microorganism (e.g., archaea) which has been adapted in culture to grow and/or survive in conditions which are low in or substantially absent of any organic carbon source. In some embodiments, the culture comprises a methanogenic microorganism which has been adapted in culture to grow and/or survive in conditions with substantially reduced amounts of carbon dioxide. In these embodiments, the methanogenic microorganisms may be adapted to exhibit an increased methanogenesis efficiency, producing the same amount of methane (as compared to the unadapted microorganism) with a reduced amount of carbon dioxide. In some embodiments, the culture comprises a methanogenic microorganism which has been adapted in culture to survive in conditions which substantially lacks carbon dioxide. In these embodiments, the methanogenic microorganisms may be in a dormant phase in which the microorganisms survive but do not produce detectable levels of methane. In some embodiments, the methanogenic microorganisms have been adapted to grow and/or survive in conditions which are low in or substantially absent of any hydrogen. In some embodiments, the methanogenic microorganisms have been adapted to grow and/or survive in conditions which are low in or substantially absent of any external source of water, e.g., the conditions do not comprise a dilution step.

In exemplary embodiments, the methanogens are adapted in culture to a nearly stationary growth phase. Such methanogens favor methane production over cell growth as measured, e.g., by the ratio of the number of CO₂ molecules converted to methane to the number of CO₂ molecules converted to cellular materials. This ratio is increased as compared to unadapted methanogens (which may exhibit, e.g., a ratio ranging from about 8:1 to about 20:1). In some embodiments, the methanogens are adapted in culture to a nearly stationary growth phase by being deprived of one or more nutrients otherwise required for optimal growth for a prolonged period of time (e.g., 1 week, 2 week, 3 weeks, 1 month, 2 months, 3 months, 4 months, 5 months, 6 months, 7 months, 8 months, 9 months, 10 months, 11 months, 1 year, 2 years, 3 years, 4 years, 5 years or more). In some embodiments, the methanogens are deprived of inorganic nutrients (e.g., hydrogen or electrons) necessary for optimum growth. In some embodiments, depriving the methanogens of hydrogen or electrons is achieved by sparging the media with an insert gas mixture such as Ar:CO₂ at a flow rate of 250 mL/min for several hours until neither hydrogen nor methane appear in the effluent gas stream. In some embodiments, the methanogenic microorganisms have been adapted to a nearly stationary growth phase in conditions which are low in or substantially absent of any external source of water, e.g., the adaptation conditions do not comprise a dilution step.

In some aspects, the culture comprises a methanogenic microorganism which has been adapted in culture to grow and/or survive in the culture medium set forth herein as Medium 1 and/or Medium 2 or a medium which is substantially similar to Medium 1 or Medium 2.

### Genetically modified archaea

In some embodiments, the culture comprises methanogenic microorganisms which have been purposefully or intentionally genetically modified to become suitable, e.g., more suitable, for the purposes of the present invention. Suitable cultures may also be obtained by genetic modification of non-methanogenic organisms in which genes essential for supporting autotrophic methanogenesis are transferred from a methanogenic microbe or from a combination of microbes that may or may not be methanogenic on their own. Suitable genetic modification may also be obtained by enzymatic or chemical synthesis of the necessary genes.

In exemplary embodiments, a host cell that is not naturally methanogenic is intentionally genetically modified to express one or more genes that are known to be important for methanogenesis. For example, the host cell in some aspects is intentionally genetically modified to express one or more coenzymes or cofactors involved in methanogenesis. In some specific aspects, the coenzymes or cofactors are selected from the group consisting of F420, coenzyme B, coenzyme M, methanofuran, and methanopterin, the structures of which are known in the art. In some aspects the organisms are modified to express the enzymes, well known in the art, that employ these cofactors in methanogenesis.

In some embodiments, the host cells that are intentionally modified are extreme halophiles. In other embodiments, the host cells that are intentionally modified are thermophiles or hyperthermophiles. In other embodiments, the host cells that are intentionally modified are non-autotrophic methanogens. In some aspects, the host cells that are intentionally modified are methanogens that are not autotrophic. In some aspects, the host cells that are intentionally modified are cells which are neither methanogenic nor autotrophic. In other embodiments, the host cells that are intentionally modified are host cells comprising synthetic genomes. In some aspects, the host cells that are intentionally modified are host cells which comprise a genome which is not native to the host cell.

In some embodiments, the culture comprises microorganisms that have been purposefully or intentionally genetically modified to express pili or altered pili, e.g., altered pili that promote cell adhesion to the cathode or other components of the electrobiological methanogenesis reactor or pili altered to become electrically conductive. Pili are thin filamentous protein complexes that form flexible filaments that are made of proteins called pilins. Pili traverse the outer membrane of microbial cells and can extend from the cell surface to attach to a variety of other surfaces. Pili formation facilitates such disparate and important functions as surface adhesion, cell-cell interactions that mediate processes such as aggregation, conjugation, and twitching motility. Recent in silico analyses of more than twenty archaeal genomes have identified a large number of archaeal genes that encode putative proteins resembling type IV pilins (Szabo et al. 2007). The expression of several archaeal pilin-like proteins has since been confirmed in vivo (Wang et al. 2008; Zolghadr et al. 2007; Frols et al. 2007, 2008). The sequence divergence of these proteins as well as the differential expression of the operons encoding these proteins suggests they play a variety of roles in distinct biological processes.

Certain microorganisms such as Geobacter and Rhodoferax species, have highly conductive pili that can function as biologically produced nanowires as described in US 20060257985. Many methanogenic organisms, including most of the Methanocaldococcus species and the Methanotorris species, have native pili and in some cases these pili are used for attachment. None of these organisms are known to have natively electrically conductive pili.

In certain embodiments of the present invention the pili of a methanogenic organism and/or surfaces in contact with pili of a methanogenic organism or other biological components can be altered in order to promote cell adhesion to the cathode or other components of the electrobiological methanogenesis reactor. Pili of a methanogenic organism can be further engineered to optimize their electrical conductivity. Pilin proteins can be engineered to bind to various complexes. For example, pilin proteins can be engineered to bind iron, mimicking the pili of Geobacter species or alternatively, they can be engineered to bind a low potential ferredoxin-like iron-sulfur cluster that occurs naturally in many hyperthermophilic methanogens. The desired complex for a particular application will be governed by the midpoint potential of the redox reaction.

The cells may be genetically modified, e.g., using recombinant DNA technology. For example, cell or strain variants or mutants may be prepared by introducing appropriate nucleotide changes into the organism's DNA. The changes may include, for example, deletions, insertions, or substitutions of, nucleotides within a nucleic acid sequence of interest. The changes may also include introduction of a DNA sequence that is not naturally found in the strain or cell type. One of ordinary skill in the art will readily be able to select an appropriate method depending upon the particular cell type being modified. Methods for introducing such changes are well known in the art and include, for example, oligonucleotide-mediated mutagenesis, transposon mutagenesis, phage transduction, transformation, random mutagenesis (which may be induced by exposure to mutagenic compounds, radiation such as X-rays, UV light, etc.), PCR-mediated mutagenesis, DNA transfection, electroporation, etc.

The ability of the pili of the methanogenic organisms to adhere to the cathode coupled with an increased ability to conduct electrons, will enable the organisms to receive directly electrons passing through the cathode from the negative electrode of the power source. The use of methanogenic organisms with genetically engineered pili attached to the cathode will greatly increase the efficiency of conversion of electric power to methane.

### Culture Media

The culture comprising the methanogenic microorganisms, e.g., the methanogenic archaea, may be maintained in or on a culture medium. In some embodiments, the culture medium is a solution or suspension (e.g., an aqueous solution). In other embodiments, the culture medium is a solid or semisolid. In yet other embodiments, the culture medium comprises or is a gel, a gelatin, or a paste.

In some embodiments, the culture medium is one that encourages the active growth phase of the methanogenic microorganisms. In exemplary aspects, the culture medium comprises materials, e.g., nutrients, in non-limiting amounts that support relatively rapid growth of the microorganisms. The materials and amounts of each material of the culture medium that supports the active phase of the methanogenic microorganisms will vary depending on the species or strain of the microorganisms of the culture. However, it is within the skill of the ordinary artisan to determine the contents of culture medium suitable for supporting the active phase of the microorganisms of the culture. In some embodiments, the culture medium encourages or permits a stationary phase of the methanogenic microorganisms. Exemplary culture medium components and concentrations are described in further detail below. Using this guidance, alternative variations can be selected for particular species for electrobiological methanogenesis in the operating state of the biological reactor using well known techniques in the field.

### Inorganic materials: Inorganic elements, minerals, and salts

In some embodiments, the medium for culturing archaea comprises one or more nutrients that are inorganic elements, or salts thereof. Common inorganic elements include but are not limited to sodium, potassium, magnesium, calcium, iron, chloride, sulfur sources such as hydrogen sulfide or elemental sulfur, phosphorus sources such as phosphate and nitrogen sources such as ammonium, nitrogen gas or nitrate. Exemplary sources include NaCl, NaHCO₃, KCl, MgCl₂, MgSO₄, CaCl₂, ferrous sulfate, Na₂HPO₄, NaH₂PO₄ H₂O, H₂S, Na₂S, NH₄OH, N₂, and NaNO₃. In some embodiments, the culture medium further comprises one or more trace elements selected from the group consisting of ions of barium, bromium, boron, cobalt, iodine, manganese, chromium, copper, nickel, selenium, vanadium, titanium, germanium, molybdenum, silicon, iron, fluorine, silver, rubidium, tin, zirconium, cadmium, zinc, tungsten and aluminum. These ions may be provided, for example, in trace element salts, such as H₃BO₃, Ba(C₂H₃O₂)₂, KBr, COCl₂-6H₂O, KI, MnCl₂-2H₂O, Cr(SO₄)₃-15H₂O, CuSO₄-5H₂O, NiSO₄-6H₂O, H₂SeO₃, NaVO₃, TiCl₄, GeO₂, (NH₄)6Mo₇O₂₄-4H₂O, Na₂SiO₃-9H₂O, FeSO₄-7H₂O, NaF, AgNO₃, RbCl, SnCl₂,ZrOCl₂-8H₂O, CdSO₄-8H₂O, ZnSO₄-7H₂O, Fe(NO₃)₃-9H₂ONa₂WO₄, AlCl₃-6H₂O.

In some embodiments, the medium comprises one or more minerals selected from the group consisting of nickel, cobalt, sodium, magnesium, iron, copper, manganese, zinc, boron, phosphorus, sulfur, nitrogen, selenium, tungsten, aluminum and potassium including any suitable non-toxic salts thereof. Thus, in some embodiments, the minerals in the medium are provided as mineral salts. Any suitable salts or hydrates may be used to make the medium. For example, and in some embodiments, the media comprises one or more of the following mineral salts: Na₃nitrilotriacetate, nitrilotriacetic acid, NiCl₂-6H₂O, CoCl₂-6H₂O, Na₂MoO₄-H₂O, MgCl₂-6H₂O, FeSO₄-H₂O, Na₂SeO₃, Na₂WO₄, KH₂PO₄, and NaCl. In some embodiments, L-cysteine may be added as a redox buffer to support early phases of growth of a low-density culture. In some embodiments, the medium comprises nickel, optionally NiCl₂-6H₂O in an amount of about 0.001 mM to about 0.01 mM, e.g. 0.002 mM, 0.003 mM, 0.004 mM, 0.005 mM, 0.006 mM, 0.007 mM, 0.008 mM, 0.009 mM, or any combination of the foregoing range endpoints. In some embodiments, the media comprises a nitrogen source, e.g., ammonium hydroxide or ammonium chloride in an amount of about 1 mM to about 10 mM, e.g. 2 mM, 3 mM, 4 mM, 5 mM, 6 mM, 7 mM, 8 mM, 9 mM, or any combination of the foregoing range endpoints. In some embodiments, the media comprises cobalt, e.g. CoCl₂-6H₂O, in an amount of about 0.001 mM to about 0.01 mM, e.g., 0.002 mM, 0.003 mM, 0.004 mM, 0.005 mM, 0.006 mM, 0.007 mM, 0.008 mM, 0.009 mM, or any combination of the foregoing range endpoints. In some embodiments, the media comprises molybdenum, a molybdenum source or molybdate, e.g. Na₂MoO₄-H₂O, in an amount of about 0.005 mM to about 0.05 mM, e.g., 0.006 mM, 0.007 mM, 0.008 mM, 0.009 mM, 0.01 mM, 0.02 mM, 0.03 mM, 0.04 mM, or any combination of the foregoing range endpoints. In some embodiments, the media comprises magnesium, e.g. MgCl₂-6H₂O, in an amount of about 0.5 mM to about 1.5 mM, e.g., 0.6 mM, 0.7 mM, 0.8 mM, 0.9 mM, 1.0 mM, 1.1 mM, 1.2 mM, 1.3 mM, 1.4 mM, or any combination of the foregoing range endpoints. In some embodiments, the media comprises iron, e.g. FeSO₄-H₂O, in an amount of about 0.05 mM to about 0.5 mM, e.g., 0.06 mM, 0.07 mM, 0.08 mM, 0.09 mM, 0.1 mM, 0.2 mM, 0.3 mM, 0.4 mM, or any combination of the foregoing range endpoints. In some embodiments, the media comprises a sulfur source or sulfate in an amount of about 0.05 mM to about 0.5 mM, e.g., 0.06 mM, 0.07 mM, 0.08 mM, 0.09 mM, 0.1 mM, 0.2 mM, 0.3 mM, 0.4 mM, or any combination of the foregoing range endpoints. In some embodiments, the media comprises selenium, a selenium source or selenate, e.g. Na₂SeO₃, in an amount of about 0.005 mM to about 0.05 mM, e.g., 0.006 mM, 0.007 mM, 0.008 mM, 0.009 mM, 0.01 mM, 0.02 mM, 0.03 mM, 0.04 mM, or any combination of the foregoing range endpoints. In some embodiments, the media comprises tungsten, a tungsten source or tungstate, e.g. Na₂WO₄, in an amount of about 0.005 mM to about 0.05 mM, e.g., 0.006 mM, 0.007 mM, 0.008 mM, 0.009 mM, 0.01 mM, 0.02 mM, 0.03 mM, 0.04 mM, or any combination of the foregoing range endpoints. In some embodiments, the media comprises potassium, e.g. KH₂PO₄, in an amount of about 5 mM to about 15 mM, e.g., 6 mM, 7 mM, 8 mM, 9 mM, 10 mM, 11 mM, 12 mM, 13 mM, 14 mM, or any combination of the foregoing range endpoints. In some embodiments, the media comprises phosphorus, a phosphorus source, or phosphate, e.g. KH₂PO₄, in an amount of about 5 mM to about 15 mM, e.g., 6 mM, 7 mM, 8 mM, 9 mM, 10 mM, 11 mM, 12 mM, 13 mM, 14 mM, or any combination of the foregoing range endpoints. In some embodiments, the media comprises NaCl in an amount of about 5 mM to about 15 mM, e.g., 6 mM, 7 mM, 8 mM, 9 mM, 10 mM, 11 mM, 12 mM, 13 mM, 14 mM, or any combination of the foregoing range endpoints.

Some archaea are extreme halophiles and prefer high salt conditions, e.g. about 1.5M to about 5.5 M NaCl, or about 3 M to about 4 M NaCl. Other archaea may be adapted to growth in higher salt conditions than their normal environment.

In some embodiments, the culture medium serves more than one purpose. Accordingly, in some aspects, the culture medium supports the growth and/or survival of the microorganisms of the culture and serves as the cathode electrolytic medium within the reactor. An electrolyte is a substance that, when dissolved in water, permits current to flow through the solution. The conductivity (or specific conductance) of an electrolytic medium is a measure of its ability to conduct electricity. The SI unit of conductivity is siemens per meter (S/m), and unless otherwise qualified, it is measured at a standard temperature of 25 °C. Deionized water may have a conductivity of about 5.5 µS/m, while sea water has a conductivity of about 5 S/m (i.e., sea water's conductivity is one million times higher than that of deionized water).

Conductivity is traditionally determined by measuring the AC resistance of the solution between two electrodes or by torroidal inductance meters.

Limiting ion conductivity in water at 298 K for exemplary ions:

| Cations | λ+0 /mS m²mol⁻¹ | anions | λ-0 /mS m²mol⁻¹ |
|---|---|---|---|
| H⁺ | 34.96 | OH | 19.91 |
| Li⁺ | 3.869 | CI | 7.634 |
| Na⁺ | 5.011 | Br | 7.84 |
| K⁺ | 7.350 | I | 7.68 |
| Mg²⁺ | 10.612 | SO42 | 15.96 |
| Ca²⁺ | 11.900 | NO₃ | 7.14 |

In some embodiments, the culture medium comprises a high salt concentration for purposes of increasing the conductivity of the culture medium/reactor cathode electrolyte. Conductivity is readily adjusted, for example, by adding NaCl until the desired conductivity is achieved. In some embodiments, the conductivity of the medium/electrolyte is in the range of 1 to 25 S/m (0.01 to 0.25 S/cm). This conductivity is readily achieved within the range of salt concentrations that are compatible with living methanogenic Archaea.

### Vitamins

In some embodiments, vitamins are substantially absent from the culture medium, to reduce contamination by non-methanogens and/or to decrease the cost of the culture medium, and thus, the overall cost of the biological reactor. However, it is possible to operate the biological reactor using media supplemented with one or more vitamins selected from the group consisting of ascorbic acid, biotin, choline chloride; D-Ca⁺⁺pantothenate, folic acid, i-inositol, menadione, niacinamide, nicotinic acid, paraaminobenzoic acid (PABA), pyridoxal, pyridoxine, riboflavin, thiamine-HCl, vitamin A acetate, vitamin B₁₂ and vitamin D₂. In some embodiments, the medium is supplemented with a vitamin that is essential to survival of the methanogenic microorganism, but other vitamins are substantially absent.

### Other materials

The culture medium in some embodiments comprises materials other than inorganic compounds and salts. For example, the culture medium in some embodiments, comprises a chelating agent. Suitable chelating agents are known in the art and include but not limited to nitrilotriacetic acid and/or salts thereof. Also, in some aspects, the culture medium comprises a redox buffer, e.g., Cys, to support an early active growth phase in a low-density culture.

### Carbon Sources

In some aspects, the culture medium comprises a carbon source, e.g., carbon dioxide, formate, or carbon monoxide. In some embodiments, the culture medium comprises a plurality of these carbon sources in combination. Preferably, organic carbon sources are substantially absent, to reduce contamination by non-methanogens.

### Nitrogen Sources

In some embodiments, the culture medium comprises a nitrogen source, e.g., ammonium, anhydrous ammonia, ammonium salts and the like. In some embodiments, the culture medium may comprise nitrate or nitrite salts as a nitrogen source, although chemically reduced nitrogen compounds are preferable. In some aspects, the culture medium substantially lacks an organic nitrogen source, e.g., urea, corn steep liquor, casein, peptone yeast extract, and meat extract. In some embodiments diatomic nitrogen (N₂) may serve as a nitrogen source, either alone or in combination with other nitrogen sources.

### Oxygen

Methanogens that are primarily anaerobic may still be capable of surviving prolonged periods of oxygen stress, e.g. exposure to ambient air for at least 6, 12, 18, or 24 hours, or 2 days, 3 days, 4 days, 5 days, 6 days, 1 week or more. Ideally, exposure to air is for 4 days or less, or 3 days or less, or 2 days or less, or 24 hours or less. Methane production may continue in the presence of oxygen concentrations as high as 2-3% of the gas phase for extended periods (at least days). However, anaerobic organisms will grow optimally in conditions of low oxygen. In some embodiments, the biological reactor substantially excludes oxygen to promote high levels of methane production.

In some embodiments, the system comprises various methods and/or features that reduce the presence of oxygen in the CO₂ stream that is fed into the biological reactor. When obligate anaerobic methanogenic microorganisms are used to catalyze methane formation, the presence of oxygen may be detrimental to the performance of the process and contaminates the product gas. Therefore, reduction of the presence of oxygen in the CO₂ stream is helpful for improving the process. In one embodiment, the oxygen is removed by pre-treatment of the gas stream in a biological reactor. In this embodiment, the reductant may be provided either by provision of a source of organic material (e.g. glucose, starch, cellulose, fermentation residue from an ethanol plant, whey residue, etc.) that can serve as substrate for an oxidative fermentation. The microbial biological catalyst is chosen to oxidatively ferment the chosen organic source, yielding CO₂ from the contaminant oxygen. In another embodiment, oxygen removal is accomplished in the main fermentation vessel via a mixed culture of microbes that includes one capable of oxidative fermentation of an added organic source in addition to the autotrophic methanogen necessary for methane production. An example of a suitable mixed culture was originally isolated as "Methanobacillus omelianskii" and is readily obtained from environmental sources (Bryant et al. Archiv Microbiol 59:20-31 (1967) "Methanobacillus omelianskii, a symbiotic association of two species of bacteria."). In another embodiment, carbon dioxide in the input gas stream is purified away from contaminating gases, including oxygen, buy selective absorption or by membrane separation. Methods for preparing carbon dioxide sufficiently free of oxygen are well known in the art.

### Exemplary media

In some embodiments, the culture medium comprises the following components: Na₃nitrilotriacetate, nitrilotriacetic acid, NiCl₂-6H₂O, CoCl₂-6H₂O, Na₂MoO₄-H₂O, MgCl₂-6H₂O, FeSO₄-H₂O, Na₂SeO₃, Na₂WO₄, KH₂PO₄, and NaCl. In some embodiments, L-cysteine may be added as a redox buffer to support early phases of growth of a low-density culture. In some embodiments, the media comprises Na₃nitrilotriacetate (0.81 mM), nitrilotriacetic acid (0.4 mM), NiCl₂-6H₂O (0.005 mM), CoCl₂-6H₂O (0.0025 mM), Na₂MoO₄-H₂O (0.0025 mM), MgCl₂-6H₂O (1.0 mM), FeSO₄-H₂O (0.2 mM), Na₂SeO₃ (0.001 mM), Na₂WO₄ (0.01 mM), KH₂PO₄ (10 mM), and NaCl (10 mM). L-cysteine (0.2 mM) may be included.

In some embodiments, the culture medium comprises the following components: KH₂PO₄, NH₄Cl, NaCl, Na₃nitrilotriacetate, NiCl₂-6H₂O, CoCl₂-H₂O, Na₂MoO₄-2H₂O, FeSO₄-7H₂O, MgCl₂-6H₂O, Na₂SeO₃, Na₂WO₄, Na₂S-9H₂O. A culture medium comprising these components may be referred to herein as Medium 1, which is capable of supporting survival and/or growth of methanogenic microorganisms originally derived from a terrestrial environment, e.g., *aMethanothermobacter* species. Thus, in some embodiments, the biological reactor comprises a culture of *Methanothermobacter* and a culture medium of Medium 1. In some aspects , the culture medium is adjusted with NH₄OH to a pH between about 6.8 and about 7.3. In some embodiments, the culture medium not only supports growth of and/or survival of and/or methane production by the methanogenic microorganisms but also serves as the cathode electrolytic medium suitable for conducting electricity within the reactor. Accordingly, in some aspects, the conductivity of the culture medium is in the range of about 1 to about 25 S/m (about 0.01 to about 0.25 S/cm).

In some embodiments, the KH₂PO₄ is present in the medium at a concentration within the range of about 1 mM to about 100 mM, e.g., about 2 mM, about 50 mM, about 5 mM to about 20 mM.

In some embodiments, the NH₄Cl is present in the culture medium at a concentration within the range of about 10 mM to about 1500 mM, e.g., about 20 mM to about 600 mM, about 60 mM to about 250 mM.

In some embodiments, the NaCl is present in the culture medium within the range of about 1 mM to about 100 mM, e.g., about 2 mM, about 50 mM, about 5 mM to about 20 mM.

In some embodiments, the Na₃nitrilotriacetate is present in the culture medium within the range of about 0.1 mM to about 10 mM, e.g., 0.20 mM to about 6 mM, about 0.5 to about 2.5 mM.

In some embodiments, the NiCl₂-6H₂O is present in the culture medium within the range of about 0.00025 to about 0.025 mM, e.g., about 0.005 mM to about 0.0125 mM, about 0.0005 mM to about 0.005 mM.

In some embodiments, the COCl₂-H₂O is present in the culture medium within the range of about 0.0005 mM to about 0.05 mM, e.g., about 0.001 mM to about 0.025 mM, about 0.0025 mM to about 0.01 mM.

In some embodiments, the Na₂MoO₄-2H₂O is present in the culture medium within the range of about 0.00025 mM to about 0.025 mM, e.g., about 0.0005 mM to about 0.0125 mM, about 0.00125 mM to about 0.005 mM.

In some embodiments, the FeSO₄-7H₂O is present in the culture medium within the range of about 0.02 mM to about 2 mM, e.g., about 0.04 mM to about 1 mM, about 0.1 mM to about 0.4 mM.

In some embodiments, the MgCl₂-6H₂O is present in the culture medium within the range of about 0.1 mM to about 10 mM, e.g., about 0.2 mM to about 5 mM, about 0.5 mM to about 2 mM.

In some embodiments, the Na₂SeO₃ is present in the culture medium within the range of about 0.0001 mM to about 0.01 mM, e.g., about 0.0002 mM to about 0.005 mM, about 0.0005 mM to about 0.002 mM.

In some embodiments, the Na₂WO₄ is present in the culture medium within the range of about 0.001 mM to about 0.1 mM, e.g., about 0.05 mM to about 0.05 mM, about 0.005 mM to about 0.02 mM.

In some embodiments, Medium 1 is supplemented with components, such as sulfide, that support the active growth phase or relatively rapid multiplication of the microorganism. Accordingly, in some aspects, the culture medium comprises a higher sulfide concentration, e.g. 0.1 mM to about 10 mM (e.g., about 0.2 mM to about 5 mM, about 0.5 mM to about 2 mM), about 0.5 to 5 mM, or about 1 mM Na2S-9H2O, and preferably greater than 0.01 mM Na2S-9H2O, optionally with a pH between about 6.8 and about 7.0. In other embodiments, Medium 1 supports the inactive or stationary or nearly-stationary growth phase of the microorganism and the medium comprises a lower sulfide concentration. Accordingly, in some aspects, the culture comprises about 0.01 mM or less Na2S-9H2O, and not 1 mM Na2S-9H2O. optionally with a pH between about 7.2 and about 7.4.

In some embodiments, the culture medium comprises the following components: KH₂PO₄, NaCl, NH₄Cl, Na₂CO₃, CaCl₂ x 2H₂O, MgCl₂ x 6H₂O, FeCl₂ x 4H₂O, NiCl₂ x 6H₂O, Na₂SeO₃ x 5 H₂O, Na₂WO₄ x H₂O, MnCl₂ x 4H₂O, ZnCl₂, H₃BO₃, CoCl₂ x 6H₂O, CuCl₂ x 2H₂O, Na₂MoO₄ x 2H₂O, Nitrilotriacetic acid, Na₃nitrilotriacetic acid, KAl(SO₄)₂ x 12 H₂O, Na₂S x 9H₂O. A culture medium comprising these components may be referred to herein as Medium 2, which is capable of supporting survival and/or growth of methanogenic microorganisms originally derived from a marine environment, e.g., *a Methanocaldooccus* species, *Methanotorris* species, *Methanopyrus* species, or *Methanothermococcus* species. In some aspects , the culture medium is adjusted with NH₄OH to a pH between about 6.3 and about 6.8 (e.g., about 6.4 to about 6.6). In some embodiments, the culture medium not only supports growth of and/or survival of and/or methane production by the methanogenic microorganisms but also serves as the cathode electrolytic medium suitable for conducting electricity within the reactor. Accordingly, in some aspects, the conductivity of the culture medium is in the range of about 1 to about 25 S/m (about 0.01 to about 0.25 S/cm).

In some embodiments, the KH₂PO₄ is present in the culture medium at a concentration within the range of about 0.35 mM to about 37 mM, e.g., about 0.7 mM to about 0.75 mM, about 1.75 mM to about 7.5 mM.

In some embodiments, the NaCl is present in the culture medium at a concentration within the range of about 17 mM to about 1750 mM, e.g., about 30 mM to about 860 mM, about 80 mM to about 350 mM.

In some embodiments, the NH₄Cl is present in the culture medium at a concentration within the range of about 0.7 mM to about 750 mM, e.g., about 1.5 mM to about 40 mM, about 3.75 mM to about 15 mM.

In some embodiments, the Na₂CO₃ is present in the culture medium at a concentration within the range of about 5 mM to about 600 mM, e.g., 10 mM to about 300 mM, about 30 mM to about 150 mM.

In some embodiments, the CaCl₂ x 2H₂O is present in the culture medium at a concentration within the range of about 0.05 to about 50 mM, e.g., 0.2 mM to about 5 mM, about 0.5 mM to about 2 mM.

In some embodiments, the MgCl₂ x 6H₂O is present in the culture medium at a concentration within the range of about 3 mM to about 350 mM, e.g., about 6.5 mM to about 175 mM, about 15 mM to about 70 mM.

In some embodiments, the FeCl₂ x 4H₂O is present in the culture medium at a concentration within the range of about 0.003 mM to about 0.3 mM, e.g., about 0.006 mM to about 0.15 mM, about 0.015 mM to about 0.06 mM.

In some embodiments, the NiCl₂ x 6H₂O is present in the culture medium at a concentration within the range of about 0.0005 mM to about 0.007 mM, e.g., 0.0012 mM to about 0.03 mM, about 0.003 mM to about 0.012 mM.

In some embodiments, the Na₂SeO₃ x 5 H₂O is present in the culture medium at a concentration within the range of about 0.0001 mM to about 0.01 mM, e.g., about 0.00025 mM to about 0.01 mM, about 0.001 mM to about 0.005 mM.

In some embodiments, the Na₂WO₄ x H₂O is present in the culture medium at a concentration within the range of about 0.0005 mM to about 0.007 mM, e.g., 0.0012 mM to about 0.03 mM, about 0.003 mM to about 0.012 mM.

In some embodiments, the MnCl₂ x 4H₂O is present in the culture medium at a concentration within the range of about 0.003 mM to about 0.4 mM, e.g., about 0.08 mM to about 2 mM, about 0.02 mM to about 0.08 mM.

In some embodiments, the ZnCl₂ is present in the culture medium at a concentration within the range of about 0.0005 mM to about 0.007 mM, e.g., 0.0012 mM to about 0.03 mM, about 0.003 mM to about 0.012 mM.

In some embodiments, the H₃BO₃ is present in the culture medium at a concentration within the range of about 0.0001 mM to about 0.01 mM, e.g., about 0.00025 mM to about 0.01 mM, about 0.001 mM to about 0.005 mM.

In some embodiments, the COC₁₂ x 6H₂O is present in the culture medium at a concentration within the range of about 0.0005 mM to about 0.007 mM, e.g., 0.0012 mM to about 0.03 mM, about 0.003 mM to about 0.012 mM.

In some embodiments, the CuCl₂ x 2H₂O is present in the culture medium at a concentration within the range of about 0.00004 mM to about 0.004 mM, e.g., 0.00008 mM to about 0.002 mM, about 0.0002 mM to about 0.0008 mM.

In some embodiments, the Na₂MoO₄ x 2H₂O is present in the culture medium at a concentration within the range of about 0.00004 mM to about 0.004 mM, e.g., 0.00008 mM to about 0.002 mM, about 0.0002 mM to about 0.0008 mM.

In some embodiments, the Nitrilotriacetic acid is present in the culture medium at a concentration within the range of about 0.003 mM to about 0.7 mM, e.g., about 0.12 mM to about 0.3 mM, about 0.03 mM to about 0.12 mM.

In some embodiments, the Na₃nitrilotriacetic acid is present in the culture medium at a concentration within the range of about 0.002 mM to about 0.2 mM, e.g., about 0.004 mM to about 0.1 mM, about 0.01 mM to about 0.04 mM.

In some embodiments, the KAl(SO₄)₂ x 12 H₂O is present in the culture medium at a concentration within the range of about 0.00004 mM to about 0.004 mM, e.g., 0.00008 mM to about 0.002 mM, about 0.0002 mM to about 0.0008 mM.

In some embodiments, the salt concentration in Medium 2 is adjusted upward to the range of 400 to 800 mM for formulation of the electrolyte in the reactor. Additionally, the sulfide concentration of relatively stationary cultures is adjusted downward to the range of <0.01mM (<1ppm sulfide in the exit gas stream).

In some examples, the media is sparged with a H2:CO2 gas mixture in a 4:1 ratio. The gas mixture can, in some embodiments, be generated with mass flow controllers at a total flow of 250 ml/minute. In some embodiments, the medium should be replenished at a rate suitable to maintain a useful concentration of essential minerals and to eliminate any metabolic products that may inhibit methanogenesis. Dilution rates below 0.1 culture volume per hour are suitable, since they yield high volumetric concentrations of active methane generation capacity.

### Culture Conditions

### Temperature

It is also disclosed that the temperature of the culture is maintained near the optimum temperature for growth of the organism used in the culture (e.g. about 35°C to about 37°C for mesophilic organisms such as *Methanosarcinia barkeri* and *Methanococcus maripaludis* or about 60°C to about 65°C for thermophiles such as *Methanothermobacter thermoautotrophicus,* and about 85°C to about 90°C for organisms such as *Methanocaldococcus jannaschii, Methanocaldococcus fervens, Methanocaldococcus indicus, Methanocaldococcus infernus,* and *Methanocaldococcus vulcanius*)*.* However, it is envisioned that temperatures above or below the temperatures for optimal growth may be used. In fact, higher conversion rates of methane may be obtained at temperatures above the optimal growth rate temperature. Temperatures of 50 °C or higher are contemplated, e.g., 51 °C or higher, 52 °C or higher, 53°C or higher, 54 °C or higher, 55 °C or higher, 56°C or higher, 57 °C or higher, 58 °C or higher, 59 °C or higher, 60°C to 150°C, 60°C to 120°C, 60°C to 100°C, 60°C to 80°C. Temperatures of 50°C or higher are contemplated, e.g. 50°C to 150°C, 50°C to 120°C, or 50°C to 100°C.

In view of the foregoing, the temperature at which the culture is maintained may be considered as a description of the methanogenic microorganisms contemplated herein. For example, when the temperature of the culture is maintained at a temperature between 55 °C and 120°C, the methanogenic microorganism is considered as one that can be cultured at this temperature. Accordingly, the methanogenic microorganism in some embodiments is a thermophile or a hyperthermophile. In some aspects, the culture of the biological reactor comprises an autotrophic thermophilic methanogenic microorganism or an autotrophic hyperthermophilic methanogenic microorganism. In some aspects, the culture of the biological reactor comprises an autotrophic thermophilic methanogenic microorganism or an autotrophic hyperthermophilic methanogenic microorganism, either of which is tolerant to high conductivity culture medium (e.g., about 1 to about 25 S/m), as described herein, e.g., a halophile.

Archaea may be capable of surviving extended periods at suboptimal temperatures. In some embodiments, a culture of archaea can naturally survive or are adapted to survive at room temperature (e.g. 22-28 °C) for a period of at least 3 weeks to 1, 2, 3, 4, 5 or 6 months.

In some embodiments, the organisms in the culture are not mesophilic. In some embodiments, the culture is not maintained at a temperature below or about 37 °C. With respect to thermophilic organisms (including, but not limited to, *Methanothermobacter thermoautotrophicus, Methanocaldococcus jannaschii, Methanocaldococcusfervens, Methanocaldococcus indicus, Methanocaldococcus infernus,* and *Methanocaldococcus vulcanius*)*,* in some embodiments, the temperature of the culture is e.g. about 60°C to about 150°C, about 60°C to about 120°C, about 60°C to about 100°C, or about 60°C to about 80°C.

### pH

Archaea can also survive under a wide range of pH conditions. In some embodiments, the pH of the culture comprising methanogenic microorganisms is between about 3.5 and about 10.0, although for growth conditions, the pH may be between about 6.5 and about 7.5. For example, the pH of the culture may be about 3.5, about 3.6., about 3.7, about 3.8, about 3.9, about 4.0, about 4.5, about 5.0, about 5.5, about 6.0, about 6.5, about 7.0, about 7.5, about 8.0, about 8.5, about 9.0, about 9.5, about 10.0. In some embodiments, the pH of the media is acidic, e.g. about 0.1 to about 5.5, about 0.1 to about 4, about 0.1 to about 3, about 1 to about 3, or about 2 to about 3. In some embodiments, the pH of the media is close to neutral, e.g. about 6 to about 8. In some embodiments, the pH of the media is alkaline, e.g. about 8.5 to about 11, or about 8 to about 10. The pH of the media can be altered by means known in the art. For example, the pH can be controlled by sparging CO₂ and/or by adding acid (e.g., HCL) or base (e.g., NaOH or NH₄OH) as needed.

### Pressure

In some embodiments, suitably pressures within the biological reactor range from about 0.5 atmospheres to about 500 atmospheres. The biological reactor can also contain a source of intermittent agitation of the culture. Also in one embodiment, the methane gas removed from the biological reactor suitably comprises less than about 450 ppm hydrogen sulfide, or alternatively less than about 400 ppm, 300 ppm, 200 ppm, 150 ppm, 100 ppm, 50 ppm or 20 ppm of hydrogen sulfide. Total gas delivery rates (CO₂) in the range of 0.2 to 4 volume of gas (STP) per volume of culture per minute are suitable, since they both maintain and exploit high volumetric concentrations of active methane generation capacity. In one embodiment, the redox potential is maintained below -100 mV or lower during methanogenesis. The method of the present invention encompasses conditions in which the redox potential is transiently increased to above -100 MV, as for example when air is added to the system.

### Culture Containers

A biological reactor, also known as a fermentor vessel, bioreactor, or simply reactor, as set forth herein may be any suitable vessel in which methanogenesis can take place. Suitable biological reactors to be used in the present invention should be sized relative to the volume of the CO₂ source. Typical streams of 2,200,000 1b CO₂/day from a 100,000,000 gal/yr ethanol plant would require a CO₂ recovery/methane production fermentor of about 750,000 gal total capacity. Fermentor vessels similar to the 750,000 gal individual fermentor units installed in such an ethanol plant would be suitable.

### Culture Volume and Density

The concentration of living cells in the culture medium (culture density) is in some embodiments maintained above 1 g dry weight/L. In certain embodiments, the density may be 30 g dry weight/L or higher. The volume of the culture is based upon the pore volume within the porous cathode structure within the reactor, plus any volume needed to fill any ancillary components of the reactor system, such as pumps and liquid/gas separators.

### Culture Medium For Reducing Contamination By Non-Methanogens

The term "non-methanogen" as used herein refers to any microorganism that is not a methanogen or is not a host cell expressing genes that permit methanogenesis. For example, in some embodiments, the archaea are cultured under conditions wherein the temperature, pH, salinity, sulfide concentration, carbon source, hydrogen concentration or electric source is altered such that growth of non-methanogens is significantly retarded under such conditions. The methanogens are cultured at a temperature that is at least 50 °C or higher, as discussed herein, e.g., 100°C or more, to avoid contamination by mesophilic non-methanogens. In other embodiments, the methanogens are cultured under conditions of high salinity (e.g., >75%) to avoid contamination by non-methanogens that are not capable of growing under high salt conditions. In still other embodiments, the methanogens are cultured under conditions in which the pH of the culture media is altered to be more acidic or more basic in order to reduce or eliminate contamination by non-methanogens that are not capable of growing under such conditions.

Contamination by non-methanogens can also be accomplished by minimizing amounts of organic carbon nutrients (e.g., sugars, fatty acids, oils, etc.) in the media. For example, in some embodiments, organic nutrients are substantially absent from the medium.

In some embodiments, components required for the growth of non-methanogenic organisms are substantially absent from the media. Such components include, but are not limited to, one or more organic carbon sources, and/or one or more organic nitrogen sources, and/or one or more vitamins. In some embodiments, formate, acetate, ethanol, methanol, methylamine, and any other metabolically available organic materials are substantially absent from the media.

In some embodiments, high salt conditions that permit survival of methanogens can retard growth of contaminating organisms.

In some embodiments, high temperatures that permit survival of methanogens can retard growth of contaminating organisms.

The term "substantially lacks" or "substantially absent" or "substantially excludes" as used herein refers to the qualitative condition of lacking an amount of a particular component significant enough to contribute to the desired function (e.g. growth of microorganisms, production of methane). In some embodiments, the term "substantially lacks" when applied to a given component of the media means that the media contains less than 5%, 4%, 3%, 2%, 1%, 0.9%, 0.8%, 0.7%, 0.6%, 0.5%, 0.4%, 0.3%, 0.2%, 0.1% or less of that component. In some embodiments, the media does not contain detectable amounts of a given component.

### Kits

The present disclosure further provides kits comprising any one or a combination of: a culture comprising methanogenic microorganisms, a reactor, and a culture medium. The culture of the kit may be in accordance with any of the teachings on cultures described herein. The kit may comprise a culture comprising an adapted strain of methanogenic microorganisms that are capable of growth and/or survival under high temperature conditions (i.e. above 50 °C, as further described herein), high salt or high conductivity conditions (as further described herein). The kit may comprise only the methanogenic microorganisms. The culture medium of the kits may be in accordance with any of the teachings on culture medium described herein. The kit may comprise a culture medium comprising the components of Medium 1 or comprising the components of Medium 2, as described herein. The kit may comprise only the culture medium. In certain of these aspects, the kit may comprise the reactor comprising an anode and cathode. The reactor may be in accordance with any of the teachings of reactors described herein.

### III. Implementations and Applications of the System

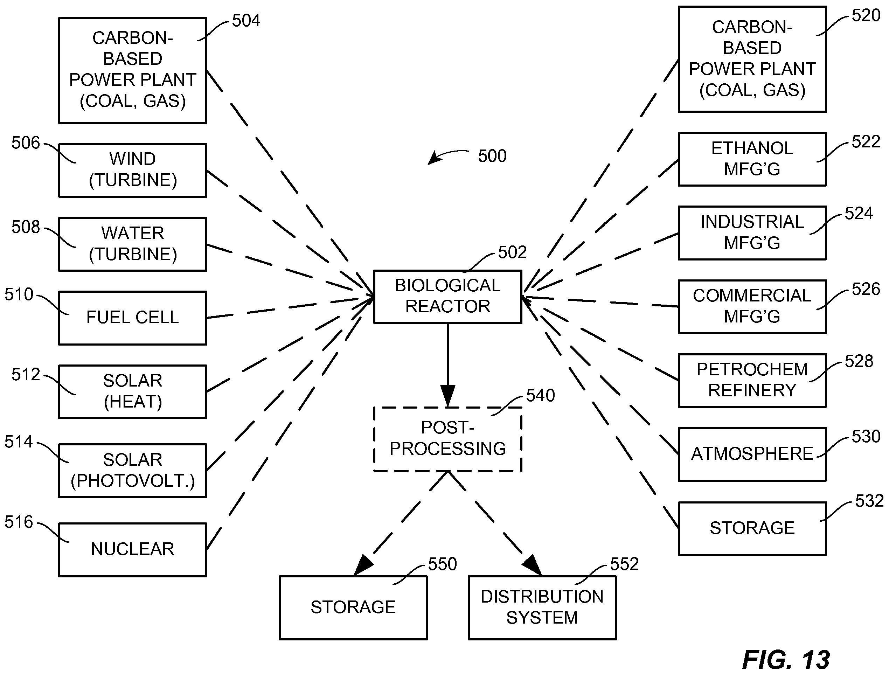

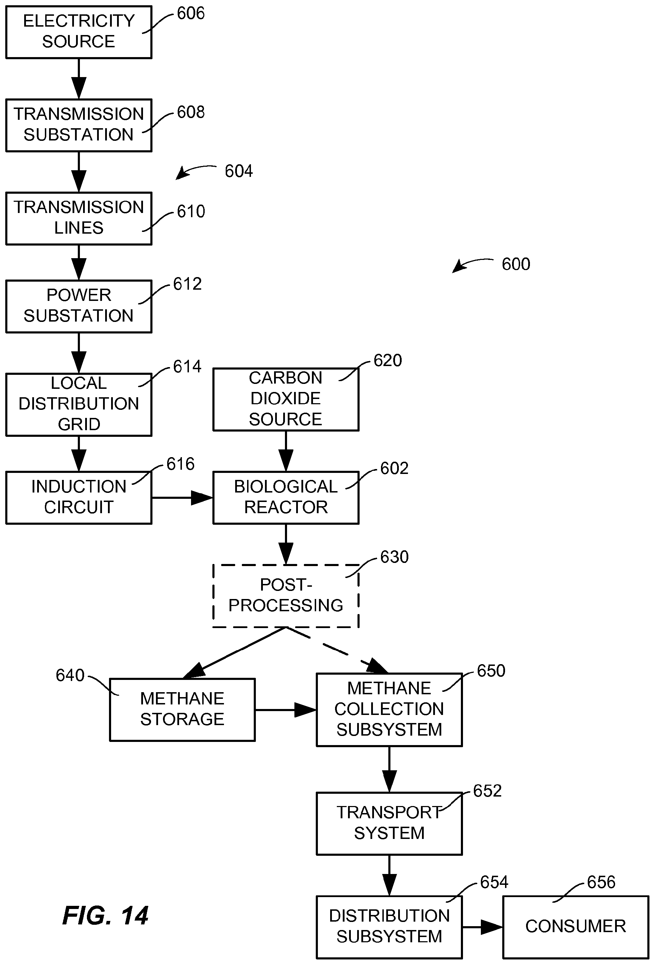

The biological reactor used in the methods for producing methane according to the claims may be used in a variety of implementations or applications, such as are illustrated in Figs. 13 and 14.

For example, a biological reactor may be used as part of a stand-alone system 500, as illustrated in Fig. 13. The system 500 may be used to provide multiple energy sources (e.g., electricity and methane), or to store electrical energy produced during favorable conditions as other energy resources (e.g., methane) for use when electrical energy cannot be generated at required levels. Such a stand-alone system 500 may be particularly useful in processing spatially or temporally stranded electricity where or when this electricity does not have preferable markets.

The system 500 may include a biological reactor 502 coupled to one or more electricity sources, for example a carbon-based power plant (e.g., coal-fired power plant, natural gas-fired power plant, or biomass-fired power plant) 504, a wind-powered turbine 506, water-powered turbine 508, a fuel cell 510, solar thermal system 512 or photovoltaic system 514, or a nuclear power plant 516. It will be recognized that other sources of electricity (e.g., a geothermal power source, or a capacitor or super capacitor used for energy storage) may be used in addition to or in substitution for those illustrated. The biological reactor 502 may be coupled directly to carbon-based power plant 504, nuclear power plant 516, or other power plant that may not be able to ramp power production up or down without significant costs and/or delays, and in such a system the biological reactor 502 uses surplus electricity to convert carbon dioxide into methane that can be used in a generator to produce sufficient electricity to meet additional demands. The biological reactor 502 may use surplus electricity (electricity that is not needed for other purposes) generated by one or more of the sources 506, 508, 510, 512, 514 to convert carbon dioxide into methane to be used in a generator to produce electricity when wind, water, solar or other conditions are unfavorable to produce electricity or to produce sufficient electricity to meet demands.

As is also illustrated in Fig. 13, the biological reactor 502 may be coupled to one or more carbon dioxide sources, for example one or more carbon-based power plants (e.g., coal-fired power plant, natural gas-fired power plant, biomass-fired power plant, or carbon-based fuel cells, which may be used as heating and power co-generation facilities or as dedicated factory power facilities) 520, which plants may be the same as or different from the plant 504. Alternatively, the stand-alone system 500 may be disposed in the vicinity of an industrial plant that provides carbon dioxide as an byproduct or a waste product, including ethanol manufacturing plants (e.g., fuel ethanol fermentation facilities) 522, industrial manufacturing plants (e.g., cement manufacturing plants or chemical manufacturing facilities) 524, commercial manufacturing plants (e.g., breweries) 526, and petrochemical refineries 528. While such significant point source emissions may serve well as a source of carbon dioxide for the biological reactor 502, it may also be possible to use atmospheric sources 530 as well (by using a carbon dioxide adsorption/desorption systems to capture atmospheric carbon dioxide, for example). As a further alternative, the carbon dioxide may be stored for use in the biological reactor (e.g., a stored source 532).

Where a significant point source of emissions is used as the carbon dioxide source (e.g., sources 520, 522, 524, 526, 528), the carbon dioxide emissions may be diverted into the biological reactor 502 to produce methane when electric power is available at prices below a pre-determined threshold. When electric power is above the pre-determined threshold, the carbon dioxide emissions may instead be emitted to the atmosphere, or it may be stored (as represented by the source 532) for later utilization in the biological reactor 502.

The carbon dioxide from a point emission source may be commingled with other gases, including carbon monoxide, hydrogen, hydrogen sulfide, nitrogen, or oxygen or other gases common to industrial processes, or it may be substantially pure. The mixture of gases can be delivered directly to the biological reactor 502, or the carbon dioxide may be separated from the gaseous mixture before delivery to the biological reactor 502. Such sources of mixed gases include landfills, trash-to-energy facilities, municipal or industrial solid waste facilities, anaerobic digesters, concentrated animal feeding operations, natural gas wells, and facilities for purifying natural gas, which sources may be considered along side the illustrated sources 520, 522, 524, 526, 528, 530, 532.

In operation, electricity and carbon dioxide may be delivered to the biological reactor 502 continuously to maintain a desired output of methane. Alternatively, the delivery rate of the electrical current, the carbon dioxide, or water to the biological reactor 502 may be varied which may cause the rate of methane production to vary. The variations in electrical current, carbon dioxide, and water may vary according to design (to modulate the rate of methane production in response to greater or lesser demand) or as the availability of these inputs varies.

As is also illustrated in Fig. 13, the system 500 may include certain post-processing equipment 540 associated with the biological reactor 502. For example, depending on the nature of the biological reactor 502, the flow of material exiting the first (cathode) chamber may be sent to a liquid-gas separator. Alternatively, it may be necessary to process the methane from a gaseous form into a liquid form, which may be more convenient for purposes of storage or transport. The gas may need to be filtered to remove byproducts, which byproducts may be stored or transported separately or may be disposed of as waste material. In any event, the methane produced by the biological reactor may be sent to a storage site 550, or optionally to a distribution or transportation system 552 such as is discussed in detail with reference to the system illustrated in Fig. 14. The methane may also be used locally, for example to replace natural gas in local operations for heat, or in chemical production.

It will be recognized that while the discussion has focused on methane as the primary product of the reactor 502, the reactor 502 also will produce oxygen, which may be referred to as a secondary product or as a byproduct. Oxygen may be stored or transported in the same fashion as methane, and as such a parallel storage site and/or distribution system may be established for the oxygen generated as well. As one such example, the oxygen may be used locally, for example to enhance the efficiency of combustion and/or fuel cell energy conversion.

In the alternative to a stand-alone implementation, an integrated system 600 may be provided wherein a reactor 602 is coupled to an electrical power distribution grid 604, or power grid for short, as illustrated in Fig. 14. The power grid 604 may connect to a source of electricity 606, for example one or more power plants discussed above, such as a carbon-based power plant (e.g., coal-fired power plant, natural gas-fired power plant, or biomass-fired power plant), a wind-powered turbine, water-powered turbine, a fuel cell, solar thermal system or photovoltaic system, or a nuclear power plant. These plants 606 may be connected, via transmission substations 608 and high-voltage transmission lines 610, to power substations 612 and associated local distribution grids 614. A local distribution grid 614 may be connected to one or more biological reactors 602 according to the present disclosure via an induction circuit 616.

As noted above, certain of these power plants, such as those combusting the carbon-based fuels, operate most efficiently at steady state (i.e., ramping power production up or down causes significant costs and/or delays). The power grid may also be connected to power plants that have a variable output, such as the wind-powered and water-powered turbines and the solar-thermal and photovoltaic systems. Additionally, power users have variable demand. As such, the electricity that power producers with the lowest marginal operating expenses desire to supply to the grid 604 can, and typically does, exceed demand during extended periods (so called off-peak periods). During those periods, the excess capacity can be used by one or more biological reactors 602 according to the present disclosure to produce methane.

As also noted above, the biological reactor 602 may be coupled to one or more carbon dioxide sources 620, for example including carbon-based power plants (e.g., coal-fired power plant, natural gas-fired power plant, biomass-fired power plant, or carbon-based fuel cells). Alternatively, the system 600 may be disposed near an industrial plant that provides carbon dioxide as a byproduct or a waste product, or may use atmospheric sources of carbon dioxide or stored carbon dioxide. In fact, while it may be possible to have a readily available source of carbon dioxide for conversion into methane when off-peak electricity is also available, it might also be necessary to store carbon dioxide during non-off-peak (or peak) periods for later conversion when the electricity is available. For example, an industrial source of carbon dioxide may typically generate most of its carbon dioxide during daylight hours, which may coincide with the typical peak demand period for electricity, causing some manner of storage to be required so that sufficient carbon dioxide is available to be used in conjunction with off-peak electricity production. Simple and inexpensive, gas impermeable tanks may be sufficient for such storage for short periods of time, such as part of a day or for several days. As to such storage issues for longer periods or for larger volumes, considerable effort is presently being devoted to sequestration of carbon dioxide in underground storage sites, and it may be possible to utilize the sequestered carbon dioxide stored in such sites as the source 620 of carbon dioxide for use in the biological reactors 602 according to the present disclosure.

As was the case with the system 500, the system 600 may include optional post-processing equipment 630 that is used to separate or treat the methane produced in the reactor 602 as required. The methane may be directed from the biological reactor 602 (with or without post-processing) into one or more containment vessels 640. In fact, the methane may be stored in aboveground storage tanks, or transported via local or interstate natural gas pipelines to underground storage locations, or reservoirs, such as depleted gas reservoirs, aquifers, and salt caverns. Additionally, the methane may be liquefied for even more compact storage, in particular where the biological reactors 602 are located where a connection to a power grid and a source of carbon dioxide are readily available, but the connection to a natural gas pipeline is uneconomical.

It will be further noted from Fig. 14 that methane may be taken from storage 640 or sent directly from the reactor 602 (optionally via the post-processing equipment 630) to a methane collection subsystem 650. From the collection subsystem 650, the methane may be introduced into a transport system 652, which system 652 may be a system of local, interstate or international pipelines for the transportation of methane, or alternatively natural gas. In this regard, the methane produced by the reactor 602 may take advantage of existing infrastructure to transport the methane from its location of production to its location of consumption. The transportation system 652 may be coupled to a distribution subsystem 654 that further facilitates its transmission to the consumer 656, which consumer may be located remote from the biological reactor 602. The consumer 656 may even be one of the sources of electricity 606 connected to the power grid 604.

It will also be recognized that a biological reactor for producing methane may be useful in a closed atmospheric environment containing carbon dioxide or in which carbon dioxide is released by respiration, or other biological processes or by chemical reactions such as combustion or by a fuel cell. The biological reactor may be operating as a stand-alone implementation (as in Fig. 13) or as part of an integrated system (as in Fig. 14). The carbon dioxide in such an environment can be combined in the biological reactor with electrical current and water to produce methane and oxygen. Production of methane by this process may occur in a building sealed for containment purposes, or underground compartment, mine or cave or in submersible vehicle such as a submarine, or any other device or compartment that has limited access to external molecular oxygen, but sufficient electrical power, water and carbon dioxide to support the production of methane and oxygen. Oxygen produced by the biological reactor may likewise be stored as a gas, or liquefied for future use, sale or distribution.

While the foregoing examples have discussed the potential uses for methane produced by the biological reactor in meeting industrial, commercial, transportation, or residential needs (e.g., conversion into electricity through combustion in a carbon-based fuel generator or other uses, such as heating or cooking, non-combustion based conversion of methane into electricity such as via fuel cells, or chemical conversion into other compounds such as via halogenation, or reaction with other reactive species), it is also possible to appreciate the use of the biological reactor used in the method according to the claims, either in a stand-alone system or as connected to a power grid, as a mechanism for carbon capture. That is, separate and apart from its uses to provide an alternative energy resource, the biological reactors may be used to remove carbon dioxide from the atmosphere, where the carbon dioxide is produced by living microorganisms, by chemical oxidation of organic material or from combustion of carbon-based fossil fuels, in particular where the carbon dioxide may be produced by large point sources such as fossil fuel power plants, cement kilns or fermentation facilities. The conversion of carbon dioxide into methane thus may produce not only methane, which has multiple other uses, but the conversion of carbon dioxide according to the present disclosure may generate or earn carbon credits for the source of the carbon dioxide, in that the carbon dioxide production of the source is decreased. These carbon credits may then be used within a regulatory scheme to offset other activities undertaken by the carbon dioxide producer, or in the life cycle of the products used or sold by the carbon dioxide producer, such as for renewable fuels derived from biomass, or gasoline refined from crude petroleum or may be used within a trading scheme to produce a separate source of revenue. Credits or offsets may be sold or conveyed in association with the methane, or oxygen, or other secondary products generated by the biological reactor or through the use of the biological reactor, or the credits may be traded independently such as on an exchange or sold directly to customers. In cases where the biological reactor functions within a business, or as part of a business contract with an entity, that uses oxygen, natural gas or methane from fossil or geologic sources, the methane produced by the biological reactor can be delivered to, or sold into a natural gas distribution system at times or in locations different from the use of natural gas and the business may retain any credits or offsets associated with the oxygen or methane produced with the biological reactor and apply such credit or offsets to natural gas or oxygen purchased from other sources and not produced directly by the biological reactor.

### IV. Other Exemplary Embodiments

According to one embodiment, a method of converting carbon dioxide to methane according to claim 1 comprises a) preparing a culture of hydrogenotrophic methanogenic archaea, b) placing the culture of hydrogenotrophic methanogenic archaea in a cathode chamber of an electrolysis chamber, the electrolysis chamber having an anode and a cathode, the cathode situated in the cathode chamber, and the cathode and anode chambers separated by a selectively permeable barrier; c) supplying carbon dioxide to the cathode chamber of the electrolysis chamber; d) supplying water to the electrolysis chamber, and e) wherein the hydrogenotrophic methanogenic archaea utilize the electrons released by the cathode and convert the carbon dioxide to methane. Additionally, step e) of such a method may only result in the production of methane gas by the hydrogenotrophic methanogenic archaea and a separate stream of oxygen gas by the anode.

According to another embodiment, a method of converting carbon dioxide to methane according to claim 1 comprises a) preparing a culture of hydrogenotrophic methanogenic archaea; b) placing the culture of hydrogenotrophic methanogenic archaea in a cathode chamber of an electrolysis chamber, the electrolysis chamber having an anode chamber and a cathode chamber wherein the anode chamber has an anode and the cathode chamber has a cathode; c) supplying carbon dioxide to the electrolysis chamber; d) supplying water to the electrolysis chamber; e) wherein an electric potential difference is maintained between the cathode and the anode; and f) wherein the hydrogenotrophic methanogenic archaea utilize the electric potential difference between the cathode and the anode to convert the carbon dioxide to methane. According to such a method, the anode chamber and the cathode chamber may be separated by a selectively permeable barrier.

### EXAMPLE 1

Vertical electrolysis chamber/cell configuration. A cylindrical electrolysis cell, 1.2 cm in internal diameter, was constructed from polysulfone plastic and arranged with an air-exposed anode on the bottom, covered by a Nafion 117 PEM and the closed cathode chamber on the top (Fig. 3). A Pt-carbon catalytic layer on a carbon mesh gas diffusion layer (GDE-LT) was used as the anode, with a titanium ring current collector around the circumference of the cell. The active area of the Nafion 117 PEM was 1.2 cm². The enclosed cathode chamber had a total internal volume of 3 ml and during operation the ∼1.5 ml of gas phase above the liquid phase was swept with a continuous flow (20 ml/min) of inert carrier gas. The composition of the exit gas, including any gases emitted within the cathode chamber, was analyzed continuously by mass spectrometry. The cathode electrode was constructed from reticulated vitreous carbon foam (ERG Materials and Aerospace Corp.) in the form of a cylinder, 1.2 cm diameter, 1 cm tall, placed in contact with the PEM, filling approximately half of the chamber, and connected electrically to the outside via a titanium wire. The cathode chamber was filled with 1.5 ml concentrated cell suspension, which settled into and filled the foam electrode. The carbon foam provided a high surface area for close contact between the cathode and the microorganisms. Occasionally, gas was released from bubbles formed in the solution by the addition of 5-10 microliters of antifoam agent.

Preparation of the cell suspension. Initial culture growth. Cells were grown in a continuously stirred tank fermenter, BioFlo 110, with a total internal volume of 1.3L and a typical liquid volume of 0.6L. An initial inoculum of the autotrophic hydrogenotrophic thermophilic methanogen, Methanothermobacter thermautotrophicus, DSMZ 3590, was grown at 60°C as a batch culture in a medium containing the following components: Na3nitrilotriacetate, 0.81 mM; nitrilotriacetic acid, 0.4 mM; NiCl₂-6H₂O, 0.005 mM; CoCl₂-6H₂O, 0.0025 mM; Na₂MoO₄-2H₂O, 0.0025 mM; MgCl₂-6H₂O, 1.0 mM; FeSO₄-7H₂O, 0.2 mM; Na₂SeO₃, 0.001 mM; Na₂WO₄, 0.01 mM; KH₂PO₄, 10 mM; NaCl, 10 mM; L-cysteine, 0.2 mM. This medium was sparged with a 4:1 H₂:CO₂ gas mixture generated with mass flow controllers at a total flow of 250 standard ml/minute. The pH of the medium was initially maintained at 6.85 via a pH stat that used 2M ammonium hydroxide to make adjustments. During the early growth phase of the culture when methane production was limited by cell concentration and increased at an exponential rate, a 0.5M sodium sulfide solution was added as needed to maintain the redox potential below -300 mV. Once the culture was grown and methane production reached a steady-state, the culture maintained the redox potential below - 450 mV on its own, using hydrogen as the reducing agent. Sulfide addition was slowed to a minimal rate (<1 ppm of H₂S in the outflow gas, as determined by mass spectrometry) needed for maintaining steady methane productivity with this strain of methanogen. Under these conditions, steady state methane production corresponds to 96-98% conversion of the input hydrogen.

Selection of a strain adapted to nearly stationary growth conditions. After steady state conditions had been established, the culture was maintained for several weeks without the addition of fresh medium. Instead, the increased volume of the culture generated by water production during methanogenesis was continually removed. The inorganic nutrients removed along with the removed medium and microorganisms were replaced from a 100x concentrated stock formulated as follows: Na₃ nitrilotriacetate, 81 mM; nitrilotriacetic acid, 40 mM; NiCl₂-6H₂O, 0.5 mM; CoCl₂-6H₂O, 0.25 mM; Na₂MoO₄-2H₂O, 0.25 mM; MgCl₂-6H₂O, 100 mM; FeSO₄-7H₂O, 20 mM; Na₂SeO₃, 0.1 mM; Na₂WO₄, 1.0 mM; KH₂PO₄, 1.0 M; NaCl, 1.0 M; L-cysteine, 20 mM. The goal of maintaining this extended culture under nearly stationary growth conditions was to select for a strain that could perform well and survive under conditions similar to those that are encountered in the electrolysis chamber.

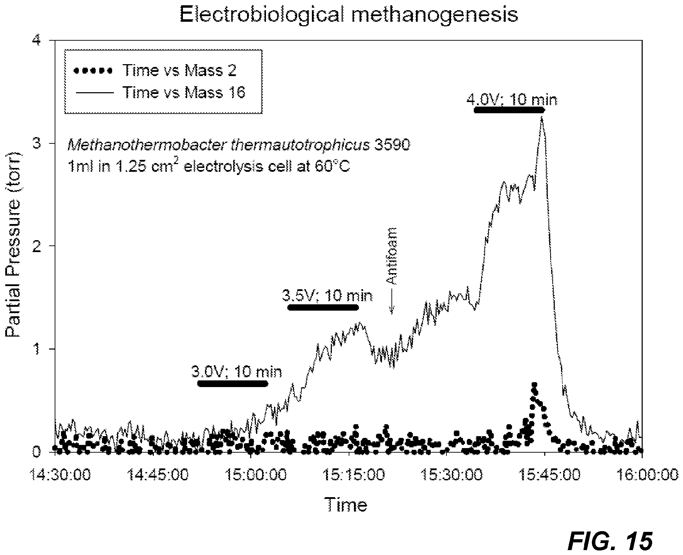

Performance under electrolysis conditions. The adapted culture, at a cell concentration of 5-7g dry weight/L, was starved for energy by sparging at 250 ml/min with a 4:1 gas mixture of Ar:CO₂ for several hours until neither hydrogen nor methane appeared in the effluent gas stream. The cells in a sample from the culture were then concentrated threefold by centrifugation under anaerobic conditions and resuspended at a final concentration of 15-21 g dry weight/L. One and one half milliliters of this concentrated suspension was placed into the chamber and impregnated into the carbon foam cathode (Fig. 3). The cathode was polarized at a voltage of 3.0 to 4.0 V, relative to the anode, and the gasses emerging from the chamber were monitored in a 20 ml/min flow of He carrier gas. As can be seen in Fig. 15, methane is the sole gas product at voltages less than 4.0V, but a minor proportion of hydrogen gas can also be produced at higher voltages. Other possible electrochemical reaction products, such as carbon monoxide, formic acid or methanol were not detected.

Various alternative improvements. Many modifications of this setup are anticipated and intended to be within the scope of the claims. Expanded graphite foam or particulate graphite or other high surface to volume electrically conductive materials may be suitable as cathode electrodes. A circulating cathode solution may allow for more rapid and complete gas exchange with the outside of the electrolysis chamber. Alternative temperatures within the scope of claim 1 may allow for more efficient charge transfer across the membrane separating the cathode and anode chamber. Alternative materials, including composite Nafion/PTFE, may be suitable for use as the selectively permeable membrane separating the cathode and anode chambers. Alternative geometries of the chambers may improve the charge and gas transport to and from the microbes. Alternative strains of methanogenic microbes may be more tolerant of the various mechanical strains, electrical demands, and metabolite exposure present in this invention.

**TABLE 1**

| **Class** | **Order** | **Family** | **Genus** | **Species** | **Taxonomy ID** |
|---|---|---|---|---|---|
| Archaeoglobi | Archaeoglobales | Archaeoglobaceae | Archaeoglobus | fulgidus | 224325 |
| Archaeoglobi | Archaeoglobales | Archaeoglobaceae | Archaeoglobus | infectus | 403219 |
| Archaeoglobi | Archaeoglobales | Archaeoglobaceae | Archaeoglobus | lithotrophicus | 138903 |
| Archaeoglobi | Archaeoglobales | Archaeoglobaceae | Archaeoglobus | profundus | 572546 |
| Archaeoglobi | Archaeoglobales | Archaeoglobaceae | Archaeoglobus | veneficus | 58290 |
| Archaeoglobi | Archaeoglobales | Archaeoglobaceae | Archaeoglobus | sp. Arc22 | 403220 |
| Archaeoglobi | Archaeoglobales | Archaeoglobaceae | Archaeoglobus | sp. Fe70 19 | 669409 |
| Archaeoglobi | Archaeoglobales | Archaeoglobaceae | Archaeoglobus | sp. Fe70 20 | 669410 |
| Archaeoglobi | Archaeoglobales | Archaeoglobaceae | Archaeoglobus | sp. N185-A | 269231 |
| Archaeoglobi | Archaeoglobales | Archaeoglobaceae | Archaeoglobus | sp. NS-tSRB-2 | 330389 |
| Archaeoglobi | Archaeoglobales | Archaeoglobaceae | Archaeoglobus | sp. NS70-A | 269230 |
| Archaeoglobi | Archaeoglobales | Archaeoglobaceae | Archaeoglobus | sp. PM70-1 | 387631 |
| Archaeoglobi | Archaeoglobales | Archaeoglobaceae | Archaeoglobus | Unclassified | 269247 |
| Archaeoglobi | Archaeoglobales | Archaeoglobaceae | Ferroglobus | placidus | 589924 |
| Archaeoglobi | Archaeoglobales | Archaeoglobaceae | Ferroglobus | Unclassified | 269249 |
| Archaeoglobi | Archaeoglobales | Archaeoglobaceae | Geoglobus | ahangari | 113653 |
| Archaeoglobi | Archaeoglobales | Archaeoglobaceae | Geoglobus | sp. AF1T1440 | 568410 |
| Archaeoglobi | Archaeoglobales | Archaeoglobaceae | Geoglobus | sp. AF1T2020 | 568411 |
| Archaeoglobi | Archaeoglobales | Archaeoglobaceae | Geoglobus | sp. AF2T1421 | 568413 |
| Archaeoglobi | Archaeoglobales | Archaeoglobaceae | Geoglobus | sp. AF2T819 | 568412 |
| Archaeoglobi | Archaeoglobales | Archaeoglobaceae | Geoglobus | sp. SBH6 | 565033 |
| Archaeoglobi | Archaeoglobales | Archaeoglobaceae | Geoglobus | sp. SN4 | 685720 |
| Archaeoglobi | Archaeoglobales | Unclassified | Unclassified | Unclassified | 309173 |
| Archaeoglobi | Unclassified | Unclassified | Unclassified | Unclassified | 763499 |
| Halobateria | Halobacteriales | Halobacteriaceae | Haladaptatus | cibarius | 453847 |
| Halobateria | Halobacteriales | Halobacteriaceae | Haladaptatus | litoreus | 553468 |
| Halobateria | Halobacteriales | Halobacteriaceae | Haladaptatus | paucihalophilus | 797209 |
| Halobateria | Halobacteriales | Halobacteriaceae | Halalkalicoccus | jeotgali | 413810 |
| Halobateria | Halobacteriales | Halobacteriaceae | Halalkalicoccus | tibetensis | 175632 |
| Halobateria | Halobacteriales | Halobacteriaceae | Halalkalicoccus | sp. C15 | 370968 |
| Halobateria | Halobacteriales | Halobacteriaceae | Halalkalicoccus | Unclassified | 663941 |
| Halobateria | Halobacteriales | Halobacteriaceae | Halarchaeum | acidiphilum | 489138 |
| Halobateria | Halobacteriales | Halobacteriaceae | Halarchaeum | sp. HY-204-1 | 744725 |
| Halobateria | Halobacteriales | Halobacteriaceae | Haloalcalophilium | atacamensis | 119862 |
| Halobateria | Halobacteriales | Halobacteriaceae | Haloalcalophilium | Unclassified | 260475 |
| Halobateria | Halobacteriales | Halobacteriaceae | Haloarcula | aidinensis | 56545 |
| Halobateria | Halobacteriales | Halobacteriaceae | Haloarcula | algeriensis | 337689 |
| Halobateria | Halobacteriales | Halobacteriaceae | Haloarcula | amylolytica | 396317 |
| Halobateria | Halobacteriales | Halobacteriaceae | Haloarcula | argentinensis | 43776 |
| Halobateria | Halobacteriales | Halobacteriaceae | Haloarcula | californiae | 662475 |
| Halobateria | Halobacteriales | Halobacteriaceae | Haloarcula | hispanica | 634497 |
| Halobateria | Halobacteriales | Halobacteriaceae | Haloarcula | japonica | 29282 |
| Halobateria | Halobacteriales | Halobacteriaceae | Haloarcula | marismortui | 272569 |
| Halobateria | Halobacteriales | Halobacteriaceae | Haloarcula | quadrata | 182779 |
| Halobateria | Halobacteriales | Halobacteriaceae | Haloarcula | siamensis | 456446 |
| Halobateria | Halobacteriales | Halobacteriaceae | Haloarcula | sinaiiensis | 662476 |
| Halobateria | Halobacteriales | Halobacteriaceae | Haloarcula | vallismortis | 662477 |
| Halobateria | Halobacteriales | Halobacteriaceae | Haloarcula | Unclassified | 44098 |
| Halobateria | Halobacteriales | Halobacteriaceae | Haloarcula | sp. 113 | 536043 |
| Halobateria | Halobacteriales | Halobacteriaceae | Haloarcula | sp. 12B-U | 584967 |
| Halobateria | Halobacteriales | Halobacteriaceae | Haloarcula | sp. 2KYS1 | 367810 |
| Halobateria | Halobacteriales | Halobacteriaceae | Haloarcula | sp. 2Sb1 | 329271 |
| Halobateria | Halobacteriales | Halobacteriaceae | Haloarcula | sp. 2TK2 | 251319 |
| Halobateria | Halobacteriales | Halobacteriaceae | Haloarcula | sp. 2TK3 | 251320 |
| Halobateria | Halobacteriales | Halobacteriaceae | Haloarcula | sp. 3TK1 | 251317 |
| Halobateria | Halobacteriales | Halobacteriaceae | Haloarcula | sp. 3TL4 | 367812 |
| Halobateria | Halobacteriales | Halobacteriaceae | Haloarcula | sp. 3TL6 | 367809 |
| Halobateria | Halobacteriales | Halobacteriaceae | Haloarcula | sp. 4TK1 | 251318 |
| Halobateria | Halobacteriales | Halobacteriaceae | Haloarcula | sp. 4TK3 | 251321 |
| Halobateria | Halobacteriales | Halobacteriaceae | Haloarcula | sp. 5Mm10 | 329272 |
| Halobateria | Halobacteriales | Halobacteriaceae | Haloarcula | sp. 9D-U | 584968 |
| Halobateria | Halobacteriales | Halobacteriaceae | Haloarcula | sp. A283 | 362893 |
| Halobateria | Halobacteriales | Halobacteriaceae | Haloarcula | sp. A337 | 362892 |
| Halobateria | Halobacteriales | Halobacteriaceae | Haloarcula | sp. A43 | 362894 |
| Halobateria | Halobacteriales | Halobacteriaceae | Haloarcula | sp. AB19 | 367757 |
| Halobateria | Halobacteriales | Halobacteriaceae | Haloarcula | sp. AJ4 | 222985 |
| Halobateria | Halobacteriales | Halobacteriaceae | Haloarcula | sp. AJ7 | 229734 |
| Halobateria | Halobacteriales | Halobacteriaceae | Haloarcula | sp. Arch325ppt-a | 682724 |
| Halobateria | Halobacteriales | Halobacteriaceae | Haloarcula | sp. ARG-2 | 69009 |
| Halobateria | Halobacteriales | Halobacteriaceae | Haloarcula | sp. AS7094 | 262078 |
| Halobateria | Halobacteriales | Halobacteriaceae | Haloarcula | sp. aus-5 | 464028 |
| Halobateria | Halobacteriales | Halobacteriaceae | Haloarcula | sp. B19-RDX | 589455 |
| Halobateria | Halobacteriales | Halobacteriaceae | Haloarcula | sp. B22-GYDX | 589454 |
| Halobateria | Halobacteriales | Halobacteriaceae | Haloarcula | sp. B44A | 370972 |
| Halobateria | Halobacteriales | Halobacteriaceae | Haloarcula | sp. C205090908-1R | 593534 |
| Halobateria | Halobacteriales | Halobacteriaceae | Haloarcula | sp. CH7 | 596417 |
| Halobateria | Halobacteriales | Halobacteriaceae | Haloarcula | sp. CHA1 | 596418 |
| Halobateria | Halobacteriales | Halobacteriaceae | Haloarcula | sp. CHB-U | 584969 |
| Halobateria | Halobacteriales | Halobacteriaceae | Haloarcula | sp. D1 | 242927 |
| Halobateria | Halobacteriales | Halobacteriaceae | Haloarcula | sp. D21 | 520558 |
| Halobateria | Halobacteriales | Halobacteriaceae | Haloarcula | sp. Ez1.2 | 655453 |
| Halobateria | Halobacteriales | Halobacteriaceae | Haloarcula | sp. FC130_ 30 | 493028 |
| Halobateria | Halobacteriales | Halobacteriaceae | Haloarcula | sp. FC134_ 14 | 493029 |
| Halobateria | Halobacteriales | Halobacteriaceae | Haloarcula | sp. FC134_ 15 | 493030 |
| Halobateria | Halobacteriales | Halobacteriaceae | Haloarcula | sp. FC137_1 | 493031 |
| Halobateria | Halobacteriales | Halobacteriaceae | Haloarcula | sp. FC137_ 10 | 493032 |
| Halobateria | Halobacteriales | Halobacteriaceae | Haloarcula | sp. FC137_11 | 493033 |
| Halobateria | Halobacteriales | Halobacteriaceae | Haloarcula | sp. FC137_ 12 | 493034 |
| Halobateria | Halobacteriales | Halobacteriaceae | Haloarcula | sp. FC137_ 13 | 493035 |
| Halobateria | Halobacteriales | Halobacteriaceae | Haloarcula | sp. FC137_ 2 | 493036 |
| Halobateria | Halobacteriales | Halobacteriaceae | Haloarcula | sp. FC137_ 3 | 493037 |
| Halobateria | Halobacteriales | Halobacteriaceae | Haloarcula | sp. FC137_ 4 | 493038 |
| Halobateria | Halobacteriales | Halobacteriaceae | Haloarcula | sp. FC137_ 5 | 493039 |
| Halobateria | Halobacteriales | Halobacteriaceae | Haloarcula | sp. FC137_ 6 | 493040 |
| Halobateria | Halobacteriales | Halobacteriaceae | Haloarcula | sp. FC137_ 7 | 493041 |
| Halobateria | Halobacteriales | Halobacteriaceae | Haloarcula | sp. FC137_ 8 | 493042 |
| Halobateria | Halobacteriales | Halobacteriaceae | Haloarcula | sp. FC137_ 9 | 493043 |
| Halobateria | Halobacteriales | Halobacteriaceae | Haloarcula | sp. FC167_ 2 | 493044 |
| Halobateria | Halobacteriales | Halobacteriaceae | Haloarcula | sp. FC168_ 1 | 493045 |
| Halobateria | Halobacteriales | Halobacteriaceae | Haloarcula | sp. FC168_ 10 | 493046 |
| Halobateria | Halobacteriales | Halobacteriaceae | Haloarcula | sp. FC168_ 11 | 493047 |
| Halobateria | Halobacteriales | Halobacteriaceae | Haloarcula | sp. FC168_12 | 493048 |
| Halobateria | Halobacteriales | Halobacteriaceae | Haloarcula | sp. FC168_ 13 | 493049 |
| Halobateria | Halobacteriales | Halobacteriaceae | Haloarcula | sp. FC168_14 | 493050 |
| Halobateria | Halobacteriales | Halobacteriaceae | Haloarcula | sp. FC168_ 15 | 493051 |
| Halobateria | Halobacteriales | Halobacteriaceae | Haloarcula | sp. FC168_ 16 | 493052 |
| Halobateria | Halobacteriales | Halobacteriaceae | Haloarcula | sp. FC168_17 | 493053 |
| Halobateria | Halobacteriales | Halobacteriaceae | Haloarcula | sp. FC168_ 18 | 493054 |
| Halobateria | Halobacteriales | Halobacteriaceae | Haloarcula | sp. FC168_19 | 493055 |
| Halobateria | Halobacteriales | Halobacteriaceae | Haloarcula | sp. FC168_ 20 | 493056 |
| Halobateria | Halobacteriales | Halobacteriaceae | Haloarcula | sp. FC168_ 21 | 493057 |
| Halobateria | Halobacteriales | Halobacteriaceae | Haloarcula | sp. FC168_ 22 | 493058 |
| Halobateria | Halobacteriales | Halobacteriaceae | Haloarcula | sp. FC168_ 23 | 493059 |
| Halobateria | Halobacteriales | Halobacteriaceae | Haloarcula | sp. FC168_24 | 493060 |
| Halobateria | Halobacteriales | Halobacteriaceae | Haloarcula | sp. FC168_ 25 | 493061 |
| Halobateria | Halobacteriales | Halobacteriaceae | Haloarcula | sp. FC168_26 | 493062 |
| Halobateria | Halobacteriales | Halobacteriaceae | Haloarcula | sp. FC168_ 27 | 493063 |
| Halobateria | Halobacteriales | Halobacteriaceae | Haloarcula | sp. FC168_ 28 | 493064 |
| Halobateria | Halobacteriales | Halobacteriaceae | Haloarcula | sp. FC168_29 | 493065 |
| Halobateria | Halobacteriales | Halobacteriaceae | Haloarcula | sp. FC168_3 | 493066 |
| Halobateria | Halobacteriales | Halobacteriaceae | Haloarcula | sp. FC168_ 31 | 493067 |
| Halobateria | Halobacteriales | Halobacteriaceae | Haloarcula | sp. FC168_ 32 | 493068 |
| Halobateria | Halobacteriales | Halobacteriaceae | Haloarcula | sp. FC168_ 33 | 493069 |
| Halobateria | Halobacteriales | Halobacteriaceae | Haloarcula | sp. FC168_ 34 | 493070 |
| Halobateria | Halobacteriales | Halobacteriaceae | Haloarcula | sp. FC168_35 | 493071 |
| Halobateria | Halobacteriales | Halobacteriaceae | Haloarcula | sp. FC168_ 36 | 493072 |
| Halobateria | Halobacteriales | Halobacteriaceae | Haloarcula | sp. FC168_37 | 493073 |
| Halobateria | Halobacteriales | Halobacteriaceae | Haloarcula | sp. FC168_ 38 | 493074 |
| Halobateria | Halobacteriales | Halobacteriaceae | Haloarcula | sp. FC168_ 39 | 493075 |
| Halobateria | Halobacteriales | Halobacteriaceae | Haloarcula | sp. FC168_4 | 493076 |
| Halobateria | Halobacteriales | Halobacteriaceae | Haloarcula | sp. FC168_ 40 | 493077 |
| Halobateria | Halobacteriales | Halobacteriaceae | Haloarcula | sp. FC168_41 | 493078 |
| Halobateria | Halobacteriales | Halobacteriaceae | Haloarcula | sp. FC168_ 5 | 493079 |
| Halobateria | Halobacteriales | Halobacteriaceae | Haloarcula | sp. FC168_ 6 | 493080 |
| Halobateria | Halobacteriales | Halobacteriaceae | Haloarcula | sp. FC168_ 7 | 493081 |
| Halobateria | Halobacteriales | Halobacteriaceae | Haloarcula | sp. FC168_ 8 | 493082 |
| Halobateria | Halobacteriales | Halobacteriaceae | Haloarcula | sp. FC168_9 | 493083 |
| Halobateria | Halobacteriales | Halobacteriaceae | Haloarcula | sp. GR3 | 574570 |
| Halobateria | Halobacteriales | Halobacteriaceae | Haloarcula | sp. GSP101 | 614216 |
| Halobateria | Halobacteriales | Halobacteriaceae | Haloarcula | sp. GSP108 | 614217 |
| Halobateria | Halobacteriales | Halobacteriaceae | Haloarcula | sp. HST01-2R | 575195 |
| Halobateria | Halobacteriales | Halobacteriaceae | Haloarcula | sp. HST03 | 575194 |
| Halobateria | Halobacteriales | Halobacteriaceae | Haloarcula | sp. I.B14 | 564675 |
| Halobateria | Halobacteriales | Halobacteriaceae | Haloarcula | sp. I.B17 | 564677 |
| Halobateria | Halobacteriales | Halobacteriaceae | Haloarcula | sp. I.B27 | 564684 |
| Halobateria | Halobacteriales | Halobacteriaceae | Haloarcula | sp. I.B29 | 564686 |
| Halobateria | Halobacteriales | Halobacteriaceae | Haloarcula | sp. I.C3 | 564689 |
| Halobateria | Halobacteriales | Halobacteriaceae | Haloarcula | sp. I.C4 | 564690 |
| Halobateria | Halobacteriales | Halobacteriaceae | Haloarcula | sp. I.C5 | 564691 |
| Halobateria | Halobacteriales | Halobacteriaceae | Haloarcula | sp. I.C6 | 564692 |
| Halobateria | Halobacteriales | Halobacteriaceae | Haloarcula | sp. I.C7 | 564693 |
| Halobateria | Halobacteriales | Halobacteriaceae | Haloarcula | sp. LCKW-Isolate15A | 338959 |
| Halobateria | Halobacteriales | Halobacteriaceae | Haloarcula | sp. LCKW-Isolate20A | 338960 |
| Halobateria | Halobacteriales | Halobacteriaceae | Haloarcula | sp. LCKW-lsolate20N | 338961 |
| Halobateria | Halobacteriales | Halobacteriaceae | Haloarcula | sp. LK1 | 796337 |
| Halobateria | Halobacteriales | Halobacteriaceae | Haloarcula | sp. M4r1 | 323740 |
| Halobateria | Halobacteriales | Halobacteriaceae | Haloarcula | sp. MGG2 | 717750 |
| Halobateria | Halobacteriales | Halobacteriaceae | Haloarcula | sp. MGG3 | 717751 |
| Halobateria | Halobacteriales | Halobacteriaceae | Haloarcula | sp. OHF-1 | 217171 |
| Halobateria | Halobacteriales | Halobacteriaceae | Haloarcula | sp. OHF-2 | 217024 |
| Halobateria | Halobacteriales | Halobacteriaceae | Haloarcula | sp. Q6 | 323742 |
| Halobateria | Halobacteriales | Halobacteriaceae | Haloarcula | sp. RBCA-10^2 | 584970 |
| Halobateria | Halobacteriales | Halobacteriaceae | Haloarcula | sp. SP2(1) | 402992 |
| Halobateria | Halobacteriales | Halobacteriaceae | Haloarcula | sp. SP2(2) | 402870 |
| Halobateria | Halobacteriales | Halobacteriaceae | Haloarcula | sp. SP4 | 402871 |
| Halobateria | Halobacteriales | Halobacteriaceae | Haloarcula | sp. YW016 | 340950 |
| Halobateria | Halobacteriales | Halobacteriaceae | Haloarcula | Unclassified | 376544 |
| Halobateria | Halobacteriales | Halobacteriaceae | Halobacterium | jilantaiense | 355548 |
| Halobateria | Halobacteriales | Halobacteriaceae | Halobacterium | noricense | 223182 |
| Halobateria | Halobacteriales | Halobacteriaceae | Halobacterium | piscisalsi | 413968 |
| Halobateria | Halobacteriales | Halobacteriaceae | Halobacterium | salina rum (strain Mex) | 33003 |
| Halobateria | Halobacteriales | Halobacteriaceae | Halobacterium | salina rum (strain Port) | 33004 |
| Halobateria | Halobacteriales | Halobacteriaceae | Halobacterium | salina rum (strain Shark) | 33005 |
| Halobateria | Halobacteriales | Halobacteriaceae | Halobacterium | salinarum R1 | 478009 |
| Halobateria | Halobacteriales | Halobacteriaceae | Halobacterium | salinarum sp. NRC-1 | 64091 |
| Halobateria | Halobacteriales | Halobacteriaceae | Halobacterium | Unclassified | 2243 |
| Halobateria | Halobacteriales | Halobacteriaceae | Halobacterium | sp. 0Cb11 | 639868 |
| Halobateria | Halobacteriales | Halobacteriaceae | Halobacterium | sp. 0Cb21 | |
| Halobateria | Halobacteriales | Halobacteriaceae | Halobacterium | sp. 0Cb22 | |
| Halobateria | Halobacteriales | Halobacteriaceae | Halobacterium | sp. 0Cb23 | |
| Halobateria | Halobacteriales | Halobacteriaceae | Halobacterium | sp. 15C0 | |
| Halobateria | Halobacteriales | Halobacteriaceae | Halobacterium | sp. 15C11 | |
| Halobateria | Halobacteriales | Halobacteriaceae | Halobacterium | sp. 15C21 | |
| Halobateria | Halobacteriales | Halobacteriaceae | Halobacterium | sp. 15C23 | |
| Halobateria | Halobacteriales | Halobacteriaceae | Halobacterium | sp. 15C31 | |
| Halobateria | Halobacteriales | Halobacteriaceae | Halobacterium | sp. 1TK1 | |
| Halobateria | Halobacteriales | Halobacteriaceae | Halobacterium | sp. 1TK2 | |
| Halobateria | Halobacteriales | Halobacteriaceae | Halobacterium | sp. 1TK3 | |
| Halobateria | Halobacteriales | Halobacteriaceae | Halobacterium | sp. 2-24-2 | |
| Halobateria | Halobacteriales | Halobacteriaceae | Halobacterium | sp. 2-24-3 | |
| Halobateria | Halobacteriales | Halobacteriaceae | Halobacterium | sp. 2-24-4 | |
| Halobateria | Halobacteriales | Halobacteriaceae | Halobacterium | sp. 2-24-5 | |
| Halobateria | Halobacteriales | Halobacteriaceae | Halobacterium | sp. 2-24-6 | |
| Halobateria | Halobacteriales | Halobacteriaceae | Halobacterium | sp. 2-24-7 | |
| Halobateria | Halobacteriales | Halobacteriaceae | Halobacterium | sp. 2Ma3 | |
| Halobateria | Halobacteriales | Halobacteriaceae | Halobacterium | sp. 3KYS1 | |
| Halobateria | Halobacteriales | Halobacteriaceae | Halobacterium | sp. 5Mm6 | |
| Halobateria | Halobacteriales | Halobacteriaceae | Halobacterium | sp. 7Sb5 | |
| Halobateria | Halobacteriales | Halobacteriaceae | Halobacterium | sp. 9R | |
| Halobateria | Halobacteriales | Halobacteriaceae | Halobacterium | sp. AS133 | |
| Halobateria | Halobacteriales | Halobacteriaceae | Halobacterium | sp. AS28 | |
| Halobateria | Halobacteriales | Halobacteriaceae | Halobacterium | sp. AS7092 | |
| Halobateria | Halobacteriales | Halobacteriaceae | Halobacterium | sp. AUS-1 | |
| Halobateria | Halobacteriales | Halobacteriaceae | Halobacterium | sp. AUS-2 | |
| Halobateria | Halobacteriales | Halobacteriaceae | Halobacterium | sp. BCCS 030 | |
| Halobateria | Halobacteriales | Halobacteriaceae | Halobacterium | sp. BCCS 039 | |
| Halobateria | Halobacteriales | Halobacteriaceae | Halobacterium | sp. BIHGY150/14 | |
| Halobateria | Halobacteriales | Halobacteriaceae | Halobacterium | sp. BIHSTY150/18 | |
| Halobateria | Halobacteriales | Halobacteriaceae | Halobacterium | sp. CH11 | |
| Halobateria | Halobacteriales | Halobacteriaceae | Halobacterium | sp. EL 001 | |
| Halobateria | Halobacteriales | Halobacteriaceae | Halobacterium | sp. EL 002 | |
| Halobateria | Halobacteriales | Halobacteriaceae | Halobacterium | sp. EL 003 | |
| Halobateria | Halobacteriales | Halobacteriaceae | Halobacterium | sp. Ez21 | |
| Halobateria | Halobacteriales | Halobacteriaceae | Halobacterium | sp. EzA | |
| Halobateria | Halobacteriales | Halobacteriaceae | Halobacterium | sp. FIC145_ 1 | |
| Halobateria | Halobacteriales | Halobacteriaceae | Halobacterium | sp. FIC145_ 2 | |
| Halobateria | Halobacteriales | Halobacteriaceae | Halobacterium | sp. FIC146_ 1 | |
| Halobateria | Halobacteriales | Halobacteriaceae | Halobacterium | sp. FIC146_ 2 | |
| Halobateria | Halobacteriales | Halobacteriaceae | Halobacterium | sp. FIC146_3 | |
| Halobateria | Halobacteriales | Halobacteriaceae | Halobacterium | sp. FIC146_ 4 | |
| Halobateria | Halobacteriales | Halobacteriaceae | Halobacterium | sp. GN101 | |
| Halobateria | Halobacteriales | Halobacteriaceae | Halobacterium | sp. GRB | |
| Halobateria | Halobacteriales | Halobacteriaceae | Halobacterium | sp. HA3 | |
| Halobateria | Halobacteriales | Halobacteriaceae | Halobacterium | sp. halo-3 | |
| Halobateria | Halobacteriales | Halobacteriaceae | Halobacterium | sp. HM01 | |
| Halobateria | Halobacteriales | Halobacteriaceae | Halobacterium | sp. HM02 | |
| Halobateria | Halobacteriales | Halobacteriaceae | Halobacterium | sp. HM11 | |
| Halobateria | Halobacteriales | Halobacteriaceae | Halobacterium | sp. HM13 | |
| Halobateria | Halobacteriales | Halobacteriaceae | Halobacterium | sp. HM3 | |
| Halobateria | Halobacteriales | Halobacteriaceae | Halobacterium | sp. HP-R1 | |
| Halobateria | Halobacteriales | Halobacteriaceae | Halobacterium | sp. HSC3 | |
| Halobateria | Halobacteriales | Halobacteriaceae | Halobacterium | sp. I.B12 | |
| Halobateria | Halobacteriales | Halobacteriaceae | Halobacterium | sp. I.C16 | |
| Halobateria | Halobacteriales | Halobacteriaceae | Halobacterium | sp. I.C17 | |
| Halobateria | Halobacteriales | Halobacteriaceae | Halobacterium | sp. I.C2 | |
| Halobateria | Halobacteriales | Halobacteriaceae | Halobacterium | sp. I.C24 | |
| Halobateria | Halobacteriales | Halobacteriaceae | Halobacterium | sp. I.C26 | |
| Halobateria | Halobacteriales | Halobacteriaceae | Halobacterium | sp. I.C29 | |
| Halobateria | Halobacteriales | Halobacteriaceae | Halobacterium | sp. I.C30 | |
| Halobateria | Halobacteriales | Halobacteriaceae | Halobacterium | sp. I.C31 | |
| Halobateria | Halobacteriales | Halobacteriaceae | Halobacterium | sp. JCM 9447 | |
| Halobateria | Halobacteriales | Halobacteriaceae | Halobacterium | sp. JP-6 | |
| Halobateria | Halobacteriales | Halobacteriaceae | Halobacterium | sp. LCKS000-Isolate10 | |
| Halobateria | Halobacteriales | Halobacteriaceae | Halobacterium | sp. LcKS000-Isolate39 | |
| Halobateria | Halobacteriales | Halobacteriaceae | Halobacterium | sp. LCKS200-Isolate33 | |
| Halobateria | Halobacteriales | Halobacteriaceae | Halobacterium | sp. MO51 | |
| Halobateria | Halobacteriales | Halobacteriaceae | Halobacterium | sp. MVBDU1 | |
| Halobateria | Halobacteriales | Halobacteriaceae | Halobacterium | sp. MVBDU2 | |
| Halobateria | Halobacteriales | Halobacteriaceae | Halobacterium | sp. NCIMB 714 | |
| Halobateria | Halobacteriales | Halobacteriaceae | Halobacterium | sp. NCIMB 718 | |
| Halobateria | Halobacteriales | Halobacteriaceae | Halobacterium | sp. NCIMB 720 | |
| Halobateria | Halobacteriales | Halobacteriaceae | Halobacterium | sp. NCIMB 733 | |
| Halobateria | Halobacteriales | Halobacteriaceae | Halobacterium | sp. NCIMB 734 | |
| Halobateria | Halobacteriales | Halobacteriaceae | Halobacterium | sp. NCIMB 741 | |
| Halobateria | Halobacteriales | Halobacteriaceae | Halobacterium | sp. NCIMB 765 | |
| Halobateria | Halobacteriales | Halobacteriaceae | Halobacterium | sp. P102070208-3O | |
| Halobateria | Halobacteriales | Halobacteriaceae | Halobacterium | sp. P102070208-3R | |
| Halobateria | Halobacteriales | Halobacteriaceae | Halobacterium | sp. P92A090908-6O | |
| Halobateria | Halobacteriales | Halobacteriaceae | Halobacterium | sp. P92A090908-6P | |
| Halobateria | Halobacteriales | Halobacteriaceae | Halobacterium | sp. SG1 | |
| Halobateria | Halobacteriales | Halobacteriaceae | Halobacterium | sp. SP3(2) | |
| Halobateria | Halobacteriales | Halobacteriaceae | Halobacterium | sp. TG1 | |
| Halobateria | Halobacteriales | Halobacteriaceae | Halobacterium | sp. IJ-EY1 | |
| Halobateria | Halobacteriales | Halobacteriaceae | Halobacterium | sp. XH3 | |
| Halobateria | Halobacteriales | Halobacteriaceae | Halobacterium | sp. Y12 | |
| Halobateria | Halobacteriales | Halobacteriaceae | Halobacterium | Unclassified | 260463 |
| Halobateria | Halobacteriales | Halobacteriaceae | Halobaculum | gomorrense | 43928 |
| Halobateria | Halobacteriales | Halobacteriaceae | Halobiforma | haloterrestris | 148448 |
| Halobateria | Halobacteriales | Halobacteriaceae | Halobiforma | lacisalsi | 358396 |
| Halobateria | Halobacteriales | Halobacteriaceae | Halobiforma | nitratireducens | 130048 |
| Halobateria | Halobacteriales | Halobacteriaceae | Halobiforma | Unclassified | 417359 |
| Halobateria | Halobacteriales | Halobacteriaceae | Halococcus | dombroskii | 179637 |
| Halobateria | Halobacteriales | Halobacteriaceae | Halococcus | hamelinensis | 332168 |
| Halobateria | Halobacteriales | Halobacteriaceae | Halococcus | morrhuae | 2250 |
| Halobateria | Halobacteriales | Halobacteriaceae | Halococcus | qingdaonensis | 224402 |
| Halobateria | Halobacteriales | Halobacteriaceae | Halococcus | saccharolyticus | 62319 |
| Halobateria | Halobacteriales | Halobacteriaceae | Halococcus | salifodinae | 36738 |
| Halobateria | Halobacteriales | Halobacteriaceae | Halococcus | thailandensis | 335952 |
| Halobateria | Halobacteriales | Halobacteriaceae | Halococcus | Unclassified | 29286 |
| Halobateria | Halobacteriales | Halobacteriaceae | Halococcus | sp. 001/2 | 481737 |
| Halobateria | Halobacteriales | Halobacteriaceae | Halococcus | sp. 004/1-2 | 481736 |
| Halobateria | Halobacteriales | Halobacteriaceae | Halococcus | sp. BIGigoW09 | 481735 |
| Halobateria | Halobacteriales | Halobacteriaceae | Halococcus | sp. BIHTY10/11 | 481734 |
| Halobateria | Halobacteriales | Halobacteriaceae | Halococcus | sp. CH8B | 596420 |
| Halobateria | Halobacteriales | Halobacteriaceae | Halococcus | sp. CH8K | 596421 |
| Halobateria | Halobacteriales | Halobacteriaceae | Halococcus | sp. FC211 | 493090 |
| Halobateria | Halobacteriales | Halobacteriaceae | Halococcus | sp. HA4 | 723882 |
| Halobateria | Halobacteriales | Halobacteriaceae | Halococcus | sp. HP-R5 | 701664 |
| Halobateria | Halobacteriales | Halobacteriaceae | Halococcus | sp. HPA-86 | 436977 |
| Halobateria | Halobacteriales | Halobacteriaceae | Halococcus | sp. HPA-87 | 436978 |
| Halobateria | Halobacteriales | Halobacteriaceae | Halococcus | sp. HSA18 | 436979 |
| Halobateria | Halobacteriales | Halobacteriaceae | Halococcus | sp. HSA19 | 436980 |
| Halobateria | Halobacteriales | Halobacteriaceae | Halococcus | sp. HSA20 | 436981 |
| Halobateria | Halobacteriales | Halobacteriaceae | Halococcus | sp. HSt 2.0 | 557878 |
| Halobateria | Halobacteriales | Halobacteriaceae | Halococcus | sp. HSt 2.2 | 557879 |
| Halobateria | Halobacteriales | Halobacteriaceae | Halococcus | sp. HSt 3.0 | 557880 |
| Halobateria | Halobacteriales | Halobacteriaceae | Halococcus | sp. HSt 3.1 | 557881 |
| Halobateria | Halobacteriales | Halobacteriaceae | Halococcus | sp. HSt 4.0 | 557882 |
| Halobateria | Halobacteriales | Halobacteriaceae | Halococcus | sp. HSt 4.1 | 557883 |
| Halobateria | Halobacteriales | Halobacteriaceae | Halococcus | sp. HSt 5.0 | 557884 |
| Halobateria | Halobacteriales | Halobacteriaceae | Halococcus | sp. IS10-2 | 335951 |
| Halobateria | Halobacteriales | Halobacteriaceae | Halococcus | sp. JCM 8979 | 228414 |
| Halobateria | Halobacteriales | Halobacteriaceae | Halococcus | Unclassified | 260465 |
| Halobateria | Halobacteriales | Halobacteriaceae | Haloferax | alexandrines | 114529 |
| Halobateria | Halobacteriales | Halobacteriaceae | Haloferax | antrum | 381855 |
| Halobateria | Halobacteriales | Halobacteriaceae | Haloferax | berberensis | 340952 |
| Halobateria | Halobacteriales | Halobacteriaceae | Haloferax | denitrificans | 662478 |
| Halobateria | Halobacteriales | Halobacteriaceae | Haloferax | elongans | 403191 |
| Halobateria | Halobacteriales | Halobacteriaceae | Haloferax | gibbonsii | 35746 |
| Halobateria | Halobacteriales | Halobacteriaceae | Haloferax | larsenii | 302484 |
| Halobateria | Halobacteriales | Halobacteriaceae | Haloferax | lucentense | 523840 |
| Halobateria | Halobacteriales | Halobacteriaceae | Haloferax | mediterranei | 523841 |
| Halobateria | Halobacteriales | Halobacteriaceae | Haloferax | mucosum | 662479 |
| Halobateria | Halobacteriales | Halobacteriaceae | Haloferax | opilio | 381854 |
| Halobateria | Halobacteriales | Halobacteriaceae | Haloferax | prahovense | 381852 |
| Halobateria | Halobacteriales | Halobacteriaceae | Haloferax | rutilus | 381853 |
| Halobateria | Halobacteriales | Halobacteriaceae | Haloferax | sulfurifontis | 662480 |
| Halobateria | Halobacteriales | Halobacteriaceae | Haloferax | viridis | 381851 |
| Halobateria | Halobacteriales | Halobacteriaceae | Haloferax | volcanii | 309800 |
| Halobateria | Halobacteriales | Halobacteriaceae | Haloferax | Unclassified | 2253 |
| Halobateria | Halobacteriales | Halobacteriaceae | Haloferax | sp. 25H4_1 | |
| Halobateria | Halobacteriales | Halobacteriaceae | Haloferax | sp. 25H4_2 | |
| Halobateria | Halobacteriales | Halobacteriaceae | Haloferax | sp. 25H4_3 | |
| Halobateria | Halobacteriales | Halobacteriaceae | Haloferax | sp. 25H4_4 | |
| Halobateria | Halobacteriales | Halobacteriaceae | Haloferax | sp. 50C21_1 | |
| Halobateria | Halobacteriales | Halobacteriaceae | Haloferax | sp. 50C21_2 | |
| Halobateria | Halobacteriales | Halobacteriaceae | Haloferax | sp. 50C21_3 | |
| Halobateria | Halobacteriales | Halobacteriaceae | Haloferax | sp. 50C21_4 | |
| Halobateria | Halobacteriales | Halobacteriaceae | Haloferax | sp. 50C21_5 | |
| Halobateria | Halobacteriales | Halobacteriaceae | Haloferax | sp. 50C21_6 | |
| Halobateria | Halobacteriales | Halobacteriaceae | Haloferax | sp. A317 | |
| Halobateria | Halobacteriales | Halobacteriaceae | Haloferax | sp. A440 | |
| Halobateria | Halobacteriales | Halobacteriaceae | Haloferax | sp. Aa2.2 | |
| Halobateria | Halobacteriales | Halobacteriaceae | Haloferax | sp. B-1 | |
| Halobateria | Halobacteriales | Halobacteriaceae | Haloferax | sp. B-3 | |
| Halobateria | Halobacteriales | Halobacteriaceae | Haloferax | sp. B-4 | |
| Halobateria | Halobacteriales | Halobacteriaceae | Haloferax | sp. Bej51 | |
| Halobateria | Halobacteriales | Halobacteriaceae | Haloferax | sp. BejS3 | |
| Halobateria | Halobacteriales | Halobacteriaceae | Haloferax | sp. BS1 | |
| Halobateria | Halobacteriales | Halobacteriaceae | Haloferax | sp. BS2a | |
| Halobateria | Halobacteriales | Halobacteriaceae | Haloferax | sp. BS2b | |
| Halobateria | Halobacteriales | Halobacteriaceae | Haloferax | sp. BV2 | |
| Halobateria | Halobacteriales | Halobacteriaceae | Haloferax | sp. CIBAARC2BR | |
| Halobateria | Halobacteriales | Halobacteriaceae | Haloferax | sp. CS1-10 | |
| Halobateria | Halobacteriales | Halobacteriaceae | Haloferax | sp. CS1-3 | |
| Halobateria | Halobacteriales | Halobacteriaceae | Haloferax | sp. CS1-4 | |
| Halobateria | Halobacteriales | Halobacteriaceae | Haloferax | sp. CS1-5 | |
| Halobateria | Halobacteriales | Halobacteriaceae | Haloferax | sp. CS1-7 | |
| Halobateria | Halobacteriales | Halobacteriaceae | Haloferax | sp. CS1-8 | |
| Halobateria | Halobacteriales | Halobacteriaceae | Haloferax | sp. CS1-9 | |
| Halobateria | Halobacteriales | Halobacteriaceae | Haloferax | sp. CS2-1 | |
| Halobateria | Halobacteriales | Halobacteriaceae | Haloferax | sp. CS3-01 | |
| Halobateria | Halobacteriales | Halobacteriaceae | Haloferax | sp. CS3-1 | |
| Halobateria | Halobacteriales | Halobacteriaceae | Haloferax | sp. CT2-4 | |
| Halobateria | Halobacteriales | Halobacteriaceae | Haloferax | sp. CT3-3 | |
| Halobateria | Halobacteriales | Halobacteriaceae | Haloferax | sp. CT3-7 | |
| Halobateria | Halobacteriales | Halobacteriaceae | Haloferax | sp. CT4-2 | |
| Halobateria | Halobacteriales | Halobacteriaceae | Haloferax | sp. CT4-3 | |
| Halobateria | Halobacteriales | Halobacteriaceae | Haloferax | sp. CT4-7 | |
| Halobateria | Halobacteriales | Halobacteriaceae | Haloferax | sp. D107 | |
| Halobateria | Halobacteriales | Halobacteriaceae | Haloferax | sp. D1227 | |
| Halobateria | Halobacteriales | Halobacteriaceae | Haloferax | sp. FB247_1 | |
| Halobateria | Halobacteriales | Halobacteriaceae | Haloferax | sp. FB247_ 10 | |
| Halobateria | Halobacteriales | Halobacteriaceae | Haloferax | sp. FB247_11 | |
| Halobateria | Halobacteriales | Halobacteriaceae | Haloferax | sp. FB247_ 12 | |
| Halobateria | Halobacteriales | Halobacteriaceae | Haloferax | sp. FB247_ 13 | |
| Halobateria | Halobacteriales | Halobacteriaceae | Haloferax | sp. FB247_ 14 | |
| Halobateria | Halobacteriales | Halobacteriaceae | Haloferax | sp. FB247_ 2 | |
| Halobateria | Halobacteriales | Halobacteriaceae | Haloferax | sp. FB247_3 | |
| Halobateria | Halobacteriales | Halobacteriaceae | Haloferax | sp. FB247_ 4 | |
| Halobateria | Halobacteriales | Halobacteriaceae | Haloferax | sp. FB247_ 5 | |
| Halobateria | Halobacteriales | Halobacteriaceae | Haloferax | sp. FB247_ 6 | |
| Halobateria | Halobacteriales | Halobacteriaceae | Haloferax | sp. FB247_ 7 | |
| Halobateria | Halobacteriales | Halobacteriaceae | Haloferax | sp. FB247_8 | |
| Halobateria | Halobacteriales | Halobacteriaceae | Haloferax | sp. FB247_ 9 | |
| Halobateria | Halobacteriales | Halobacteriaceae | Haloferax | sp. FB25_1 | |
| Halobateria | Halobacteriales | Halobacteriaceae | Haloferax | sp. FB25_ 2 | |
| Halobateria | Halobacteriales | Halobacteriaceae | Haloferax | sp. FB25_3 | |
| Halobateria | Halobacteriales | Halobacteriaceae | Haloferax | sp. FB25_4 | |
| Halobateria | Halobacteriales | Halobacteriaceae | Haloferax | sp. FB25_5 | |
| Halobateria | Halobacteriales | Halobacteriaceae | Haloferax | sp. FB25_6 | |
| Halobateria | Halobacteriales | Halobacteriaceae | Haloferax | sp. FB25_7 | |
| Halobateria | Halobacteriales | Halobacteriaceae | Haloferax | sp. FB25_8 | |
| Halobateria | Halobacteriales | Halobacteriaceae | Haloferax | sp. FB25_9 | |
| Halobateria | Halobacteriales | Halobacteriaceae | Haloferax | sp. FC28_ 1 | |
| Halobateria | Halobacteriales | Halobacteriaceae | Haloferax | sp. FC28_10 | |
| Halobateria | Halobacteriales | Halobacteriaceae | Haloferax | sp. FC28_11 | |
| Halobateria | Halobacteriales | Halobacteriaceae | Haloferax | sp. FC28_12 | |
| Halobateria | Halobacteriales | Halobacteriaceae | Haloferax | sp. FC28_ 13 | |
| Halobateria | Halobacteriales | Halobacteriaceae | Haloferax | sp. FC28_14 | |
| Halobateria | Halobacteriales | Halobacteriaceae | Haloferax | sp. FC28_15 | |
| Halobateria | Halobacteriales | Halobacteriaceae | Haloferax | sp. FC28_16 | |
| Halobateria | Halobacteriales | Halobacteriaceae | Haloferax | sp. FC28_17 | |
| Halobateria | Halobacteriales | Halobacteriaceae | Haloferax | sp. FC28_18 | |
| Halobateria | Halobacteriales | Halobacteriaceae | Haloferax | sp. FC28_ 19 | |
| Halobateria | Halobacteriales | Halobacteriaceae | Haloferax | sp. FC28 2 | |
| Halobateria | Halobacteriales | Halobacteriaceae | Haloferax | sp. FC28_20 | |
| Halobateria | Halobacteriales | Halobacteriaceae | Haloferax | sp. FC28_21 | |
| Halobateria | Halobacteriales | Halobacteriaceae | Haloferax | sp. FC28_3 | |
| Halobateria | Halobacteriales | Halobacteriaceae | Haloferax | sp. FC28_ 4 | |
| Halobateria | Halobacteriales | Halobacteriaceae | Haloferax | sp. FC28_ 5 | |
| Halobateria | Halobacteriales | Halobacteriaceae | Haloferax | sp. FC28_6 | |
| Halobateria | Halobacteriales | Halobacteriaceae | Haloferax | sp. FC28_ 7 | |
| Halobateria | Halobacteriales | Halobacteriaceae | Haloferax | sp. FC28_8 | |
| Halobateria | Halobacteriales | Halobacteriaceae | Haloferax | sp. FC28_ 9 | |
| Halobateria | Halobacteriales | Halobacteriaceae | Haloferax | sp. FIB210_ 1 | |
| Halobateria | Halobacteriales | Halobacteriaceae | Haloferax | sp. FIB210_ 2 | |
| Halobateria | Halobacteriales | Halobacteriaceae | Haloferax | sp. FIB210_ 3 | |
| Halobateria | Halobacteriales | Halobacteriaceae | Haloferax | sp. FIB210_4 | |
| Halobateria | Halobacteriales | Halobacteriaceae | Haloferax | sp. FIB210_ 5 | |
| Halobateria | Halobacteriales | Halobacteriaceae | Haloferax | sp. FIB210_ 6 | |
| Halobateria | Halobacteriales | Halobacteriaceae | Haloferax | sp. FIB210_ 7 | |
| Halobateria | Halobacteriales | Halobacteriaceae | Haloferax | sp. FIB210_ 8 | |
| Halobateria | Halobacteriales | Halobacteriaceae | Haloferax | sp. FIB210_9 | |
| Halobateria | Halobacteriales | Halobacteriaceae | Haloferax | sp. GSP103 | |
| Halobateria | Halobacteriales | Halobacteriaceae | Haloferax | sp. GSP104 | |
| Halobateria | Halobacteriales | Halobacteriaceae | Haloferax | sp. GSP105 | |
| Halobateria | Halobacteriales | Halobacteriaceae | Haloferax | sp. GSP106 | |
| Halobateria | Halobacteriales | Halobacteriaceae | Haloferax | sp. GSP107 | |
| Halobateria | Halobacteriales | Halobacteriaceae | Haloferax | sp. H4 | |
| Halobateria | Halobacteriales | Halobacteriaceae | Haloferax | sp. HA1 | |
| Halobateria | Halobacteriales | Halobacteriaceae | Haloferax | sp. HA2 | |
| Halobateria | Halobacteriales | Halobacteriaceae | Haloferax | sp. HSC4 | |
| Halobateria | Halobacteriales | Halobacteriaceae | Haloferax | sp. LSC3 | |
| Halobateria | Halobacteriales | Halobacteriaceae | Haloferax | sp. LWp2.1 | |
| Halobateria | Halobacteriales | Halobacteriaceae | Haloferax | sp. M14 | |
| Halobateria | Halobacteriales | Halobacteriaceae | Haloferax | sp. M16 | |
| Halobateria | Halobacteriales | Halobacteriaceae | Haloferax | sp. M5 | |
| Halobateria | Halobacteriales | Halobacteriaceae | Haloferax | sp. M6 | |
| Halobateria | Halobacteriales | Halobacteriaceae | Haloferax | sp. M7 | |
| Halobateria | Halobacteriales | Halobacteriaceae | Haloferax | sp. M8 | |
| Halobateria | Halobacteriales | Halobacteriaceae | Haloferax | sp. MO20 | |
| Halobateria | Halobacteriales | Halobacteriaceae | Haloferax | sp. MO25 | |
| Halobateria | Halobacteriales | Halobacteriaceae | Haloferax | sp. MO52 | |
| Halobateria | Halobacteriales | Halobacteriaceae | Haloferax | sp. MO55 | |
| Halobateria | Halobacteriales | Halobacteriaceae | Haloferax | sp. N5 | |
| Halobateria | Halobacteriales | Halobacteriaceae | Haloferax | sp. PA13 | |
| Halobateria | Halobacteriales | Halobacteriaceae | Haloferax | sp. PA14 | |
| Halobateria | Halobacteriales | Halobacteriaceae | Haloferax | sp. PA15 | |
| Halobateria | Halobacteriales | Halobacteriaceae | Haloferax | sp. PA16 | |
| Halobateria | Halobacteriales | Halobacteriaceae | Haloferax | sp. PA17 | |
| Halobateria | Halobacteriales | Halobacteriaceae | Haloferax | sp. SA1 | |
| Halobateria | Halobacteriales | Halobacteriaceae | Haloferax | sp. SA2 | |
| Halobateria | Halobacteriales | Halobacteriaceae | Haloferax | sp. SA3 | |
| Halobateria | Halobacteriales | Halobacteriaceae | Haloferax | sp. SA4 | |
| Halobateria | Halobacteriales | Halobacteriaceae | Haloferax | sp. SA6 | |
| Halobateria | Halobacteriales | Halobacteriaceae | Haloferax | sp. SA7 | |
| Halobateria | Halobacteriales | Halobacteriaceae | Haloferax | sp. Set21 | |
| Halobateria | Halobacteriales | Halobacteriaceae | Haloferax | sp. SOP | |
| Halobateria | Halobacteriales | Halobacteriaceae | Haloferax | sp. SP1(2a) | |
| Halobateria | Halobacteriales | Halobacteriaceae | Haloferax | sp. SP2(3) | |
| Halobateria | Halobacteriales | Halobacteriaceae | Haloferax | sp. SP3(1) | |
| Halobateria | Halobacteriales | Halobacteriaceae | Haloferax | sp. VKMM004 | |
| Halobateria | Halobacteriales | Halobacteriaceae | Haloferax | sp. VKMM006 | |
| Halobateria | Halobacteriales | Halobacteriaceae | Haloferax | sp. VKMM009 | |
| Halobateria | Halobacteriales | Halobacteriaceae | Haloferax | sp. VKMM01 | |
| Halobateria | Halobacteriales | Halobacteriaceae | Haloferax | sp. VKMM010 | |
| Halobateria | Halobacteriales | Halobacteriaceae | Haloferax | sp. VKMM011 | |
| Halobateria | Halobacteriales | Halobacteriaceae | Haloferax | sp. VKMM015 | |
| Halobateria | Halobacteriales | Halobacteriaceae | Haloferax | sp. YT216 | |
| Halobateria | Halobacteriales | Halobacteriaceae | Haloferax | sp. YT226 | |
| Halobateria | Halobacteriales | Halobacteriaceae | Haloferax | sp. YT228 | |
| Halobateria | Halobacteriales | Halobacteriaceae | Haloferax | sp. YT236 | |
| Halobateria | Halobacteriales | Halobacteriaceae | Haloferax | sp. YT247 | |
| Halobateria | Halobacteriales | Halobacteriaceae | Haloferax | sp. ZJ203 | |
| Halobateria | Halobacteriales | Halobacteriaceae | Haloferax | Unclassified | 260473 |
| Halobateria | Halobacteriales | Halobacteriaceae | Halogeometricum | borinquense | 469382 |
| Halobateria | Halobacteriales | Halobacteriaceae | Halogeometricum | sp. 50C25 | 493144 |
| Halobateria | Halobacteriales | Halobacteriaceae | Halogeometricum | sp. 5Sa3 | 329275 |
| Halobateria | Halobacteriales | Halobacteriaceae | Halogeometricum | sp. KL | 627706 |
| Halobateria | Halobacteriales | Halobacteriaceae | Halogeometricum | sp. P29B070208-1P | 593539 |
| Halobateria | Halobacteriales | Halobacteriaceae | Halogeometricum | sp. SS1 | 484017 |
| Halobateria | Halobacteriales | Halobacteriaceae | Halogeometricum | Unclassified | 436947 |
| Halobateria | Halobacteriales | Halobacteriaceae | Halogranum | rubrum | 553466 |
| Halobateria | Halobacteriales | Halobacteriaceae | Halomicrobium | katesii | 437163 |
| Halobateria | Halobacteriales | Halobacteriaceae | Halomicrobium | mukohataei | 485914 |
| Halobateria | Halobacteriales | Halobacteriaceae | Halomicrobium | sp. A191 | 362891 |
| Halobateria | Halobacteriales | Halobacteriaceae | Halomicrobium | sp. Bet58 | 643748 |
| Halobateria | Halobacteriales | Halobacteriaceae | Halomicrobium | sp. KM | 627707 |
| Halobateria | Halobacteriales | Halobacteriaceae | Halopiger | xanaduensis | 797210 |
| Halobateria | Halobacteriales | Halobacteriaceae | Haloplanus | natans | 376171 |
| Halobateria | Halobacteriales | Halobacteriaceae | Haloplanus | sp. RO5-8 | 555874 |
| Halobateria | Halobacteriales | Halobacteriaceae | Haloplanus | Unclassified | 682717 |
| Halobateria | Halobacteriales | Halobacteriaceae | Haloquadratum | walsbyi - C23 | 768065 |
| Halobateria | Halobacteriales | Halobacteriaceae | Haloquadratum | walsbyi - DSM 16790 | 362976 |
| Halobateria | Halobacteriales | Halobacteriaceae | Haloquadratum | Unclassified | 329270 |
| Halobateria | Halobacteriales | Halobacteriaceae | Halorhabdus | tiamatea | 430914 |
| Halobateria | Halobacteriales | Halobacteriaceae | Halorhabdus | utahensis | 519442 |
| Halobateria | Halobacteriales | Halobacteriaceae | Halorhabdus | Unclassified | 643678 |
| Halobateria | Halobacteriales | Halobacteriaceae | Halorubrum | africanae | |
| Halobateria | Halobacteriales | Halobacteriaceae | Halorubrum | aidingense | |
| Halobateria | Halobacteriales | Halobacteriaceae | Halorubrum | alkaliphilum | |
| Halobateria | Halobacteriales | Halobacteriaceae | Halorubrum | arcis | |
| Halobateria | Halobacteriales | Halobacteriaceae | Halorubrum | californiense | |
| Halobateria | Halobacteriales | Halobacteriaceae | Halorubrum | cibarium | |
| Halobateria | Halobacteriales | Halobacteriaceae | Halorubrum | cibi | |
| Halobateria | Halobacteriales | Halobacteriaceae | Halorubrum | constantinense | |
| Halobateria | Halobacteriales | Halobacteriaceae | Halorubrum | coriense | |
| Halobateria | Halobacteriales | Halobacteriaceae | Halorubrum | distributum | |
| Halobateria | Halobacteriales | Halobacteriaceae | Halorubrum | ejinorense | |
| Halobateria | Halobacteriales | Halobacteriaceae | Halorubrum | ezzemoulense | |
| Halobateria | Halobacteriales | Halobacteriaceae | Halorubrum | halophilum | |
| Halobateria | Halobacteriales | Halobacteriaceae | Halorubrum | jeotgali | |
| Halobateria | Halobacteriales | Halobacteriaceae | Halorubrum | lacusprofundi | |
| Halobateria | Halobacteriales | Halobacteriaceae | Halorubrum | lipolyticum | |
| Halobateria | Halobacteriales | Halobacteriaceae | Halorubrum | litoreum | |
| Halobateria | Halobacteriales | Halobacteriaceae | Halorubrum | luteum | |
| Halobateria | Halobacteriales | Halobacteriaceae | Halorubrum | orientale | |
| Halobateria | Halobacteriales | Halobacteriaceae | Halorubrum | saccharovorum | |
| Halobateria | Halobacteriales | Halobacteriaceae | Halorubrum | sodomense | |
| Halobateria | Halobacteriales | Halobacteriaceae | Halorubrum | tebenquichense | |
| Halobateria | Halobacteriales | Halobacteriaceae | Halorubrum | terrestre | |
| Halobateria | Halobacteriales | Halobacteriaceae | Halorubrum | tibetense | |
| Halobateria | Halobacteriales | Halobacteriaceae | Halorubrum | trapanicum | |
| Halobateria | Halobacteriales | Halobacteriaceae | Halorubrum | vacuolatum | |
| Halobateria | Halobacteriales | Halobacteriaceae | Halorubrum | xinjiangense | |
| Halobateria | Halobacteriales | Halobacteriaceae | Halorubrum | sp. 106 | |
| Halobateria | Halobacteriales | Halobacteriaceae | Halorubrum | sp. 11-10^6 | |
| Halobateria | Halobacteriales | Halobacteriaceae | Halorubrum | sp. 11GM-10^3 | |
| Halobateria | Halobacteriales | Halobacteriaceae | Halorubrum | sp. 12-10^3 | |
| Halobateria | Halobacteriales | Halobacteriaceae | Halorubrum | sp. 2TL9 | |
| Halobateria | Halobacteriales | Halobacteriaceae | Halorubrum | sp. 5TL6 | |
| Halobateria | Halobacteriales | Halobacteriaceae | Halorubrum | sp. 9B-U | |
| Halobateria | Halobacteriales | Halobacteriaceae | Halorubrum | sp. A29 | |
| Halobateria | Halobacteriales | Halobacteriaceae | Halorubrum | sp. A33 | |
| Halobateria | Halobacteriales | Halobacteriaceae | Halorubrum | sp. A407 | |
| Halobateria | Halobacteriales | Halobacteriaceae | Halorubrum | sp. A87B | |
| Halobateria | Halobacteriales | Halobacteriaceae | Halorubrum | sp. AC 1 | |
| Halobateria | Halobacteriales | Halobacteriaceae | Halorubrum | sp. Arch265ppt-f | |
| Halobateria | Halobacteriales | Halobacteriaceae | Halorubrum | sp. Arch270ppt-f | |
| Halobateria | Halobacteriales | Halobacteriaceae | Halorubrum | sp. Arch325ppt-b | |
| Halobateria | Halobacteriales | Halobacteriaceae | Halorubrum | sp. AS2-1 | |
| Halobateria | Halobacteriales | Halobacteriaceae | Halorubrum | sp. AS2-11 | |
| Halobateria | Halobacteriales | Halobacteriaceae | Halorubrum | sp. AS2-14 | |
| Halobateria | Halobacteriales | Halobacteriaceae | Halorubrum | sp. AS2-17 | |
| Halobateria | Halobacteriales | Halobacteriaceae | Halorubrum | sp. AS2-2 | |
| Halobateria | Halobacteriales | Halobacteriaceae | Halorubrum | sp. AS2-3 | |
| Halobateria | Halobacteriales | Halobacteriaceae | Halorubrum | sp. AS2-5 | |
| Halobateria | Halobacteriales | Halobacteriaceae | Halorubrum | sp. AS2-6 | |
| Halobateria | Halobacteriales | Halobacteriaceae | Halorubrum | sp. AS2-7 | |
| Halobateria | Halobacteriales | Halobacteriaceae | Halorubrum | sp. B10-MWDX | |
| Halobateria | Halobacteriales | Halobacteriaceae | Halorubrum | sp. B12-RDX | |
| Halobateria | Halobacteriales | Halobacteriaceae | Halorubrum | sp. B13-MW | |
| Halobateria | Halobacteriales | Halobacteriaceae | Halorubrum | sp. B20-RDX | |
| Halobateria | Halobacteriales | Halobacteriaceae | Halorubrum | sp. B23 | |
| Halobateria | Halobacteriales | Halobacteriaceae | Halorubrum | sp. B36 | |
| Halobateria | Halobacteriales | Halobacteriaceae | Halorubrum | sp. B43 | |
| Halobateria | Halobacteriales | Halobacteriaceae | Halorubrum | sp. B6 | |
| Halobateria | Halobacteriales | Halobacteriaceae | Halorubrum | sp. B6-RDX | |
| Halobateria | Halobacteriales | Halobacteriaceae | Halorubrum | sp. Beja5 | |
| Halobateria | Halobacteriales | Halobacteriaceae | Halorubrum | sp. Bet217 | |
| Halobateria | Halobacteriales | Halobacteriaceae | Halorubrum | sp. Bet25 | |
| Halobateria | Halobacteriales | Halobacteriaceae | Halorubrum | sp. Bet512 | |
| Halobateria | Halobacteriales | Halobacteriaceae | Halorubrum | sp. BG-1 | |
| Halobateria | Halobacteriales | Halobacteriaceae | Halorubrum | sp. Bitterns-10^3 | |
| Halobateria | Halobacteriales | Halobacteriaceae | Halorubrum | sp. C35 | |
| Halobateria | Halobacteriales | Halobacteriaceae | Halorubrum | sp. CGSA-14 | |
| Halobateria | Halobacteriales | Halobacteriaceae | Halorubrum | sp. CGSA-42 | |
| Halobateria | Halobacteriales | Halobacteriaceae | Halorubrum | sp. CH2 | |
| Halobateria | Halobacteriales | Halobacteriaceae | Halorubrum | sp. CH3 | |
| Halobateria | Halobacteriales | Halobacteriaceae | Halorubrum | sp. CHA-MPN-10^6 | |
| Halobateria | Halobacteriales | Halobacteriaceae | Halorubrum | sp. CHC | |
| Halobateria | Halobacteriales | Halobacteriaceae | Halorubrum | sp. CHC-U | |
| Halobateria | Halobacteriales | Halobacteriaceae | Halorubrum | sp. Crystal-Bi-White-U | |
| Halobateria | Halobacteriales | Halobacteriaceae | Halorubrum | sp. Crystal-Bii-Red-U | |
| Halobateria | Halobacteriales | Halobacteriaceae | Halorubrum | sp. CS1-2 | |
| Halobateria | Halobacteriales | Halobacteriaceae | Halorubrum | sp. CS4-4 | |
| Halobateria | Halobacteriales | Halobacteriaceae | Halorubrum | sp. CT4-02 | |
| Halobateria | Halobacteriales | Halobacteriaceae | Halorubrum | sp. CY | |
| Halobateria | Halobacteriales | Halobacteriaceae | Halorubrum | sp. D1A | |
| Halobateria | Halobacteriales | Halobacteriaceae | Halorubrum | sp. D24 | |
| Halobateria | Halobacteriales | Halobacteriaceae | Halorubrum | sp. DS10 | |
| Halobateria | Halobacteriales | Halobacteriaceae | Halorubrum | sp. DV427 | |
| Halobateria | Halobacteriales | Halobacteriaceae | Halorubrum | sp. DW6 | |
| Halobateria | Halobacteriales | Halobacteriaceae | Halorubrum | sp. E4 | |
| Halobateria | Halobacteriales | Halobacteriaceae | Halorubrum | sp. EN-2 | |
| Halobateria | Halobacteriales | Halobacteriaceae | Halorubrum | sp. ETD6 | |
| Halobateria | Halobacteriales | Halobacteriaceae | Halorubrum | sp. Ez228 | |
| Halobateria | Halobacteriales | Halobacteriaceae | Halorubrum | sp. Ez24 | |
| Halobateria | Halobacteriales | Halobacteriaceae | Halorubrum | sp. Ez26 | |
| Halobateria | Halobacteriales | Halobacteriaceae | Halorubrum | sp. Ez5-1 | |
| Halobateria | Halobacteriales | Halobacteriaceae | Halorubrum | sp. Ez5-2 | |
| Halobateria | Halobacteriales | Halobacteriaceae | Halorubrum | sp. Ez522 | |
| Halobateria | Halobacteriales | Halobacteriaceae | Halorubrum | sp. Ez526 | |
| Halobateria | Halobacteriales | Halobacteriaceae | Halorubrum | sp. Ez59 | |
| Halobateria | Halobacteriales | Halobacteriaceae | Halorubrum | sp. Ez5RB | |
| Halobateria | Halobacteriales | Halobacteriaceae | Halorubrum | sp. EzA1 | |
| Halobateria | Halobacteriales | Halobacteriaceae | Halorubrum | sp. Eza4 | |
| Halobateria | Halobacteriales | Halobacteriaceae | Halorubrum | sp. EzB1 | |
| Halobateria | Halobacteriales | Halobacteriaceae | Halorubrum | sp. EzS2 | |
| Halobateria | Halobacteriales | Halobacteriaceae | Halorubrum | sp. EzS6 | |
| Halobateria | Halobacteriales | Halobacteriaceae | Halorubrum | sp. F100 | |
| Halobateria | Halobacteriales | Halobacteriaceae | Halorubrum | sp. F23A | |
| Halobateria | Halobacteriales | Halobacteriaceae | Halorubrum | sp. F42A | |
| Halobateria | Halobacteriales | Halobacteriaceae | Halorubrum | sp. FIC145 | |
| Halobateria | Halobacteriales | Halobacteriaceae | Halorubrum | sp. FIC234 | |
| Halobateria | Halobacteriales | Halobacteriaceae | Halorubrum | sp. GS1 | |
| Halobateria | Halobacteriales | Halobacteriaceae | Halorubrum | sp. GSL5.48 | |
| Halobateria | Halobacteriales | Halobacteriaceae | Halorubrum | sp. GSP100 | |
| Halobateria | Halobacteriales | Halobacteriaceae | Halorubrum | sp. HALO-G* | |
| Halobateria | Halobacteriales | Halobacteriaceae | Halorubrum | sp. HBCC-2 | |
| Halobateria | Halobacteriales | Halobacteriaceae | Halorubrum | sp. HEN2-25 | |
| Halobateria | Halobacteriales | Halobacteriaceae | Halorubrum | sp. HEN2-39 | |
| Halobateria | Halobacteriales | Halobacteriaceae | Halorubrum | sp. IB11 | |
| Halobateria | Halobacteriales | Halobacteriaceae | Halorubrum | sp. IB15 | |
| Halobateria | Halobacteriales | Halobacteriaceae | Halorubrum | sp. I.B18 | |
| Halobateria | Halobacteriales | Halobacteriaceae | Halorubrum | sp. I.B19 | |
| Halobateria | Halobacteriales | Halobacteriaceae | Halorubrum | sp. I.B2 | |
| Halobateria | Halobacteriales | Halobacteriaceae | Halorubrum | sp. I.B20 | |
| Halobateria | Halobacteriales | Halobacteriaceae | Halorubrum | sp. I.B21 | |
| Halobateria | Halobacteriales | Halobacteriaceae | Halorubrum | sp. I.B23 | |
| Halobateria | Halobacteriales | Halobacteriaceae | Halorubrum | sp. I.B26 | |
| Halobateria | Halobacteriales | Halobacteriaceae | Halorubrum | sp. I.B28 | |
| Halobateria | Halobacteriales | Halobacteriaceae | Halorubrum | sp. I.B3 | |
| Halobateria | Halobacteriales | Halobacteriaceae | Halorubrum | sp. I.B30 | |
| Halobateria | Halobacteriales | Halobacteriaceae | Halorubrum | sp. I.B4 | |
| Halobateria | Halobacteriales | Halobacteriaceae | Halorubrum | sp. I.B5 | |
| Halobateria | Halobacteriales | Halobacteriaceae | Halorubrum | sp. I.B6 | |
| Halobateria | Halobacteriales | Halobacteriaceae | Halorubrum | sp. I.B8 | |
| Halobateria | Halobacteriales | Halobacteriaceae | Halorubrum | sp. I.B9 | |
| Halobateria | Halobacteriales | Halobacteriaceae | Halorubrum | sp. I.C11 | |
| Halobateria | Halobacteriales | Halobacteriaceae | Halorubrum | sp. I.C12 | |
| Halobateria | Halobacteriales | Halobacteriaceae | Halorubrum | sp. I.C28 | |
| Halobateria | Halobacteriales | Halobacteriaceae | Halorubrum | sp. I.C8 | |
| Halobateria | Halobacteriales | Halobacteriaceae | Halorubrum | sp. I.C9 | |
| Halobateria | Halobacteriales | Halobacteriaceae | Halorubrum | sp. IMCC2547A | |
| Halobateria | Halobacteriales | Halobacteriaceae | Halorubrum | sp. IMCC2607 | |
| Halobateria | Halobacteriales | Halobacteriaceae | Halorubrum | sp. IMCC8195 | |
| Halobateria | Halobacteriales | Halobacteriaceae | Halorubrum | sp. L56 | |
| Halobateria | Halobacteriales | Halobacteriaceae | Halorubrum | sp. MG215 | |
| Halobateria | Halobacteriales | Halobacteriaceae | Halorubrum | sp. MG23 | |
| Halobateria | Halobacteriales | Halobacteriaceae | Halorubrum | sp. MG25 | |
| Halobateria | Halobacteriales | Halobacteriaceae | Halorubrum | sp. MG525 | |
| Halobateria | Halobacteriales | Halobacteriaceae | Halorubrum | sp. MG526 | |
| Halobateria | Halobacteriales | Halobacteriaceae | Halorubrum | sp. PV6 | |
| Halobateria | Halobacteriales | Halobacteriaceae | Halorubrum | sp. RBC-10^4 | |
| Halobateria | Halobacteriales | Halobacteriaceae | Halorubrum | sp. RBCA-10^3 | |
| Halobateria | Halobacteriales | Halobacteriaceae | Halorubrum | sp. RBCB-10^2 | |
| Halobateria | Halobacteriales | Halobacteriaceae | Halorubrum | sp. RBCB-10^3 | |
| Halobateria | Halobacteriales | Halobacteriaceae | Halorubrum | sp. s1-1 | |
| Halobateria | Halobacteriales | Halobacteriaceae | Halorubrum | sp. SC1.2 | |
| Halobateria | Halobacteriales | Halobacteriaceae | Halorubrum | sp. SH4 | |
| Halobateria | Halobacteriales | Halobacteriaceae | Halorubrum | sp. Slt1 | |
| Halobateria | Halobacteriales | Halobacteriaceae | Halorubrum | sp. SYM | |
| Halobateria | Halobacteriales | Halobacteriaceae | Halorubrum | sp. T1/2S95 | |
| Halobateria | Halobacteriales | Halobacteriaceae | Halorubrum | sp. TP001 | |
| Halobateria | Halobacteriales | Halobacteriaceae | Halorubrum | sp. TP003 | |
| Halobateria | Halobacteriales | Halobacteriaceae | Halorubrum | sp. TP004 | |
| Halobateria | Halobacteriales | Halobacteriaceae | Halorubrum | sp. TP008 | |
| Halobateria | Halobacteriales | Halobacteriaceae | Halorubrum | sp. TP009 | |
| Halobateria | Halobacteriales | Halobacteriaceae | Halorubrum | sp. TP015 | |
| Halobateria | Halobacteriales | Halobacteriaceae | Halorubrum | sp. TP017 | |
| Halobateria | Halobacteriales | Halobacteriaceae | Halorubrum | sp. TP018 | |
| Halobateria | Halobacteriales | Halobacteriaceae | Halorubrum | sp. TP019 | |
| Halobateria | Halobacteriales | Halobacteriaceae | Halorubrum | sp. TP020 | |
| Halobateria | Halobacteriales | Halobacteriaceae | Halorubrum | sp. TP023 | |
| Halobateria | Halobacteriales | Halobacteriaceae | Halorubrum | sp. TP024 | |
| Halobateria | Halobacteriales | Halobacteriaceae | Halorubrum | sp. TP025 | |
| Halobateria | Halobacteriales | Halobacteriaceae | Halorubrum | sp. TP026 | |
| Halobateria | Halobacteriales | Halobacteriaceae | Halorubrum | sp. TP028 | |
| Halobateria | Halobacteriales | Halobacteriaceae | Halorubrum | sp. TP029 | |
| Halobateria | Halobacteriales | Halobacteriaceae | Halorubrum | sp. TP033 | |
| Halobateria | Halobacteriales | Halobacteriaceae | Halorubrum | sp. TP034 | |
| Halobateria | Halobacteriales | Halobacteriaceae | Halorubrum | sp. TP036 | |
| Halobateria | Halobacteriales | Halobacteriaceae | Halorubrum | sp. TP037 | |
| Halobateria | Halobacteriales | Halobacteriaceae | Halorubrum | sp. TP041 | |
| Halobateria | Halobacteriales | Halobacteriaceae | Halorubrum | sp. TP042 | |
| Halobateria | Halobacteriales | Halobacteriaceae | Halorubrum | sp. TP044 | |
| Halobateria | Halobacteriales | Halobacteriaceae | Halorubrum | sp. TP045 | |
| Halobateria | Halobacteriales | Halobacteriaceae | Halorubrum | sp. TP046 | |
| Halobateria | Halobacteriales | Halobacteriaceae | Halorubrum | sp. TP048 | |
| Halobateria | Halobacteriales | Halobacteriaceae | Halorubrum | sp. TP050 | |
| Halobateria | Halobacteriales | Halobacteriaceae | Halorubrum | sp. TP051 | |
| Halobateria | Halobacteriales | Halobacteriaceae | Halorubrum | sp. TP053 | |
| Halobateria | Halobacteriales | Halobacteriaceae | Halorubrum | sp. TP054 | |
| Halobateria | Halobacteriales | Halobacteriaceae | Halorubrum | sp. TP055 | |
| Halobateria | Halobacteriales | Halobacteriaceae | Halorubrum | sp. TP056 | |
| Halobateria | Halobacteriales | Halobacteriaceae | Halorubrum | sp. TP057 | |
| Halobateria | Halobacteriales | Halobacteriaceae | Halorubrum | sp. TP058 | |
| Halobateria | Halobacteriales | Halobacteriaceae | Halorubrum | sp. TP059 | |
| Halobateria | Halobacteriales | Halobacteriaceae | Halorubrum | sp. TP060 | |
| Halobateria | Halobacteriales | Halobacteriaceae | Halorubrum | sp. TP062 | |
| Halobateria | Halobacteriales | Halobacteriaceae | Halorubrum | sp. TP063 | |
| Halobateria | Halobacteriales | Halobacteriaceae | Halorubrum | sp. TP071 | |
| Halobateria | Halobacteriales | Halobacteriaceae | Halorubrum | sp. TP074 | |
| Halobateria | Halobacteriales | Halobacteriaceae | Halorubrum | sp. TP081 | |
| Halobateria | Halobacteriales | Halobacteriaceae | Halorubrum | sp. TP082 | |
| Halobateria | Halobacteriales | Halobacteriaceae | Halorubrum | sp. TP084 | |
| Halobateria | Halobacteriales | Halobacteriaceae | Halorubrum | sp. TP085 | |
| Halobateria | Halobacteriales | Halobacteriaceae | Halorubrum | sp. TP086 | |
| Halobateria | Halobacteriales | Halobacteriaceae | Halorubrum | sp. TP094 | |
| Halobateria | Halobacteriales | Halobacteriaceae | Halorubrum | sp. TP099 | |
| Halobateria | Halobacteriales | Halobacteriaceae | Halorubrum | sp. TP101 | |
| Halobateria | Halobacteriales | Halobacteriaceae | Halorubrum | sp. TP103 | |
| Halobateria | Halobacteriales | Halobacteriaceae | Halorubrum | sp. TP105 | |
| Halobateria | Halobacteriales | Halobacteriaceae | Halorubrum | sp. TP109 | |
| Halobateria | Halobacteriales | Halobacteriaceae | Halorubrum | sp. TP115 | |
| Halobateria | Halobacteriales | Halobacteriaceae | Halorubrum | sp. TP116 | |
| Halobateria | Halobacteriales | Halobacteriaceae | Halorubrum | sp. TP117 | |
| Halobateria | Halobacteriales | Halobacteriaceae | Halorubrum | sp. TP118 | |
| Halobateria | Halobacteriales | Halobacteriaceae | Halorubrum | sp. TP121 | |
| Halobateria | Halobacteriales | Halobacteriaceae | Halorubrum | sp. TP124 | |
| Halobateria | Halobacteriales | Halobacteriaceae | Halorubrum | sp. TP125 | |
| Halobateria | Halobacteriales | Halobacteriaceae | Halorubrum | sp. TP126 | |
| Halobateria | Halobacteriales | Halobacteriaceae | Halorubrum | sp. TP132 | |
| Halobateria | Halobacteriales | Halobacteriaceae | Halorubrum | sp. TP133 | |
| Halobateria | Halobacteriales | Halobacteriaceae | Halorubrum | sp. TP135 | |
| Halobateria | Halobacteriales | Halobacteriaceae | Halorubrum | sp. TP143 | |
| Halobateria | Halobacteriales | Halobacteriaceae | Halorubrum | sp. TP145 | |
| Halobateria | Halobacteriales | Halobacteriaceae | Halorubrum | sp. TP146 | |
| Halobateria | Halobacteriales | Halobacteriaceae | Halorubrum | sp. TP148 | |
| Halobateria | Halobacteriales | Halobacteriaceae | Halorubrum | sp. TP149 | |
| Halobateria | Halobacteriales | Halobacteriaceae | Halorubrum | sp. TP150 | |
| Halobateria | Halobacteriales | Halobacteriaceae | Halorubrum | sp. TP151 | |
| Halobateria | Halobacteriales | Halobacteriaceae | Halorubrum | sp. TP152 | |
| Halobateria | Halobacteriales | Halobacteriaceae | Halorubrum | sp. TP153 | |
| Halobateria | Halobacteriales | Halobacteriaceae | Halorubrum | sp. TP154 | |
| Halobateria | Halobacteriales | Halobacteriaceae | Halorubrum | sp. TP158 | |
| Halobateria | Halobacteriales | Halobacteriaceae | Halorubrum | sp. TP159 | |
| Halobateria | Halobacteriales | Halobacteriaceae | Halorubrum | sp. TP160 | |
| Halobateria | Halobacteriales | Halobacteriaceae | Halorubrum | sp. TP162 | |
| Halobateria | Halobacteriales | Halobacteriaceae | Halorubrum | sp. TP163 | |
| Halobateria | Halobacteriales | Halobacteriaceae | Halorubrum | sp. TP165 | |
| Halobateria | Halobacteriales | Halobacteriaceae | Halorubrum | sp. TP167 | |
| Halobateria | Halobacteriales | Halobacteriaceae | Halorubrum | sp. TP168 | |
| Halobateria | Halobacteriales | Halobacteriaceae | Halorubrum | sp. TP169 | |
| Halobateria | Halobacteriales | Halobacteriaceae | Halorubrum | sp. TP172 | |
| Halobateria | Halobacteriales | Halobacteriaceae | Halorubrum | sp. TP173 | |
| Halobateria | Halobacteriales | Halobacteriaceae | Halorubrum | sp. TP175 | |
| Halobateria | Halobacteriales | Halobacteriaceae | Halorubrum | sp. TP176 | |
| Halobateria | Halobacteriales | Halobacteriaceae | Halorubrum | sp. TP177 | |
| Halobateria | Halobacteriales | Halobacteriaceae | Halorubrum | sp. TP178 | |
| Halobateria | Halobacteriales | Halobacteriaceae | Halorubrum | sp. TP179 | |
| Halobateria | Halobacteriales | Halobacteriaceae | Halorubrum | sp. TP181 | |
| Halobateria | Halobacteriales | Halobacteriaceae | Halorubrum | sp. TP182 | |
| Halobateria | Halobacteriales | Halobacteriaceae | Halorubrum | sp. TP183 | |
| Halobateria | Halobacteriales | Halobacteriaceae | Halorubrum | sp. TP188 | |
| Halobateria | Halobacteriales | Halobacteriaceae | Halorubrum | sp. TP189 | |
| Halobateria | Halobacteriales | Halobacteriaceae | Halorubrum | sp. TP192 | |
| Halobateria | Halobacteriales | Halobacteriaceae | Halorubrum | sp. TP196 | |
| Halobateria | Halobacteriales | Halobacteriaceae | Halorubrum | sp. TP197 | |
| Halobateria | Halobacteriales | Halobacteriaceae | Halorubrum | sp. TP198 | |
| Halobateria | Halobacteriales | Halobacteriaceae | Halorubrum | sp. TP201 | |
| Halobateria | Halobacteriales | Halobacteriaceae | Halorubrum | sp. TP202 | |
| Halobateria | Halobacteriales | Halobacteriaceae | Halorubrum | sp. TP203 | |
| Halobateria | Halobacteriales | Halobacteriaceae | Halorubrum | sp. TP205 | |
| Halobateria | Halobacteriales | Halobacteriaceae | Halorubrum | sp. TP207 | |
| Halobateria | Halobacteriales | Halobacteriaceae | Halorubrum | sp. TP208 | |
| Halobateria | Halobacteriales | Halobacteriaceae | Halorubrum | sp. TP209 | |
| Halobateria | Halobacteriales | Halobacteriaceae | Halorubrum | sp. TP210 | |
| Halobateria | Halobacteriales | Halobacteriaceae | Halorubrum | sp. TP212 | |
| Halobateria | Halobacteriales | Halobacteriaceae | Halorubrum | sp. TP214 | |
| Halobateria | Halobacteriales | Halobacteriaceae | Halorubrum | sp. TP215 | |
| Halobateria | Halobacteriales | Halobacteriaceae | Halorubrum | sp. TP217 | |
| Halobateria | Halobacteriales | Halobacteriaceae | Halorubrum | sp. TP218 | |
| Halobateria | Halobacteriales | Halobacteriaceae | Halorubrum | sp. TP219 | |
| Halobateria | Halobacteriales | Halobacteriaceae | Halorubrum | sp. TP220 | |
| Halobateria | Halobacteriales | Halobacteriaceae | Halorubrum | sp. TP222 | |
| Halobateria | Halobacteriales | Halobacteriaceae | Halorubrum | sp. TP224 | |
| Halobateria | Halobacteriales | Halobacteriaceae | Halorubrum | sp. TP225 | |
| Halobateria | Halobacteriales | Halobacteriaceae | Halorubrum | sp. TP226 | |
| Halobateria | Halobacteriales | Halobacteriaceae | Halorubrum | sp. TP227 | |
| Halobateria | Halobacteriales | Halobacteriaceae | Halorubrum | sp. TP228 | |
| Halobateria | Halobacteriales | Halobacteriaceae | Halorubrum | sp. TP229 | |
| Halobateria | Halobacteriales | Halobacteriaceae | Halorubrum | sp. TP230 | |
| Halobateria | Halobacteriales | Halobacteriaceae | Halorubrum | sp. TP231 | |
| Halobateria | Halobacteriales | Halobacteriaceae | Halorubrum | sp. TP233 | |
| Halobateria | Halobacteriales | Halobacteriaceae | Halorubrum | sp. TP235 | |
| Halobateria | Halobacteriales | Halobacteriaceae | Halorubrum | sp. TP240 | |
| Halobateria | Halobacteriales | Halobacteriaceae | Halorubrum | sp. TP244 | |
| Halobateria | Halobacteriales | Halobacteriaceae | Halorubrum | sp. TP245 | |
| Halobateria | Halobacteriales | Halobacteriaceae | Halorubrum | sp. TP246 | |
| Halobateria | Halobacteriales | Halobacteriaceae | Halorubrum | sp. TP247 | |
| Halobateria | Halobacteriales | Halobacteriaceae | Halorubrum | sp. TP249 | |
| Halobateria | Halobacteriales | Halobacteriaceae | Halorubrum | sp. TP250 | |
| Halobateria | Halobacteriales | Halobacteriaceae | Halorubrum | sp. TP252 | |
| Halobateria | Halobacteriales | Halobacteriaceae | Halorubrum | sp. TP254 | |
| Halobateria | Halobacteriales | Halobacteriaceae | Halorubrum | sp. TP260 | |
| Halobateria | Halobacteriales | Halobacteriaceae | Halorubrum | sp. TP261 | |
| Halobateria | Halobacteriales | Halobacteriaceae | Halorubrum | sp. TP263 | |
| Halobateria | Halobacteriales | Halobacteriaceae | Halorubrum | sp. TP265 | |
| Halobateria | Halobacteriales | Halobacteriaceae | Halorubrum | sp. TP267 | |
| Halobateria | Halobacteriales | Halobacteriaceae | Halorubrum | sp. TP269 | |
| Halobateria | Halobacteriales | Halobacteriaceae | Halorubrum | sp. TP271 | |
| Halobateria | Halobacteriales | Halobacteriaceae | Halorubrum | sp. TP272 | |
| Halobateria | Halobacteriales | Halobacteriaceae | Halorubrum | sp. TP273 | |
| Halobateria | Halobacteriales | Halobacteriaceae | Halorubrum | sp. TP277 | |
| Halobateria | Halobacteriales | Halobacteriaceae | Halorubrum | sp. TP300 | |
| Halobateria | Halobacteriales | Halobacteriaceae | Halorubrum | sp. TP302 | |
| Halobateria | Halobacteriales | Halobacteriaceae | Halorubrum | sp. TP303 | |
| Halobateria | Halobacteriales | Halobacteriaceae | Halorubrum | sp. TP306 | |
| Halobateria | Halobacteriales | Halobacteriaceae | Halorubrum | sp. TP309 | |
| Halobateria | Halobacteriales | Halobacteriaceae | Halorubrum | sp. TP312 | |
| Halobateria | Halobacteriales | Halobacteriaceae | Halorubrum | sp. TP313 | |
| Halobateria | Halobacteriales | Halobacteriaceae | Halorubrum | sp. TP315 | |
| Halobateria | Halobacteriales | Halobacteriaceae | Halorubrum | sp. TP317 | |
| Halobateria | Halobacteriales | Halobacteriaceae | Halorubrum | sp. TP318 | |
| Halobateria | Halobacteriales | Halobacteriaceae | Halorubrum | sp. TP319 | |
| Halobateria | Halobacteriales | Halobacteriaceae | Halorubrum | sp. TP320 | |
| Halobateria | Halobacteriales | Halobacteriaceae | Halorubrum | sp. TP329 | |
| Halobateria | Halobacteriales | Halobacteriaceae | Halorubrum | sp. TP341 | |
| Halobateria | Halobacteriales | Halobacteriaceae | Halorubrum | sp. VKMM017 | |
| Halobateria | Halobacteriales | Halobacteriaceae | Halorubrum | sp. VKMM019 | |
| Halobateria | Halobacteriales | Halobacteriaceae | Halorubrum | sp. VKMM033 | |
| Halobateria | Halobacteriales | Halobacteriaceae | Halorubrum | sp. YT245 | |
| Halobateria | Halobacteriales | Halobacteriaceae | Halorubrum | sp. YW059 | |
| Halobateria | Halobacteriales | Halobacteriaceae | Halorubrum | sp. YYJ | |
| Halobateria | Halobacteriales | Halobacteriaceae | Halorubrum | sp. YYJ21 | |
| Halobateria | Halobacteriales | Halobacteriaceae | Halorubrum | archaeon DEEP-1 | 97928 |
| Halobateria | Halobacteriales | Halobacteriaceae | Halorubrum | archaeon ORGANIC1_A | 98847 |
| Halobateria | Halobacteriales | Halobacteriaceae | Halorubrum | Unclassified | 399555 |
| Halobateria | Halobacteriales | Halobacteriaceae | Halosarcina | pallida | 411361 |
| Halobateria | Halobacteriales | Halobacteriaceae | Halosarcina | sp. RO1-4 | 553469 |
| Halobateria | Halobacteriales | Halobacteriaceae | Halosarcina | sp. RO1-64 | 671107 |
| Halobateria | Halobacteriales | Halobacteriaceae | Halosimplex | carlsbadense | 797114 |
| Halobateria | Halobacteriales | Halobacteriaceae | Halosimplex | Unclassified | 260471 |
| Halobateria | Halobacteriales | Halobacteriaceae | Halostagnicola | larsenii | 353800 |
| Halobateria | Halobacteriales | Halobacteriaceae | Haloterrigena | daqingensis | 588898 |
| Halobateria | Halobacteriales | Halobacteriaceae | Haloterrigena | hispanica | 392421 |
| Halobateria | Halobacteriales | Halobacteriaceae | Haloterrigena | jeotgali | 413811 |
| Halobateria | Halobacteriales | Halobacteriaceae | Haloterrigena | limicola | 370323 |
| Halobateria | Halobacteriales | Halobacteriaceae | Haloterrigena | longa | 370324 |
| Halobateria | Halobacteriales | Halobacteriaceae | Haloterrigena | saccharevitans | 301967 |
| Halobateria | Halobacteriales | Halobacteriaceae | Haloterrigena | salina | 504937 |
| Halobateria | Halobacteriales | Halobacteriaceae | Haloterrigena | thermotolerans | 121872 |
| Halobateria | Halobacteriales | Halobacteriaceae | Haloterrigena | turkmenica | 543526 |
| Halobateria | Halobacteriales | Halobacteriaceae | Haloterrigena | turpansis | 239108 |
| Halobateria | Halobacteriales | Halobacteriaceae | Haloterrigena | sp. A206A | |
| Halobateria | Halobacteriales | Halobacteriaceae | Haloterrigena | sp. A82 | |
| Halobateria | Halobacteriales | Halobacteriaceae | Haloterrigena | sp. AB30 | |
| Halobateria | Halobacteriales | Halobacteriaceae | Haloterrigena | sp. arg-4 | |
| Halobateria | Halobacteriales | Halobacteriaceae | Haloterrigena | sp. D113 | |
| Halobateria | Halobacteriales | Halobacteriaceae | Haloterrigena | sp. D83A | |
| Halobateria | Halobacteriales | Halobacteriaceae | Haloterrigena | sp. DV582A-1 | |
| Halobateria | Halobacteriales | Halobacteriaceae | Haloterrigena | sp. DV582B-3 | |
| Halobateria | Halobacteriales | Halobacteriaceae | Haloterrigena | sp. DV582c2 | |
| Halobateria | Halobacteriales | Halobacteriaceae | Haloterrigena | sp. DV582c4 | |
| Halobateria | Halobacteriales | Halobacteriaceae | Haloterrigena | sp. E49 | |
| Halobateria | Halobacteriales | Halobacteriaceae | Haloterrigena | sp. E57B | |
| Halobateria | Halobacteriales | Halobacteriaceae | Haloterrigena | sp. EzB3 | |
| Halobateria | Halobacteriales | Halobacteriaceae | Haloterrigena | sp. EzSm | |
| Halobateria | Halobacteriales | Halobacteriaceae | Haloterrigena | sp. FIC147 | |
| Halobateria | Halobacteriales | Halobacteriaceae | Haloterrigena | sp. FIC148 1 | |
| Halobateria | Halobacteriales | Halobacteriaceae | Haloterrigena | sp. FIC148 2 | |
| Halobateria | Halobacteriales | Halobacteriaceae | Haloterrigena | sp. GSL-11 | |
| Halobateria | Halobacteriales | Halobacteriaceae | Haloterrigena | sp. L52 | |
| Halobateria | Halobacteriales | Halobacteriaceae | Haloterrigena | sp. LPNTC | |
| Halobateria | Halobacteriales | Halobacteriaceae | Haloterrigena | sp. MO19 | |
| Halobateria | Halobacteriales | Halobacteriaceae | Haloterrigena | sp. MO23 | |
| Halobateria | Halobacteriales | Halobacteriaceae | Haloterrigena | sp. MO24 | |
| Halobateria | Halobacteriales | Halobacteriaceae | Haloterrigena | sp. XJNU-19 | |
| Halobateria | Halobacteriales | Halobacteriaceae | Haloterrigena | sp. XJNU-45 | |
| Halobateria | Halobacteriales | Halobacteriaceae | Haloterrigena | sp. XJNU-45-4 | |
| Halobateria | Halobacteriales | Halobacteriaceae | Haloterrigena | sp. XJNU-86-2 | |
| Halobateria | Halobacteriales | Halobacteriaceae | Haloterrigena | sp. XJNU-97 | |
| Halobateria | Halobacteriales | Halobacteriaceae | Halovivax | asiaticus | 332953 |
| Halobateria | Halobacteriales | Halobacteriaceae | Halovivax | ruber | 387341 |
| Halobateria | Halobacteriales | Halobacteriaceae | Halovivax | sp. A21 | 520557 |
| Halobateria | Halobacteriales | Halobacteriaceae | Halovivax | sp. B45 | 596429 |
| Halobateria | Halobacteriales | Halobacteriaceae | Halovivax | sp. E107 | 596430 |
| Halobateria | Halobacteriales | Halobacteriaceae | Halovivax | sp. EN-4 | 388350 |
| Halobateria | Halobacteriales | Halobacteriaceae | Natrialba | aegyptia | 129789 |
| Halobateria | Halobacteriales | Halobacteriaceae | Natrialba | aibiensis | 248371 |
| Halobateria | Halobacteriales | Halobacteriaceae | Natrialba | asiatica | 64602 |
| Halobateria | Halobacteriales | Halobacteriaceae | Natrialba | chahannaoensis | 68911 |
| Halobateria | Halobacteriales | Halobacteriaceae | Natrialba | hulunbeirensis | 123783 |
| Halobateria | Halobacteriales | Halobacteriaceae | Natrialba | magadii | 547559 |
| Halobateria | Halobacteriales | Halobacteriaceae | Natrialba | taiwanensis | 160846 |
| Halobateria | Halobacteriales | Halobacteriaceae | Natrialba | wudunaoensis | 70318 |
| Halobateria | Halobacteriales | Halobacteriaceae | Natrialba | sp. A137 | 362883 |
| Halobateria | Halobacteriales | Halobacteriaceae | Natrialba | sp. ATCC 43988 | 63741 |
| Halobateria | Halobacteriales | Halobacteriaceae | Natrialba | sp. B49 | 370966 |
| Halobateria | Halobacteriales | Halobacteriaceae | Natrialba | sp. F4A | 362882 |
| Halobateria | Halobacteriales | Halobacteriaceae | Natrialba | sp. F5 | 359307 |
| Halobateria | Halobacteriales | Halobacteriaceae | Natrialba | sp. Tunisia HMg-25 | 138615 |
| Halobateria | Halobacteriales | Halobacteriaceae | Natrialba | sp. Tunisia HMg-27 | 138616 |
| Halobateria | Halobacteriales | Halobacteriaceae | Natrialba | Unclassified | 549377 |
| Halobateria | Halobacteriales | Halobacteriaceae | Natrinema | aidingensis | |
| Halobateria | Halobacteriales | Halobacteriaceae | Natrinema | altunense | |
| Halobateria | Halobacteriales | Halobacteriaceae | Natrinema | ejinorense | |
| Halobateria | Halobacteriales | Halobacteriaceae | Natrinema | gari | |
| Halobateria | Halobacteriales | Halobacteriaceae | Natrinema | pallidum | |
| Halobateria | Halobacteriales | Halobacteriaceae | Natrinema | pellirubrum | |
| Halobateria | Halobacteriales | Halobacteriaceae | Natrinema | versiforme | |
| Halobateria | Halobacteriales | Halobacteriaceae | Natrinema | xinjiang | |
| Halobateria | Halobacteriales | Halobacteriaceae | Natrinema | sp. 2TK1 | |
| Halobateria | Halobacteriales | Halobacteriaceae | Natrinema | sp. 5TK1 | |
| Halobateria | Halobacteriales | Halobacteriaceae | Natrinema | sp. A85 | |
| Halobateria | Halobacteriales | Halobacteriaceae | Natrinema | sp. ABDH11 | |
| Halobateria | Halobacteriales | Halobacteriaceae | Natrinema | sp. ABDH17 | |
| Halobateria | Halobacteriales | Halobacteriaceae | Natrinema | sp. B19 | |
| Halobateria | Halobacteriales | Halobacteriaceae | Natrinema | sp. B5-RDX | |
| Halobateria | Halobacteriales | Halobacteriaceae | Natrinema | sp. B77A | |
| Halobateria | Halobacteriales | Halobacteriaceae | Natrinema | sp. CX2021 | |
| Halobateria | Halobacteriales | Halobacteriaceae | Natrinema | sp. CY21 | |
| Halobateria | Halobacteriales | Halobacteriaceae | Natrinema | sp. D74 | |
| Halobateria | Halobacteriales | Halobacteriaceae | Natrinema | sp. E92B | |
| Halobateria | Halobacteriales | Halobacteriaceae | Natrinema | sp. FP1R | |
| Halobateria | Halobacteriales | Halobacteriaceae | Natrinema | sp. GSP102 | |
| Halobateria | Halobacteriales | Halobacteriaceae | Natrinema | sp. GSP109 | |
| Halobateria | Halobacteriales | Halobacteriaceae | Natrinema | sp. HA33DX | |
| Halobateria | Halobacteriales | Halobacteriaceae | Natrinema | sp. HDS1-1 | |
| Halobateria | Halobacteriales | Halobacteriaceae | Natrinema | sp. HM06 | |
| Halobateria | Halobacteriales | Halobacteriaceae | Natrinema | sp. J7 | |
| Halobateria | Halobacteriales | Halobacteriaceae | Natrinema | sp. LPN89 | |
| Halobateria | Halobacteriales | Halobacteriaceae | Natrinema | sp. XA3-1 | |
| Halobateria | Halobacteriales | Halobacteriaceae | Natrinema | sp. XJNU-10 | |
| Halobateria | Halobacteriales | Halobacteriaceae | Natrinema | sp. XJBU-49 | |
| Halobateria | Halobacteriales | Halobacteriaceae | Natrinema | sp. XJNU-57 | |
| Halobateria | Halobacteriales | Halobacteriaceae | Natrinema | sp. enrichment culture clone ABDH17 | 630201 |
| Halobateria | Halobacteriales | Halobacteriaceae | Natrinema | sp. enrichment culture clone ABDH2 | 630202 |
| Halobateria | Halobacteriales | Halobacteriaceae | Natrinema | sp. enrichment culture clone ABDH34 | 630203 |
| Halobateria | Halobacteriales | Halobacteriaceae | Natrinema | sp. enrichment culture clone ABDH37 | 630204 |
| Halobateria | Halobacteriales | Halobacteriaceae | Natrinema | Unclassified | 261026 |
| Halobateria | Halobacteriales | Halobacteriaceae | Natronobacterium | gregoryi | |
| Halobateria | Halobacteriales | Halobacteriaceae | Natrionobacterium | innermongoliae | |
| Halobateria | Halobacteriales | Halobacteriaceae | Natronobacterium | sp. 2-24-1 | |
| Halobateria | Halobacteriales | Halobacteriaceae | Natronobacterium | sp. 2-24-8 | |
| Halobateria | Halobacteriales | Halobacteriaceae | Natrionobacterium | sp. AS-7091 | |
| Halobateria | Halobacteriales | Halobacteriaceae | Natronobacterium | sp. B-MWDX | |
| Halobateria | Halobacteriales | Halobacteriaceae | Natronobacterium | sp. SSL-6 | |
| Halobateria | Halobacteriales | Halobacteriaceae | Natrionobacterium | sp. SSL6 | |
| Halobateria | Halobacteriales | Halobacteriaceae | Natronobacterium | sp. XJNU-101 | |
| Halobateria | Halobacteriales | Halobacteriaceae | Natronobacterium | sp. XJNU-12 | |
| Halobateria | Halobacteriales | Halobacteriaceae | Natrionobacterium | sp. XJNU-13 | |
| Halobateria | Halobacteriales | Halobacteriaceae | Natronobacterium | sp. XJNU-22 | |
| Halobateria | Halobacteriales | Halobacteriaceae | Natronobacterium | sp. XJNU-36 | |
| Halobateria | Halobacteriales | Halobacteriaceae | Natrionobacterium | sp. XJNU-39 | |
| Halobateria | Halobacteriales | Halobacteriaceae | Natronobacterium | sp. XJNU-43 | |
| Halobateria | Halobacteriales | Halobacteriaceae | Natronobacterium | sp. XJNU-46 | |
| Halobateria | Halobacteriales | Halobacteriaceae | Natrionobacterium | sp. XJNU-62 | |
| Halobateria | Halobacteriales | Halobacteriaceae | Natronobacterium | sp. XJNU-74 | |
| Halobateria | Halobacteriales | Halobacteriaceae | Natronobacterium | sp. XJNU-75 | |
| Halobateria | Halobacteriales | Halobacteriaceae | Natrionobacterium | sp. XJNU-77 | |
| Halobateria | Halobacteriales | Halobacteriaceae | Natronobacterium | sp. XJNU-96 | |
| Halobateria | Halobacteriales | Halobacteriaceae | Natronobacterium | sp. XJNU-99 | |
| Halobateria | Halobacteriales | Halobacteriaceae | Natrionobacterium | Unclassified | 523723 |
| Halobateria | Halobacteriales | Halobacteriaceae | Natronococcus | aibiensis | |
| Halobateria | Halobacteriales | Halobacteriaceae | Natronococcus | amylolyticus | |
| Halobateria | Halobacteriales | Halobacteriaceae | Natronococcus | jeotgali | |
| Halobateria | Halobacteriales | Halobacteriaceae | Natronococcus | occultus | |
| Halobateria | Halobacteriales | Halobacteriaceae | Natronococcus | occultus SP4 | |
| Halobateria | Halobacteriales | Halobacteriaceae | Natronococcus | xinjiangense | |
| Halobateria | Halobacteriales | Halobacteriaceae | Natronococcus | yunnanense | |
| Halobateria | Halobacteriales | Halobacteriaceae | Natronococcus | zabuyensis | |
| Halobateria | Halobacteriales | Halobacteriaceae | Natronococcus | sp. | |
| Halobateria | Halobacteriales | Halobacteriaceae | Natronococcus | sp. Ah-36 | |
| Halobateria | Halobacteriales | Halobacteriaceae | Natronococcus | sp. D50 | |
| Halobateria | Halobacteriales | Halobacteriaceae | Natronococcus | sp. D58A | |
| Halobateria | Halobacteriales | Halobacteriaceae | Natronococcus | sp. E7 | |
| Halobateria | Halobacteriales | Halobacteriaceae | Natronococcus | sp. F30Al | |
| Halobateria | Halobacteriales | Halobacteriaceae | Natronococcus | sp. GS3 | |
| Halobateria | Halobacteriales | Halobacteriaceae | Natronococcus | sp. M13 | |
| Halobateria | Halobacteriales | Halobacteriaceae | Natronococcus | sp. Sua-E41 | |
| Halobateria | Halobacteriales | Halobacteriaceae | Natronococcus | sp. Sua-E43 | |
| Halobateria | Halobacteriales | Halobacteriaceae | Natronococcus | sp. TC6 | |
| Halobateria | Halobacteriales | Halobacteriaceae | Natronococcus | sp. XH4 | |
| Halobateria | Halobacteriales | Halobacteriaceae | Natronococcus | sp. XJNU-111 | |
| Halobateria | Halobacteriales | Halobacteriaceae | Natronococcus | sp. enrichment culture clone ABDH12 | 630205 |
| Halobateria | Halobacteriales | Halobacteriaceae | Natronococcus | Unclassified | 236503 |
| Halobateria | Halobacteriales | Halobacteriaceae | Natronolimnobius | baerhuensis | 253108 |
| Halobateria | Halobacteriales | Halobacteriaceae | Natronolimnobius | innermongolicus | 253107 |
| Halobateria | Halobacteriales | Halobacteriaceae | Natronolimnobius | Unclassified | 549379 |
| Halobateria | Halobacteriales | Halobacteriaceae | Natronomonas | pharaonis | 348780 |
| Halobateria | Halobacteriales | Halobacteriaceae | Natronomonas | sp. DV462A | 585976 |
| Halobateria | Halobacteriales | Halobacteriaceae | Natronomonas | Unclassified | 436949 |
| Halobateria | Halobacteriales | Halobacteriaceae | Natronorubum | aibiense | 348826 |
| Halobateria | Halobacteriales | Halobacteriaceae | Natronorubum | bangense | 61858 |
| Halobateria | Halobacteriales | Halobacteriaceae | Natronorubum | sulfidifaciens | 388259 |
| Halobateria | Halobacteriales | Halobacteriaceae | Natronorubum | thiooxidans | 308853 |
| Halobateria | Halobacteriales | Halobacteriaceae | Natronorubum | tibetense | 63128 |
| Halobateria | Halobacteriales | Halobacteriaceae | Natronorubum | sp. CG-4 | 640944 |
| Halobateria | Halobacteriales | Halobacteriaceae | Natronorubum | sp. CG-6 | 640943 |
| Halobateria | Halobacteriales | Halobacteriaceae | Natronorubum | sp. Sua-E01 | 549372 |
| Halobateria | Halobacteriales | Halobacteriaceae | Natronorubum | sp. Tenzan-10 | 134815 |
| Halobateria | Halobacteriales | Halobacteriaceae | Natronorubum | sp. Wadi Natrun-19 | 134814 |
| Halobateria | Halobacteriales | Halobacteriaceae | Natronorubum | sp. XJNU-14 | 642520 |
| Halobateria | Halobacteriales | Halobacteriaceae | Natronorubum | sp. XJNU-92 | 642521 |
| Halobateria | Halobacteriales | Halobacteriaceae | Natronorubum | Unclassified | 260478 |
| Halobateria | Halobacteriales | Halobacteriaceae | Unclassified | haloarchaeon 10AH | |
| Halobateria | Halobacteriales | Halobacteriaceae | Unclassified | haloarchaeon 14AHG | |
| Halobateria | Halobacteriales | Halobacteriaceae | Unclassified | haloarchaeon 30AH | |
| Halobateria | Halobacteriales | Halobacteriaceae | Unclassified | haloarchaeon 82M4 | |
| Halobateria | Halobacteriales | Halobacteriaceae | Unclassified | haloarchaeon 86M4 | |
| Halobateria | Halobacteriales | Halobacteriaceae | Unclassified | haloarchaeon 89M4 | |
| Halobateria | Halobacteriales | Halobacteriaceae | Unclassified | haloarchaeon 8AHG | |
| Halobateria | Halobacteriales | Halobacteriaceae | Unclassified | haloarchaeon 93dLM4 | |
| Halobateria | Halobacteriales | Halobacteriaceae | Unclassified | haloarchaeon 93ILM4 | |
| Halobateria | Halobacteriales | Halobacteriaceae | Unclassified | haloarchaeon 98NT4 | |
| Halobateria | Halobacteriales | Halobacteriaceae | Unclassified | haloarchaeon 9AH | |
| Halobateria | Halobacteriales | Halobacteriaceae | Unclassified | haloarchaeon B13-RDX | |
| Halobateria | Halobacteriales | Halobacteriaceae | Unclassified | haloarchaeon CSW1.15.5 | |
| Halobateria | Halobacteriales | Halobacteriaceae | Unclassified | haloarchaeon CSW2.24.4 | |
| Halobateria | Halobacteriales | Halobacteriaceae | Unclassified | haloarchaeon CSW2.25.5 | |
| Halobateria | Halobacteriales | Halobacteriaceae | Unclassified | haloarchaeon CSW2.27.5 | |
| Halobateria | Halobacteriales | Halobacteriaceae | Unclassified | haloarchaeon CSW4.03.5 | |
| Halobateria | Halobacteriales | Halobacteriaceae | Unclassified | haloarchaeon CSW4.05.5 | |
| Halobateria | Halobacteriales | Halobacteriaceae | Unclassified | haloarchaeon CSW4.11.5 | |
| Halobateria | Halobacteriales | Halobacteriaceae | Unclassified | haloarchaeon CSW4.22.4 | |
| Halobateria | Halobacteriales | Halobacteriaceae | Unclassified | haloarchaeon CSW5.28.5 | |
| Halobateria | Halobacteriales | Halobacteriaceae | Unclassified | haloarchaeon CSW6.14.5 | |
| Halobateria | Halobacteriales | Halobacteriaceae | Unclassified | haloarchaeon CSW8.8.11 | |
| Halobateria | Halobacteriales | Halobacteriaceae | Unclassified | haloarchaeon HA15 | |
| Halobateria | Halobacteriales | Halobacteriaceae | Unclassified | haloarchaeon HA25 | |
| Halobateria | Halobacteriales | Halobacteriaceae | Unclassified | haloarchaeon S8a | |
| Halobateria | Halobacteriales | Halobacteriaceae | Unclassified | haloarchaeon SC4 | |
| Halobateria | Halobacteriales | Halobacteriaceae | Unclassified | haloarchaeon SC7 | |
| Halobateria | Halobacteriales | Halobacteriaceae | Unclassified | haloarchaeon SC8 | |
| Halobateria | Halobacteriales | Halobacteriaceae | Unclassified | haloarchaeon SC9 | |
| Halobateria | Halobacteriales | Halobacteriaceae | Unclassified | haloarchaeon sech10 | |
| Halobateria | Halobacteriales | Halobacteriaceae | Unclassified | haloarchaeon sech14 | |
| Halobateria | Halobacteriales | Halobacteriaceae | Unclassified | haloarchaeon sech4 | |
| Halobateria | Halobacteriales | Halobacteriaceae | Unclassified | haloarchaeon sech6 | |
| Halobateria | Halobacteriales | Halobacteriaceae | Unclassified | haloarchaeon sech7a | |
| Halobateria | Halobacteriales | Halobacteriaceae | Unclassified | haloarchaeon sech8 | |
| Halobateria | Halobacteriales | Halobacteriaceae | Unclassified | haloarchaeon sech9 | |
| Halobateria | Halobacteriales | Halobacteriaceae | Unclassified | haloarchaeon W1 | |
| Halobateria | Halobacteriales | Halobacteriaceae | Unclassified | haloarchaeon YNPASCul | |
| Halobateria | Halobacteriales | Halobacteriaceae | Unclassified | archaeon 309 | |
| Halobateria | Halobacteriales | Halobacteriaceae | Unclassified | archaeon GLYP1 | |
| Halobateria | Halobacteriales | Halobacteriaceae | Unclassified | archaeon GX1 | |
| Halobateria | Halobacteriales | Halobacteriaceae | Unclassified | archaeon GX10 | |
| Halobateria | Halobacteriales | Halobacteriaceae | Unclassified | archaeon GX21 | |
| Halobateria | Halobacteriales | Halobacteriaceae | Unclassified | archaeon GX26 | |
| Halobateria | Halobacteriales | Halobacteriaceae | Unclassified | archaeon GX3 | |
| Halobateria | Halobacteriales | Halobacteriaceae | Unclassified | archaeon GX31 | |
| Halobateria | Halobacteriales | Halobacteriaceae | Unclassified | archaeon GX48 | |
| Halobateria | Halobacteriales | Halobacteriaceae | Unclassified | archaeon GX60 | |
| Halobateria | Halobacteriales | Halobacteriaceae | Unclassified | archaeon GX7 | |
| Halobateria | Halobacteriales | Halobacteriaceae | Unclassified | archaeon GX71 | |
| Halobateria | Halobacteriales | Halobacteriaceae | Unclassified | archaeon GX74 | |
| Halobateria | Halobacteriales | Halobacteriaceae | Unclassified | archaeon HO2-1 | |
| Halobateria | Halobacteriales | Halobacteriaceae | Unclassified | archaeon IMCC2586B | |
| Halobateria | Halobacteriales | Halobacteriaceae | Unclassified | archaeon IMCC8204 | |
| Halobateria | Halobacteriales | Halobacteriaceae | Unclassified | archaeon KeC-11 | |
| Halobateria | Halobacteriales | Halobacteriaceae | Unclassified | archaeon L1 | |
| Halobateria | Halobacteriales | Halobacteriaceae | Unclassified | archaeon RO1-6 | |
| Halobateria | Halobacteriales | Halobacteriaceae | Unclassified | archaeon RO3-11 | |
| Halobateria | Halobacteriales | Halobacteriaceae | Unclassified | archaeon RO5-14 | |
| Halobateria | Halobacteriales | Halobacteriaceae | Unclassified | archaeon RO5-2 | |
| Halobateria | Halobacteriales | Halobacteriaceae | Unclassified | archaeon Ston11 | |
| Halobateria | Halobacteriales | Halobacteriaceae | Unclassified | archaeon Ston12 | |
| Halobateria | Halobacteriales | Halobacteriaceae | Unclassified | archaeon Ston16 | |
| Halobateria | Halobacteriales | Halobacteriaceae | Unclassified | archaeon Ston2 | |
| Halobateria | Halobacteriales | Halobacteriaceae | Unclassified | archaeon Ston28 | |
| Halobateria | Halobacteriales | Halobacteriaceae | Unclassified | archaeon Ston3 | |
| Halobateria | Halobacteriales | Halobacteriaceae | Unclassified | archaeon Ston5 | |
| Halobateria | Halobacteriales | Halobacteriaceae | Unclassified | archaeon Ston6 | |
| Halobateria | Halobacteriales | Halobacteriaceae | Unclassified | archaeon TBN12 | |
| Halobateria | Halobacteriales | Halobacteriaceae | Unclassified | archaeon TBN19 | |
| Halobateria | Halobacteriales | Halobacteriaceae | Unclassified | archaeon TBN21 | |
| Halobateria | Halobacteriales | Halobacteriaceae | Unclassified | archaeon TBN37 | |
| Halobateria | Halobacteriales | Halobacteriaceae | Unclassified | archaeon TBN4 | |
| Halobateria | Halobacteriales | Halobacteriaceae | Unclassified | archaeon TBN49 | |
| Halobateria | Halobacteriales | Halobacteriaceae | Unclassified | archaeon TBN5 | |
| Halobateria | Halobacteriales | Halobacteriaceae | Unclassified | archaeon TBN51 | |
| Halobateria | Halobacteriales | Halobacteriaceae | Unclassified | archaeon TBN53 | |
| Halobateria | Halobacteriales | Halobacteriaceae | Unclassified | archaeon TNN10 | |
| Halobateria | Halobacteriales | Halobacteriaceae | Unclassified | archaeon TNN18 | |
| Halobateria | Halobacteriales | Halobacteriaceae | Unclassified | archaeon TNN28 | |
| Halobateria | Halobacteriales | Halobacteriaceae | Unclassified | archaeon TNN44 | |
| Halobateria | Halobacteriales | Halobacteriaceae | Unclassified | archaeon TNN50 | |
| Halobateria | Halobacteriales | Halobacteriaceae | Unclassified | archaeon TNN58 | |
| Halobateria | Halobacteriales | Halobacteriaceae | Unclassified | halophilic archaeon 194-10 | |
| Halobateria | Halobacteriales | Halobacteriaceae | Unclassified | halophilic archaeon 1DH38 | |
| Halobateria | Halobacteriales | Halobacteriaceae | Unclassified | halophilic archaeon 1SC5 | |
| Halobateria | Halobacteriales | Halobacteriaceae | Unclassified | halophilic archaeon 2DH35 | |
| Halobateria | Halobacteriales | Halobacteriaceae | Unclassified | halophilic archaeon DH34 | |
| Halobateria | Halobacteriales | Halobacteriaceae | Unclassified | halophilic archaeon HS47 | |
| Halobateria | Halobacteriales | Halobacteriaceae | Unclassified | halophilic archaeon MK13-1 | |
| Halobateria | Halobacteriales | Halobacteriaceae | Unclassified | halophilic archaeon Naxosll | |
| Halobateria | Halobacteriales | Halobacteriaceae | Unclassified | halophilic archaeon Palaell | |
| Halobateria | Halobacteriales | Halobacteriaceae | Unclassified | halophilic archaeon SC3 | |
| Halobateria | Halobacteriales | Halobacteriaceae | Unclassified | halophilic archaeon SC6 | |
| Halobateria | Halobacteriales | Halobacteriaceae | Unclassified | mixed culture haloarchaeon CDI-271 | |
| Halobateria | Halobacteriales | Halobacteriaceae | Unclassified | mixed culture haloarchaeon CDI-276 | |
| Halobateria | Halobacteriales | Halobacteriaceae | Unclassified | mixed culture haloarchaeon CDII-272 | |
| Halobateria | Halobacteriales | Halobacteriaceae | Unclassified | mixed culture haloarchaeon CDIII-273 | |
| Halobateria | Halobacteriales | Halobacteriaceae | Unclassified | mixed culture haloarchaeon CLI-248 | |
| Halobateria | Halobacteriales | Halobacteriaceae | Unclassified | mixed culture haloarchaeon CLI-250 | |
| Halobateria | Halobacteriales | Halobacteriaceae | Unclassified | mixed culture haloarchaeon CLI-257 | |
| Halobateria | Halobacteriales | Halobacteriaceae | Unclassified | mixed culture haloarchaeon CLI-265 | |
| Halobateria | Halobacteriales | Halobacteriaceae | Unclassified | mixed culture haloarchaeon YE.LI-230 | |
| Halobateria | Halobacteriales | Halobacteriaceae | Unclassified | Sambhar Salt Lake archaeon HA1 | |
| Halobateria | Halobacteriales | Halobacteriaceae | Unclassified | Sambhar Salt Lake archaeon HA6 | |
| Halobateria | Halobacteriales | Halobacteriaceae | Unclassified | Halobacterium sp. NCIMB 763 | |
| Halobateria | Halobacteriales | Halobacteriaceae | Unclassified | gen. sp. | |
| Halobateria | Halobacteriales | Halobacteriaceae | Unclassified | gen. sp. 524 | |
| Halobateria | Halobacteriales | Halobacteriaceae | Unclassified | gen. sp. 56 | |
| Halobateria | Halobacteriales | Halobacteriaceae | Unclassified | gen. sp. 600 | |
| Halobateria | Halobacteriales | Halobacteriaceae | Unclassified | gen. sp. H1 | |
| Halobateria | Halobacteriales | Halobacteriaceae | Unclassified | gen. sp. SR1.5 | |
| Halobateria | Halobacteriales | Halobacteriaceae | Unclassified | gen. sp. T5.7 | |
| Halobateria | Halobacteriales | Halobacteriaceae | Unclassified | enrichment culture clone SLAb1_archaeon | |
| Halobateria | Halobacteriales | Halobacteriaceae | Unclassified | uncultured archaeon DEEP-10 | |
| Halobateria | Halobacteriales | Halobacteriaceae | Unclassified | uncultured archaeon DEEP-2 | |
| Halobateria | Halobacteriales | Halobacteriaceae | Unclassified | uncultured archaeon DEEP-5 | |
| Halobateria | Halobacteriales | Halobacteriaceae | Unclassified | uncultured archaeon DEEP-6 | |
| Halobateria | Halobacteriales | Halobacteriaceae | Unclassified | uncultured archaeon DEEP-7 | |
| Halobateria | Halobacteriales | Halobacteriaceae | Unclassified | uncultured archaeon DEEP-8 | |
| Halobateria | Halobacteriales | Halobacteriaceae | Unclassified | uncultured archaeon DEEP-9 | |
| Halobateria | Halobacteriales | Halobacteriaceae | Unclassified | uncultured haloarchaeon | |
| Halobateria | Halobacteriales | Halobacteriaceae | Unclassified | uncultured haloarchaeon CDI-271 | |
| Halobateria | Halobacteriales | Halobacteriaceae | Unclassified | uncultured haloarchaeon CDII-272 | |
| Halobateria | Halobacteriales | Halobacteriaceae | Unclassified | uncultured haloarchaeon CDIII-273 | |
| Halobateria | Halobacteriales | Halobacteriaceae | Unclassified | uncultured haloarchaeon CLI-248 | |
| Halobateria | Halobacteriales | Halobacteriaceae | Unclassified | uncultured haloarchaeon CLI-250 | |
| Halobateria | Halobacteriales | Halobacteriaceae | Unclassified | uncultured haloarchaeon CLI-257 | |
| Halobateria | Halobacteriales | Halobacteriaceae | Unclassified | uncultured haloarchaeon Envl-181 | |
| Halobateria | Halobacteriales | Halobacteriaceae | Unclassified | uncultured haloarchaeon Envl-182 | |
| Halobateria | Halobacteriales | Halobacteriaceae | Unclassified | uncultured haloarchaeon Envl-184 | |
| Halobateria | Halobacteriales | Halobacteriaceae | Unclassified | uncultured haloarchaeon FLAS10H9 | |
| Halobateria | Halobacteriales | Halobacteriaceae | Unclassified | uncultured haloarchaeon YE.LI-230 | |
| Methanobacteria | Methanobacteriales | Methanobacteriaceae | Methanobacterium | aarhusense | |
| Methanobacteria | Methanobacteriales | Methanobacteriaceae | Methanobacterium | alcaliphilum | |
| Methanobacteria | Methanobacteriales | Methanobacteriaceae | Methanobacterium | beijingense | |
| Methanobacteria | Methanobacteriales | Methanobacteriaceae | Methanobacterium | bryantii | |
| Methanobacteria | Methanobacteriales | Methanobacteriaceae | Methanobacterium | congolense | |
| Methanobacteria | Methanobacteriales | Methanobacteriaceae | Methanobacterium | curvum | |
| Methanobacteria | Methanobacteriales | Methanobacteriaceae | Methanobacterium | espanolae | |
| Methanobacteria | Methanobacteriales | Methanobacteriaceae | Methanobacterium | formicicum | |
| Methanobacteria | Methanobacteriales | Methanobacteriaceae | Methanobacterium | ivanovii | |
| Methanobacteria | Methanobacteriales | Methanobacteriaceae | Methanobacterium | oryzae | |
| Methanobacteria | Methanobacteriales | Methanobacteriaceae | Methanobacterium | palustre | |
| Methanobacteria | Methanobacteriales | Methanobacteriaceae | Methanobacterium | subterraneum | |
| Methanobacteria | Methanobacteriales | Methanobacteriaceae | Methanobacterium | thermaggregans | |
| Methanobacteria | Methanobacteriales | Methanobacteriaceae | Methanobacterium | uliginosum | |
| Methanobacteria | Methanobacteriales | Methanobacteriaceae | Methanobacterium | sp. | |
| Methanobacteria | Methanobacteriales | Methanobacteriaceae | Methanobacterium | sp. 0372-D1 | |
| Methanobacteria | Methanobacteriales | Methanobacteriaceae | Methanobacterium | sp. 169 | |
| Methanobacteria | Methanobacteriales | Methanobacteriaceae | Methanobacterium | sp. 25 | |
| Methanobacteria | Methanobacteriales | Methanobacteriaceae | Methanobacterium | sp. 28 | |
| Methanobacteria | Methanobacteriales | Methanobacteriaceae | Methanobacterium | sp. 8-1 | |
| Methanobacteria | Methanobacteriales | Methanobacteriaceae | Methanobacterium | sp. AH1 | |
| Methanobacteria | Methanobacteriales | Methanobacteriaceae | Methanobacterium | sp. BRM9 | |
| Methanobacteria | Methanobacteriales | Methanobacteriaceae | Methanobacterium | sp. C5/51 | |
| Methanobacteria | Methanobacteriales | Methanobacteriaceae | Methanobacterium | sp. Ch | |
| Methanobacteria | Methanobacteriales | Methanobacteriaceae | Methanobacterium | sp. F | |
| Methanobacteria | Methanobacteriales | Methanobacteriaceae | Methanobacterium | sp. GH | |
| Methanobacteria | Methanobacteriales | Methanobacteriaceae | Methanobacterium | sp. HD-1 | |
| Methanobacteria | Methanobacteriales | Methanobacteriaceae | Methanobacterium | sp. IM1 | |
| Methanobacteria | Methanobacteriales | Methanobacteriaceae | Methanobacterium | sp. M03 | |
| Methanobacteria | Methanobacteriales | Methanobacteriaceae | Methanobacterium | sp. M2 | |
| Methanobacteria | Methanobacteriales | Methanobacteriaceae | Methanobacterium | sp. MB4 | |
| Methanobacteria | Methanobacteriales | Methanobacteriaceae | Methanobacterium | sp. Mg38 | |
| Methanobacteria | Methanobacteriales | Methanobacteriaceae | Methanobacterium | sp. Mic5c12 | |
| Methanobacteria | Methanobacteriales | Methanobacteriaceae | Methanobacterium | sp. Mic6c05 | |
| Methanobacteria | Methanobacteriales | Methanobacteriaceae | Methanobacterium | sp. MK4 | |
| Methanobacteria | Methanobacteriales | Methanobacteriaceae | Methanobacterium | sp. OM15 | |
| Methanobacteria | Methanobacteriales | Methanobacteriaceae | Methanobacterium | sp. Ps21 | |
| Methanobacteria | Methanobacteriales | Methanobacteriaceae | Methanobacterium | sp. SA-12 | |
| Methanobacteria | Methanobacteriales | Methanobacteriaceae | Methanobacterium | sp. T01 | |
| Methanobacteria | Methanobacteriales | Methanobacteriaceae | Methanobacterium | sp. T11 | |
| Methanobacteria | Methanobacteriales | Methanobacteriaceae | Methanobacterium | sp. Tc3 | |
| Methanobacteria | Methanobacteriales | Methanobacteriaceae | Methanobacterium | sp. TM-8 | |
| Methanobacteria | Methanobacteriales | Methanobacteriaceae | Methanobacterium | sp. TS2 | |
| Methanobacteria | Methanobacteriales | Methanobacteriaceae | Methanobacterium | sp. XJ-3a | |
| Methanobacteria | Methanobacteriales | Methanobacteriaceae | Methanobacterium | sp. YCM1 | |
| Methanobacteria | Methanobacteriales | Methanobacteriaceae | Methanobacterium | Unclassified | 176306 |
| Methanobacteria | Methanobacteriales | Methanobacteriaceae | Methanobrevibacter | acididurans | |
| Methanobacteria | Methanobacteriales | Methanobacteriaceae | Methanobrevibacter | arboriphilus | |
| Methanobacteria | Methanobacteriales | Methanobacteriaceae | Methanobrevibacter | curvatus | |
| Methanobacteria | Methanobacteriales | Methanobacteriaceae | Methanobrevibacter | cuticularis | |
| Methanobacteria | Methanobacteriales | Methanobacteriaceae | Methanobrevibacter | filiformis | |
| Methanobacteria | Methanobacteriales | Methanobacteriaceae | Methanobrevibacter | gottschalkii | |
| Methanobacteria | Methanobacteriales | Methanobacteriaceae | Methanobrevibacter | millerae | |
| Methanobacteria | Methanobacteriales | Methanobacteriaceae | Methanobrevibacter | olleyae | |
| Methanobacteria | Methanobacteriales | Methanobacteriaceae | Methanobrevibacter | oralis | |
| Methanobacteria | Methanobacteriales | Methanobacteriaceae | Methanobrevibacter | ruminantium | |
| Methanobacteria | Methanobacteriales | Methanobacteriaceae | Methanobrevibacter | smithii ATCC 35061 | |
| Methanobacteria | Methanobacteriales | Methanobacteriaceae | Methanobrevibacter | smithii DSM 11975 | |
| Methanobacteria | Methanobacteriales | Methanobacteriaceae | Methanobrevibacter | smithii DSM 2374 | |
| Methanobacteria | Methanobacteriales | Methanobacteriaceae | Methanobrevibacter | smithii DSM 2375 | |
| Methanobacteria | Methanobacteriales | Methanobacteriaceae | Methanobrevibacter | thaueri | |
| Methanobacteria | Methanobacteriales | Methanobacteriaceae | Methanobrevibacter | woesei | |
| Methanobacteria | Methanobacteriales | Methanobacteriaceae | Methanobrevibacter | wolinii | |
| Methanobacteria | Methanobacteriales | Methanobacteriaceae | Methanobrevibacter | Unclassified | |
| Methanobacteria | Methanobacteriales | Methanobacteriaceae | Methanobrevibacter | sp. 110 | |
| Methanobacteria | Methanobacteriales | Methanobacteriaceae | Methanobrevibacter | sp. 1Y | |
| Methanobacteria | Methanobacteriales | Methanobacteriaceae | Methanobrevibacter | sp. 30Y | |
| Methanobacteria | Methanobacteriales | Methanobacteriaceae | Methanobrevibacter | sp. 62 | |
| Methanobacteria | Methanobacteriales | Methanobacteriaceae | Methanobrevibacter | sp. 87.7 | |
| Methanobacteria | Methanobacteriales | Methanobacteriaceae | Methanobrevibacter | sp. AbM4 | |
| Methanobacteria | Methanobacteriales | Methanobacteriaceae | Methanobrevibacter | sp. AK-87 | |
| Methanobacteria | Methanobacteriales | Methanobacteriaceae | Methanobrevibacter | sp. CIRG-GMbb01 | |
| Methanobacteria | Methanobacteriales | Methanobacteriaceae | Methanobrevibacter | sp. CIRG-GMbb02 | |
| Methanobacteria | Methanobacteriales | Methanobacteriaceae | Methanobrevibacter | sp. FM1 | |
| Methanobacteria | Methanobacteriales | Methanobacteriaceae | Methanobrevibacter | sp. FMB1 | |
| Methanobacteria | Methanobacteriales | Methanobacteriaceae | Methanobrevibacter | sp. FMB2 | |
| Methanobacteria | Methanobacteriales | Methanobacteriaceae | Methanobrevibacter | sp. FMB3 | |
| Methanobacteria | Methanobacteriales | Methanobacteriaceae | Methanobrevibacter | sp. FMBK1 | |
| Methanobacteria | Methanobacteriales | Methanobacteriaceae | Methanobrevibacter | sp. FMBK2 | |
| Methanobacteria | Methanobacteriales | Methanobacteriaceae | Methanobrevibacter | sp. FMBK3 | |
| Methanobacteria | Methanobacteriales | Methanobacteriaceae | Methanobrevibacter | sp. FMBK4 | |
| Methanobacteria | Methanobacteriales | Methanobacteriaceae | Methanobrevibacter | sp. FMBK5 | |
| Methanobacteria | Methanobacteriales | Methanobacteriaceae | Methanobrevibacter | sp. FMBK6 | |
| Methanobacteria | Methanobacteriales | Methanobacteriaceae | Methanobrevibacter | sp. FMBK7 | |
| Methanobacteria | Methanobacteriales | Methanobacteriaceae | Methanobrevibacter | sp. HW23 | |
| Methanobacteria | Methanobacteriales | Methanobacteriaceae | Methanobrevibacter | sp. LRsD4 | |
| Methanobacteria | Methanobacteriales | Methanobacteriaceae | Methanobrevibacter | sp. Mc30 | |
| Methanobacteria | Methanobacteriales | Methanobacteriaceae | Methanobrevibacter | sp. MCTS 1-B | |
| Methanobacteria | Methanobacteriales | Methanobacteriaceae | Methanobrevibacter | sp. MCTS 2-G | |
| Methanobacteria | Methanobacteriales | Methanobacteriaceae | Methanobrevibacter | sp. MD101 | |
| Methanobacteria | Methanobacteriales | Methanobacteriaceae | Methanobrevibacter | sp. MD102 | |
| Methanobacteria | Methanobacteriales | Methanobacteriaceae | Methanobrevibacter | sp. MD103 | |
| Methanobacteria | Methanobacteriales | Methanobacteriaceae | Methanobrevibacter | sp. MD104 | |
| Methanobacteria | Methanobacteriales | Methanobacteriaceae | Methanobrevibacter | sp. MD105 | |
| Methanobacteria | Methanobacteriales | Methanobacteriaceae | Methanobrevibacter | sp. OCP | |
| Methanobacteria | Methanobacteriales | Methanobacteriaceae | Methanobrevibacter | sp. Rsl3 | |
| Methanobacteria | Methanobacteriales | Methanobacteriaceae | Methanobrevibacter | sp. RsW3 | |
| Methanobacteria | Methanobacteriales | Methanobacteriaceae | Methanobrevibacter | sp. SM9 | |
| Methanobacteria | Methanobacteriales | Methanobacteriaceae | Methanobrevibacter | sp. WBY1 | |
| Methanobacteria | Methanobacteriales | Methanobacteriaceae | Methanobrevibacter | sp. XT106 | |
| Methanobacteria | Methanobacteriales | Methanobacteriaceae | Methanobrevibacter | sp. XT108 | |
| Methanobacteria | Methanobacteriales | Methanobacteriaceae | Methanobrevibacter | sp. XT109 | |
| Methanobacteria | Methanobacteriales | Methanobacteriaceae | Methanobrevibacter | sp. YE286 | |
| Methanobacteria | Methanobacteriales | Methanobacteriaceae | Methanobrevibacter | sp. YE287 | |
| Methanobacteria | Methanobacteriales | Methanobacteriaceae | Methanobrevibacter | sp. YE288 | |
| Methanobacteria | Methanobacteriales | Methanobacteriaceae | Methanobrevibacter | sp. YE300 | |
| Methanobacteria | Methanobacteriales | Methanobacteriaceae | Methanobrevibacter | sp. YE301 | |
| Methanobacteria | Methanobacteriales | Methanobacteriaceae | Methanobrevibacter | sp. YE302 | |
| Methanobacteria | Methanobacteriales | Methanobacteriaceae | Methanobrevibacter | sp. YE303 | |
| Methanobacteria | Methanobacteriales | Methanobacteriaceae | Methanobrevibacter | sp. YE304 | |
| Methanobacteria | Methanobacteriales | Methanobacteriaceae | Methanobrevibacter | sp. YLM1 | |
| Methanobacteria | Methanobacteriales | Methanobacteriaceae | Methanobrevibacter | sp. Z4 | |
| Methanobacteria | Methanobacteriales | Methanobacteriaceae | Methanobrevibacter | sp. Z6 | |
| Methanobacteria | Methanobacteriales | Methanobacteriaceae | Methanobrevibacter | sp. Z8 | |
| Methanobacteria | Methanobacteriales | Methanobacteriaceae | Methanobrevibacter | endosymbiont 'TS1' of Trimyema compressum | |
| Methanobacteria | Methanobacteriales | Methanobacteriaceae | Methanobrevibacter | methanogenic endosymbiont of Nyctotherus cordiformis | |
| Methanobacteria | Methanobacteriales | Methanobacteriaceae | Methanobrevibacter | methanogenic endosymbiont of Nyctotherus ovalis | |
| Methanobacteria | Methanobacteriales | Methanobacteriaceae | Methanobrevibacter | methanogenic endosymbiont of Nyctotherus velox | |
| Methanobacteria | Methanobacteriales | Methanobacteriaceae | Methanobrevibacter | methanogenic symbiont RS104 | |
| Methanobacteria | Methanobacteriales | Methanobacteriaceae | Methanobrevibacter | methanogenic symbiont RS105 | |
| Methanobacteria | Methanobacteriales | Methanobacteriaceae | Methanobrevibacter | methanogenic symbiont RS208 | |
| Methanobacteria | Methanobacteriales | Methanobacteriaceae | Methanobrevibacter | methanogenic symbiont RS301 | |
| Methanobacteria | Methanobacteriales | Methanobacteriaceae | Methanobrevibacter | methanogenic symbiont RS404 | |
| Methanobacteria | Methanobacteriales | Methanobacteriaceae | Methanobrevibacter | methanogenic symbiont RS801 | |
| Methanobacteria | Methanobacteriales | Methanobacteriaceae | Methanobrevibacter | methanogenic symbiont RS802 | |
| Methanobacteria | Methanobacteriales | Methanobacteriaceae | Methanobrevibacter | sp. HI1 | |
| Methanobacteria | Methanobacteriales | Methanobacteriaceae | Methanobrevibacter | sp. HI26 | |
| Methanobacteria | Methanobacteriales | Methanobacteriaceae | Methanobrevibacter | sp. HI28 | |
| Methanobacteria | Methanobacteriales | Methanobacteriaceae | Methanobrevibacter | sp. HW1 | |
| Methanobacteria | Methanobacteriales | Methanobacteriaceae | Methanobrevibacter | sp. HW2 | |
| Methanobacteria | Methanobacteriales | Methanobacteriaceae | Methanobrevibacter | sp. HW3 | |
| Methanobacteria | Methanobacteriales | Methanobacteriaceae | Methanobrevibacter | sp. LHD12 | |
| Methanobacteria | Methanobacteriales | Methanobacteriaceae | Methanobrevibacter | sp. LHD2 | |
| Methanobacteria | Methanobacteriales | Methanobacteriaceae | Methanobrevibacter | sp. LHM8 | |
| Methanobacteria | Methanobacteriales | Methanobacteriaceae | Methanobrevibacter | sp. LRsD2 | |
| Methanobacteria | Methanobacteriales | Methanobacteriaceae | Methanobrevibacter | sp. LRsD3 | |
| Methanobacteria | Methanobacteriales | Methanobacteriaceae | Methanobrevibacter | sp. LRsM1 | |
| Methanobacteria | Methanobacteriales | Methanobacteriaceae | Methanobrevibacter | sp. R1 | |
| Methanobacteria | Methanobacteriales | Methanobacteriaceae | Methanobrevibacter | sp. R2 | |
| Methanobacteria | Methanobacteriales | Methanobacteriaceae | Methanobrevibacter | sp. R3 | |
| Methanobacteria | Methanobacteriales | Methanobacteriaceae | Methanobrevibacter | sp. R4 | |
| Methanobacteria | Methanobacteriales | Methanobacteriaceae | Methanobrevibacter | sp. R5 | |
| Methanobacteria | Methanobacteriales | Methanobacteriaceae | Methanobrevibacter | sp. RsI12 | |
| Methanobacteria | Methanobacteriales | Methanobacteriaceae | Methanobrevibacter | sp. RsI17 | |
| Methanobacteria | Methanobacteriales | Methanobacteriaceae | Methanobrevibacter | sp. Rsl4 | |
| Methanobacteria | Methanobacteriales | Methanobacteriaceae | Methanobrevibacter | sp. RsW10 | |
| Methanobacteria | Methanobacteriales | Methanobacteriaceae | Methanobrevibacter | sp. RsW2 | |
| Methanobacteria | Methanobacteriales | Methanobacteriaceae | Methanobrevibacter | uncultured archaeon Ar40 | |
| Methanobacteria | Methanobacteriales | Methanobacteriaceae | Methanobrevibacter | uncultured Methanobrevibacter sp. | |
| Methanobacteria | Methanobacteriales | Methanobacteriaceae | Methanobrevibacter | uncultured termite gut bacterium Cd30 | |
| Methanobacteria | Methanobacteriales | Methanobacteriaceae | Methanobrevibacter | unidentified methanogen ARC1 | |
| Methanobacteria | Methanobacteriales | Methanobacteriaceae | Methanobrevibacter | unidentified methanogen ARC12 | |
| Methanobacteria | Methanobacteriales | Methanobacteriaceae | Methanobrevibacter | unidentified methanogen ARC13 | |
| Methanobacteria | Methanobacteriales | Methanobacteriaceae | Methanobrevibacter | unidentified methanogen ARC15 | |
| Methanobacteria | Methanobacteriales | Methanobacteriaceae | Methanobrevibacter | unidentified methanogen ARC19 | |
| Methanobacteria | Methanobacteriales | Methanobacteriaceae | Methanobrevibacter | unidentified methanogen ARC20 | |
| Methanobacteria | Methanobacteriales | Methanobacteriaceae | Methanobrevibacter | unidentified methanogen ARC23 | |
| Methanobacteria | Methanobacteriales | Methanobacteriaceae | Methanobrevibacter | unidentified methanogen ARC24 | |
| Methanobacteria | Methanobacteriales | Methanobacteriaceae | Methanobrevibacter | unidentified methanogen ARC25 | |
| Methanobacteria | Methanobacteriales | Methanobacteriaceae | Methanobrevibacter | unidentified methanogen ARC26 | |
| Methanobacteria | Methanobacteriales | Methanobacteriaceae | Methanobrevibacter | unidentified methanogen ARC27 | |
| Methanobacteria | Methanobacteriales | Methanobacteriaceae | Methanobrevibacter | unidentified methanogen ARC28 | |
| Methanobacteria | Methanobacteriales | Methanobacteriaceae | Methanobrevibacter | unidentified methanogen ARC32 | |
| Methanobacteria | Methanobacteriales | Methanobacteriaceae | Methanobrevibacter | unidentified methanogen ARC33 | |
| Methanobacteria | Methanobacteriales | Methanobacteriaceae | Methanobrevibacter | unidentified methanogen ARC40 | |
| Methanobacteria | Methanobacteriales | Methanobacteriaceae | Methanobrevibacter | unidentified methanogen ARC41 | |
| Methanobacteria | Methanobacteriales | Methanobacteriaceae | Methanobrevibacter | unidentified methanogen ARC44 | |
| Methanobacteria | Methanobacteriales | Methanobacteriaceae | Methanobrevibacter | unidentified methanogen ARC50 | |
| Methanobacteria | Methanobacteriales | Methanobacteriaceae | Methanobrevibacter | unidentified methanogen ARC51 | |
| Methanobacteria | Methanobacteriales | Methanobacteriaceae | Methanobrevibacter | unidentified methanogen ARC52 | |
| Methanobacteria | Methanobacteriales | Methanobacteriaceae | Methanobrevibacter | unidentified methanogen ARC53 | |
| Methanobacteria | Methanobacteriales | Methanobacteriaceae | Methanobrevibacter | unidentified methanogen ARC61 | |
| Methanobacteria | Methanobacteriales | Methanobacteriaceae | Methanobrevibacter | unidentified methanogen ARC65 | |
| Methanobacteria | Methanobacteriales | Methanobacteriaceae | Methanobrevibacter | unidentified methanogen ARC66 | |
| Methanobacteria | Methanobacteriales | Methanobacteriaceae | Methanosphaera | Stadtmanae | 339860 |
| Methanobacteria | Methanobacteriales | Methanobacteriaceae | Methanosphaera | sp. R6 | |
| Methanobacteria | Methanobacteriales | Methanobacteriaceae | Methanosphaera | Uncultured Methanosphaera sp. | |
| Methanobacteria | Methanobacteriales | Methanobacteriaceae | Methanosphaera | unidentified methanogen ARC14 | |
| Methanobacteria | Methanobacteriales | Methanobacteriaceae | Methanosphaera | unidentified methanogen ARC17 | |
| Methanobacteria | Methanobacteriales | Methanobacteriaceae | Methanosphaera | unidentified methanogen ARC18 | |
| Methanobacteria | Methanobacteriales | Methanobacteriaceae | Methanosphaera | unidentified methanogen ARC21 | |
| Methanobacteria | Methanobacteriales | Methanobacteriaceae | Methanosphaera | unidentified methanogen ARC29 | |
| Methanobacteria | Methanobacteriales | Methanobacteriaceae | Methanosphaera | unidentified methanogen ARC30 | |
| Methanobacteria | Methanobacteriales | Methanobacteriaceae | Methanosphaera | unidentified methanogen ARC39 | |
| Methanobacteria | Methanobacteriales | Methanobacteriaceae | Methanosphaera | unidentified methanogen ARC43 | |
| Methanobacteria | Methanobacteriales | Methanobacteriaceae | Methanosphaera | unidentified methanogen ARC49 | |
| Methanobacteria | Methanobacteriales | Methanobacteriaceae | Methanosphaera | unidentified methanogen ARC62 | |
| Methanobacteria | Methanobacteriales | Methanobacteriaceae | Methanosphaera | Unidentified methanogen ARC8 | |
| Methanobacteria | Methanobacteriales | Methanobacteriaceae | Methanothermobacter | defluvii | |
| Methanobacteria | Methanobacteriales | Methanobacteriaceae | Methanothermobacter | marburgensis | |
| Methanobacteria | Methanobacteriales | Methanobacteriaceae | Methanothermobacter | thermautotrophicus str. Delta H | |
| Methanobacteria | Methanobacteriales | Methanobacteriaceae | Methanothermobacter | thermautotrophicus str. Winter | |
| Methanobacteria | Methanobacteriales | Methanobacteriaceae | Methanothermobacter | thermoflexus | |
| Methanobacteria | Methanobacteriales | Methanobacteriaceae | Methanothermobacter | thermophilus | |
| Methanobacteria | Methanobacteriales | Methanobacteriaceae | Methanothermobacter | wolfeii | |
| Methanobacteria | Methanobacteriales | Methanobacteriaceae | Methanothermobacter | sp. RY3 | |
| Methanobacteria | Methanobacteriales | Methanobacteriaceae | Methanothermobacter | sp. THUT3 | |
| Methanobacteria | Methanobacteriales | Methanobacteriaceae | Methanothermobacter | sp. enrichment clone M2 | |
| Methanobacteria | Methanobacteriales | Methanobacteriaceae | Methanothermobacter | sp. enrichment clone PY1 | |
| Methanobacteria | Methanobacteriales | Methanobacteriaceae | Methanothermobacter | sp. enrichment clone PY2 | |
| Methanobacteria | Methanobacteriales | Methanobacteriaceae | Methanothermobacter | sp. enrichment clone SA11 | |
| Methanobacteria | Methanobacteriales | Methanobacteriaceae | Methanothermobacter | sp. enrichment clone SA2 | |
| Methanobacteria | Methanobacteriales | Methanobacteriaceae | Unclassified | archaeon A2.95.53 | |
| Methanobacteria | Methanobacteriales | Methanobacteriaceae | Unclassified | archaeon A8.96.15 | |
| Methanobacteria | Methanobacteriales | Methanobacteriaceae | Unclassified | archaeon A9.96.64 | |
| Methanobacteria | Methanobacteriales | Methanobacteriaceae | Unclassified | archaeon 12aF | |
| Methanobacteria | Methanobacteriales | Methanobacteriaceae | Unclassified | archaeon 14aZ | |
| Methanobacteria | Methanobacteriales | Methanobacteriaceae | Unclassified | archaeon 15aZ | |
| Methanobacteria | Methanobacteriales | Methanobacteriaceae | Unclassified | archaeon 1aR | |
| Methanobacteria | Methanobacteriales | Methanobacteriaceae | Unclassified | archaeon 1aZ | |
| Methanobacteria | Methanobacteriales | Methanobacteriaceae | Unclassified | archaeon 25aG | |
| Methanobacteria | Methanobacteriales | Methanobacteriaceae | Unclassified | archaeon 26aM | |
| Methanobacteria | Methanobacteriales | Methanobacteriaceae | Unclassified | archaeon 2aG | |
| Methanobacteria | Methanobacteriales | Methanobacteriaceae | Unclassified | archaeon 36aR | |
| Methanobacteria | Methanobacteriales | Methanobacteriaceae | Unclassified | archaeon 37aM | |
| Methanobacteria | Methanobacteriales | Methanobacteriaceae | Unclassified | archaeon 3aG | |
| Methanobacteria | Methanobacteriales | Methanobacteriaceae | Unclassified | archaeon 40aM | |
| Methanobacteria | Methanobacteriales | Methanobacteriaceae | Unclassified | archaeon 55aZ | |
| Methanobacteria | Methanobacteriales | Methanobacteriaceae | Unclassified | archaeon 58aZ | |
| Methanobacteria | Methanobacteriales | Methanobacteriaceae | Unclassified | archaeon 77aZ | |
| Methanobacteria | Methanobacteriales | Methanobacteriaceae | Unclassified | archaeon RMAS | |
| Methanobacteria | Methanobacteriales | Methanobacteriaceae | Unclassified | methanogen 5c | |
| Methanobacteria | Methanobacteriales | Methanobacteriaceae | Unclassified | methanogen MEm1 associated with Eudiplodinium maggii | |
| Methanobacteria | Methanobacteriales | Methanobacteriaceae | Unclassified | methanogen MEm2 associated with Eudiplodinium maggii | |
| Methanobacteria | Methanobacteriales | Methanobacteriaceae | Unclassified | methanogen MEm3 associated with Eudiplodinium maggii | |
| Methanobacteria | Methanobacteriales | Methanobacteriaceae | Unclassified | methanogen MIp1 associated with Isotricha prostoma | |
| Methanobacteria | Methanobacteriales | Methanobacteriaceae | Unclassified | methanogen Mlp2 associated with Isotricha prostoma | |
| Methanobacteria | Methanobacteriales | Methanobacteriaceae | Unclassified | methanogen MPm1 associated with Polyplastron multivesiculatum | |
| Methanobacteria | Methanobacteriales | Methanobacteriaceae | Unclassified | methanogen MPm2 associated with Polyplastron multivesiculatum | |
| Methanobacteria | Methanobacteriales | Methanobacteriaceae | Unclassified | methanogen MPm3 associated with Polyplastron multivesiculatum | |
| Methanobacteria | Methanobacteriales | Methanobacteriaceae | Unclassified | Methanobacteriaceae archaeon enrichment clone M13 | |
| Methanobacteria | Methanobacteriales | Methanobacteriaceae | Unclassified | Methanobacteriaceae archaeon enrichment culture clone MBT-13 | |
| Methanobacteria | Methanobacteriales | Methanobacteriaceae | Unclassified | uncultured Methanobacteriaceae archaeon | |
| Methanobacteria | Methanobacteriales | Methanobacteriaceae | Unclassified | uncultured methanogen MRE08 | |
| Methanobacteria | Methanobacteriales | Methanobacteriaceae | Unclassified | uncultured methanogen RS-MCR07 | |
| Methanobacteria | Methanobacteriales | Methanobacteriaceae | Unclassified | uncultured methanogen RS-MCR09 | |
| Methanobacteria | Methanobacteriales | Methanobacteriaceae | Unclassified | uncultured methanogen RS-MCR11 | |
| Methanobacteria | Methanobacteriales | Methanobacteriaceae | Unclassified | uncultured methanogen RS-MCR12 | |
| Methanobacteria | Methanobacteriales | Methanobacteriaceae | Unclassified | uncultured methanogen RS-MCR16 | |
| Methanobacteria | Methanobacteriales | Methanobacteriaceae | Unclassified | uncultured methanogen RS-MCR18 | |
| Methanobacteria | Methanobacteriales | Methanobacteriaceae | Unclassified | uncultured methanogen RS-MCR21 | |
| Methanobacteria | Methanobacteriales | Methanobacteriaceae | Unclassified | uncultured methanogen RS-MCR23 | |
| Methanobacteria | Methanobacteriales | Methanobacteriaceae | Unclassified | uncultured methanogen RS-MCR26 | |
| Methanobacteria | Methanobacteriales | Methanobacteriaceae | Unclassified | uncultured methanogen RS-MCR27 | |
| Methanobacteria | Methanobacteriales | Methanobacteriaceae | Unclassified | uncultured methanogen RS-MCR29 | |
| Methanobacteria | Methanobacteriales | Methanobacteriaceae | Unclassified | uncultured methanogen RS-MCR37 | |
| Methanobacteria | Methanobacteriales | Methanobacteriaceae | Unclassified | uncultured methanogen RS-MCR44 | |
| Methanobacteria | Methanobacteriales | Methanobacteriaceae | Unclassified | uncultured methanogen RS-MCR45 | |
| Methanobacteria | Methanobacteriales | Methanobacteriaceae | Unclassified | uncultured methanogen RS-ME01 | |
| Methanobacteria | Methanobacteriales | Methanobacteriaceae | Unclassified | uncultured methanogen RS-ME05 | |
| Methanobacteria | Methanobacteriales | Methanobacteriaceae | Unclassified | uncultured methanogen RS-ME07 | |
| Methanobacteria | Methanobacteriales | Methanobacteriaceae | Unclassified | uncultured methanogen RS-ME09 | |
| Methanobacteria | Methanobacteriales | Methanobacteriaceae | Unclassified | uncultured methanogen RS-ME10 | |
| Methanobacteria | Methanobacteriales | Methanobacteriaceae | Unclassified | uncultured methanogen RS-ME15 | |
| Methanobacteria | Methanobacteriales | Methanobacteriaceae | Unclassified | uncultured methanogen RS-ME29 | |
| Methanobacteria | Methanobacteriales | Methanobacteriaceae | Unclassified | uncultured methanogen RS-ME31 | |
| Methanobacteria | Methanobacteriales | Methanobacteriaceae | Unclassified | uncultured methanogen RS-ME36 | |
| Methanobacteria | Methanobacteriales | Methanobacteriaceae | Unclassified | uncultured methanogen RS-ME38 | |
| Methanobacteria | Methanobacteriales | Methanobacteriaceae | Unclassified | uncultured methanogen RS-ME44 | |
| Methanobacteria | Methanobacteriales | Methanobacteriaceae | Unclassified | uncultured methanogen RS-ME45 | |
| Methanobacteria | Methanobacteriales | Methanobacteriaceae | Unclassified | uncultured methanogen RS-ME47 | |
| Methanobacteria | Methanobacteriales | Methanobacteriaceae | Unclassified | uncultured methanogen RS-ME50 | |
| Methanobacteria | Methanobacteriales | Methanobacteriaceae | Methanothermus | fervidus | 523846 |
| Methanobacteria | Methanobacteriales | Methanobacteriaceae | Methanothermus | sociabilis | 2181 |
| Methanobacteria | Methanobacteriales | Methanobacteriaceae | Unclassified | enrichment culture E21A2 | 114581 |
| Methanobacteria | Methanobacteriales | Methanobacteriaceae | Unclassified | archaeon enrichment clone M65 | 388592 |
| Methanobacteria | Methanobacteriales | Methanobacteriaceae | Unclassified | uncultured Methanobacteriales archaeon | 194842 |
| Methanobacteria | Methanobacteriales | Methanobacteriaceae | Unclassified | Unidentified Methanobacteriales | 58668 |
| Methanococci | Methanococcales | Methanocaldococcaceae | Methanocaldococcus | fervens | 573064 |
| Methanococci | Methanococcales | Methanocaldococcaceae | Methanocaldococcus | indicus | 213231 |
| Methanococci | Methanococcales | Methanocaldococcaceae | Methanocaldococcus | infernus | 573063 |
| Methanococci | Methanococcales | Methanocaldococcaceae | Methanocaldococcus | jannaschii | 243232 |
| Methanococci | Methanococcales | Methanocaldococcaceae | Methanocaldococcus | vulcanius | 579137 |
| Methanococci | Methanococcales | Methanocaldococcaceae | Methanocaldococcus | sp. 70-8-3 | 345590 |
| Methanococci | Methanococcales | Methanocaldococcaceae | Methanocaldococcus | sp. E1885-M | 269226 |
| Methanococci | Methanococcales | Methanocaldococcaceae | Methanocaldococcus | sp. FS406-22 | 644281 |
| Methanococci | Methanococcales | Methanocaldococcaceae | Methanocaldococcus | sp. KIN24-T80 | 667126 |
| Methanococci | Methanococcales | Methanocaldococcaceae | Methanocaldococcus | sp. Mc-1-85 | 213203 |
| Methanococci | Methanococcales | Methanocaldococcaceae | Methanocaldococcus | sp. Mc-2-70 | 213204 |
| Methanococci | Methanococcales | Methanocaldococcaceae | Methanocaldococcus | sp. Mc-2-85 | 213203 |
| Methanococci | Methanococcales | Methanocaldococcaceae | Methanocaldococcus | sp. Mc-365-70 | 213206 |
| Methanococci | Methanococcales | Methanocaldococcaceae | Methanocaldococcus | sp. Mc-365-85 | 213207 |
| Methanococci | Methanococcales | Methanocaldococcaceae | Methanocaldococcus | sp. Mc-I-85 | 213208 |
| Methanococci | Methanococcales | Methanocaldococcaceae | Methanocaldococcus | sp. Mc-S-85 | 213209 |
| Methanococci | Methanococcales | Methanocaldococcaceae | Methanocaldococcus | Unclassified | 328406 |
| Methanococci | Methanococcales | Methanocaldococcaceae | Methanotorris | formicicus | 213185 |
| Methanococci | Methanococcales | Methanocaldococcaceae | Methanotorris | igneus | 2189 |
| Methanococci | Methanococcales | Methanocaldococcaceae | Methanotorris | sp. Mc-I-70 | 213186 |
| Methanococci | Methanococcales | Methanocaldococcaceae | Methanotorris | sp. Mc-S-70 | 213187 |
| Methanococci | Methanococcales | Methanocaldococcaceae | Methanotorris | Unclassified | 549381 |
| Methanococci | Methanococcales | Methanococcaceae | Methanococcus | aeolicus | |
| Methanococci | Methanococcales | Methanococcaceae | Methanococcus | maripaludis | |
| Methanococci | Methanococcales | Methanococcaceae | Methanococcus | vannielii | |
| Methanococci | Methanococcales | Methanococcaceae | Methanococcus | voltae | |
| Methanococci | Methanococcales | Methanococcaceae | Methanococcus | sp. Mc55_ 19 | |
| Methanococci | Methanococcales | Methanococcaceae | Methanococcus | sp. Mc55_ 2 | |
| Methanococci | Methanococcales | Methanococcaceae | Methanococcus | sp. Mc55_ 20 | |
| Methanococci | Methanococcales | Methanococcaceae | Methanococcus | sp. Mc70_1 | |
| Methanococci | Methanococcales | Methanococcaceae | Methanococcus | sp. Mc70_ 2 | |
| Methanococci | Methanococcales | Methanococcaceae | Methanococcus | sp. Mc85_2 | |
| Methanococci | Methanococcales | Methanococcaceae | Methanococcus | sp. Ms33_19 | |
| Methanococci | Methanococcales | Methanococcaceae | Methanococcus | sp. Ms33_ 20 | |
| Methanococci | Methanococcales | Methanococcaceae | Methanococcus | sp. Ms55_19 | |
| Methanococci | Methanococcales | Methanococcaceae | Methanococcus | sp. Ms55_ 20 | |
| Methanococci | Methanococcales | Methanococcaceae | Methanococcus | sp. P2F9701 a | |
| Methanococci | Methanococcales | Methanococcaceae | Methanococcus | Unclassified | 262498 |
| Methanococci | Methanococcales | Methanococcaceae | Methanothermococcus | okinawensis | |
| Methanococci | Methanococcales | Methanococcaceae | Methanothermococcus | thermolithotrophicus | |
| Methanococci | Methanococcales | Methanococcaceae | Methanothermococcus | sp. E1855-M | |
| Methanococci | Methanococcales | Methanococcaceae | Methanothermococcus | sp. Ep55 | |
| Methanococci | Methanococcales | Methanococcaceae | Methanothermococcus | sp. Ep70 | |
| Methanococci | Methanococcales | Methanococcaceae | Methanothermococcus | sp. Mc-1-55 | |
| Methanococci | Methanococcales | Methanococcaceae | Methanothermococcus | sp. Mc37 | |
| Methanococci | Methanococcales | Methanococcaceae | Methanothermococcus | sp. Mc55 | |
| Methanococci | Methanococcales | Methanococcaceae | Methanothermococcus | sp. Mc70 | |
| Methanococci | Methanococcales | Methanococcaceae | Methanothermococcus | sp. Mc70_19 | |
| Methanococci | Methanococcales | Methanococcaceae | Methanothermococcus | sp. PaI55-Mc | |
| Methanococci | Methanococcales | Methanococcaceae | Methanothermococcus | sp. enrichment clone M11 | 388596 |
| Methanococci | Methanococcales | Methanococcaceae | Methanothermococcus | sp. enrichment clone M37 | 388597 |
| Methanococci | Methanococcales | Methanococcaceae | Methanothermococcus | Unclassified | 269251 |
| Methanococci | Methanococcales | Unclassified | Unclassified | archaeal str. vp183 | 114585 |
| Methanococci | Methanococcales | Unclassified | Unclassified | archaeal str. vp21 | 114587 |
| Methanococci | Methanococcales | Unclassified | Unclassified | hyperthermophilic methanogen FS406-22 | 412882 |
| Methanococci | Methanococcales | Unclassified | Unclassified | Unclassified | 345627 |
| Methanomicrobia | Methanocellales | Methanocellaceae | Methanocella | paludicola | 304371 |
| Methanomicrobia | Methanomicrobiales | Methanocorpusculaceae | Methanocorpusculum | aggregans | 176294 |
| Methanomicrobia | Methanomicrobiales | Methanocorpusculaceae | Methanocorpusculum | bavaricum | |
| Methanomicrobia | Methanomicrobiales | Methanocorpusculaceae | Methanocorpusculum | labreanum | |
| Methanomicrobia | Methanomicrobiales | Methanocorpusculaceae | Methanocorpusculum | parvum | |
| Methanomicrobia | Methanomicrobiales | Methanocorpusculaceae | Methanocorpusculum | sinense | |
| Methanomicrobia | Methanomicrobiales | Methanocorpusculaceae | Methanocorpusculum | sp. MSP | |
| Methanomicrobia | Methanomicrobiales | Methanocorpusculaceae | Methanocorpusculum | sp. T07 | |
| Methanomicrobia | Methanomicrobiales | Methanocorpusculaceae | Methanocorpusculum | sp. T08 | |
| Methanomicrobia | Methanomicrobiales | Methanocorpusculaceae | Methanocorpusculum | Metopus contortus archaeal symbiont | |
| Methanomicrobia | Methanomicrobiales | Methanocorpusculaceae | Methanocorpusculum | Metopus palaeformis endosymbiont | |
| Methanomicrobia | Methanomicrobiales | Methanocorpusculaceae | Methanocorpusculum | Trimyema sp. archaeal symbiont | |
| Methanomicrobia | Methanomicrobiales | Methanocorpusculaceae | Methanocorpusculum | Unclassified | 176309 |
| Methanomicrobia | Methanomicrobiales | Methanocorpusculaceae | Methanocorpusculum | uncultured archaeon Ar37 | 97121 |
| Methanomicrobia | Methanomicrobiales | Methanomicrobiaceae | Methanoculleus | bourgensis | |
| Methanomicrobia | Methanomicrobiales | Methanomicrobiaceae | Methanoculleus | chikugoensis | |
| Methanomicrobia | Methanomicrobiales | Methanomicrobiaceae | Methanoculleus | marisnigri | |
| Methanomicrobia | Methanomicrobiales | Methanomicrobiaceae | Methanoculleus | marisnigri JR1 | |
| Methanomicrobia | Methanomicrobiales | Methanomicrobiaceae | Methanoculleus | palmolei | |
| Methanomicrobia | Methanomicrobiales | Methanomicrobiaceae | Methanoculleus | receptaculi | |
| Methanomicrobia | Methanomicrobiales | Methanomicrobiaceae | Methanoculleus | submarinus | |
| Methanomicrobia | Methanomicrobiales | Methanomicrobiaceae | Methanoculleus | thermophilus | |
| Methanomicrobia | Methanomicrobiales | Methanomicrobiaceae | Methanoculleus | Unclassified | |
| Methanomicrobia | Methanomicrobiales | Methanomicrobiaceae | Methanoculleus | sp. 10 | |
| Methanomicrobia | Methanomicrobiales | Methanomicrobiaceae | Methanoculleus | sp. 20 | |
| Methanomicrobia | Methanomicrobiales | Methanomicrobiaceae | Methanoculleus | sp. 22 | |
| Methanomicrobia | Methanomicrobiales | Methanomicrobiaceae | Methanoculleus | sp. BA1 | |
| Methanomicrobia | Methanomicrobiales | Methanomicrobiaceae | Methanoculleus | sp. dm2 | |
| Methanomicrobia | Methanomicrobiales | Methanomicrobiaceae | Methanoculleus | sp. HC | |
| Methanomicrobia | Methanomicrobiales | Methanomicrobiaceae | Methanoculleus | sp. HC-1 | |
| Methanomicrobia | Methanomicrobiales | Methanomicrobiaceae | Methanoculleus | sp. IIE1 | |
| Methanomicrobia | Methanomicrobiales | Methanomicrobiaceae | Methanoculleus | sp. LH | |
| Methanomicrobia | Methanomicrobiales | Methanomicrobiaceae | Methanoculleus | sp. LH2 | |
| Methanomicrobia | Methanomicrobiales | Methanomicrobiaceae | Methanoculleus | sp. M06 | |
| Methanomicrobia | Methanomicrobiales | Methanomicrobiaceae | Methanoculleus | sp. M07 | |
| Methanomicrobia | Methanomicrobiales | Methanomicrobiaceae | Methanoculleus | sp. M11 | |
| Methanomicrobia | Methanomicrobiales | Methanomicrobiaceae | Methanoculleus | sp. MAB1 | |
| Methanomicrobia | Methanomicrobiales | Methanomicrobiaceae | Methanoculleus | sp. MAB2 | |
| Methanomicrobia | Methanomicrobiales | Methanomicrobiaceae | Methanoculleus | sp. MAB3 | |
| Methanomicrobia | Methanomicrobiales | Methanomicrobiaceae | Methanoculleus | sp. MQ-4 | |
| Methanomicrobia | Methanomicrobiales | Methanomicrobiaceae | Methanoculleus | sp. RPS4 | |
| Methanomicrobia | Methanomicrobiales | Methanomicrobiaceae | Methanoculleus | sp. T02 | |
| Methanomicrobia | Methanomicrobiales | Methanomicrobiaceae | Methanoculleus | sp. T03 | |
| Methanomicrobia | Methanomicrobiales | Methanomicrobiaceae | Methanoculleus | sp. T05 | |
| Methanomicrobia | Methanomicrobiales | Methanomicrobiaceae | Methanoculleus | sp. T10 | |
| Methanomicrobia | Methanomicrobiales | Methanomicrobiaceae | Methanoculleus | sp. T14 | |
| Methanomicrobia | Methanomicrobiales | Methanomicrobiaceae | Methanoculleus | sp. enrichment culture clone BAMC-1 | |
| Methanomicrobia | Methanomicrobiales | Methanomicrobiaceae | Methanoculleus | sp. enrichment culture clone BAMC-2 | |
| Methanomicrobia | Methanomicrobiales | Methanomicrobiaceae | Methanoculleus | uncultured sp. | 183762 |
| Methanomicrobia | Methanomicrobiales | Methanomicrobiaceae | Methanofollis | aquaemaris | |
| Methanomicrobia | Methanomicrobiales | Methanomicrobiaceae | Methanofollis | ethanolicus | |
| Methanomicrobia | Methanomicrobiales | Methanomicrobiaceae | Methanofollis | formosanus | |
| Methanomicrobia | Methanomicrobiales | Methanomicrobiaceae | Methanofollis | liminatans | |
| Methanomicrobia | Methanomicrobiales | Methanomicrobiaceae | Methanofollis | tationis | |
| Methanomicrobia | Methanomicrobiales | Methanomicrobiaceae | Methanofollis | sp. YCM2 | |
| Methanomicrobia | Methanomicrobiales | Methanomicrobiaceae | Methanofollis | sp. YCM3 | |
| Methanomicrobia | Methanomicrobiales | Methanomicrobiaceae | Methanofollis | sp. YCM4 | |
| Methanomicrobia | Methanomicrobiales | Methanomicrobiaceae | Methanofollis | Unclassified | 262500 |
| Methanomicrobia | Methanomicrobiales | Methanomicrobiaceae | Methanogenium | boonei | |
| Methanomicrobia | Methanomicrobiales | Methanomicrobiaceae | Methanogenium | cariaci | |
| Methanomicrobia | Methanomicrobiales | Methanomicrobiaceae | Methanogenium | frigidum | |
| Methanomicrobia | Methanomicrobiales | Methanomicrobiaceae | Methanogenium | marinum | |
| Methanomicrobia | Methanomicrobiales | Methanomicrobiaceae | Methanogenium | organophilum | |
| Methanomicrobia | Methanomicrobiales | Methanomicrobiaceae | Methanogenium | sp. AK-8 | |
| Methanomicrobia | Methanomicrobiales | Methanomicrobiaceae | Methanogenium | archaeon ACE1_A | |
| Methanomicrobia | Methanomicrobiales | Methanomicrobiaceae | Methanogenium | archaeon SCALE-14 | |
| Methanomicrobia | Methanomicrobiales | Methanomicrobiaceae | Methanogenium | Unclassified | 292409 |
| | | | | | |
| Methanomicrobia | Methanomicrobiales | Methanomicrobiaceae | Methanoplanus | endosymbiosus | |
| Methanomicrobia | Methanomicrobiales | Methanomicrobiaceae | Methanoplanus | limicola | |
| Methanomicrobia | Methanomicrobiales | Methanomicrobiaceae | Methanoplanus | petrolearius | |
| Methanomicrobia | Methanomicrobiales | Methanomicrobiaceae | Methanoplanus | sp. MobH | |
| Methanomicrobia | Methanomicrobiales | Methanomicrobiaceae | Methanoplanus | Unclassified | 404323 |
| Methanomicrobia | Methanomicrobiales | Methanomicrobiaceae | Unclassified | archaeon 11aR | |
| Methanomicrobia | Methanomicrobiales | Methanomicrobiaceae | Unclassified | archaeon 22aZ | |
| Methanomicrobia | Methanomicrobiales | Methanomicrobiaceae | Unclassified | archaeon 29aM | |
| Methanomicrobia | Methanomicrobiales | Methanomicrobiaceae | Unclassified | archaeon 34aM | |
| Methanomicrobia | Methanomicrobiales | Methanomicrobiaceae | Unclassified | archaeon 56aR | |
| Methanomicrobia | Methanomicrobiales | Methanomicrobiaceae | Unclassified | archaeon 66aM | |
| Methanomicrobia | Methanomicrobiales | Methanomicrobiaceae | Unclassified | archaeon 6aM | |
| Methanomicrobia | Methanomicrobiales | Methanomicrobiaceae | Unclassified | strain EBac | |
| Methanomicrobia | Methanomicrobiales | Methanomicrobiaceae | Unclassified | Nasutitermes takasagoensis symbiont MNt1 | |
| Methanomicrobia | Methanomicrobiales | Methanomicrobiaceae | Unclassified | Nasutitermes takasagoensis symbiont MNt2 | |
| Methanomicrobia | Methanomicrobiales | Methanomicrobiaceae | Unclassified | Pericapritermes nitobei symbiont MPn1 | |
| Methanomicrobia | Methanomicrobiales | Methanomicrobiaceae | Unclassified | Plagiopyla nasuta symbiont | |
| Methanomicrobia | Methanomicrobiales | Methanomicrobiaceae | Unclassified | methanogenic endosymbiont of Brachonella sp. | |
| Methanomicrobia | Methanomicrobiales | Methanomicrobiaceae | Unclassified | methanogenic endosymbiont of Caenomorpha sp. | |
| Methanomicrobia | Methanomicrobiales | Methanomicrobiaceae | Unclassified | methanogenic endosymbiont of Caenomorpha sp. 10 | |
| Methanomicrobia | Methanomicrobiales | Methanomicrobiaceae | Unclassified | methanogenic endosymbiont of Caenomorpha sp. 2 | |
| Methanomicrobia | Methanomicrobiales | Methanomicrobiaceae | Unclassified | methanogenic endosymbiont of Caenomorpha-like sp. 1 | |
| Methanomicrobia | Methanomicrobiales | Methanomicrobiaceae | Unclassified | methanogenic endosymbiont of Caenomorpha-like sp. 4 | |
| Methanomicrobia | Methanomicrobiales | Methanomicrobiaceae | Unclassified | methanogenic endosymbiont of Caenomorpha-like sp. 8 | |
| Methanomicrobia | Methanomicrobiales | Methanomicrobiaceae | Unclassified | uncultured archaeon ACE4_A | |
| Methanomicrobia | Methanomicrobiales | Methanomicrobiaceae | Unclassified | uncultured archaeon Ar27 | |
| Methanomicrobia | Methanomicrobiales | Methanomicrobiaceae | Unclassified | uncultured archaeon Ar32 | |
| Methanomicrobia | Methanomicrobiales | Methanomicrobiaceae | Unclassified | uncultured archaeon BURTON24_ A | |
| Methanomicrobia | Methanomicrobiales | Methanomicrobiaceae | Unclassified | uncultured Methanomicrobiaceae archaeon | |
| Methanomicrobia | Methanomicrobiales | Methanomicrobiaceae | Unclassified | uncultured sheep rumen methanogen clone 10 | |
| Methanomicrobia | Methanomicrobiales | Methanomicrobiaceae | Unclassified | uncultured sheep rumen methanogen clone 12 | |
| Methanomicrobia | Methanomicrobiales | Methanomicrobiaceae | Unclassified | uncultured sheep rumen methanogen clone 19 | |
| Methanomicrobia | Methanomicrobiales | Methanomicrobiaceae | Unclassified | uncultured sheep rumen methanogen clone 20 | |
| Methanomicrobia | Methanomicrobiales | Methanomicrobiaceae | Unclassified | uncultured sheep rumen methanogen clone 22 | |
| Methanomicrobia | Methanomicrobiales | Methanomicrobiaceae | Unclassified | uncultured sheep rumen methanogen clone 31 | |
| Methanomicrobia | Methanomicrobiales | Methanomicrobiaceae | Unclassified | uncultured sheep rumen methanogen clone 34 | |
| Methanomicrobia | Methanomicrobiales | Methanomicrobiaceae | Unclassified | uncultured sheep rumen methanogen clone 36 | |
| Methanomicrobia | Methanomicrobiales | Methanomicrobiaceae | Unclassified | uncultured sheep rumen methanogen clone 38 | |
| Methanomicrobia | Methanomicrobiales | Methanomicrobiaceae | Unclassified | uncultured sheep rumen methanogen clone 41 | |
| Methanomicrobia | Methanomicrobiales | Methanomicrobiaceae | Unclassified | uncultured sheep rumen methanogen clone 46 | |
| Methanomicrobia | Methanomicrobiales | Methanomicrobiaceae | Unclassified | uncultured sheep rumen methanogen clone 47 | |
| Methanomicrobia | Methanomicrobiales | Methanomicrobiaceae | Unclassified | uncultured sheep rumen methanogen clone 48 | |
| Methanomicrobia | Methanomicrobiales | Methanomicrobiaceae | Unclassified | uncultured sheep rumen | |
| | | | | methanogen clone 49 | |
| Methanomicrobia | Methanomicrobiales | Methanomicrobiaceae | Unclassified | uncultured sheep rumen methanogen clone 52 | |
| Methanomicrobia | Methanomicrobiales | Methanomicrobiaceae | Unclassified | uncultured sheep rumen methanogen clone 55 | |
| Methanomicrobia | Methanomicrobiales | Methanomicrobiaceae | Unclassified | uncultured sheep rumen methanogen clone 57 | |
| Methanomicrobia | Methanomicrobiales | Methanomicrobiaceae | Unclassified | uncultured sheep rumen methanogen clone 58 | |
| Methanomicrobia | Methanomicrobiales | Methanomicrobiaceae | Unclassified | uncultured sheep rumen methanogen clone 60 | |
| Methanomicrobia | Methanomicrobiales | Methanomicrobiaceae | Unclassified | uncultured sheep rumen methanogen clone 9 | |
| Methanomicrobia | Methanomicrobiales | Methanomicrobiaceae | Methanospirillum | hungatei | |
| Methanomicrobia | Methanomicrobiales | Methanomicrobiaceae | Methanospirillum | Unclassified | |
| Methanomicrobia | Methanomicrobiales | Methanomicrobiaceae | Methanospirillum | sp. K18-1 | |
| Methanomicrobia | Methanomicrobiales | Methanomicrobiaceae | Methanospirillum | sp. TM20-1 | |
| Methanomicrobia | Methanomicrobiales | Methanomicrobiaceae | Methanospirillum | sp. enrichment culture clone D2CL_Arch_16S_clone2A | |
| Methanomicrobia | Methanomicrobiales | Methanomicrobiaceae | Methanospirillum | sp. enrichment culture clone D2CL_Arch_16S_clone2B | |
| Methanomicrobia | Methanomicrobiales | Methanomicrobiaceae | Methanospirillum | sp. enrichment culture clone D2CL_ mvrD_ Clone1 | |
| Methanomicrobia | Methanomicrobiales | Methanomicrobiaceae | Methanospirillum | Unclassified | 262503 |
| Methanomicrobia | Methanomicrobiales | Methanomicrobiaceae | Methanospirillum | Unclassified | 346907 |
| Methanomicrobia | Methanomicrobiales | Methanomicrobiaceae | Methanomicrobium | Methanomicrobium mobile | |
| Methanomicrobia | Methanomicrobiales | Methanomicrobiaceae | Methanomicrobium | Methanobacterium sp. enrichment culture clone MBT-1 | |
| Methanomicrobia | Methanomicrobiales | Methanomicrobiaceae | Methanomicrobium | Methanobacterium sp. enrichment culture clone MBT-10 | |
| Methanomicrobia | Methanomicrobiales | Methanomicrobiaceae | Methanomicrobium | Methanobacterium sp. enrichment culture clone MBT-12 | |
| Methanomicrobia | Methanomicrobiales | Methanomicrobiaceae | Methanomicrobium | Methanobacterium sp. enrichment culture clone MBT-2 | |
| Methanomicrobia | Methanomicrobiales | Methanomicrobiaceae | Methanomicrobium | Methanobacterium sp. enrichment culture clone MBT-3 | |
| Methanomicrobia | Methanomicrobiales | Methanomicrobiaceae | Methanomicrobium | Methanobacterium sp. enrichment culture clone MBT-5 | |
| Methanomicrobia | Methanomicrobiales | Methanomicrobiaceae | Methanomicrobium | Methanobacterium sp. enrichment culture clone MBT-6 | |
| Methanomicrobia | Methanomicrobiales | Methanomicrobiaceae | Methanomicrobium | Methanobacterium sp. enrichment culture clone MBT-7 | |
| Methanomicrobia | Methanomicrobiales | Methanomicrobiaceae | Methanomicrobium | Methanobacterium sp. enrichment culture clone MBT-8 | |
| Methanomicrobia | Methanomicrobiales | Methanomicrobiaceae | Methanomicrobium | Methanobacterium sp. enrichment culture clone MBT-9 | |
| Methanomicrobia | Methanomicrobiales | Methanomicrobiaceae | Methanomicrobium | Methanomicrobium sp. enrichment culture clone MBT-4 | |
| Methanomicrobia | Methanomicrobiales | Methanomicrobiaceae | Methanomicrobium | uncultured Methanomicrobium sp. | |
| Methanomicrobia | Methanomicrobiales | Methanomicrobiaceae | Methanolacinia | Methanolacinia paynteri | |
| Methanopyri | Methanopyrales | Methanopyraceae | | uncultured Methanopyrales archaeon | 345629 |
| Thermococci | Thermococcales | Thermococcaceae | Palaeococcus | ferrophilus | |
| Thermococci | Thermococcales | Thermococcaceae | Palaeococcus | Helgesonii | |
| Thermococci | Thermococcales | Thermococcaceae | Palaeococcus | Sp. Ax00-33 | |
| Thermococci | Thermococcales | Thermococcaceae | Pyrococcus | abyssi | |
| Thermococci | Thermococcales | Thermococcaceae | Pyrococcus | Furiosus | |
| Thermococci | Thermococcales | Thermococcaceae | Pyrococcus | Glycovorans | |
| Thermococci | Thermococcales | Thermococcaceae | Pyrococcus | Horikoshii | |
| Thermococci | Thermococcales | Thermococcaceae | Pyrococcus | Pyrococcus woesei | |
| | | | | Pyrococcus sp. | |
| | | | | Pyrococcus sp. 12/1 | |
| | | | | Pyrococcus sp. 121 | |
| | | | | Pyrococcus sp. 303 | |
| | | | | Pyrococcus sp. 304 | |
| | | | | Pyrococcus sp. 312 | |
| | | | | Pyrococcus sp. 32-4 | |
| | | | | Pyrococcus sp. 321 | |
| | | | | Pyrococcus sp. 322 | |
| | | | | Pyrococcus sp. 323 | |
| | | | | Pyrococcus sp. 324 | |
| | | | | Pyrococcus sp. 95-12-1 | |
| | | | | Pyrococcus sp. AV5 | |
| | | | | Pyrococcus sp. Ax99-7 | |
| | | | | Pyrococcus sp. C2 | |
| | | | | Pyrococcus sp. CH1 | |
| | | | | Pyrococcus sp. ES4 | |
| | | | | Pyrococcus sp. EX2 | |
| | | | | Pyrococcus sp. FIa95-Pc | |
| | | | | Pyrococcus sp. GB-3A | |
| | | | | Pyrococcus sp. GB-D | |
| | | | | Pyrococcus sp. GBD | |
| | | | | Pyrococcus sp. GI-H | |
| | | | | Pyrococcus sp. GI-J | |
| | | | | Pyrococcus sp. GIL | |
| | | | | Pyrococcus sp. HT3 | |
| | | | | Pyrococcus sp. JT1 | |
| | | | | Pyrococcus sp. MA2.31 | |
| | | | | Pyrococcus sp. MA2.32 | |
| | | | | Pyrococcus sp. MA2.34 | |
| | | | | Pyrococcus sp. MV1019 | |
| | | | | Pyrococcus sp. MV4 | |
| | | | | Pyrococcus sp. MV7 | |
| | | | | Pyrococcus sp. MZ14 | |
| | | | | Pyrococcus sp. MZ4 | |
| | | | | Pyrococcus sp. NA2 | |
| | | | | Pyrococcus sp. NS102-T | |
| | | | | Pyrococcus sp. Pikanate 5017 | |
| | | | | Pyrococcus sp. ST700 | |
| | | | | Pyrococcus sp. Tc-2-70 | |
| | | | | Pyrococcus sp. Tc95-7C-I | |
| | | | | Pyrococcus sp. TC95-7C-S | |
| | | | | Pyrococcus sp. Tc95_6 | |
| | | | | Pyrococcus sp. V211 | |
| | | | | Pyrococcus sp. V212 | |
| | | | | Pyrococcus sp. V221 | |
| | | | | Pyrococcus sp. V222 | |
| | | | | Pyrococcus sp. V231 | |
| | | | | Pyrococcus sp. V232 | |
| | | | | Pyrococcus sp. V61 | |
| | | | | Pyrococcus sp. V62 | |
| | | | | Pyrococcus sp. V63 | |
| | | | | Pyrococcus sp. V72 | |
| | | | | Pyrococcus sp. V73 | |
| | | | | Pyrococcus sp. VB112 | |
| | | | | Pyrococcus sp. VB113 | |
| | | | | Pyrococcus sp. VB81 | |
| | | | | Pyrococcus sp. VB82 | |
| | | | | Pyrococcus sp. VB83 | |
| | | | | Pyrococcus sp. VB85 | |
| | | | | Pyrococcus sp. VB86 | |
| | | | | Pyrococcus sp. VB93 | |
| | | | Thermococcus | Thermococcus acidaminovorans | |
| | | | | Thermococcus aegaeus | |
| | | | | Thermococcus aggregans | |
| | | | | Thermococcus alcaliphilus | |
| | | | | Thermococcus atlanticus | |
| | | | | Thermococcus barophilus | |
| | | | | Thermococcus barophilus MP | |
| | | | | Thermococcus barossii | |
| | | | | Thermococcus celer | |
| | | | | Thermococcus celericrescens | |
| | | | | Thermococcus chitonophagus | |
| | | | | Thermococcus coalescens | |
| | | | | Thermococcus fumicolans | |
| | | | | Thermococcus gammatolerans | |
| | | | | Thermococcus gammatolerans EJ3 | |
| | | | | Thermococcus gorgonarius | |
| | | | | Thermococcus guaymasensis | |
| | | | | Thermococcus hydrothermalis | |
| | | | | Thermococcus kodakarensis | |
| | | | | Thermococcus kodakarensis KOD1 | |
| | | | | Thermococcus litoralis | |
| | | | | Thermococcus litoralis DSM 5473 | |
| | | | | Thermococcus marinus | |
| | | | | Thermococcus mexicalis | |
| | | | | Thermococcus nautilus | |
| | | | | Thermococcus onnurineus | |
| | | | | Thermococcus onnurineus NA1 | |
| | | | | Thermococcus pacificus | |
| | | | | Thermococcus peptonophilus | |
| | | | | Thermococcus peptonophilus JCM 9653 | |
| | | | | Thermococcus profundus | |
| | | | | Thermococcus radiotolerans | |
| | | | | Thermococcus sibiricus | |
| | | | | Thermococcus sibiricus MM 739 | |
| | | | | Thermococcus siculi | |
| | | | | Thermococcus stetteri | |
| | | | | Thermococcus thioreducens | |
| | | | | Thermococcus waimanguensis | |
| | | | | Thermococcus waiotapuensis | |
| | | | | Thermococcus zilligii | |
| | | | | Thermococcus sp. | |
| | | | | Thermococcus sp. 'AEPII 1a' | |
| | | | | Thermococcus sp. 'Bio pl 0405IT2' | |
| | | | | Thermococcus sp. 11N.A5 | |
| | | | | Thermococcus sp. 12-4 | |
| | | | | Thermococcus sp. 13-2 | |
| | | | | Thermococcus sp. 13-3 | |
| | | | | Thermococcus sp. 1519 | |
| | | | | Thermococcus sp. 21-1 | |
| | | | | Thermococcus sp. 23-1 | |
| | | | | Thermococcus sp. 23-2 | |
| | | | | Thermococcus sp. 26-2 | |
| | | | | Thermococcus sp. 26-3 | |
| | | | | Thermococcus sp. 26/2 | |
| | | | | Thermococcus sp. 28-1 | |
| | | | | Thermococcus sp. 29-1 | |
| | | | | Thermococcus sp. 300-Tc | |
| | | | | Thermococcus sp. 31-1 | |
| | | | | Thermococcus sp. 31-3 | |
| | | | | Thermococcus sp. 5-1 | |
| | | | | Thermococcus sp. 70-4-2 | |
| | | | | Thermococcus sp. 83-5-2 | |
| | | | | Thermococcus sp. 9N2 | |
| | | | | Thermococcus sp. 9N2.20 | |
| | | | | Thermococcus sp. 9N2.21 | |
| | | | | Thermococcus sp. 9N3 | |
| | | | | Thermococcus sp. 9oN-7 | |
| | | | | Thermococcus sp. A4 | |
| | | | | Thermococcus sp. AF1T14.13 | |
| | | | | Thermococcus sp. AF1T1423 | |
| | | | | Thermococcus sp. AF1T20.11 | |
| | | | | Thermococcus sp. AF1T6.10 | |
| | | | | Thermococcus sp. AF1T6.12 | |
| | | | | Thermococcus sp. AF1T6.63 | |
| | | | | Thermococcus sp. AF2T511 | |
| | | | | Thermococcus sp. Ag85-vw | |
| | | | | Thermococcus sp. AM4 | |
| | | | | Thermococcus sp. AMT11 | |
| | | | | Thermococcus sp. Anhete70-I78 | |
| | | | | Thermococcus sp. Anhete70-SCI | |
| | | | | Thermococcus sp. Anhete85-I78 | |
| | | | | Thermococcus sp. Anhete85-SCI | |
| | | | | Thermococcus sp. AT1273 | |
| | | | | Thermococcus sp. Ax00-17 | |
| | | | | Thermococcus sp. Ax00-27 | |
| | | | | Thermococcus sp. Ax00-39 | |
| | | | | Thermococcus sp. Ax00-45 | |
| | | | | Thermococcus sp. Ax01-2 | |
| | | | | Thermococcus sp. Ax01-3 | |
| | | | | Thermococcus sp. Ax01-37 | |
| | | | | Thermococcus sp. Ax01-39 | |
| | | | | Thermococcus sp. Ax01-61 | |
| | | | | Thermococcus sp. Ax01-62 | |
| | | | | Thermococcus sp. Ax01-65 | |
| | | | | Thermococcus sp. Ax98-43 | |
| | | | | Thermococcus sp. Ax98-46 | |
| | | | | Thermococcus sp. Ax98-48 | |
| | | | | Thermococcus sp. Ax99-47 | |
| | | | | Thermococcus sp. Ax99-57 | |
| | | | | Thermococcus sp. Ax99-67 | |
| | | | | Thermococcus sp. B1 | |
| | | | | Thermococcus sp. B1001 | |
| | | | | Thermococcus sp. B4 | |
| | | | | Thermococcus sp. BHI60a21 | |
| | | | | Thermococcus sp. BHI80a28 | |
| | | | | Thermococcus sp. BHI80a40 | |
| | | | | Thermococcus sp. CAR-80 | |
| | | | | Thermococcus sp. CKU-1 | |
| | | | | Thermococcus sp. CKU-199 | |
| | | | | Thermococcus sp. CL1 | |
| | | | | Thermococcus sp. CL2 | |
| | | | | Thermococcus sp. CMI | |
| | | | | Thermococcus sp. CNR-5 | |
| | | | | Thermococcus sp. CX1 | |
| | | | | Thermococcus sp. CX2 | |
| | | | | Thermococcus sp. CX3 | |
| | | | | Thermococcus sp. CX4 | |
| | | | | Thermococcus sp. CYA | |
| | | | | Thermococcus sp. Dex80a71 | |
| | | | | Thermococcus sp. Dex80a75 | |
| | | | | Thermococcus sp. ES1 | |
| | | | | Thermococcus sp. Fe85_1_2 | |
| | | | | Thermococcus sp. GB18 | |
| | | | | Thermococcus sp. GB20 | |
| | | | | Thermococcus sp. GE8 | |
| | | | | Thermococcus sp. Gorda2 | |
| | | | | Thermococcus sp. Gorda3 | |
| | | | | Thermococcus sp. Gorda4 | |
| | | | | Thermococcus sp. Gorda5 | |
| | | | | Thermococcus sp. Gorda6 | |
| | | | | Thermococcus sp. GT | |
| | | | | Thermococcus sp. GU5L5 | |
| | | | | Thermococcus sp. HJ21 | |
| | | | | Thermococcus sp. JDF-3 | |
| | | | | Thermococcus sp. KI | |
| | | | | Thermococcus sp. KS-1 | |
| | | | | Thermococcus sp. KS-8 | |
| | | | | Thermococcus sp. MA2.27 | |
| | | | | Thermococcus sp. MA2.28 | |
| | | | | Thermococcus sp. MA2.29 | |
| | | | | Thermococcus sp. MA2.33 | |
| | | | | Thermococcus sp. MV1031 | |
| | | | | Thermococcus sp. MV1049 | |
| | | | | Thermococcus sp. MV1083 | |
| | | | | Thermococcus sp. MV1092 | |
| | | | | Thermococcus sp. MV1099 | |
| | | | | Thermococcus sp. MZ1 | |
| | | | | Thermococcus sp. MZ10 | |
| | | | | Thermococcus sp. MZ11 | |
| | | | | Thermococcus sp. MZ12 | |
| | | | | Thermococcus sp. MZ13 | |
| | | | | Thermococcus sp. MZ2 | |
| | | | | Thermococcus sp. MZ3 | |
| | | | | Thermococcus sp. MZ5 | |
| | | | | Thermococcus sp. MZ6 | |
| | | | | Thermococcus sp. MZ8 | |
| | | | | Thermococcus sp. MZ9 | |
| | | | | Thermococcus sp. NS85-T | |
| | | | | Thermococcus sp. P6 | |
| | | | | Thermococcus sp. Pd70 | |
| | | | | Thermococcus sp. Pd85 | |
| | | | | Thermococcus sp. Rt3 | |
| | | | | Thermococcus sp. SB611 | |
| | | | | Thermococcus sp. SN531 | |
| | | | | Thermococcus sp. SRB55_1 | |
| | | | | Thermococcus sp. SRB70_1 | |
| | | | | Thermococcus sp. SRB70_10 | |
| | | | | Thermococcus sp. Tc-1-70 | |
| | | | | Thermococcus sp. Tc-1-85 | |
| | | | | Thermococcus sp. Tc-1-95 | |
| | | | | Thermococcus sp. Tc-2-85 | |
| | | | | Thermococcus sp. Tc-2-95 | |
| | | | | Thermococcus sp. Tc-365-70 | |
| | | | | Thermococcus sp. Tc-365-85 | |
| | | | | Thermococcus sp. Tc-365-95 | |
| | | | | Thermococcus sp. Tc-4-70 | |
| | | | | Thermococcus sp. Tc-4-85 | |
| | | | | Thermococcus sp. Tc-I-70 | |
| | | | | Thermococcus sp. Tc-I-85 | |
| | | | | Thermococcus sp. Tc-S-70 | |
| | | | | Thermococcus sp. Tc-S-85 | |
| | | | | Thermococcus sp. Tc55_1 | |
| | | | | Thermococcus sp. Tc55_12 | |
| | | | | Thermococcus sp. Tc70-4C-I | |
| | | | | Thermococcus sp. Tc70-4C-S | |
| | | | | Thermococcus sp. Tc70-7C-I | |
| | | | | Thermococcus sp. Tc70-7C-S | |
| | | | | Thermococcus sp. Tc70-CRC-I | |
| | | | | Thermococcus sp. Tc70-CRC-S | |
| | | | | Thermococcus sp. Tc70-MC-S | |
| | | | | Thermococcus sp. Tc70-SC-1 | |
| | | | | Thermococcus sp. Tc70-SC-S | |
| | | | | Thermococcus sp. Tc70-vw | |
| | | | | Thermococcus sp. Tc70_1 | |
| | | | | Thermococcus sp. Tc70_10 | |
| | | | | Thermococcus sp. Tc70_11 | |
| | | | | Thermococcus sp. Tc70_12 | |
| | | | | Thermococcus sp. Tc70_20 | |
| | | | | Thermococcus sp. Tc70_6 | |
| | | | | Thermococcus sp. Tc70_9 | |
| | | | | Thermococcus sp. Tc85-0 age SC | |
| | | | | Thermococcus sp. Tc85-4C-I | |
| | | | | Thermococcus sp. Tc85-4C-S | |
| | | | | Thermococcus sp. Tc85-7C-S | |
| | | | | Thermococcus sp. Tc85-CRC-I | |
| | | | | Thermococcus sp. Tc85-CRC-S | |
| | | | | Thermococcus sp. Tc85-MC-I | |
| | | | | Thermococcus sp. Tc85-MC-S | |
| | | | | Thermococcus sp. Tc85-SC-I | |
| | | | | Thermococcus sp. Tc85-SC-ISCS | |
| | | | | Thermococcus sp. Tc85-SC-S | |
| | | | | Thermococcus sp. Tc85_1 | |
| | | | | Thermococcus sp. Tc85_10 | |
| | | | | Thermococcus sp. Tc85_11 | |
| | | | | Thermococcus sp. Tc85_12 | |
| | | | | Thermococcus sp. Tc85_13 | |
| | | | | Thermococcus sp. Tc85_19 | |
| | | | | Thermococcus sp. Tc85_2 | |
| | | | | Thermococcus sp. Tc85_20 | |
| | | | | Thermococcus sp. Tc85_9 | |
| | | | | Thermococcus sp. Tc95-CRC-I | |
| | | | | Thermococcus sp. Tc95-CRC-S | |
| | | | | Thermococcus sp. Tc95-MC-1 | |
| | | | | Thermococcus sp. Tc95-MC-S | |
| | | | | Thermococcus sp. Tc95-SC-S | |
| | | | | Thermococcus sp. TK1 | |
| | | | | Thermococcus sp. TM1 | |
| | | | | Thermococcus sp. TS3 | |
| | | | | Thermococcus sp. vp197 | |
| | | | | environmental samples | |
| | | | | Thermococcus sp. enrichment clone SA3 | |
| | | | | uncultured Thermococcus sp. | |
| Thermoplasmata | Thermoplasmatales | Ferroplasmaceae | Acidiplasma | aeolicum | 507754 |
| | | Ferroplasmaceae | Ferroplasma | acidarmanus | |
| | | Ferroplasmaceae | Ferroplasma | acidiphilum | |
| | | Ferroplasmaceae | Ferroplasma | Cupricumulans | |
| | | Ferroplasmaceae | Ferroplasma | Thermophilum | |
| | | Ferroplasmaceae | Ferroplasma | Sp. JTC3 | |
| | | Ferroplasmaceae | Ferroplasma | Sp. clone E8A015 | |
| | | Ferroplasmaceae | Ferroplasma | Sp. Type II | |
| | | Ferroplasmaceae | Ferroplasma | Uncultured Ferroplasma sp. | |
| | | Picrophilaceae | Picrophilus | Oshimae | |
| | | Picrophilaceae | Picrophilus | torrid us | |
| | | Thermoplasmataceae | Thermoplasmataceae | Acidophilum | |
| | | Thermoplasmataceae | Thermoplasmataceae | Volcanium | |
| | | Thermoplasmataceae | Thermoplasmataceae | Sp. 67.1 | |
| | | Thermoplasmataceae | Thermoplasmataceae | Sp. P61 | |
| | | Thermoplasmataceae | Thermoplasmataceae | Sp. S01 | |
| | | Thermoplasmataceae | Thermoplasmataceae | Sp. S02 | |
| | | Thermoplasmataceae | Thermoplasmataceae | Sp. Xt101 | |
| | | Thermoplasmataceae | Thermoplasmataceae | Xt102 | |
| | | Thermoplasmataceae | Thermoplasmataceae | XT103 | |
| | | Thermoplasmataceae | Thermoplasmataceae | XT107 | |
| | | Thermoplasmataceae | Thermogymnomonas | acidicola | |
| unclassified | unclassified | unclassified | unclassified | uncultured SA2 group euryarchaeote uncultured SA1 group euryarchaeote | |
| | | | | uncultured marine euryarchaeote DH148-Y15 | |
| | | | | uncultured marine euryarchaeote DH148-Y16 | |
| | | | | uncultured marine euryarchaeote DH148-Y19 | |
| | | | | uncultured marine euryarchaeote DH148-Y2 | |
| | | | | uncultured marine euryarchaeote DH148-Y4 | |
| | | | | uncultured marine euryarchaeote DH148-Z1 | |
| | | | | uncultured marine group IV euryarchaeote | |
| | | | | uncultured marine group III euryarchaeote | |
| | | | | uncultured marine group III euryarchaeote AD1000-40-D7 | |
| | | | | uncultured marine group III euryarchaeote HF10_21C05 | |
| | | | | uncultured marine group III euryarchaeote HF130_ 43E12 | |
| | | | | uncultured marine group III euryarchaeote HF130_95B02 | |
| | | | | uncultured marine group III euryarchaeote HF200_25B11 | |
| | | | | uncultured marine group III euryarchaeote HF500_17G02 | |
| | | | | uncultured marine group III euryarchaeote KM3-28-E8 | |
| | | | | uncultured marine group III euryarchaeote SAT1000-53-B3 | |
| | | | | uncultured marine group II euryarchaeote | |
| | | | | uncultured marine group II euryarchaeote 37F11 | |
| | | | | uncultured marine group II euryarchaeote AD1000-18-D2 | |
| | | | | uncultured marine group II euryarchaeote DeepAnt-15E7 | |
| | | | | uncultured marine group II euryarchaeote DeepAnt-JyKC7 | |
| | | | | uncultured marine group II euryarchaeote EF1 00_57A08 | |
| | | | | uncultured marine group II euryarchaeote HF10_15F03 | |
| | | | | uncultured marine group II euryarchaeote HF10_15F05 | |
| | | | | uncultured marine group II euryarchaeote HF10_15G04 | |
| | | | | uncultured marine group II euryarchaeote HF10_20F02 | |
| | | | | uncultured marine group II euryarchaeote HF10_24E03 | |
| | | | | uncultured marine group II euryarchaeote HF10_25H10 | |
| | | | | uncultured marine group II euryarchaeote HF10_27F06 | |
| | | | | uncultured marine group II euryarchaeote HF10_28B09 | |
| | | | | uncultured marine group II euryarchaeote HF10_29E05 | |
| | | | | uncultured marine group II euryarchaeote HF10_30E08 | |
| | | | | uncultured marine group II euryarchaeote HF10_30F11 | |
| | | | | uncultured marine group II euryarchaeote HF10_35F06 | |
| | | | | uncultured marine group II euryarchaeote HF10_36B02 | |
| | | | | uncultured marine group II euryarchaeote HF10_39E10 | |
| | | | | uncultured marine group II | |
| | | | | euryarchaeote HF10_43A09 | |
| | | | | uncultured marine group II euryarchaeote HF10_48G07 | |
| | | | | uncultured marine group II euryarchaeote HF10_53B05 | |
| | | | | uncultured marine group II euryarchaeote HF10_61D03 | |
| | | | | uncultured marine group II euryarchaeote HF10_65D04 | |
| | | | | uncultured marine group II euryarchaeote HF10_73E12 | |
| | | | | uncultured marine group II euryarchaeote HF10_8H07 | |
| | | | | uncultured marine group II euryarchaeote HF10_90C09 | |
| | | | | uncultured marine group II euryarchaeote HF130_17B12 | |
| | | | | uncultured marine group II euryarchaeote HF130_17D07 | |
| | | | | uncultured marine group II euryarchaeote HF130_21B04 | |
| | | | | uncultured marine group II euryarchaeote HF130_26G06 | |
| | | | | uncultured marine group II euryarchaeote HF130_27A09 | |
| | | | | uncultured marine group II euryarchaeote HF130_28F07 | |
| | | | | uncultured marine group II euryarchaeote HF130_29F10 | |
| | | | | uncultured marine group II euryarchaeote HF130_30F08 | |
| | | | | uncultured marine group II euryarchaeote HF130_31B11 | |
| | | | | uncultured marine group II euryarchaeote HF130_32D03 | |
| | | | | uncultured marine group II euryarchaeote HF130_33C02 | |
| | | | | uncultured marine group II euryarchaeote HF130_34E01 | |
| | | | | uncultured marine group II euryarchaeote HF130_35A10 | |
| | | | | uncultured marine group II euryarchaeote HF130_37H07 | |
| | | | | uncultured marine group II euryarchaeote HF130_40B01 | |
| | | | | uncultured marine group II euryarchaeote HF130_40B02 | |
| | | | | uncultured marine group II euryarchaeote HF130_ 40G07 | |
| | | | | uncultured marine group II euryarchaeote HF130_ 40G09 | |
| | | | | uncultured marine group II euryarchaeote HF130_44A02 | |
| | | | | uncultured marine group II euryarchaeote HF130_49F04 | |
| | | | | uncultured marine group II euryarchaeote HF130_4G08 | |
| | | | | uncultured marine group II euryarchaeote HF130_56E12 | |
| | | | | uncultured marine group II euryarchaeote HF130_67F08 | |
| | | | | uncultured marine group II euryarchaeote HF130_6E07 | |
| | | | | uncultured marine group II euryarchaeote HF130_71B05 | |
| | | | | uncultured marine group II euryarchaeote HF130_73G01 | |
| | | | | uncultured marine group II euryarchaeote HF130_75B06 | |
| | | | | uncultured marine group II euryarchaeote HF130_76G06 | |
| | | | | uncultured marine group II euryarchaeote HF130 83E02 | |
| | | | | uncultured marine group II euryarchaeote HF130_88G10 | |
| | | | | uncultured marine group II euryarchaeote HF130_90E09 | |
| | | | | uncultured marine group II euryarchaeote HF130_94A03 | |
| | | | | uncultured marine group II euryarchaeote HF200_101H01 | |
| | | | | uncultured marine group II euryarchaeote HF200_102F03 | |
| | | | | uncultured marine group II euryarchaeote HF200_103E03 | |
| | | | | uncultured marine group II euryarchaeote HF200_15F06 | |
| | | | | uncultured marine group II euryarchaeote HF200_25F07 | |
| | | | | uncultured marine group II euryarchaeote HF200_35B05 | |
| | | | | uncultured marine group II euryarchaeote HF200_35E02 | |
| | | | | uncultured marine group II euryarchaeote HF200 _43D02 | |
| | | | | uncultured marine group II euryarchaeote HF200 _44E05 | |
| | | | | uncultured marine group II euryarchaeote HF200 _49H12 | |
| | | | | uncultured marine group II euryarchaeote HF200_50D06 | |
| | | | | uncultured marine group II euryarchaeote HF200_63E02 | |
| | | | | uncultured marine group II euryarchaeote HF200_64G08 | |
| | | | | uncultured marine group II euryarchaeote HF200_65H08 | |
| | | | | uncultured marine group II euryarchaeote HF200_66A10 | |
| | | | | uncultured marine group II euryarchaeote HF200_70E08 | |
| | | | | uncultured marine group II euryarchaeote HF200_71A04 | |
| | | | | uncultured marine group II euryarchaeote HF200_72A06 | |
| | | | | uncultured marine group II euryarchaeote HF200_78D05 | |
| | | | | uncultured marine group II euryarchaeote HF200_84A01 | |
| | | | | uncultured marine group II euryarchaeote HF200_89A11 | |
| | | | | uncultured marine group II euryarchaeote HF200_97B09 | |
| | | | | uncultured marine group II euryarchaeote HF500_100E05 | |
| | | | | uncultured marine group II euryarchaeote HF500_11 G07 | |
| | | | | uncultured marine group II euryarchaeote HF500_22F05 | |
| | | | | uncultured marine group II euryarchaeote HF500_24F01 | |
| | | | | uncultured marine group II euryarchaeote HF500_25E08 | |
| | | | | uncultured marine group II euryarchaeote HF500_26A05 | |
| | | | | uncultured marine group II euryarchaeote HF500_30A08 | |
| | | | | uncultured marine group II euryarchaeote HF500_ 47D04 | |
| | | | | uncultured marine group II euryarchaeote HF500_56B09 | |
| | | | | uncultured marine group II euryarchaeote HF500_58A11 | |
| | | | | uncultured marine group II euryarchaeote HF500_67F10 | |
| | | | | uncultured marine group II | |
| | | | | euryarchaeote HF70_105F02 | |
| | | | | uncultured marine group II euryarchaeote HF70_106D07 | |
| | | | | uncultured marine group II euryarchaeote HF70_14F12 | |
| | | | | uncultured marine group II euryarchaeote HF70_25A 12 | |
| | | | | uncultured marine group II euryarchaeote HF70_39H11 | |
| | | | | uncultured marine group II euryarchaeote HF70_ 41E01 | |
| | | | | uncultured marine group II euryarchaeote HF70_48A05 | |
| | | | | uncultured marine group II euryarchaeote HF70_48G03 | |
| | | | | uncultured marine group II euryarchaeote HF70_51 B02 | |
| | | | | uncultured marine group II euryarchaeote HF70_53G11 | |
| | | | | uncultured marine group II euryarchaeote HF70_59C08 | |
| | | | | uncultured marine group II euryarchaeote HF70_89B11 | |
| | | | | uncultured marine group II euryarchaeote HF70_91 G08 | |
| | | | | uncultured marine group II euryarchaeote HF70_95E04 | |
| | | | | uncultured marine group II euryarchaeote HF70_97E04 | |
| | | | | uncultured marine group II euryarchaeote KM3-130-D10 | |
| | | | | uncultured marine group II euryarchaeote KM3-136-D10 | |
| | | | | uncultured marine group II euryarchaeote KM3-72-G3 | |
| | | | | uncultured marine group II euryarchaeote KM3-85-F5 | |
| | | | | uncultured marine group II euryarchaeote SAT1000-15-B12 | |
| | | | | uncultured archaeon ACE-6 | |
| | | | | uncultured archaeon BURTON-41 | |
| | | | | uncultured archaeon BURTON-47 | |
| | | | | uncultured archaeon CLEAR-15 | |
| | | | | uncultured archaeon CLEAR-24 | |
| | | | | uncultured archaeon PENDANT-17 | |
| | | | | uncultured archaeon PENDANT-33 | |
| | | | | euryarchaeote J3.25-8 | |
| | | | | euryarchaeote D4.75-18 115532 | |
| | | | | euryarchaeote D4.75-4 | |
| | | | | euryarchaeote DJ3.25-13 | |
| | | | | euryarchaeote J4.75-12 | |
| | | | | euryarchaeote J4.75-15 | |
| | | | | euryarchaeote J4.75-24 | |
| | | | | euryarchaeote SvA99MeOH | |
| | | | | euryarchaeote SvA99TMA | |
| | | | | methanogenic archaeon CH1270 | |
| | | | | methanogenic archaeon F1/B-1 | |
| | | | | methanogenic archaeon F1/B-2 | |
| | | | | methanogenic archaeon F1/B-3 | |
| | | | | methanogenic archaeon F1/P-1 | |
| | | | | methanogenic archaeon F1/P-2 | |
| | | | | methanogenic archaeon F1/P-3 | |
| | | | | methanogenic archaeon F4/B-1 | |
| | | | | methanogenic archaeon F4/B-2 | |
| | | | | methanogenic archaeon F4/B-3 | |
| | | | | methanogenic archaeon F4/P-1 | |
| | | | | methanogenic archaeon F4/P-2 | |
| | | | | methanogenic archaeon F4/P-3 | |
| | | | | methanogenic archaeon U3/B-1 | |
| | | | | methanogenic archaeon U3/P-1 | |
| | | | | methanogen sp. | |

**TABLE 2**

| Name of Unclassified Species | Taxonomy ID No. |
|---|---|
| euryarchaeote enrichment culture clone BAMC-3 | 679265 |
| euryarchaeote enrichment culture clone MST-5 | 645575 |
| euryarchaeote enrichment culture clone T-RF243d | 670234 |
| euryarchaeote enrichment culture clone T-RF243e | 670235 |
| euryarchaeote enrichment culture clone T-RF259 | 670236 |
| planktonic euryarchaeote | 110443 |
| uncultured archaeon BA1a1 | 92881 |
| uncultured archaeon BA1a2 | 92882 |
| uncultured archaeon BA1b1 | 92883 |
| uncultured archaeon BA2e8 | 92884 |
| uncultured archaeon BA2F4fin | 92893 |
| uncultured archaeon BA2H11fin | 92894 |
| uncultured archaeon O23F7 | 114539 |
| uncultured archaeon TA1a4 | 92885 |
| uncultured archaeon TA1a6 | 92890 |
| uncultured archaeon TA1a9 | 92888 |
| uncultured archaeon TA1b12 | 92886 |
| uncultured archaeon TA1c9 | 92889 |
| uncultured archaeon TA1e6 | 92890 |
| uncultured archaeon TA1f2 | 92891 |
| uncultured archaeon TA2e12 | 92892 |
| uncultured archaeon WCHA1-57 | 74278 |
| uncultured archaeon WCHA2-08 | 74279 |
| uncultured archaeon WCHD3-02 | 74273 |
| uncultured archaeon WCHD3-07 | 74274 |
| uncultured archaeon WCHD3-16 | 74275 |
| uncultured archaeon WCHD3-30 | 74263 |
| uncultured archaeon WCHD3-33 | 74276 |
| uncultured archaeon WCHD3-34 | 74277 |
| uncultured euryarchaeote | 114243 |
| uncultured euryarchaeote a118ev | 117334 |
| uncultured euryarchaeote a50ev | 117335 |
| uncultured euryarchaeote a60ev | 117333 |
| uncultured euryarchaeote Alv-FOS1 | 337892 |
| uncultured euryarchaeote Alv-FOS4 | 337893 |
| uncultured euryarchaeote Alv-FOS5 | 337891 |
| uncultured euryarchaeote AM-20A_122 | 115055 |
| uncultured euryarchaeote AM-20A_123 | 115056 |
| uncultured euryarchaeote ARMAN-1 | 425594 |
| uncultured euryarchaeote AT-200_29 | 115057 |
| uncultured euryarchaeote AT-200_P25 | 115058 |
| uncultured euryarchaeote AT-5_21 | 115059 |
| uncultured euryarchaeote AT-5_P24 | 115060 |
| uncultured euryarchaeote CA-15_22 | 115061 |
| uncultured euryarchaeote CA-15_23 | 115062 |
| uncultured euryarchaeote CA-15_27 | 115063 |
| uncultured euryarchaeote CA-15_32 | 115064 |
| uncultured euryarchaeote CA-15_P4 | 115065 |
| uncultured euryarchaeote DF-5_21 | 115066 |
| uncultured euryarchaeote June4.75-16 | 134989 |
| uncultured euryarchaeote ME-450_21 | 115067 |
| uncultured euryarchaeote ME-450_30 | 115068 |
| uncultured euryarchaeote ME-450_38 | 115069 |
| uncultured euryarchaeote ME-450_P14 | 115070 |
| uncultured euryarchaeote ME-450_P9 | 115071 |
| uncultured euryarchaeote pBRKC101 | 91308 |
| uncultured euryarchaeote pBRKC112 | 91309 |
| uncultured euryarchaeote pBRKC128 | 91310 |
| uncultured euryarchaeote pBRKC134 | 91312 |
| uncultured euryarchaeote pBRKC22 | 91306 |
| uncultured euryarchaeote pBRKC84 | 91307 |
| uncultured euryarchaeote pBRKC91 | 91311 |
| uncultured euryarchaeote SB95-35 | 115072 |
| uncultured euryarchaeote SB95-48 | 115073 |
| uncultured euryarchaeote SB95-87 | 115074 |
| uncultured euryarchaeote VAL1 | 85383 |
| uncultured euryarchaeote VAL112 | 85386 |
| uncultured euryarchaeote VAL125 | 85387 |
| uncultured euryarchaeote VAL147 | 85395 |
| uncultured euryarchaeote VAL2 | 85384 |
| uncultured euryarchaeote VAL28 | 85394 |
| uncultured euryarchaeote VAL31-1 | 85396 |
| uncultured euryarchaeote VAL33-1 | 85397 |
| uncultured euryarchaeote VAL35-1 | 85398 |
| uncultured euryarchaeote VAL40 | 85392 |
| uncultured euryarchaeote VAL47 | 85388 |
| uncultured euryarchaeote VAL68 | 85385 |
| uncultured euryarchaeote VAL78 | 85389 |
| uncultured euryarchaeote VAL84 | 85390 |
| uncultured euryarchaeote VAL9 | 85393 |
| uncultured euryarchaeote VAL90 | 85391 |
| uncultured Green Bay ferromanganous micronodule archaeon ARB5 | 140616 |
| uncultured Green Bay ferromanganous micronodule archaeon ARC3 | 140617 |
| uncultured Green Bay ferromanganous micronodule archaeon ARF3 | 140614 |
| uncultured Green Bay ferromanganous micronodule archaeon ARG4 | 140615 |
| uncultured haloarchaeon MSP1 | 75449 |
| uncultured haloarchaeon MSP11 | 75452 |
| uncultured haloarchaeon MSP12 | 75453 |
| uncultured haloarchaeon MSP14 | 75454 |
| uncultured haloarchaeon MSP16 | 75455 |
| uncultured haloarchaeon MSP17 | 75456 |
| uncultured haloarchaeon MSP22 | 75457 |
| uncultured haloarchaeon MSP23 | 75458 |
| uncultured haloarchaeon MSP41 | 75459 |
| uncultured haloarchaeon MSP8 | 75450 |
| uncultured haloarchaeon MSP9 | 75451 |
| uncultured marine archaeon AEGEAN_50 | 147164 |
| uncultured marine archaeon AEGEAN_51 | 147166 |
| uncultured marine archaeon AEGEAN_52 | 147167 |
| uncultured marine archaeon AEGEAN_53 | 147168 |
| uncultured marine archaeon AEGEAN_54 | 147174 |
| uncultured marine archaeon AEGEAN_55 | 147169 |
| uncultured marine archaeon AEGEAN_57 | 147175 |
| uncultured marine archaeon AEGEAN_58 | 147176 |
| uncultured marine archaeon AEGEAN_59 | 147177 |
| uncultured marine archaeon AEGEAN_60 | 147178 |
| uncultured marine archaeon AEGEAN_62 | 147170 |
| uncultured marine archaeon AEGEAN_63 | 147171 |
| uncultured marine archaeon AEGEAN_64 | 147172 |
| uncultured marine archaeon AEGEAN_65 | 147173 |
| uncultured marine archaeon AEGEAN_66 | 147181 |
| uncultured marine archaeon AEGEAN_68 | 147179 |
| uncultured marine archaeon AEGEAN_71 | 147180 |
| uncultured marine archaeon AEGEAN_73 | 147182 |
| uncultured marine archaeon AEGEAN_74 | 147183 |
| uncultured marine euryarchaeote | 257466 |
| uncultured marine euryarchaeote dh148-A14 | 149701 |
| uncultured marine euryarchaeote dh148-A18 | 149702 |
| uncultured marine euryarchaeote dh148-A7 | 149700 |
| uncultured marine euryarchaeote DH148-W1 | 123945 |
| uncultured marine euryarchaeote dh148-W10 | 149705 |
| uncultured marine euryarchaeote dh148-W15 | 149706 |
| uncultured marine euryarchaeote dh148-W16 | 149707 |
| uncultured marine euryarchaeote dh148-W17 | 149708 |
| uncultured marine euryarchaeote dh148-W23 | 149709 |
| uncultured marine euryarchaeote DH148-W24 | 123946 |
| uncultured marine euryarchaeote dh148-W3 | 149703 |
| uncultured marine euryarchaeote dh148-W9 | 149704 |
| uncultured methanoqen CIBARC-1 | 153143 |
| uncultured methanoqen CRARC-3 | 153144 |
| uncultured methanoqen MRE-MCR1 | 143132 |
| uncultured methanoqen MRE-MCR2 | 143133 |
| uncultured methanoqen MRE-MCR3 | 143134 |
| uncultured methanoqen MRE-MCR4 | 143135 |
| uncultured methanoqen MRE-MCR5 | 143136 |
| uncultured methanoqen MRE-MCR6 | 143137 |
| uncultured methanoqen MRE-ME1 | 143138 |
| uncultured methanoqen MRE-ME3 | 143139 |
| uncultured methanoqen MRE-ME4 | 143140 |
| uncultured methanoqen MRE-ME5 | 143141 |
| uncultured methanoqen MRE01 | 143138 |
| uncultured methanoqen MRE02 | 143139 |
| uncultured methanoqen MRE03 | 143144 |
| uncultured methanoqen MRE04 | 143145 |
| uncultured methanoqen MRE05 | 143146 |
| uncultured methanoqen MRE06 | 143147 |
| uncultured methanoqen MRE07 | 143148 |
| uncultured methanoqen MRE09 | 143149 |
| uncultured methanoqen MRE10 | 143150 |
| uncultured methanoqen MRE11 | 143151 |
| uncultured methanoqen MRE12 | 143152 |
| uncultured methanoqen RS-MCR01 | 143109 |
| uncultured methanoqen RS-MCR04 | 143110 |
| uncultured methanoqen RS-MCR06 | 143111 |
| uncultured methanoqen RS-MCR08 | 143112 |
| uncultured methanoqen RS-MCR10 | 143113 |
| uncultured methanoqen RS-MCR15 | 143114 |
| uncultured methanoqen RS-MCR22 | 143115 |
| uncultured methanoqen RS-MCR25 | 143116 |
| uncultured methanoqen RS-MCR40 | 143117 |
| uncultured methanoqen RS-MCR41 | 143118 |
| uncultured methanoqen RS-MCR43 | 143119 |
| uncultured methanoqen RS-MCR46 | 143120 |
| uncultured methanoqen RS-ME11 | 143121 |
| uncultured methanoqen RS-ME19 | 143122 |
| uncultured methanoqen RS-ME22 | 143123 |
| uncultured methanoqen RS-ME24 | 143124 |
| uncultured methanoqen RS-ME26 | 143125 |
| uncultured methanoqen RS-ME30 | 143126 |
| uncultured methanoqen RS-ME32 | 143127 |
| uncultured methanoqen RS-ME33 | 143128 |
| uncultured methanoqen RS-ME34 | 143129 |
| uncultured methanoqen RS-ME39 | 143130 |
| uncultured methanoqen RS-ME49 | 143131 |
| uncultured methanoqen VIARC-0 | 153145 |
| uncultured methanoqen VIARC-4 | 153146 |
| uncultured methanoqenic archaeon | 198240 |
| uncultured methanogenic archaeon 'South African gold mine' | 260753 |
| uncultured methanoqenic archaeon RC-I | 351160 |
| uncultured methanoqenic symbiont PA101 | 161327 |
| uncultured methanoqenic symbiont PA102 | 161319 |
| uncultured methanoqenic symbiont PA103 | 161318 |
| uncultured methanoqenic symbiont PA104 | 161306 |
| uncultured methanoqenic symbiont PA105 | 161336 |
| uncultured methanoqenic symbiont PA112 | 161305 |
| uncultured methanoqenic symbiont PA114 | 161320 |
| uncultured methanoqenic symbiont PA119 | 161303 |
| uncultured methanoqenic symbiont PA123 | 161302 |
| uncultured methanoqenic symbiont PA124 | 161329 |
| uncultured methanoqenic symbiont PA127 | 161299 |
| uncultured methanoqenic symbiont PJ101 | 161314 |
| uncultured methanoqenic symbiont PJ102 | 161335 |
| uncultured methanoqenic symbiont PJ109 | 161315 |
| uncultured methanoqenic symbiont PJ118 | 161330 |
| uncultured methanoqenic symbiont ST102 | 161337 |
| uncultured methanoqenic symbiont ST103 | 161322 |
| uncultured methanoqenic symbiont ST104 | 161300 |
| uncultured methanoqenic symbiont ST105 | 161323 |
| uncultured methanoqenic symbiont ST107 | 161308 |
| uncultured methanoqenic symbiont ST109 | 161317 |
| uncultured methanoqenic symbiont ST111 | 161325 |
| uncultured methanoqenic symbiont ST113 | 161328 |
| uncultured methanoqenic symbiont ST117 | 161326 |
| uncultured methanoqenic symbiont ST126 | 161324 |
| uncultured methanoqenic symbiont ST129 | 161334 |
| uncultured methanoqenic symbiont ST131 | 161298 |
| uncultured methanoqenic symbiont ST140 | 161313 |
| uncultured methanoqenic symbiont ST143 | 161333 |
| uncultured methanoqenic symbiont ST144 | 161307 |
| uncultured methanoqenic symbiont ST152 | 161301 |
| uncultured methanoqenic symbiont ST153 | 161311 |
| uncultured methanoqenic symbiont ST154 | 161321 |
| uncultured methanoqenic symbiont ST155 | 161296 |
| uncultured methanoqenic symbiont ST157 | 161297 |
| uncultured methanoqenic symbiont ST158 | 161310 |
| uncultured methanoqenic symbiont ST159 | 161316 |
| uncultured methanoqenic symbiont ST162 | 161309 |
| uncultured methanoqenic symbiont ST164 | 161304 |
| uncultured methanoqenic symbiont ST165 | 161312 |
| uncultured methanoqenic symbiont ST167 | 161332 |
| uncultured methanoqenic symbiont ST168 | 161331 |
| unidentified euryarchaeote | 29293 |
| unidentified methanoqen ARC31 | 68396 |
| unidentified methanoqen ARC45 | 68397 |
| unidentified methanoqen ARC46 | 68398 |
| unidentified methanoqen ARC63 | 68399 |
| unidentified methanoqen ARC64 | 68400 |
| unidentified methanoqen ARC9 | 68395 |

**TABLE 3**

| Name of Unclassified Species | Taxonomy ID |
|---|---|
| anaerobic methanoqenic archaeon E15-1 | 93517 |
| anaerobic methanoqenic archaeon E15-10 | 93526 |
| anaerobic methanoqenic archaeon E15-2 | 93518 |
| anaerobic methanoqenic archaeon E15-3 | 93519 |
| anaerobic methanoqenic archaeon E15-4 | 93520 |
| anaerobic methanoqenic archaeon E15-5 | 93521 |
| anaerobic methanoqenic archaeon E15-6 | 93522 |
| anaerobic methanoqenic archaeon E15-7 | 93523 |
| anaerobic methanoqenic archaeon E15-8 | 93524 |
| anaerobic methanoqenic archaeon E15-9 | 93525 |
| anaerobic methanoqenic archaeon E30-1 | 93527 |
| anaerobic methanoqenic archaeon E30-10 | 93536 |
| anaerobic methanoqenic archaeon E30-2 | 93528 |
| anaerobic methanoqenic archaeon E30-3 | 93529 |
| anaerobic methanoqenic archaeon E30-4 | 93530 |
| anaerobic methanoqenic archaeon E30-5 | 93531 |
| anaerobic methanoqenic archaeon E30-6 | 93532 |
| anaerobic methanoqenic archaeon E30-7 | 93533 |
| anaerobic methanoqenic archaeon E30-8 | 93534 |
| anaerobic methanoqenic archaeon E30-9 | 93535 |
| anaerobic methanoqenic archaeon ET1-1 | 93507 |
| anaerobic methanoqenic archaeon ET1-10 | 93516 |
| anaerobic methanoqenic archaeon ET1-2 | 93508 |
| anaerobic methanoqenic archaeon ET1-3 | 93509 |
| anaerobic methanoqenic archaeon ET1-4 | 93510 |
| anaerobic methanoqenic archaeon ET1-5 | 93511 |
| anaerobic methanoqenic archaeon ET1-6 | 93512 |
| anaerobic methanoqenic archaeon ET1-7 | 93513 |
| anaerobic methanoqenic archaeon ET1-8 | 93514 |
| anaerobic methanoqenic archaeon ET1-9 | 93515 |
| anaerobic methanoqenic archaeon SN15 | 548434 |
| anaerobic methanoqenic archaeon SN20 | 548432 |
| anaerobic methanoqenic archaeon SN22 | 548433 |
| archaeon #33-9 | 328513 |
| archaeon 'A215-UMH 22% pond' | 199002 |
| archaeon 'A311-UMH 31% pond' | 199004 |
| archaeon 'A315-UMH 31% pond' | 199005 |
| archaeon 'A319-UMH 31% pond' | 199006 |
| archaeon 'A356-UMH 31% pond' | 199007 |
| archaeon 'A363-UMH 31% pond' | 199008 |
| archaeon 'AN201-UMH 22% pond' | 199003 |
| archaeon 26-4a1 | 210392 |
| archaeon 26-4a6 | 210393 |
| archaeon 26-5a1 | 210394 |
| archaeon 26-a101 | 210395 |
| archaeon 26-a134 | 210396 |
| archaeon 4R | 323739 |
| archaeon A1 | 631354 |
| archaeon Bitterns-U | 584993 |
| archaeon CP.B3 | 413980 |
| archaeon D3.5-B | 115530 |
| archaeon G70 | 288910 |
| archaeon G76.1 | 378395 |
| archaeon G76.3 | 378397 |
| archaeon G76.4 | 378396 |
| archaeon G80 | 288911 |
| archaeon GSL1A | 378398 |
| archaeon GSL1C | 378400 |
| archaeon GSL1D | 378399 |
| Name of Unclassified Species | |
| archaeon HR3812-Enrichment-017 | |
| archaeon HR3812-Enrichment-018 | |
| archaeon HR3812-Enrichment-019 | |
| archaeon HR3812-Enrichment-020 | |
| archaeon HR3812-Enrichment-021 | |
| archaeon HR3812-Enrichment-022 | |
| archaeon K-4a2archaeon K-5a2 | |
| archaeon LL25A1archaeon LL25A10 | |
| archaeon LL25A2archaeon LL25A3 | |
| archaeon LL25A4archaeon LL25A6 | |
| archaeon LL25A7archaeon LL25A8 | |
| archaeon LL37A1archaeon LL37A19 | |
| archaeon LL37A2archaeon LL37A20 | |
| archaeon LL37A29 | |
| archaeon LL37A3 | |
| archaeonLL37A33 | |
| archaeon LL37A35 | |
| archaeon SL1.19 | |
| archaeon SL1.60 | |
| archaeon SL1.61 | |
| archaeon SL2.43 | |
| archaeon SL2.45 | |
| archaeon SVAL2.51 | |
| archaeon SVAL2.52 | |
| archaeon SVAL2.53 | |
| archaeon SVAL2.54 | |
| archaeon SVAL2.55 | |
| archaeon SVAL2.56 | |
| archaeon enrichment clone M21 | |
| archaeon enrichment clone M33 | |
| archaeon enrichment culture clone 10P | |
| Aarchaeon enrichment culture clone 1TP | |
| Aarchaeon enrichment culture clone 2TP | |
| Aarchaeon enrichment culture clone AOM-Clone-A11 | |
| archaeon enrichment culture clone AOM-Clone-A2 | |
| archaeon enrichment culture clone AOM-Clone-B2 | |
| archaeon enrichment culture clone AOM-Clone-B6 | |
| archaeon enrichment culture clone AOM-Clone-C3 | |
| archaeon enrichment culture clone AOM-Clone-C9 | |
| archaeon enrichment culture clone AOM-Clone-D10 | |
| archaeon enrichment culture clone AOM-Clone-E10 | |
| archaeon enrichment culture clone AOM-Clone-E7 | |
| archaeon enrichment culture clone AOM-Clone-E9 | |
| archaeon enrichment culture clone AOM-Clone-F5 | |
| archaeon enrichment culture clone AOM-Clone-F9 | |
| archaeon enrichment culture clone AOM-Clone-G10 | |
| archaeon enrichment culture clone AOM-Clone-H9 | |
| archaeon enrichment culture clone Arch10 | |
| archaeon enrichment culture clone ARQ17-JL | |
| archaeon enrichment culture clone ARQ19-JL | |
| archaeon enrichment culture clone ARQ2-JL | |
| archaeon enrichment culture clone ARQ9-JL | |
| archaeon enrichment culture clone C1-10C-A | |
| archaeon enrichment culture clone C1-11C-A | |
| archaeon enrichment culture clone C1-13C-A | |
| archaeon enrichment culture clone C1-16C-A | |
| archaeon enrichment culture clone C1-18C-A | |
| archaeon enrichment culture clone C1-19C-A | |
| archaeon enrichment culture clone C1-1C-A | |
| archaeon enrichment culture clone C1-20C-A | |
| archaeon enrichment culture clone C1-24C-A | |
| archaeon enrichment culture clone C1-26C-A | |
| archaeon enrichment culture clone C1-29C-A | |
| archaeon enrichment culture clone C1-2C-A | |
| archaeon enrichment culture clone C1-30C-A | |
| archaeon enrichment culture clone C1-31C-A | |
| archaeon enrichment culture clone C1-32C-A | |
| archaeon enrichment culture clone C1-34C-A | |
| archaeon enrichment culture clone C1-38C-A | |
| archaeon enrichment culture clone C1-3C-A | |
| archaeon enrichment culture clone C1-42C-A | |
| archaeon enrichment culture clone C1-44C-A | |
| archaeon enrichment culture clone C1-46C-A | |
| archaeon enrichment culture clone C1-47C-A | |
| archaeon enrichment culture clone C1-48C-A | |
| archaeon enrichment culture clone C1-4C-A | |
| archaeon enrichment culture clone C1-52C-A | |
| archaeon enrichment culture clone C1-5C-A | |
| archaeon enrichment culture clone C1-8C-A | |
| archaeon enrichment culture clone C3-15C-A | |
| archaeon enrichment culture clone C3-18C-A | |
| archaeon enrichment culture clone C3-1C-A | |
| archaeon enrichment culture clone C3-20C-A | |
| archaeon enrichment culture clone C3-22C-A | |
| archaeon enrichment culture clone C3-26C-A | |
| archaeon enrichment culture clone C3-33C-A | |
| archaeon enrichment culture clone C3-37C-A | |
| archaeon enrichment culture clone C3-41C-A | |
| archaeon enrichment culture clone C3-42C-A | |
| archaeon enrichment culture clone C3-47C-A | |
| archaeon enrichment culture clone C3-54C-A | |
| archaeon enrichment culture clone C3-56C-A | |
| archaeon enrichment culture clone C3-5C-A | |
| archaeon enrichment culture clone C3-7C-A | |
| archaeon enrichment culture clone C4-10C-A | |
| archaeon enrichment culture clone C4-11C-A | |
| archaeon enrichment culture clone C4-12C-A | |
| archaeon enrichment culture clone C4-14C-A | |
| archaeon enrichment culture clone C4-15C-A | |
| archaeon enrichment culture clone C4-16C-A | |
| archaeon enrichment culture clone C4-17C-A | |
| archaeon enrichment culture clone C4-18C-A | |
| archaeon enrichment culture clone C4-20C-A | |
| archaeon enrichment culture clone C4-21C-A | |
| archaeon enrichment culture clone C4-22C-A | |
| archaeon enrichment culture clone C4-23C-A | |
| archaeon enrichment culture clone C4-24C-A | |
| archaeon enrichment culture clone C4-26C-A | |
| archaeon enrichment culture clone C4-27C-A | |
| archaeon enrichment culture clone C4-28C-A | |
| archaeon enrichment culture clone C4-29C-A | |
| archaeon enrichment culture clone C4-2C-A | |
| archaeon enrichment culture clone C4-30C-A | |
| archaeon enrichment culture clone C4-32C-A | |
| archaeon enrichment culture clone C4-34C-A | |
| archaeon enrichment culture clone C4-35C-A | |
| archaeon enrichment culture clone C4-37C-A | |
| archaeon enrichment culture clone C4-38C-A | |
| archaeon enrichment culture clone C4-3C-A | |
| archaeon enrichment culture clone C4-40C-A | |
| archaeon enrichment culture clone C4-41C-A | |
| archaeon enrichment culture clone C4-42C-A | |
| archaeon enrichment culture clone C4-43C-A | |
| archaeon enrichment culture clone C4-45C-A | |
| archaeon enrichment culture clone C4-46C-A | |
| archaeon enrichment culture clone C4-47C-A | |
| archaeon enrichment culture clone C4-48C-A | |
| archaeon enrichment culture clone C4-49C-A | |
| archaeon enrichment culture clone C4-4C-A | |
| archaeon enrichment culture clone C4-50C-A | |
| archaeon enrichment culture clone C4-51C-A | |
| archaeon enrichment culture clone C4-52C-A | |
| archaeon enrichment culture clone C4-54C-A | |
| archaeon enrichment culture clone C4-6C-A | |
| archaeon enrichment culture clone C4-8C-A | |
| archaeon enrichment culture clone C4-9C-A | |
| archaeon enrichment culture clone Dan60_A10E | |
| archaeon enrichment culture clone Dan60_A11E | |
| archaeon enrichment culture clone Dan60_A12E | |
| archaeon enrichment culture clone Dan60_A13E | |
| archaeon enrichment culture clone Dan60_A14E | |
| archaeon enrichment culture clone Dan60_A15E | |
| archaeon enrichment culture clone Dan60_A16E | |
| archaeon enrichment culture clone Dan60_A17E | |
| archaeon enrichment culture clone Dan60_A18E | |
| archaeon enrichment culture clone Dan60_A19E | |
| archaeon enrichment culture clone Dan60_A1E | |
| archaeon enrichment culture clone Dan60_A20E | |
| archaeon enrichment culture clone Dan60_A2E | |
| archaeon enrichment culture clone Dan60_A3E | |
| archaeon enrichment culture clone Dan60_A4E | |
| archaeon enrichment culture clone Dan60_A5E | |
| archaeon enrichment culture clone Dan60_A6E | |
| archaeon enrichment culture clone Dan60_A7E | |
| archaeon enrichment culture clone Dan60_A8E | |
| archaeon enrichment culture clone Dan60_A9E | |
| archaeon enrichment culture clone Dan60S_A10E | |
| archaeon enrichment culture clone Dan60S_A11E | |
| archaeon enrichment culture clone Dan60S_A12E | |
| archaeon enrichment culture clone Dan60S_A13E | |
| archaeon enrichment culture clone Dan60S_A14E | |
| archaeon enrichment culture clone Dan60S_A15E | |
| archaeon enrichment culture clone Dan60S_A16E | |
| archaeon enrichment culture clone Dan60S_A17E | |
| archaeon enrichment culture clone Dan60S_A18E | |
| archaeon enrichment culture clone Dan60S_A19E | |
| archaeon enrichment culture clone Dan60S_A1E | |
| archaeon enrichment culture clone Dan60S_A20E | |
| archaeon enrichment culture clone Dan60S_A2E | |
| archaeon enrichment culture clone Dan60S_A3E | |
| archaeon enrichment culture clone Dan60S_A4E | |
| archaeon enrichment culture clone Dan60S_A5E | |
| archaeon enrichment culture clone Dan60S_A6E | |
| archaeon enrichment culture clone Dan60S_A7E | |
| archaeon enrichment culture clone Dan60S_A8E | |
| archaeon enrichment culture clone Dan60S_A9E | |
| archaeon enrichment culture clone DGGE-1A | |
| archaeon enrichment culture clone DGGE-2A | |
| archaeon enrichment culture clone DGGE-4A | |
| archaeon enrichment culture clone EA17.1 | |
| archaeon enrichment culture clone EA29.3 | |
| archaeon enrichment culture clone EA29.6 | |
| archaeon enrichment culture clone EA3.11 | |
| archaeon enrichment culture clone EA3.3 | |
| archaeon enrichment culture clone EA3.5 | |
| archaeon enrichment culture clone EA8.1 | |
| archaeon enrichment culture clone EA8.8 | |
| archaeon enrichment culture clone EA8.9 | |
| archaeon enrichment culture clone HS25_1 | |
| archaeon enrichment culture clone HS7_1 | |
| archaeon enrichment culture clone LCB_A1C7 | |
| archaeon enrichment culture clone LCB_A1C9 | |
| archaeon enrichment culture clone MBT-11 | |
| archaeon enrichment culture clone McrA2 | |
| archaeon enrichment culture clone MST-4 | |
| archaeon enrichment culture clone Nye-0_2-enr40 | |
| archaeon enrichment culture clone PW15.4A | |
| archaeon enrichment culture clone PW15.6 | |
| archaeon enrichment culture clone PW15.7A | |
| archaeon enrichment culture clone PW20.4A | |
| archaeon enrichment culture clone PW25.2A | |
| archaeon enrichment culture clone PW25.9 | |
| archaeon enrichment culture clone PW30.6A | |
| archaeon enrichment culture clone PW32.12A | |
| archaeon enrichment culture clone PW32.5A | |
| archaeon enrichment culture clone PW45.1 | |
| archaeon enrichment culture clone PW45.7A | |
| archaeon enrichment culture clone PW45.9A | |
| archaeon enrichment culture clone PW5.2A | |
| archaeon enrichment culture clone PW54.3A | |
| archaeon enrichment culture clone PW54.4 | |
| archaeon enrichment culture clone PW68.8A | |
| archaeon enrichment culture clone R3-1a11 | |
| archaeon enrichment culture clone R3-1a2 | |
| archaeon enrichment culture clone R3-1a3 | |
| archaeon enrichment culture clone R3-1a6 | |
| archaeon enrichment culture clone R3-1b6 | |
| archaeon enrichment culture clone R3-1d10 | |
| archaeon enrichment culture clone R3-1e5 | |
| archaeon enrichment culture clone R3-1e8 | |
| archaeon enrichment culture clone R3-1f5 | |
| archaeon enrichment culture clone R3-1g4 | |
| archaeon enrichment culture clone R3-1h9 | |
| archaeon enrichment culture clone T-RF321 | |
| archaeon enrichment culture clone VNC3A001 | |
| archaeon enrichment culture clone VNC3A005 | |
| archaeon enrichment culture clone YE25_1 | |
| archaeon enrichment culture clone YE7_1 | |
| archaeon enrichment culture clone YE7_5 | |
| archaeon enrichment culture DGGE band 1507aq 1 | |
| archaeon enrichment culture DGGE band 1507cas 1 | |
| archaeon enrichment culture DGGE band 1507cas 2 | |
| archaeon enrichment culture DGGE band 1510b-ker 1 | |
| archaeon enrichment culture DGGE band 1510b-ker 2 | |
| archaeon enrichment culture DGGE band 1521cmc 1 | |
| archaeon enrichment culture DGGE band 1521cmc 2 | |
| archaeon enrichment culture DGGE band 1523rope 1 | |
| archaeon enrichment culture DGGE band 1523rope 2 | |
| archaeon enrichment culture DGGE band DS13 | |
| archaeon enrichment culture DGGE band DS18 | |
| archaeon enrichment culture DGGE band DS19 | |
| methanoqenic archaeon enrichment culture clone 4.17a | Arc Band 1 |
| methanoqenic archaeon enrichment culture clone 4.17b | Arc Band 1 |
| methanoqenic archaeon enrichment culture clone | BAMC-4 |
| methanoqenic archaeon enrichment culture clone | BAMC-5 |
| methanogenic archaeon enrichment culture clone | NapK-0_20-enr35 |
| methanogenic archaeon enrichment culture clone | NapK-0_20-enr36 |
| methanogenic archaeon enrichment culture clone | Napk-0_20-enr74 |
| methanogenic archaeon enrichment culture clone | NapK-80_100-enr37 |
| toluene-deqradinq methanoqenic consortium archaeon | |
| uncultured ammonia-oxidizinq archaeon | |
| uncultured archaeal symbiont PA110 | |
| uncultured archaeal symbiont PA111 | |
| uncultured archaeal symbiont PA115 | |
| uncultured archaeal symbiont PA120 | |
| uncultured archaeal symbiont PA121 | |
| uncultured archaeal symbiont PA122 | |
| uncultured archaeal symbiont PA201 | |
| uncultured archaeal symbiont PA202 | |
| uncultured archaeal symbiont PA203 | |
| uncultured archaeal symbiont PA204 | |
| uncultured archaeal symbiont ST101 | |
| uncultured archaeal symbiont ST119 | |
| uncultured archaeal symbiont ST120 | |
| uncultured archaeal symbiont ST123 | |
| uncultured archaeal symbiont ST124 | |
| uncultured archaeal symbiont ST141 | |
| uncultured archaeal symbiont ST150 | |
| uncultured archaeon | |
| uncultured archaeon MedDCM-OCT-S02-C115 | |
| uncultured archaeon MedDCM-OCT-S04-C14 | |
| uncultured archaeon MedDCM-OCT-S04-C140 | |
| uncultured archaeon MedDCM-OCT-S04-C163 | |
| uncultured archaeon MedDCM-OCT-S04-C246 | |
| uncultured archaeon MedDCM-OCT-S05-C10 | |
| uncultured archaeon MedDCM-OCT-S05-C205 | |
| uncultured archaeon MedDCM-OCT-S05-C32 | |
| uncultured archaeon MedDCM-OCT-S05-C418 | |
| uncultured archaeon MedDCM-OCT-S05-C57 | |
| uncultured archaeon MedDCM-OCT-S05-C724 | |
| uncultured archaeon MedDCM-OCT-S06-C18 | |
| uncultured archaeon MedDCM-OCT-S08-C16 | |
| uncultured archaeon MedDCM-OCT-S08-C282 | |
| uncultured archaeon MedDCM-OCT-S08-C37 | |
| uncultured archaeon MedDCM-OCT-S08-C54 | |
| uncultured archaeon MedDCM-OCT-S08-C82 | |
| uncultured archaeon MedDCM-OCT-S08-C92 | |
| uncultured archaeon MedDCM-OCT-S09-C13 | |
| uncultured archaeon MedDCM-OCT-S09-C50 | |
| uncultured archaeon MedDCM-OCT-S11-C441 | |
| uncultured archaeon MedDCM-OCT-S11-C473 | |
| uncultured archaeon 'Antarctica #17' | |
| uncultured archaeon 'Norris Geyer Basin #1' | |
| uncultured archaeon 'Norris Geyer Basin #13' | |
| uncultured archaeon 'Norris Geyer Basin #14' | |
| uncultured archaeon 'Norris Geyer Basin #16' | |
| uncultured archaeon 'Norris Geyer Basin #4' | |
| uncultured archaeon 'Norris Geyer Basin #6' | |
| uncultured archaeon 'Norris Geyer Basin #8' | |
| uncultured archaeon 'Norris Geyer Basin #9' | |
| uncultured archaeon 'Obsidian Pool #1' | |
| uncultured archaeon 'Obsidian Pool #10' | |
| uncultured archaeon 'Obsidian Pool #3' | |
| uncultured archaeon 'Obsidian Pool #4' | |
| uncultured archaeon 'Obsidian Pool #6' | |
| uncultured archaeon 'Obsidian Pool #9' | |
| uncultured archaeon 'Queen's Laundry #28' | |
| uncultured archaeon 101B | |
| uncultured archaeon 102A | |
| uncultured archaeon 103D | |
| uncultured archaeon 112D | |
| uncultured archaeon 113C | |
| uncultured archaeon 130D | |
| uncultured archaeon 142C | |
| uncultured archaeon 145B | |
| uncultured archaeon 15A | |
| uncultured archaeon 17B | |
| uncultured archaeon 19a-1 | |
| uncultured archaeon 19a-14 | |
| uncultured archaeon 19a-18 | |
| uncultured archaeon 19a-19 | |
| uncultured archaeon 19a-23 | |
| uncultured archaeon 19a-27 | |
| uncultured archaeon 19a-29 | |
| uncultured archaeon 19a-4 | |
| uncultured archaeon 19a-5 | |
| uncultured archaeon 19b-1 | |
| uncultured archaeon 19b-16 | |
| uncultured archaeon 19b-17 | |
| uncultured archaeon 19b-2 | |
| uncultured archaeon 19b-23 | |
| uncultured archaeon 19b-24 | |
| uncultured archaeon 19b-26 | |
| uncultured archaeon 19b-30 | |
| uncultured archaeon 19b-31 | |
| uncultured archaeon 19b-32 | |
| uncultured archaeon 19b-34 | |
| uncultured archaeon 19b-35 | |
| uncultured archaeon 19b-37 | |
| uncultured archaeon 19b-38 | |
| uncultured archaeon 19b-39 | |
| uncultured archaeon 19b-41 | |
| uncultured archaeon 19b-42 | |
| uncultured archaeon 19b-46 | |
| uncultured archaeon 19b-5 | |
| uncultured archaeon 19b-52 | |
| uncultured archaeon 19b-7 | |
| uncultured archaeon 19b-8 | |
| uncultured archaeon 19b-9 | |
| uncultured archaeon 19c-1 | |
| uncultured archaeon 19c-10 | |
| uncultured archaeon 19c-12 | |
| uncultured archaeon 19c-17 | |
| uncultured archaeon 19c-18 | |
| uncultured archaeon 19c-27 | |
| uncultured archaeon 19c-31 | |
| uncultured archaeon 19c-33 | |
| uncultured archaeon 19c-35 | |
| uncultured archaeon 19c-36 | |
| uncultured archaeon 19c-45 | |
| uncultured archaeon 19c-49 | |
| uncultured archaeon 19c-5 | |
| uncultured archaeon 19c-50 | |
| uncultured archaeon 19c-51 | |
| uncultured archaeon 19c-52 | |
| uncultured archaeon 19c-53 | |
| uncultured archaeon 19c-54 | |
| uncultured archaeon 1MT315 | |
| uncultured archaeon 1MT325 | |
| uncultured archaeon 20a-1 | |
| uncultured archaeon 20a-10 | |
| uncultured archaeon 20a-12 | |
| uncultured archaeon 20a-15 | |
| uncultured archaeon 20a-17 | |
| uncultured archaeon 20a-2 | |
| uncultured archaeon 20a-25 | |
| uncultured archaeon 20a-28 | |
| uncultured archaeon 20a-3 | |
| uncultured archaeon 20a-6 | |
| uncultured archaeon 20a-7 | |
| uncultured archaeon 20a-9 | |
| uncultured archaeon 20B | |
| uncultured archaeon 20b-1 | |
| uncultured archaeon 20b-10 | |
| uncultured archaeon 20b-14 | |
| uncultured archaeon 20b-15 | |
| uncultured archaeon 20b-16 | |
| uncultured archaeon 20b-18 | |
| uncultured archaeon 20b-22 | |
| uncultured archaeon 20b-25 | |
| uncultured archaeon 20b-26 | |
| uncultured archaeon 20b-27 | |
| uncultured archaeon 20b-28 | |
| uncultured archaeon 20b-30 | |
| uncultured archaeon 20b-31 | |
| uncultured archaeon 20b-37 | |
| uncultured archaeon 20b-39 | |
| uncultured archaeon 20b-4 | |
| uncultured archaeon 20b-40 | |
| uncultured archaeon 20b-47 | |
| uncultured archaeon 20b-53 | |
| uncultured archaeon 20b-54 | |
| uncultured archaeon 20b-7 | |
| uncultured archaeon 20b-9 | |
| uncultured archaeon 20c-10 | |
| uncultured archaeon 20c-12 | |
| uncultured archaeon 20c-16 | |
| uncultured archaeon 20c-17 | |
| uncultured archaeon 20c-18 | |
| uncultured archaeon 20c-19 | |
| uncultured archaeon 20c-20 | |
| uncultured archaeon 20c-22 | |
| uncultured archaeon 20c-23 | |
| uncultured archaeon 20c-25 | |
| uncultured archaeon 20c-29 | |
| uncultured archaeon 20c-3 | |
| uncultured archaeon 20c-37 | |
| uncultured archaeon 20c-39 | |
| uncultured archaeon 20c-4 | |
| uncultured archaeon 20c-42 | |
| uncultured archaeon 20c-43 | |
| uncultured archaeon 20c-47 | |
| uncultured archaeon 20c-52 | |
| uncultured archaeon 20c-54 | |
| uncultured archaeon 20c-8 | |
| uncultured archaeon 20D | |
| uncultured archaeon 22C | |
| uncultured archaeon 26A | |
| uncultured archaeon 27 | |
| uncultured archaeon 27A | |
| uncultured archaeon 27D | |
| uncultured archaeon 2C100 | |
| uncultured archaeon 2C129 | |
| uncultured archaeon 2C130 | |
| uncultured archaeon 2C169 | |
| uncultured archaeon 2C174 | |
| uncultured archaeon 2C25 | |
| uncultured archaeon 2C30 | |
| uncultured archaeon 2C300X | |
| uncultured archaeon 2C46 | |
| uncultured archaeon 2C8 | |
| uncultured archaeon 2C82 | |
| uncultured archaeon 2C83 | |
| uncultured archaeon 2C84 | |
| uncultured archaeon 2C87 | |
| uncultured archaeon 2C9 | |
| uncultured archaeon 2MT1 | |
| uncultured archaeon 2MT103 | |
| uncultured archaeon 2MT120 | |
| uncultured archaeon 2MT16 | |
| uncultured archaeon 2MT196 | |
| uncultured archaeon 2MT198 | |
| uncultured archaeon 2MT22 | |
| uncultured archaeon 2MT310 | |
| uncultured archaeon 2MT320 | |
| uncultured archaeon 2MT53 | |
| uncultured archaeon 2MT7 | |
| uncultured archaeon 2MT8 | |
| uncultured archaeon 2MT98 | |
| uncultured archaeon 60B | |
| uncultured archaeon 61B | |
| uncultured archaeon 61D | |
| uncultured archaeon 63-A1 | |
| uncultured archaeon 63-A10 | |
| uncultured archaeon 63-A11 | |
| uncultured archaeon 63-A12 | |
| uncultured archaeon 63-A14 | |
| uncultured archaeon 63-A15 | |
| uncultured archaeon 63-A16 | |
| uncultured archaeon 63-A17 | |
| uncultured archaeon 63-A18 | |
| uncultured archaeon 63-A19 | |
| uncultured archaeon 63-A20 | |
| uncultured archaeon 63-A21 | |
| uncultured archaeon 63-A22 | |
| uncultured archaeon 63-A23 | |
| uncultured archaeon 63-A24 | |
| uncultured archaeon 63-A3 | |
| uncultured archaeon 63-A4 | |
| uncultured archaeon 63-A5 | |
| uncultured archaeon 63-A6 | |
| uncultured archaeon 63-A7 | |
| uncultured archaeon 63-A8 | |
| uncultured archaeon 63-A9 | |
| uncultured archaeon 63D | |
| uncultured archaeon 64D | |
| uncultured archaeon 65B | |
| uncultured archaeon 65C | |
| uncultured archaeon 66D | |
| uncultured archaeon 70A | |
| uncultured archaeon 71A | |
| uncultured archaeon 71C | |
| uncultured archaeon 73A | |
| uncultured archaeon 73D | |
| uncultured archaeon 76A | |
| uncultured archaeon 80B | |
| uncultured archaeon 82D | |
| uncultured archaeon 83D | |
| uncultured archaeon 84C | |
| uncultured archaeon 85A | |
| uncultured archaeon 90C | |
| uncultured archaeon 91B | |
| uncultured archaeon 93A | |
| uncultured archaeon 94B | |
| uncultured archaeon 94D | |
| uncultured archaeon 95A | |
| uncultured archaeon A016 | |
| uncultured archaeon A140 | |
| uncultured archaeon A145 | |
| uncultured archaeon A148 | |
| uncultured archaeon A151 | |
| uncultured archaeon A153 | |
| uncultured archaeon A154 | |
| uncultured archaeon A157 | |
| uncultured archaeon A174 | |
| uncultured archaeon A175 | |
| uncultured archaeon A177 | |
| uncultured archaeon A178 | |
| uncultured archaeon ACA1-0cm | |
| uncultured archaeon ACA1-9cm | |
| uncultured archaeon ACA10-0cm | |
| uncultured archaeon ACA16-9cm | |
| uncultured archaeon ACA17-9cm | |
| uncultured archaeon ACA3-0cm | |
| uncultured archaeon ACA4-0cm | |
| uncultured archaeon AM-1 | |
| uncultured archaeon AM-10 | |
| uncultured archaeon AM-11 | |
| uncultured archaeon AM-12 | |
| uncultured archaeon AM-13 | |
| uncultured archaeon AM-14 | |
| uncultured archaeon AM-15 | |
| uncultured archaeon AM-16 | |
| uncultured archaeon AM-17 | |
| uncultured archaeon AM-18 | |
| uncultured archaeon AM-19 | |
| uncultured archaeon AM-2 | |
| uncultured archaeon AM-20 | |
| uncultured archaeon AM-21 | |
| uncultured archaeon AM-22 | |
| uncultured archaeon AM-3 | |
| uncultured archaeon AM-4 | |
| uncultured archaeon AM-5 | |
| uncultured archaeon AM-6 | |
| uncultured archaeon AM-7 | |
| uncultured archaeon AM-8 | |
| uncultured archaeon AM-9 | |
| uncultured archaeon APA1-0cm | |
| uncultured archaeon APA2-17cm | |
| uncultured archaeon APA3-0cm | |
| uncultured archaeon APA3-11cm | |
| uncultured archaeon APA4-0cm | |
| uncultured archaeon APA6-17cm | |
| uncultured archaeon APA7-17cm | |
| uncultured archaeon Ar21 | |
| uncultured archaeon Ar26 | |
| uncultured archaeon Ar28 | |
| uncultured archaeon Arc.1 | |
| uncultured archaeon Arc.118 | |
| uncultured archaeon Arc.119 | |
| uncultured archaeon Arc.148 | |
| uncultured archaeon Arc.168 | |
| uncultured archaeon Arc.171 | |
| uncultured archaeon Arc.2 | |
| uncultured archaeon Arc.201 | |
| uncultured archaeon Arc.212 | |
| uncultured archaeon Arc.22 | |
| uncultured archaeon Arc.3 | |
| uncultured archaeon Arc.4 | |
| uncultured archaeon Arc.43 | |
| uncultured archaeon Arc.75 | |
| uncultured archaeon Arc.9 | |
| uncultured archaeon Arc.98 | |
| uncultured archaeon Cas14#1 | |
| uncultured archaeon Cas14#2 | |
| uncultured archaeon Cas14#3 | |
| uncultured archaeon Cas14#4 | |
| uncultured archaeon Cas14#5 | |
| uncultured archaeon Cas14#6 | |
| uncultured archaeon Cas18#1 | |
| uncultured archaeon Cas18#2 | |
| uncultured archaeon Cas18#3 | |
| uncultured archaeon Cas18#4 | |
| uncultured archaeon Cas19#1 | |
| uncultured archaeon Cas19#2 | |
| uncultured archaeon Cas19#3 | |
| uncultured archaeon Cas19#4 | |
| uncultured archaeon Cas19#5 | |
| uncultured archaeon Cas19#6 | |
| uncultured archaeon Cas20#1 | |
| uncultured archaeon Cas20#2 | |
| uncultured archaeon Cas20#3 | |
| uncultured archaeon Cas20#4 | |
| uncultured archaeon Cas20#5 | |
| uncultured archaeon CR-PA10a | |
| uncultured archaeon CR-PA12a | |
| uncultured archaeon CR-PA13a | |
| uncultured archaeon CR-PA15a | |
| uncultured archaeon CR-PA16a | |
| uncultured archaeon CR-PA1a | |
| uncultured archaeon CR-PA2a | |
| uncultured archaeon CR-PA4a | |
| uncultured archaeon CR-PA6a | |
| uncultured archaeon CR-PA7a | |
| uncultured archaeon CR-PA8a | |
| uncultured archaeon CRA12-27cm | |
| uncultured archaeon CRA13-11cm | |
| uncultured archaeon CRA20-0cm | |
| uncultured archaeon CRA36-0cm | |
| uncultured archaeon CRA4-23cm | |
| uncultured archaeon CRA7-0cm | |
| uncultured archaeon CRA7-11cm | |
| uncultured archaeon CRA8-11cm | |
| uncultured archaeon CRA8-23cm | |
| uncultured archaeon CRA8-27cm | |
| uncultured archaeon CRA9-27cm | |
| uncultured archaeon CRE-FL10a | |
| uncultured archaeon CRE-FL11a | |
| uncultured archaeon CRE-FL1a | |
| uncultured archaeon CRE-FL2a | |
| uncultured archaeon CRE-FL3a | |
| uncultured archaeon CRE-FL4a | |
| uncultured archaeon CRE-FL5a | |
| uncultured archaeon CRE-FL6a | |
| uncultured archaeon CRE-FL7a | |
| uncultured archaeon CRE-FL8a | |
| uncultured archaeon CRE-FL9a | |
| uncultured archaeon CRE-PA10a | |
| uncultured archaeon CRE-PA11a | |
| uncultured archaeon CRE-PA2a | |
| uncultured archaeon CRE-PA3a | |
| uncultured archaeon CRE-PA4a | |
| uncultured archaeon CRE-PA5a | |
| uncultured archaeon CRE-PA6a | |
| uncultured archaeon CRE-PA7a | |
| uncultured archaeon CRE-PA8a | |
| uncultured archaeon CRE-PA9a | |
| uncultured archaeon CRO-11a | |
| uncultured archaeon CRO-12a | |
| uncultured archaeon CRO-14a | |
| uncultured archaeon CRO-1a | |
| uncultured archaeon CRO-2a | |
| uncultured archaeon CRO-3a | |
| uncultured archaeon CRO-4a | |
| uncultured archaeon CRO-5a | |
| uncultured archaeon CRO-6a | |
| uncultured archaeon CRO-7a | |
| uncultured archaeon CRO-8a | |
| uncultured archaeon DGGE band PSARC-1 | |
| uncultured archaeon DGGE band PSARC-2 | |
| uncultured archaeon E1b | |
| uncultured archaeon ER-E | |
| uncultured archaeon ER-H | |
| uncultured archaeon GA01 | |
| uncultured archaeon GA02 | |
| uncultured archaeon GA04 | |
| uncultured archaeon GA10 | |
| uncultured archaeon GA32 | |
| uncultured archaeon GA42 | |
| uncultured archaeon GA54 | |
| uncultured archaeon GA55 | |
| uncultured archaeon GA67 | |
| uncultured archaeon GA77 | |
| uncultured archaeon GZfos10C7 | |
| uncultured archaeon GZfos11A10 | |
| uncultured archaeon GZfos11H11 | |
| uncultured archaeon GZfos12E1 | |
| uncultured archaeon GZfos12E2 | |
| uncultured archaeon GZfos13E1 | |
| uncultured archaeon GZfos14B8 | |
| uncultured archaeon GZfos17A3 | |
| uncultured archaeon GZfos17C7 | |
| uncultured archaeon GZfos17F1 | |
| uncultured archaeon GZfos17G11 | |
| uncultured archaeon GZfos18B6 | |
| uncultured archaeon GZfos18C8 | |
| uncultured archaeon GZfos18F2 | |
| uncultured archaeon GZfos18H11 | |
| uncultured archaeon GZfos19A5 | |
| uncultured archaeon GZfos19C7 | |
| uncultured archaeon GZfos19C8 | |
| uncultured archaeon GZfos1C11 | |
| uncultured archaeon GZfos1D1 | |
| uncultured archaeon GZfos21B5 | |
| uncultured archaeon GZfos22D9 | |
| uncultured archaeon GZfos23H7 | |
| uncultured archaeon GZfos23H9 | |
| uncultured archaeon GZfos24D9 | |
| uncultured archaeon GZfos26B2 | |
| uncultured archaeon GZfos26D6 | |
| uncultured archaeon GZfos26D8 | |
| uncultured archaeon GZfos26E7 | |
| uncultured archaeon GZfos26F9 | |
| uncultured archaeon GZfos26G2 | |
| uncultured archaeon GZfos27A8 | |
| uncultured archaeon GZfos27B6 | |
| uncultured archaeon GZfos27E6 | |
| uncultured archaeon GZfos27E7 | |
| uncultured archaeon GZfos27G5 | |
| uncultured archaeon GZfos28B8 | |
| uncultured archaeon GZfos28G7 | |
| uncultured archaeon GZfos29E12 | |
| uncultured archaeon GZfos30H9 | |
| uncultured archaeon GZfos31B6 | |
| uncultured archaeon GZfos32E4 | |
| uncultured archaeon GZfos32E7 | |
| uncultured archaeon GZfos32G12 | |
| uncultured archaeon GZfos33E1 | |
| uncultured archaeon GZfos33H6 | |
| uncultured archaeon GZfos34A6 | |
| uncultured archaeon GZfos34G5 | |
| uncultured archaeon GZfos34H10 | |
| uncultured archaeon GZfos34H9 | |
| uncultured archaeon GZfos35A2 | |
| uncultured archaeon GZfos35B7 | |
| uncultured archaeon GZfos35D7 | |
| uncultured archaeon GZfos36D8 | |
| uncultured archaeon GZfos37B2 | |
| uncultured archaeon GZfos37D1 | |
| uncultured archaeon GZfos3D4 | |
| uncultured archaeon GZfos9C4 | |
| uncultured archaeon GZfos9D1 | |
| uncultured archaeon GZfos9D8 | |
| uncultured archaeon GZfos9E5 | |
| uncultured archaeon HA01 | |
| uncultured archaeon HA03 | |
| uncultured archaeon HA04 | |
| uncultured archaeon HA05 | |
| uncultured archaeon HA06 | |
| uncultured archaeon HA08 | |
| uncultured archaeon HA09 | |
| uncultured archaeon HA10 | |
| uncultured archaeon HA11 | |
| uncultured archaeon HA19 | |
| uncultured archaeon HA25 | |
| uncultured archaeon HA48 | |
| uncultured archaeon HA55 | |
| uncultured archaeon KNIA11 | |
| uncultured archaeon KNIA12 | |
| uncultured archaeon KNIA13 | |
| uncultured archaeon KNIA14 | |
| uncultured archaeon KNIA15 | |
| uncultured archaeon n1d | |
| uncultured archaeon n41r | |
| uncultured archaeon OS-1 | |
| uncultured archaeon OS-10 | |
| uncultured archaeon OS-11 | |
| uncultured archaeon OS-12 | |
| uncultured archaeon OS-13 | |
| uncultured archaeon OS-14 | |
| uncultured archaeon OS-15 | |
| uncultured archaeon OS-16 | |
| uncultured archaeon OS-17 | |
| uncultured archaeon OS-18 | |
| uncultured archaeon OS-19 | |
| uncultured archaeon OS-2 | |
| uncultured archaeon OS-20 | |
| uncultured archaeon OS-21 | |
| uncultured archaeon OS-22 | |
| uncultured archaeon OS-23 | |
| uncultured archaeon OS-24 | |
| uncultured archaeon OS-25 | |
| uncultured archaeon OS-26 | |
| uncultured archaeon OS-27 | |
| uncultured archaeon OS-28 | |
| uncultured archaeon OS-29 | |
| uncultured archaeon OS-3 | |
| uncultured archaeon OS-30 | |
| uncultured archaeon OS-31 | |
| uncultured archaeon OS-32 | |
| uncultured archaeon OS-33 | |
| uncultured archaeon OS-4 | |
| uncultured archaeon OS-5 | |
| uncultured archaeon OS-6 | |
| uncultured archaeon OS-7 | |
| uncultured archaeon OS-8 | |
| uncultured archaeon OS-9 | |
| uncultured archaeon pHGPA1 | |
| uncultured archaeon pHGPA13 | |
| uncultured archaeon pPACMA-A | |
| uncultured archaeon pPACMA-B | |
| uncultured archaeon pPACMA-C | |
| uncultured archaeon pPACMA-E | |
| uncultured archaeon pPACMA-F | |
| uncultured archaeon pPACMA-G | |
| uncultured archaeon pPACMA-H | |
| uncultured archaeon pPACMA-I | |
| uncultured archaeon pPACMA-J | |
| uncultured archaeon pPACMA-K | |
| uncultured archaeon pPACMA-L | |
| uncultured archaeon pPACMA-M | |
| uncultured archaeon pPACMA-N | |
| uncultured archaeon pPACMA-P | |
| uncultured archaeon pPACMA-Q | |
| uncultured archaeon pPACMA-S | |
| uncultured archaeon pPACMA-T | |
| uncultured archaeon pPACMA-U | |
| uncultured archaeon pPACMA-V | |
| uncultured archaeon pPACMA-W | |
| uncultured archaeon pPACMA-X | |
| uncultured archaeon pPACMA-Y | |
| uncultured archaeon pPCA10.1 | |
| uncultured archaeon pPCA12.14 | |
| uncultured archaeon pPCA12.6 | |
| uncultured archaeon pPCA13.4 | |
| uncultured archaeon pPCA13.5 | |
| uncultured archaeon pPCA14.16 | |
| uncultured archaeon pPCA14.17 | |
| uncultured archaeon pPCA14.18 | |
| uncultured archaeon pPCA14.41 | |
| uncultured archaeon pPCA15.21 | |
| uncultured archaeon pPCA17.1 | |
| uncultured archaeon pPCA19.6 | |
| uncultured archaeon pPCA2.4 | |
| uncultured archaeon pPCA4.21 | |
| uncultured archaeon pPCA4.4 | |
| uncultured archaeon pPCA4.9 | |
| uncultured archaeon pPCA7.13 | |
| uncultured archaeon pPCA7.17 | |
| uncultured archaeon pPCA7.21 | |
| uncultured archaeon pPCA7.30 | |
| uncultured archaeon pPCA7.34 | |
| uncultured archaeon pPCA7.6 | |
| uncultured archaeon pPCA7.8 | |
| uncultured archaeon pPCA8.3 | |
| uncultured archaeon RSS50-1 | |
| uncultured archaeon RSS50-10 | |
| uncultured archaeon RSS50-11 | |
| uncultured archaeon RSS50-2 | |
| uncultured archaeon RSS50-3 | |
| uncultured archaeon RSS50-4 | |
| uncultured archaeon RSS50-5 | |
| uncultured archaeon RSS50-6 | |
| uncultured archaeon RSS50-7 | |
| uncultured archaeon RSS50-8 | |
| uncultured archaeon RSS50-9 | |
| uncultured archaeon S15-1 | |
| uncultured archaeon S15-10 | |
| uncultured archaeon S15-11 | |
| uncultured archaeon S15-12 | |
| uncultured archaeon S15-13 | |
| uncultured archaeon S15-14 | |
| uncultured archaeon S15-15 | |
| uncultured archaeon S15-16 | |
| uncultured archaeon S15-17 | |
| uncultured archaeon S15-18 | |
| uncultured archaeon S15-19 | |
| uncultured archaeon S15-2 | |
| uncultured archaeon S15-20 | |
| uncultured archaeon S15-21 | |
| uncultured archaeon S15-22 | |
| uncultured archaeon S15-23 | |
| uncultured archaeon S15-24 | |
| uncultured archaeon S15-25 | |
| uncultured archaeon S15-26 | |
| uncultured archaeon S15-27 | |
| uncultured archaeon S15-28 | |
| uncultured archaeon S15-29 | |
| uncultured archaeon S15-3 | |
| uncultured archaeon S15-30 | |
| uncultured archaeon S15-4 | |
| uncultured archaeon S15-5 | |
| uncultured archaeon S15-6 | |
| uncultured archaeon S15-7 | |
| uncultured archaeon S15-8 | |
| uncultured archaeon S15-9 | |
| uncultured archaeon S30-1 | |
| uncultured archaeon S30-10 | |
| uncultured archaeon S30-11 | |
| uncultured archaeon S30-12 | |
| uncultured archaeon S30-13 | |
| uncultured archaeon S30-14 | |
| uncultured archaeon S30-15 | |
| uncultured archaeon S30-16 | |
| uncultured archaeon S30-17 | |
| uncultured archaeon S30-18 | |
| uncultured archaeon S30-19 | |
| uncultured archaeon S30-2 | |
| uncultured archaeon S30-20 | |
| uncultured archaeon S30-21 | |
| uncultured archaeon S30-22 | |
| uncultured archaeon S30-23 | |
| uncultured archaeon S30-24 | |
| uncultured archaeon S30-25 | |
| uncultured archaeon S30-26 | |
| uncultured archaeon S30-27 | |
| uncultured archaeon S30-28 | |
| uncultured archaeon S30-29 | |
| uncultured archaeon S30-3 | |
| uncultured archaeon S30-30 | |
| uncultured archaeon S30-4 | |
| uncultured archaeon S30-5 | |
| uncultured archaeon S30-6 | |
| uncultured archaeon S30-7 | |
| uncultured archaeon S30-8 | |
| uncultured archaeon S30-9 | |
| uncultured archaeon SAGMA-1 | |
| uncultured archaeon SAGMA-10 | |
| uncultured archaeon SAGMA-11 | |
| uncultured archaeon SAGMA-12 | |
| uncultured archaeon SAGMA-13 | |
| uncultured archaeon SAGMA-14 | |
| uncultured archaeon SAGMA-15 | |
| uncultured archaeon SAGMA-16 | |
| uncultured archaeon SAGMA-17 | |
| uncultured archaeon SAGMA-2 | |
| uncultured archaeon SAGMA-3 | |
| uncultured archaeon SAGMA-4 | |
| uncultured archaeon SAGMA-6 | |
| uncultured archaeon SAGMA-7 | |
| uncultured archaeon SAGMA-8 | |
| uncultured archaeon SAGMA-9 | |
| uncultured archaeon SAGMA-A | |
| uncultured archaeon SAGMA-B | |
| uncultured archaeon SAGMA-C | |
| uncultured archaeon SAGMA-D | |
| uncultured archaeon SAGMA-E | |
| uncultured archaeon SAGMA-F | |
| uncultured archaeon SAGMA-G | |
| uncultured archaeon SAGMA-H | |
| uncultured archaeon SAGMA-I | |
| uncultured archaeon SAGMA-J | |
| uncultured archaeon SAGMA-J2 | |
| uncultured archaeon SAGMA-K | |
| uncultured archaeon SAGMA-L | |
| uncultured archaeon SAGMA-M | |
| uncultured archaeon SAGMA-N | |
| uncultured archaeon SAGMA-O | |
| uncultured archaeon SAGMA-P | |
| uncultured archaeon SAGMA-Q | |
| uncultured archaeon SAGMA-R | |
| uncultured archaeon SAGMA-S | |
| uncultured archaeon SAGMA-T | |
| uncultured archaeon SAGMA-U | |
| uncultured archaeon SAGMA-V | |
| uncultured archaeon SAGMA-W | |
| uncultured archaeon SAGMA-X | |
| uncultured archaeon SAGMA-Y | |
| uncultured archaeon SAGMA-Z | |
| uncultured archaeon SC1 | |
| uncultured archaeon SC2 | |
| uncultured archaeon SC4 | |
| uncultured archaeon SC6 | |
| uncultured archaeon SC7 | |
| uncultured archaeon SJC-11b | |
| uncultured archaeon SJC-125a | |
| uncultured archaeon SJD-102 | |
| uncultured archaeon SJD-103 | |
| uncultured archaeon SJD-105 | |
| uncultured archaeon SJD-107 | |
| uncultured archaeon SJD-111 | |
| uncultured archaeon SJD-114 | |
| uncultured archaeon SL-C | |
| uncultured archaeon SL1-1 | |
| uncultured archaeon SL2-d | |
| uncultured archaeon SM1 | |
| uncultured archaeon SM1K20 | |
| uncultured archaeon ST1-1 | |
| uncultured archaeon ST1-10 | |
| uncultured archaeon ST1-11 | |
| uncultured archaeon ST1-12 | |
| uncultured archaeon ST1-13 | |
| uncultured archaeon ST1-14 | |
| uncultured archaeon ST1-15 | |
| uncultured archaeon ST1-16 | |
| uncultured archaeon ST1-17 | |
| uncultured archaeon ST1-18 | |
| uncultured archaeon ST1-19 | |
| uncultured archaeon ST1-2 | |
| uncultured archaeon ST1-20 | |
| uncultured archaeon ST1-21 | |
| uncultured archaeon ST1-22 | |
| uncultured archaeon ST1-23 | |
| uncultured archaeon ST1-24 | |
| uncultured archaeon ST1-25 | |
| uncultured archaeon ST1-26 | |
| uncultured archaeon ST1-27 | |
| uncultured archaeon ST1-28 | |
| uncultured archaeon ST1-29 | |
| uncultured archaeon ST1-3 | |
| uncultured archaeon ST1-30 | |
| uncultured archaeon ST1-4 | |
| uncultured archaeon ST1-5 | |
| uncultured archaeon ST1-6 | |
| uncultured archaeon ST1-7 | |
| uncultured archaeon ST1-8 | |
| uncultured archaeon ST1-9 | |
| uncultured archaeon SW1 | |
| uncultured archaeon SW14 | |
| uncultured archaeon SW3 | |
| uncultured archaeon SW9 | |
| uncultured archaeon SWY | |
| uncultured archaeon SYA-1 | |
| uncultured archaeon SYA-106 | |
| uncultured archaeon SYA-112 | |
| uncultured archaeon SYA-12 | |
| uncultured archaeon SYA-122 | |
| uncultured archaeon SYA-125 | |
| uncultured archaeon SYA-127 | |
| uncultured archaeon SYA-13 | |
| uncultured archaeon SYA-130 | |
| uncultured archaeon SYA-133 | |
| uncultured archaeon SYA-136 | |
| uncultured archaeon SYA-141 | |
| uncultured archaeon SYA-20 | |
| uncultured archaeon SYA-26 | |
| uncultured archaeon SYA-30 | |
| uncultured archaeon SYA-32 | |
| uncultured archaeon SYA-39 | |
| uncultured archaeon SYA-45 | |
| uncultured archaeon SYA-5 | |
| uncultured archaeon SYA-50 | |
| uncultured archaeon SYA-62 | |
| uncultured archaeon SYA-7 | |
| uncultured archaeon SYA-70 | |
| uncultured archaeon SYA-74 | |
| uncultured archaeon SYA-75 | |
| uncultured archaeon SYA-77 | |
| uncultured archaeon SYA-78 | |
| uncultured archaeon SYA-8 | |
| uncultured archaeon SYA-80 | |
| uncultured archaeon SYA-81 | |
| uncultured archaeon SYA-94 | |
| uncultured archaeon SYA_2000_10 | |
| uncultured archaeon SYA_2000_11 | |
| uncultured archaeon SYA_2000_12 | |
| uncultured archaeon SYA_2000_13 | |
| uncultured archaeon SYA_2000_14 | |
| uncultured archaeon SYA 2000 15 | |
| uncultured archaeon SYA_2000_16 | |
| uncultured archaeon SYA_2000_17 | |
| uncultured archaeon SYA_2000_18 | |
| uncultured archaeon SYA_2000_19 | |
| uncultured archaeon SYA_2000_2 | |
| uncultured archaeon SYA_2000_20 | |
| uncultured archaeon SYA_2000_21 | |
| uncultured archaeon SYA_2000_24 | |
| uncultured archaeon SYA_2000_26 | |
| uncultured archaeon SYA_2000_27 | |
| uncultured archaeon SYA_2000_28 | |
| uncultured archaeon SYA_2000_30 | |
| uncultured archaeon SYA_2000_31 | |
| uncultured archaeon SYA_2000_32 | |
| uncultured archaeon SYA_2000_35 | |
| uncultured archaeon SYA_2000_36 | |
| uncultured archaeon SYA_2000_37 | |
| uncultured archaeon SYA_2000_39 | |
| uncultured archaeon SYA_2000_40 | |
| uncultured archaeon SYA_2000_41 | |
| uncultured archaeon SYA_2000_43 | |
| uncultured archaeon SYA_2000_44 | |
| uncultured archaeon SYA_2000_45 | |
| uncultured archaeon SYA_2000_46 | |
| uncultured archaeon SYA_2000_47 | |
| uncultured archaeon SYA_2000_5 | |
| uncultured archaeon SYA_2000_51 | |
| uncultured archaeon SYA_2000_52 | |
| uncultured archaeon SYA_2000_54 | |
| uncultured archaeon SYA_2000_55 | |
| uncultured archaeon SYA_2000_57 | |
| uncultured archaeon SYA_2000_58 | |
| uncultured archaeon SYA_2000_59 | |
| uncultured archaeon SYA_2000_6 | |
| uncultured archaeon SYA_2000_60 | |
| uncultured archaeon SYA_2000_61 | |
| uncultured archaeon SYA_2000_62 | |
| uncultured archaeon SYA_2000_63 | |
| uncultured archaeon SYA_2000_66 | |
| uncultured archaeon SYA_2000_67 | |
| uncultured archaeon SYA_2000_68 | |
| uncultured archaeon SYA_2000_7 | |
| uncultured archaeon SYA_2000_70 | |
| uncultured archaeon SYA_2000_8 | |
| uncultured archaeon SYA_2000_9 | |
| uncultured archaeon TA01 | |
| uncultured archaeon TA02 | |
| uncultured archaeon TA03 | |
| uncultured archaeon TA04 | |
| uncultured archaeon TA05 | |
| uncultured archaeon VC2.1 Arc1 | |
| uncultured archaeon VC2.1 Arc13 | |
| uncultured archaeon VC2.1 Arc16 | |
| uncultured archaeon VC2.1 Arc2 | |
| uncultured archaeon VC2.1 Arc31 | |
| uncultured archaeon VC2.1 Arc35 | |
| uncultured archaeon VC2.1 Arc36 | |
| uncultured archaeon VC2.1 Arc4 | |
| uncultured archaeon VC2.1 Arc5 | |
| uncultured archaeon VC2.1 Arc6 | |
| uncultured archaeon VC2.1 Arc7 | |
| uncultured archaeon VC2.1 Arc8 | |
| uncultured archaeon WSB-1 | |
| uncultured archaeon WSB-10 | |
| uncultured archaeon WSB-11 | |
| uncultured archaeon WSB-12 | |
| uncultured archaeon WSB-13 | |
| uncultured archaeon WSB-14 | |
| uncultured archaeon WSB-15 | |
| uncultured archaeon WSB-16 | |
| uncultured archaeon WSB-17 | |
| uncultured archaeon WSB-18 | |
| uncultured archaeon WSB-19 | |
| uncultured archaeon WSB-2 | |
| uncultured archaeon WSB-20 | |
| uncultured archaeon WSB-21 | |
| uncultured archaeon WSB-3 | |
| uncultured archaeon WSB-4 | |
| uncultured archaeon WSB-5 | |
| uncultured archaeon WSB-6 | |
| uncultured archaeon WSB-7 | |
| uncultured archaeon WSB-8 | |
| uncultured archaeon WSB-9 | |
| uncultured archeon 'KTK 18A' | |
| uncultured archeon 'KTK 28A' | |
| uncultured archeon 'KTK 31A' | |
| uncultured archeon 'KTK 4A' | |
| uncultured archeon 'KTK 9A' | |
| uncultured Banisveld landfill archaeon BVIowarchb2 | |
| uncultured Banisveld landfill archaeon BVupparchb1 | |
| uncultured compost archaeon | |
| uncultured deep-sea archaeon | |
| uncultured endolithic archaeon | |
| uncultured equine intestinal archaeal sp. DL11 | |
| uncultured maize rhizosphere archaeon c9_45(Cr) | |
| uncultured maize root archaeon ZmrA1 | |
| uncultured maize root archaeon ZmrA19 | |
| uncultured maize root archaeon ZmrA30 | |
| uncultured maize root archaeon ZmrA38 | |
| uncultured maize root archaeon ZmrA4 | |
| uncultured maize root archaeon ZmrA42 | |
| uncultured marine archaeon | |
| uncultured marine archaeon DCM3921 | |
| uncultured marine archaeon DCM6515 | |
| uncultured marine archaeon DCM65231 | |
| uncultured marine archaeon DCM74159 | |
| uncultured marine archaeon DCM74161 | |
| uncultured marine archaeon DCM858 | |
| uncultured marine archaeon DCM860 | |
| uncultured marine archaeon DCM861 | |
| uncultured marine archaeon DCM862 | |
| uncultured marine archaeon DCM863 | |
| uncultured marine archaeon DCM865 | |
| uncultured marine archaeon DCM866 | |
| uncultured marine archaeon DCM867 | |
| uncultured marine archaeon DCM871 | |
| uncultured marine archaeon DCM873 | |
| uncultured marine archaeon DCM874 | |
| uncultured marine archaeon DCM875 | |
| uncultured marine archaeon FF619 | |
| uncultured marine archaeon FF620 | |
| uncultured marine archaeon FIN625 | |
| uncultured marine archaeon FIN654 | |
| uncultured marine archaeon GIN492 | |
| uncultured marine archaeon TS10C286 | |
| uncultured marine archaeon TS10C294 | |
| uncultured marine archaeon TS10C298 | |
| uncultured marine archaeon TS10C299 | |
| uncultured marine archaeon TS235C302 | |
| uncultured marine archaeon TS235C306 | |
| uncultured marine archaeon TS235C310 | |
| uncultured methane-oxidizinq archaeon | |
| uncultured methanoqen R5 | |
| uncultured methanoqen R8 | |
| uncultured methanoqen R9 | |
| uncultured rumen archaeon | |
| uncultured rumen archaeon M1 | |
| uncultured rumen archaeon M2 | |
| uncultured rumen archaeon M7 | |
| uncultured rumen methanoqen | |
| uncultured rumen methanoqen 15 | |
| uncultured rumen methanoqen 2 | |
| uncultured rumen methanoqen 956 | |
| uncultured rumen methanoqen Hole9 | |
| uncultured rumen methanoqen M6 | |
| uncultured soil archaeon | |
| uncultured sponge symbiont PAAR2 | |
| uncultured sponge symbiont PAAR4 | |
| uncultured sponge symbiont PAAR8 | |
| uncultured sponge symbiont PAAR9 | |
| uncultured thermal soil archaeon | |
| uncultured vent archaeon | |
| unidentified archaeon | |
| unidentified archaeon H1-B1 | |
| unidentified archaeon H1-K16 | |
| unidentified archaeon H1-K19 | |
| unidentified archaeon H1-K2 | |
| unidentified archaeon H1-K9 | |
| unidentified archaeon H6-B1 | |
| unidentified archaeon H6-K5 | |
| unidentified archaeon H6-K6 | |
| unidentified archaeon HB3-1 | |
| unidentified archaeon S3-K14 | |
| unidentified archaeon S3-K15 | |
| unidentified archaeon S3-K25 | |
| unidentified archaeon S3-K5 | |
| unidentified archaeon S3-K9 | |
| unidentified hydrothermal vent archaeon PVA_OTU_1 | |
| unidentified hydrothermal vent archaeon PVA_OTU_3 | |
| unidentified marine archaeon p712-12 | |
| unidentified marine archaeon p712-13 | |
| unidentified marine archaeon p712-24 | |
| unidentified marine archaeon p712-3 | |
| unidentified marine archaeon p712-37 | |
| unidentified marine archaeon p712-63 | |

**TABLE 4**

| **Class** | **Order** | **Family** | **Genus** | **Species** | **Taxonomy ID** |
|---|---|---|---|---|---|
| Thermoprotei | Caldisphaerales | Caldisphaeraceae | Caldisphaera | draconis | 671066 |
| Thermoprotei | Caldisphaerales | Caldisphaeraceae | Caldisphaera | lagunensis | 200415 |
| Thermoprotei | Caldisphaerales | Caldisphaeraceae | Caldisphaera | | 282527 |
| Thermoprotei | Desulfurococcales | Desulfurococcaceae | Acidilobus | aceticus | 105851 |
| Thermoprotei | Desulfurococcales | Desulfurococcaceae | Acidilobus | saccharovorans | 666510 |
| Thermoprotei | Desulfurococcales | Desulfurococcaceae | Acidilobus | sulfurireducens | 411357 |
| Thermoprotei | Desulfurococcales | Desulfurococcaceae | Acidilobus | sp. 124-87 | 242702 |
| Thermoprotei | Desulfurococcales | Desulfurococcaceae | Acidilobus | sp. 405-16 | 242704 |
| Thermoprotei | Desulfurococcales | Desulfurococcaceae | Acidilobus | sp. 722-67 | 242705 |
| Thermoprotei | Desulfurococcales | Desulfurococcaceae | Acidilobus | | 242694 |
| Thermoprotei | Fervidicoccales | Fervidicoccaceae | Fervidicoccus | fontis | 683846 |
| Thermoprotei | Sulfolobales | Sulfolobaceae | Acidianus | ambivalens | 2283 |
| Thermoprotei | Sulfolobales | Sulfolobaceae | Acidianus | brierleyi | 41673 |
| Thermoprotei | Sulfolobales | Sulfolobaceae | Acidianus | convivator | 269667 |
| Thermoprotei | Sulfolobales | Sulfolobaceae | Acidianus | hospitalis | 563177 |
| Thermoprotei | Sulfolobales | Sulfolobaceae | Acidianus | infernus | 12915 |
| Thermoprotei | Sulfolobales | Sulfolobaceae | Acidianus | manzaensis | 282676 |
| Thermoprotei | Sulfolobales | Sulfolobaceae | Acidianus | pozzuoliensis | 314564 |
| Thermoprotei | Sulfolobales | Sulfolobaceae | Acidianus | sulfidivorans | 619593 |
| Thermoprotei | Sulfolobales | Sulfolobaceae | Acidianus | tengchongensis | 146920 |
| Thermoprotei | Sulfolobales | Sulfolobaceae | Acidianus | sp. Acii18 | 315459 |
| Thermoprotei | Sulfolobales | Sulfolobaceae | Acidianus | sp. Acii19 | 315462 |
| Thermoprotei | Sulfolobales | Sulfolobaceae | Acidianus | sp. Acii25 | 315461 |
| Thermoprotei | Sulfolobales | Sulfolobaceae | Acidianus | sp. Acii26 | 315460 |
| Thermoprotei | Sulfolobales | Sulfolobaceae | Acidianus | sp. BT | 513519 |
| Thermoprotei | Sulfolobales | Sulfolobaceae | Acidianus | sp. F28 | 315458 |
| Thermoprotei | Sulfolobales | Sulfolobaceae | Acidianus | | 310069 |
| Thermoprotei | Thermoproteales | Thermofilaceae | Thermofilum | librum | 54255 |
| Thermoprotei | Thermoproteales | Thermofilaceae | Thermofilum | pendens | 368408 |
| Thermoprotei | Thermoproteales | Thermofilaceae | Thermofilum | sp. 1505 | 697581 |
| Thermoprotei | Thermoproteales | Thermofilaceae | Thermofilum | | 310083 |
| Thermoprotei | Unclassified | Unclassified | Unclassified | Unclassified | 476105 |
| Unclassified | Unclassified | Unclassified | Candidatus Nitrosocaldus | yellowstonii | 498375 |
| Unclassified | Unclassified | Unclassified | Candidatus Nitrosocaldus | Unclassified | 766501 |
| Unclassified | Unclassified | Unclassified | Candidatus Nitrososphaera | gargensis | 497727 |
| Unclassified | Unclassified | Unclassified | Candidatus Nitrososphaera | Unclassified | 759874 |
| Unclassified | Unclassified | Unclassified | Unclassified | crenarchaeote 768-28 | 242701 |
| Unclassified | Unclassified | Unclassified | Unclassified | crenarchaeote OIA-40 | 161243 |
| Unclassified | Unclassified | Unclassified | Unclassified | crenarchaeote OIA-444 | 161244 |
| Unclassified | Unclassified | Unclassified | Unclassified | crenarchaeote OIA-592 | 161245 |
| Unclassified | Unclassified | Unclassified | Unclassified | crenarchaeote OIA-6 | 161242 |
| Unclassified | Unclassified | Unclassified | Unclassified | crenarchaeote SRI-298 | 132570 |
| Unclassified | Unclassified | Unclassified | Unclassified | crenarchaeote symbiont of Axinella damicornis | 171717 |
| Unclassified | Unclassified | Unclassified | Unclassified | crenarchaeote symbiont of Axinella verrucosa | 171716 |
| Unclassified | Unclassified | Unclassified | Unclassified | marine crenarchaeote RS.Sph.032 | 340702 |
| Unclassified | Unclassified | Unclassified | Unclassified | marine crenarchaeote RS.Sph.033 | 340703 |
| Unclassified | Unclassified | Unclassified | Unclassified | Octopus Spring nitrifying crenarchaeote OS70 | 498372 |
| Unclassified | Unclassified | Unclassified | Unclassified | crenarchaeote symbiont of Axinella sp. | 173517 |
| Unclassified | Unclassified | Unclassified | Unclassified | crenarchaeote enrichment clone CULT1196a | 442104 |
| Unclassified | Unclassified | Unclassified | Unclassified | crenarchaeote enrichment clone CULT1196b | 442105 |
| Unclassified | Unclassified | Unclassified | Unclassified | crenarchaeote enrichment clone CULT1198a | 442106 |
| Unclassified | Unclassified | Unclassified | Unclassified | crenarchaeote enrichment clone CULT1219a | 442107 |
| Unclassified | Unclassified | Unclassified | Unclassified | crenarchaeote enrichment clone CULT1224a | 442108 |
| Unclassified | Unclassified | Unclassified | Unclassified | crenarchaeote enrichment clone CULT1225a | 442109 |
| Unclassified | Unclassified | Unclassified | Unclassified | crenarchaeote enrichment clone CULT1231a | 442112 |
| Unclassified | Unclassified | Unclassified | Unclassified | crenarchaeote enrichment clone CULT1233a | 442110 |
| Unclassified | Unclassified | Unclassified | Unclassified | crenarchaeote enrichment clone CULT1537a | 442111 |
| Unclassified | Unclassified | Unclassified | Unclassified | crenarchaeote enrichment clone CULT1537b | 442113 |
| Unclassified | Unclassified | Unclassified | Unclassified | crenarchaeote enrichment clone CULT1539a | 442114 |
| Unclassified | Unclassified | Unclassified | Unclassified | crenarchaeote enrichment clone CULT1572a | 442115 |
| Unclassified | Unclassified | Unclassified | Unclassified | crenarchaeote enrichment clone CULT1580a | 442116 |
| Unclassified | Unclassified | Unclassified | Unclassified | crenarchaeote enrichment clone CULT1581a | 442117 |
| Unclassified | Unclassified | Unclassified | Unclassified | crenarchaeote enrichment clone CULT1587a | 442119 |
| Unclassified | Unclassified | Unclassified | Unclassified | crenarchaeote enrichment clone CULT1592a | 442118 |
| Unclassified | Unclassified | Unclassified | Unclassified | crenarchaeote enrichment clone F81 | 485627 |
| Unclassified | Unclassified | Unclassified | Unclassified | crenarchaeote enrichment culture clone SF06E-BA10-A01 | 550545 |
| Unclassified | Unclassified | Unclassified | Unclassified | crenarchaeote enrichment culture clone SF06E-BA10-A02 | 550546 |
| Unclassified | Unclassified | Unclassified | Unclassified | crenarchaeote enrichment culture clone SF06E-BA10-A03 | 550547 |
| Unclassified | Unclassified | Unclassified | Unclassified | crenarchaeote enrichment culture clone SF06E-BA10-B01 | 550548 |
| Unclassified | Unclassified | Unclassified | Unclassified | crenarchaeote enrichment culture clone SF06E-BA10-B02 | 550549 |
| Unclassified | Unclassified | Unclassified | Unclassified | crenarchaeote enrichment culture clone SF06E-BA10-B03 | 550550 |
| Unclassified | Unclassified | Unclassified | Unclassified | crenarchaeote enrichment culture clone SF06E-BA10-C01 | 550551 |
| Unclassified | Unclassified | Unclassified | Unclassified | crenarchaeote enrichment culture clone SF06E-BA10-C02 | 550552 |
| Unclassified | Unclassified | Unclassified | Unclassified | crenarchaeote enrichment culture clone SF06E-BA10-C03 | 550553 |
| Unclassified | Unclassified | Unclassified | Unclassified | crenarchaeote enrichment culture clone SF06E-BA10-D01 | 550554 |
| Unclassified | Unclassified | Unclassified | Unclassified | crenarchaeote enrichment culture clone SF06E-BA10-D02 | 550555 |
| Unclassified | Unclassified | Unclassified | Unclassified | crenarchaeote enrichment culture clone SF06E-BA10-D03 | 550556 |
| Unclassified | Unclassified | Unclassified | Unclassified | crenarchaeote enrichment culture clone SF06E-BA10-E01 | 550557 |
| Unclassified | Unclassified | Unclassified | Unclassified | crenarchaeote enrichment culture clone SF06E-BA10-E02 | 550558 |
| Unclassified | Unclassified | Unclassified | Unclassified | crenarchaeote enrichment culture clone SF06E-BA10-E03 | 550559 |
| Unclassified | Unclassified | Unclassified | Unclassified | crenarchaeote enrichment culture clone SF06E-BA10-F01 | 550560 |
| Unclassified | Unclassified | Unclassified | Unclassified | crenarchaeote enrichment culture clone SF06E-BA10-F02 | 550561 |
| Unclassified | Unclassified | Unclassified | Unclassified | crenarchaeote enrichment culture clone SF06E-BA10-F03 | 550562 |
| Unclassified | Unclassified | Unclassified | Unclassified | crenarchaeote enrichment culture clone SF06E-BA10-G01 | 550563 |
| Unclassified | Unclassified | Unclassified | Unclassified | crenarchaeote enrichment culture clone SF06E-BA10-G02 | 550564 |
| Unclassified | Unclassified | Unclassified | Unclassified | crenarchaeote enrichment culture clone SF06E-BA10-G03 | 550565 |
| Unclassified | Unclassified | Unclassified | Unclassified | crenarchaeote enrichment culture clone SF06E-BA10-H01 | 550566 |
| Unclassified | Unclassified | Unclassified | Unclassified | crenarchaeote enrichment culture clone SF06E-BA10-H02 | 550567 |
| Unclassified | Unclassified | Unclassified | Unclassified | crenarchaeote enrichment culture clone SF06E-BA10-H03 | 550568 |
| Unclassified | Unclassified | Unclassified | Unclassified | crenarchaeote enrichment culture clone SF06E-BA41-A01 | 550569 |
| Unclassified | Unclassified | Unclassified | Unclassified | crenarchaeote enrichment culture clone SF06E-BA41-A02 | 550570 |
| Unclassified | Unclassified | Unclassified | Unclassified | crenarchaeote enrichment culture clone SF06E-BA41-B01 | 550572 |
| Unclassified | Unclassified | Unclassified | Unclassified | crenarchaeote enrichment culture clone SF06E-BA41-B02 | 550573 |
| Unclassified | Unclassified | Unclassified | Unclassified | crenarchaeote enrichment culture clone SF06E-BA41-C01 | 550575 |
| Unclassified | Unclassified | Unclassified | Unclassified | crenarchaeote enrichment culture clone SF06E-BA41-C02 | 550576 |
| Unclassified | Unclassified | Unclassified | Unclassified | crenarchaeote enrichment culture clone SF06E-BA41-D01 | 550578 |
| Unclassified | Unclassified | Unclassified | Unclassified | crenarchaeote enrichment culture clone SF06E-BA41-D02 | 550579 |
| Unclassified | Unclassified | Unclassified | Unclassified | crenarchaeote enrichment culture clone SF06E-BA41-E01 | 550581 |
| Unclassified | Unclassified | Unclassified | Unclassified | crenarchaeote enrichment culture clone SF06E-BA41-F01 | 550584 |
| Unclassified | Unclassified | Unclassified | Unclassified | crenarchaeote enrichment culture clone SF06E-BA41-F02 | 550585 |
| Unclassified | Unclassified | Unclassified | Unclassified | crenarchaeote enrichment culture clone SF06E-BA41-G01 | 550587 |
| Unclassified | Unclassified | Unclassified | Unclassified | crenarchaeote enrichment culture clone SF06E-BA41-G02 | 550588 |
| Unclassified | Unclassified | Unclassified | Unclassified | crenarchaeote enrichment culture clone SF06E-BA41-H01 | 550590 |
| Unclassified | Unclassified | Unclassified | Unclassified | crenarchaeote enrichment culture clone SF06E-BA41-H02 | 550591 |
| Unclassified | Unclassified | Unclassified | Unclassified | crenarchaeote enrichment culture clone SF06E-BC11-A01 | 550593 |
| Unclassified | Unclassified | Unclassified | Unclassified | crenarchaeote enrichment culture clone SF06E-BC11-A02 | 550594 |
| Unclassified | Unclassified | Unclassified | Unclassified | crenarchaeote enrichment culture clone SF06E-BC11-B01 | 550596 |
| Unclassified | Unclassified | Unclassified | Unclassified | crenarchaeote enrichment culture clone SF06E-BC11-B02 | 550597 |
| Unclassified | Unclassified | Unclassified | Unclassified | crenarchaeote enrichment culture clone SF06E-BC11-C01 | 550599 |
| Unclassified | Unclassified | Unclassified | Unclassified | crenarchaeote enrichment culture clone SF06E-BC11-C02 | 550600 |
| Unclassified | Unclassified | Unclassified | Unclassified | crenarchaeote enrichment culture clone SF06E-BC11-D01 | 550601 |
| Unclassified | Unclassified | Unclassified | Unclassified | crenarchaeote enrichment culture clone SF06E-BC11-D02 | 550602 |
| Unclassified | Unclassified | Unclassified | Unclassified | crenarchaeote enrichment culture clone SF06E-BC11-E01 | 550603 |
| Unclassified | Unclassified | Unclassified | Unclassified | crenarchaeote enrichment culture clone SF06E-BC11-E02 | 550604 |
| Unclassified | Unclassified | Unclassified | Unclassified | crenarchaeote enrichment culture clone SF06E-BC11-F01 | 550606 |
| Unclassified | Unclassified | Unclassified | Unclassified | crenarchaeote enrichment culture clone SF06E-BC11-F02 | 550607 |
| Unclassified | Unclassified | Unclassified | Unclassified | crenarchaeote enrichment culture clone SF06E-BC11-G01 | 550609 |
| Unclassified | Unclassified | Unclassified | Unclassified | crenarchaeote enrichment culture clone SF06E-BC11-G02 | 550610 |
| Unclassified | Unclassified | Unclassified | Unclassified | crenarchaeote enrichment culture clone SF06E-BC11-H01 | 550612 |
| Unclassified | Unclassified | Unclassified | Unclassified | crenarchaeote enrichment culture clone SF06E-BC11-H02 | 550613 |
| Unclassified | Unclassified | Unclassified | Unclassified | crenarchaeote enrichment culture clone SF06E-BD31-A01 | 550615 |
| Unclassified | Unclassified | Unclassified | Unclassified | crenarchaeote enrichment culture clone SF06E-BD31-A02 | 550616 |
| Unclassified | Unclassified | Unclassified | Unclassified | crenarchaeote enrichment culture clone SF06E-BD31-B01 | 550618 |
| Unclassified | Unclassified | Unclassified | Unclassified | crenarchaeote enrichment culture clone SF06E-BD31-B02 | 550619 |
| Unclassified | Unclassified | Unclassified | Unclassified | crenarchaeote enrichment culture clone SF06E-BD31-C01 | 550621 |
| Unclassified | Unclassified | Unclassified | Unclassified | crenarchaeote enrichment culture clone SF06E-BD31-C02 | 550622 |
| Unclassified | Unclassified | Unclassified | Unclassified | crenarchaeote enrichment culture clone SF06E-BD31-D01 | 550624 |
| Unclassified | Unclassified | Unclassified | Unclassified | crenarchaeote enrichment culture clone SF06E-BD31-D02 | 550625 |
| Unclassified | Unclassified | Unclassified | Unclassified | crenarchaeote enrichment culture clone SF06E-BD31-E01 | 550627 |
| Unclassified | Unclassified | Unclassified | Unclassified | crenarchaeote enrichment culture clone SF06E-BD31-E02 | 550628 |
| Unclassified | Unclassified | Unclassified | Unclassified | crenarchaeote enrichment culture clone SF06E-BD31-F01 | 550630 |
| Unclassified | Unclassified | Unclassified | Unclassified | crenarchaeote enrichment culture clone SF06E-BD31-F02 | 550631 |
| Unclassified | Unclassified | Unclassified | Unclassified | crenarchaeote enrichment culture clone SF06E-BD31-G01 | 550633 |
| Unclassified | Unclassified | Unclassified | Unclassified | crenarchaeote enrichment culture clone SF06E-BD31-G02 | 550634 |
| Unclassified | Unclassified | Unclassified | Unclassified | crenarchaeote enrichment culture clone SF06E-BD31-H01 | 550636 |
| Unclassified | Unclassified | Unclassified | Unclassified | crenarchaeote enrichment culture clone SF06E-BD31-H02 | 550637 |
| Unclassified | Unclassified | Unclassified | Unclassified | crenarchaeote enrichment culture clone SF06E-BG30-A01 | 550639 |
| Unclassified | Unclassified | Unclassified | Unclassified | crenarchaeote enrichment culture clone SF06E-BG30-A02 | 550640 |
| Unclassified | Unclassified | Unclassified | Unclassified | crenarchaeote enrichment culture clone SF06E-BG30-A03 | 550641 |
| Unclassified | Unclassified | Unclassified | Unclassified | crenarchaeote enrichment culture clone SF06E-BG30-B01 | 550642 |
| Unclassified | Unclassified | Unclassified | Unclassified | crenarchaeote enrichment culture clone SF06E-BG30-B02 | 550643 |
| Unclassified | Unclassified | Unclassified | Unclassified | crenarchaeote enrichment culture clone SF06E-BG30-C01 | 550644 |
| Unclassified | Unclassified | Unclassified | Unclassified | crenarchaeote enrichment culture clone SF06E-BG30-C02 | 550645 |
| Unclassified | Unclassified | Unclassified | Unclassified | crenarchaeote enrichment culture clone SF06E-BG30-C03 | 550646 |
| Unclassified | Unclassified | Unclassified | Unclassified | crenarchaeote enrichment culture clone SF06E-BG30-D01 | 550647 |
| Unclassified | Unclassified | Unclassified | Unclassified | crenarchaeote enrichment culture clone SF06E-BG30-D02 | 550648 |
| Unclassified | Unclassified | Unclassified | Unclassified | crenarchaeote enrichment culture clone SF06E-BG30-E01 | 550649 |
| Unclassified | Unclassified | Unclassified | Unclassified | crenarchaeote enrichment culture clone SF06E-BG30-E02 | 550650 |
| Unclassified | Unclassified | Unclassified | Unclassified | crenarchaeote enrichment culture clone SF06E-BG30-F01 | 550651 |
| Unclassified | Unclassified | Unclassified | Unclassified | crenarchaeote enrichment culture clone SF06E-BG30-F02 | 550652 |
| Unclassified | Unclassified | Unclassified | Unclassified | crenarchaeote enrichment culture clone SF06E-BG30-F03 | 550653 |
| Unclassified | Unclassified | Unclassified | Unclassified | crenarchaeote enrichment culture clone SF06E-BG30-G01 | 550654 |
| Unclassified | Unclassified | Unclassified | Unclassified | crenarchaeote enrichment culture clone SF06E-BG30-G02 | 550655 |
| Unclassified | Unclassified | Unclassified | Unclassified | crenarchaeote enrichment culture clone SF06E-BG30-G03 | 550656 |
| Unclassified | Unclassified | Unclassified | Unclassified | crenarchaeote enrichment culture clone SF06E-BG30-H01 | 550657 |
| Unclassified | Unclassified | Unclassified | Unclassified | crenarchaeote enrichment culture clone SF06E-BG30-H02 | 550658 |
| Unclassified | Unclassified | Unclassified | Unclassified | planktonic crenarchaeote | 110442 |
| Unclassified | Unclassified | Unclassified | Unclassified | unculturable Mariana archaeon no. 1 | 73126 |
| Unclassified | Unclassified | Unclassified | Unclassified | unculturable Mariana archaeon no. 11 | 73127 |
| Unclassified | Unclassified | Unclassified | Unclassified | unculturable Mariana archaeon no. 15 | 73128 |
| Unclassified | Unclassified | Unclassified | Unclassified | uncultured ammonia-oxidizing crenarchaeote | 666997 |
| Unclassified | Unclassified | Unclassified | Unclassified | uncultured archaeon WCHA1-38 | 74272 |
| Unclassified | Unclassified | Unclassified | Unclassified | uncultured Crater Lake archaeon CL500-AR1 | 148262 |
| Unclassified | Unclassified | Unclassified | Unclassified | uncultured Crater Lake archaeon CL500-AR12 | 148263 |
| Unclassified | Unclassified | Unclassified | Unclassified | uncultured crenarchaeote | 29281 |
| Unclassified | Unclassified | Unclassified | Unclassified | uncultured crenarchaeote 10-H-08 | 311458 |
| Unclassified | Unclassified | Unclassified | Unclassified | uncultured crenarchaeote 29d5 | 684057 |
| Unclassified | Unclassified | Unclassified | Unclassified | uncultured crenarchaeote 57a5 | 684058 |
| Unclassified | Unclassified | Unclassified | Unclassified | uncultured crenarchaeote 76h13 | 684059 |
| Unclassified | Unclassified | Unclassified | Unclassified | uncultured crenarchaeote AM-20A 101 | 115035 |
| Unclassified | Unclassified | Unclassified | Unclassified | uncultured crenarchaeote AM-20A_102 | 115036 |
| Unclassified | Unclassified | Unclassified | Unclassified | uncultured crenarchaeote AM-20A_103 | 115037 |
| Unclassified | Unclassified | Unclassified | Unclassified | uncultured crenarchaeote AM-20A_104 | 115038 |
| Unclassified | Unclassified | Unclassified | Unclassified | uncultured crenarchaeote AM-20A_117 | 115039 |
| Unclassified | Unclassified | Unclassified | Unclassified | uncultured crenarchaeote AT-200_1 | 115040 |
| Unclassified | Unclassified | Unclassified | Unclassified | uncultured crenarchaeote AT-200_7 | 115041 |
| Unclassified | Unclassified | Unclassified | Unclassified | uncultured crenarchaeote AT-5_1 | 115042 |
| Unclassified | Unclassified | Unclassified | Unclassified | uncultured crenarchaeote CA-15_P18 | 115043 |
| Unclassified | Unclassified | Unclassified | Unclassified | uncultured crenarchaeote DeepAnt-EC39 | 247023 |
| Unclassified | Unclassified | Unclassified | Unclassified | uncultured crenarchaeote DN-200_1 | 115044 |
| Unclassified | Unclassified | Unclassified | Unclassified | uncultured crenarchaeote DN-5_1 | 115045 |
| Unclassified | Unclassified | Unclassified | Unclassified | uncultured crenarchaeote DS-5_1 | 115046 |
| Unclassified | Unclassified | Unclassified | Unclassified | uncultured crenarchaeote DS-5_P21 | 115047 |
| Unclassified | Unclassified | Unclassified | Unclassified | uncultured crenarchaeote FFSC1 | 78160 |
| Unclassified | Unclassified | Unclassified | Unclassified | uncultured crenarchaeote FFSC2 | 77776 |
| Unclassified | Unclassified | Unclassified | Unclassified | uncultured crenarchaeote FFSC3 | 78161 |
| Unclassified | Unclassified | Unclassified | Unclassified | uncultured crenarchaeote FFSC4 | 78162 |
| Unclassified | Unclassified | Unclassified | Unclassified | uncultured crenarchaeote FRD0 | 88890 |
| Unclassified | Unclassified | Unclassified | Unclassified | uncultured crenarchaeote | 75613 |
| | | | | KBSCul1 | |
| Unclassified | Unclassified | Unclassified | Unclassified | uncultured crenarchaeote KBSCul13 | 75618 |
| Unclassified | Unclassified | Unclassified | Unclassified | uncultured crenarchaeote KBSCul4 | 75614 |
| Unclassified | Unclassified | Unclassified | Unclassified | uncultured crenarchaeote KBSCul5 | 75615 |
| Unclassified | Unclassified | Unclassified | Unclassified | uncultured crenarchaeote KBSCul7 | 75616 |
| Unclassified | Unclassified | Unclassified | Unclassified | uncultured crenarchaeote KBSCul9 | 75617 |
| Unclassified | Unclassified | Unclassified | Unclassified | uncultured crenarchaeote KBSNat1 | 75619 |
| Unclassified | Unclassified | Unclassified | Unclassified | uncultured crenarchaeote KBSNat11 | 75622 |
| Unclassified | Unclassified | Unclassified | Unclassified | uncultured crenarchaeote KBSNat12 | 75623 |
| Unclassified | Unclassified | Unclassified | Unclassified | uncultured crenarchaeote KBSNat2 | 75620 |
| Unclassified | Unclassified | Unclassified | Unclassified | uncultured crenarchaeote KBSNat20 | 75624 |
| Unclassified | Unclassified | Unclassified | Unclassified | uncultured crenarchaeote KBSNat4 | 75621 |
| Unclassified | Unclassified | Unclassified | Unclassified | uncultured crenarchaeote MCG | 529375 |
| Unclassified | Unclassified | Unclassified | Unclassified | uncultured crenarchaeote ME-450 20 | 115048 |
| Unclassified | Unclassified | Unclassified | Unclassified | uncultured crenarchaeote ME-450_5 | 115049 |
| Unclassified | Unclassified | Unclassified | Unclassified | uncultured crenarchaeote ME-450_9 | 115050 |
| Unclassified | Unclassified | Unclassified | Unclassified | uncultured crenarchaeote ME-450 _P3 | 115051 |
| Unclassified | Unclassified | Unclassified | Unclassified | uncultured crenarchaeote ME-450 _P5 | 115052 |
| Unclassified | Unclassified | Unclassified | Unclassified | uncultured crenarchaeote ODPB-A12 | 95929 |
| Unclassified | Unclassified | Unclassified | Unclassified | uncultured crenarchaeote ODPB-A18 | 95930 |
| Unclassified | Unclassified | Unclassified | Unclassified | uncultured crenarchaeote ODPB-A2 | 95924 |
| Unclassified | Unclassified | Unclassified | Unclassified | uncultured crenarchaeote ODPB-A3 | 95925 |
| Unclassified | Unclassified | Unclassified | Unclassified | uncultured crenarchaeote ODPB-A6 | 95926 |
| Unclassified | Unclassified | Unclassified | Unclassified | uncultured crenarchaeote ODPB-A7 | 95927 |
| Unclassified | Unclassified | Unclassified | Unclassified | uncultured crenarchaeote ODPB-A9 | 95928 |
| Unclassified | Unclassified | Unclassified | Unclassified | uncultured crenarchaeote pBRKC108 | 91318 |
| Unclassified | Unclassified | Unclassified | Unclassified | uncultured crenarchaeote pBRKC125 | 91319 |
| Unclassified | Unclassified | Unclassified | Unclassified | uncultured crenarchaeote pBRKC129 | 91315 |
| Unclassified | Unclassified | Unclassified | Unclassified | uncultured crenarchaeote pBRKC135 | 91314 |
| Unclassified | Unclassified | Unclassified | Unclassified | uncultured crenarchaeote pBRKC82 | 91316 |
| Unclassified | Unclassified | Unclassified | Unclassified | uncultured crenarchaeote pBRKC86 | 91313 |
| Unclassified | Unclassified | Unclassified | Unclassified | uncultured crenarchaeote pBRKC88 | 91317 |
| Unclassified | Unclassified | Unclassified | Unclassified | uncultured crenarchaeote SB95_1 | 115053 |
| Unclassified | Unclassified | Unclassified | Unclassified | uncultured crenarchaeote SB95_20 | 115054 |
| Unclassified | Unclassified | Unclassified | Unclassified | uncultured crenarchaeote TRC132-3 | 115020 |
| Unclassified | Unclassified | Unclassified | Unclassified | uncultured crenarchaeote TRC132-6 | 115021 |
| Unclassified | Unclassified | Unclassified | Unclassified | uncultured crenarchaeote | 115022 |
| | | | | TRC132-7 | |
| Unclassified | Unclassified | Unclassified | Unclassified | uncultured crenarchaeote TRC132-8 | 115023 |
| Unclassified | Unclassified | Unclassified | Unclassified | uncultured crenarchaeote TRC132-9 | 115024 |
| Unclassified | Unclassified | Unclassified | Unclassified | uncultured crenarchaeote TRC23-10 | 115025 |
| Unclassified | Unclassified | Unclassified | Unclassified | uncultured crenarchaeote TRC23-28 | 115026 |
| Unclassified | Unclassified | Unclassified | Unclassified | uncultured crenarchaeote TRC23-30 | 115027 |
| Unclassified | Unclassified | Unclassified | Unclassified | uncultured crenarchaeote TRC23-31 | 115028 |
| Unclassified | Unclassified | Unclassified | Unclassified | uncultured crenarchaeote TRC23-38 | 115029 |
| Unclassified | Unclassified | Unclassified | Unclassified | uncultured crenarchaeote TREC16-1 | 258888 |
| Unclassified | Unclassified | Unclassified | Unclassified | uncultured crenarchaeote TREC16-10 | 258884 |
| Unclassified | Unclassified | Unclassified | Unclassified | uncultured crenarchaeote TREC16-12 | 258883 |
| Unclassified | Unclassified | Unclassified | Unclassified | uncultured crenarchaeote TREC16-14 | 258882 |
| Unclassified | Unclassified | Unclassified | Unclassified | uncultured crenarchaeote TREC16-16 | 258880 |
| Unclassified | Unclassified | Unclassified | Unclassified | uncultured crenarchaeote TREC16-18 | 258881 |
| Unclassified | Unclassified | Unclassified | Unclassified | uncultured crenarchaeote TREC16-3 | 258887 |
| Unclassified | Unclassified | Unclassified | Unclassified | uncultured crenarchaeote TREC16-6 | 258886 |
| Unclassified | Unclassified | Unclassified | Unclassified | uncultured crenarchaeote TREC16-9 | 258885 |
| Unclassified | Unclassified | Unclassified | Unclassified | uncultured crenarchaeote TREC89-10 | 258879 |
| Unclassified | Unclassified | Unclassified | Unclassified | uncultured crenarchaeote TREC89-11 | 258878 |
| Unclassified | Unclassified | Unclassified | Unclassified | uncultured crenarchaeote TREC89-136 | 258870 |
| Unclassified | Unclassified | Unclassified | Unclassified | uncultured crenarchaeote TREC89-17 | 258874 |
| Unclassified | Unclassified | Unclassified | Unclassified | uncultured crenarchaeote TREC89-20 | 258877 |
| Unclassified | Unclassified | Unclassified | Unclassified | uncultured crenarchaeote TREC89-24 | 258876 |
| Unclassified | Unclassified | Unclassified | Unclassified | uncultured crenarchaeote TREC89-30 | 258875 |
| Unclassified | Unclassified | Unclassified | Unclassified | uncultured crenarchaeote TREC89-34 | 258873 |
| Unclassified | Unclassified | Unclassified | Unclassified | uncultured crenarchaeote TREC89-36 | 258872 |
| Unclassified | Unclassified | Unclassified | Unclassified | uncultured crenarchaeote TREC89-44 | 258871 |
| Unclassified | Unclassified | Unclassified | Unclassified | uncultured crenarchaeote VAL11 | 85495 |
| Unclassified | Unclassified | Unclassified | Unclassified | uncultured crenarchaeote VAL114 | 85494 |
| Unclassified | Unclassified | Unclassified | Unclassified | uncultured crenarchaeote VAL151 | 85499 |
| Unclassified | Unclassified | Unclassified | Unclassified | uncultured crenarchaeote VAL159 | 85504 |
| Unclassified | Unclassified | Unclassified | Unclassified | uncultured crenarchaeote VAL18 | 85496 |
| Unclassified | Unclassified | Unclassified | Unclassified | uncultured crenarchaeote VAL20 | 85500 |
| Unclassified | Unclassified | Unclassified | Unclassified | uncultured crenarchaeote VAL29 | 85503 |
| Unclassified | Unclassified | Unclassified | Unclassified | uncultured crenarchaeote VAL42 | 85501 |
| Unclassified | Unclassified | Unclassified | Unclassified | uncultured crenarchaeote VAL48 | 85502 |
| Unclassified | Unclassified | Unclassified | Unclassified | uncultured crenarchaeote | 85505 |
| | | | | VAL76 | |
| Unclassified | Unclassified | Unclassified | Unclassified | uncultured crenarchaeote VAL81 | 85498 |
| Unclassified | Unclassified | Unclassified | Unclassified | uncultured crenarchaeote VAL96 | 85497 |
| Unclassified | Unclassified | Unclassified | Unclassified | uncultured Front Range soil crenarchaeote FRA0 | 147499 |
| Unclassified | Unclassified | Unclassified | Unclassified | uncultured Front Range soil crenarchaeote FRA1 | 147500 |
| Unclassified | Unclassified | Unclassified | Unclassified | uncultured Front Range soil crenarchaeote FRA27 | 147501 |
| Unclassified | Unclassified | Unclassified | Unclassified | uncultured Front Range soil crenarchaeote FRA27x2 | 147502 |
| Unclassified | Unclassified | Unclassified | Unclassified | uncultured Front Range soil crenarchaeote FRA31B | 147503 |
| Unclassified | Unclassified | Unclassified | Unclassified | uncultured Front Range soil crenarchaeote FRA32 | 147504 |
| Unclassified | Unclassified | Unclassified | Unclassified | uncultured Front Range soil crenarchaeote FRA33 | 147505 |
| Unclassified | Unclassified | Unclassified | Unclassified | uncultured Front Range soil crenarchaeote FRA9 | 147506 |
| Unclassified | Unclassified | Unclassified | Unclassified | uncultured Front Range soil crenarchaeote FRB1 | 147507 |
| Unclassified | Unclassified | Unclassified | Unclassified | uncultured Front Range soil crenarchaeote FRB15 | 147508 |
| Unclassified | Unclassified | Unclassified | Unclassified | uncultured Front Range soil crenarchaeote FRB25 | 147509 |
| Unclassified | Unclassified | Unclassified | Unclassified | uncultured Front Range soil crenarchaeote FRB27 | 147510 |
| Unclassified | Unclassified | Unclassified | Unclassified | uncultured Front Range soil crenarchaeote FRB31 | 147512 |
| Unclassified | Unclassified | Unclassified | Unclassified | uncultured Front Range soil crenarchaeote FRB32B | 147511 |
| Unclassified | Unclassified | Unclassified | Unclassified | uncultured Front Range soil crenarchaeote FRB32x2 | 147513 |
| Unclassified | Unclassified | Unclassified | Unclassified | uncultured Front Range soil crenarchaeote FRB33 | 147514 |
| Unclassified | Unclassified | Unclassified | Unclassified | uncultured Front Range soil crenarchaeote FRB38 | 147515 |
| Unclassified | Unclassified | Unclassified | Unclassified | uncultured Front Range soil crenarchaeote FRB9A | 147516 |
| Unclassified | Unclassified | Unclassified | Unclassified | uncultured Front Range soil crenarchaeote FRC0 | 147517 |
| Unclassified | Unclassified | Unclassified | Unclassified | uncultured Front Range soil crenarchaeote FRC15 | 147518 |
| Unclassified | Unclassified | Unclassified | Unclassified | uncultured Front Range soil crenarchaeote FRC1B | 147519 |
| Unclassified | Unclassified | Unclassified | Unclassified | uncultured Front Range soil crenarchaeote FRC1x2 | 147520 |
| Unclassified | Unclassified | Unclassified | Unclassified | uncultured Front Range soil crenarchaeote FRC27 | 147521 |
| Unclassified | Unclassified | Unclassified | Unclassified | uncultured Front Range soil crenarchaeote FRC32 | 147522 |
| Unclassified | Unclassified | Unclassified | Unclassified | uncultured Front Range soil crenarchaeote FRC33A | 147523 |
| Unclassified | Unclassified | Unclassified | Unclassified | uncultured Front Range soil crenarchaeote FRC33B | 147524 |
| Unclassified | Unclassified | Unclassified | Unclassified | uncultured Front Range soil crenarchaeote FRC38 | 147525 |
| Unclassified | Unclassified | Unclassified | Unclassified | uncultured Front Range soil crenarchaeote FRC9 | 147526 |
| Unclassified | Unclassified | Unclassified | Unclassified | uncultured Front Range soil crenarchaeote FRD0 | 147527 |
| Unclassified | Unclassified | Unclassified | Unclassified | uncultured Front Range soil crenarchaeote FRD15 | 147528 |
| Unclassified | Unclassified | Unclassified | Unclassified | uncultured Front Range soil crenarchaeote FRD25B | 147529 |
| Unclassified | Unclassified | Unclassified | Unclassified | uncultured Front Range soil crenarchaeote FRD25x2 | 147530 |
| Unclassified | Unclassified | Unclassified | Unclassified | uncultured Front Range soil crenarchaeote FRD31 | 147531 |
| Unclassified | Unclassified | Unclassified | Unclassified | uncultured Front Range soil | 147532 |
| | | | | crenarchaeote FRD32 | |
| Unclassified | Unclassified | Unclassified | Unclassified | uncultured Front Range soil crenarchaeote FRD33 | 147533 |
| Unclassified | Unclassified | Unclassified | Unclassified | uncultured Front Range soil crenarchaeote FRD38 | 147534 |
| Unclassified | Unclassified | Unclassified | Unclassified | uncultured Front Range soil crenarchaeote FRD9 | 147535 |
| Unclassified | Unclassified | Unclassified | Unclassified | uncultured Front Range soil crenarchaeote FRD9x2 | 147536 |
| Unclassified | Unclassified | Unclassified | Unclassified | uncultured Green Bay ferromanganous micronodule archaeon ARA7 | 140618 |
| Unclassified | Unclassified | Unclassified | Unclassified | uncultured Green Bay ferromanganous micronodule archaeon ARC12 | 140619 |
| Unclassified | Unclassified | Unclassified | Unclassified | uncultured marine archaeon AEGEAN_56 | 147159 |
| Unclassified | Unclassified | Unclassified | Unclassified | uncultured marine archaeon AEGEAN_67 | 147162 |
| Unclassified | Unclassified | Unclassified | Unclassified | uncultured marine archaeon AEGEAN_69 | 147160 |
| Unclassified | Unclassified | Unclassified | Unclassified | uncultured marine archaeon AEGEAN_70 | 147161 |
| Unclassified | Unclassified | Unclassified | Unclassified | uncultured marine crenarchaeote | 115413 |
| Unclassified | Unclassified | Unclassified | Unclassified | uncultured marine group I crenarchaeote | 360837 |
| Unclassified | Unclassified | Unclassified | Unclassified | unidentified archaeon Antarctic12 | 33863 |
| Unclassified | Unclassified | Unclassified | Unclassified | unidentified archaeon Antarctic5 | 33864 |
| Unclassified | Unclassified | Unclassified | Unclassified | unidentified archaeon C11 | 52260 |
| Unclassified | Unclassified | Unclassified | Unclassified | unidentified archaeon C20 | 52261 |
| Unclassified | Unclassified | Unclassified | Unclassified | unidentified archaeon C33 | 52262 |
| Unclassified | Unclassified | Unclassified | Unclassified | unidentified archaeon C35 | 52263 |
| Unclassified | Unclassified | Unclassified | Unclassified | unidentified archaeon C46 | 52264 |
| Unclassified | Unclassified | Unclassified | Unclassified | unidentified archaeon C48 | 52265 |
| Unclassified | Unclassified | Unclassified | Unclassified | unidentified archaeon C6 | 52266 |
| Unclassified | Unclassified | Unclassified | Unclassified | unidentified archaeon ICHT | 43688 |
| Unclassified | Unclassified | Unclassified | Unclassified | unidentified archaeon LMA137 | 57672 |
| Unclassified | Unclassified | Unclassified | Unclassified | unidentified archaeon LMA226 | 57674 |
| Unclassified | Unclassified | Unclassified | Unclassified | unidentified archaeon LMA229 | 57673 |
| Unclassified | Unclassified | Unclassified | Unclassified | unidentified archaeon LMA238 | 57671 |
| Unclassified | Unclassified | Unclassified | Unclassified | unidentified archaeon OARB | 33862 |
| Unclassified | Unclassified | Unclassified | Unclassified | unidentified archaeon PM23 | 52267 |
| Unclassified | Unclassified | Unclassified | Unclassified | unidentified archaeon PM7 | 52268 |
| Unclassified | Unclassified | Unclassified | Unclassified | unidentified archaeon PM8 | 52269 |
| Unclassified | Unclassified | Unclassified | Unclassified | unidentified archaeon SCA11 | 50858 |
| Unclassified | Unclassified | Unclassified | Unclassified | unidentified archaeon SCA1145 | 50793 |
| Unclassified | Unclassified | Unclassified | Unclassified | unidentified archaeon SCA1150 | 50850 |
| Unclassified | Unclassified | Unclassified | Unclassified | unidentified archaeon SCA1151 | 50851 |
| Unclassified | Unclassified | Unclassified | Unclassified | unidentified archaeon SCA1154 | 50852 |
| Unclassified | Unclassified | Unclassified | Unclassified | unidentified archaeon SCA1158 | 50853 |
| Unclassified | Unclassified | Unclassified | Unclassified | unidentified archaeon SCA1166 | 50854 |
| Unclassified | Unclassified | Unclassified | Unclassified | unidentified archaeon SCA1170 | 50855 |
| Unclassified | Unclassified | Unclassified | Unclassified | unidentified archaeon SCA1173 | 50856 |
| Unclassified | Unclassified | Unclassified | Unclassified | unidentified archaeon SCA1175 | 50857 |
| Unclassified | Unclassified | Unclassified | Unclassified | unidentified hydrothermal vent archaeon PVA _OTU_2 | 45967 |
| Unclassified | Unclassified | Unclassified | Unclassified | unidentified hydrothermal vent archaeon PVA_OTU_4 | 45969 |

**TABLE 5**

| Name of Unclassified Species | Taxonomy ID |
|---|---|
| crenarchaeote 768-28 | (242701) |
| crenarchaeote OIA-40 | (161243) |
| crenarchaeote OIA-444 | (161244) |
| crenarchaeote OIA-592 | (161245) |
| crenarchaeote OIA-6 | (161242) |
| crenarchaeote SRI-298 | (132570) |
| crenarchaeote symbiont of Axinella damicornis | (171717) |
| crenarchaeote symbiont of Axinella verrucosa | (171716) |
| marine crenarchaeote RS.Sph.032 | (340702) |
| marine crenarchaeote RS.Sph.033 | (340703) |
| Octopus Spring nitrifying crenarchaeote OS70 | (498372) |
| crenarchaeote symbiont of Axinella sp. | (173517) |
| crenarchaeote enrichment clone CULT1196a | (442104) |
| crenarchaeote enrichment clone CULT1196b | (442105) |
| crenarchaeote enrichment clone CULT1198a | (442106) |
| crenarchaeote enrichment clone CULT1219a | (442107) |
| crenarchaeote enrichment clone CULT1224a | (442108) |
| crenarchaeote enrichment clone CULT1225a | (442109) |
| crenarchaeote enrichment clone CULT1231a | (442112) |
| crenarchaeote enrichment clone CULT1233a | (442110) |
| crenarchaeote enrichment clone CULT1537a | (442111) |
| crenarchaeote enrichment clone CULT1537b | (442113) |
| crenarchaeote enrichment clone CULT1539a | (442114) |
| crenarchaeote enrichment clone CULT1572a | (442115) |
| crenarchaeote enrichment clone CULT1580a | (442116) |
| crenarchaeote enrichment clone CULT1581a | (442117) |
| crenarchaeote enrichment clone CULT1587a | (442119) |
| crenarchaeote enrichment clone CULT1592a | 442118) |
| crenarchaeote enrichment clone F81 | 485627 |
| crenarchaeote enrichment culture clone SF06E-BA10-A01 | 550545 |
| crenarchaeote enrichment culture clone SF06E-BA10-A02 | 550546 |
| crenarchaeote enrichment culture clone SF06E-BA10-A03 | 550547 |
| crenarchaeote enrichment culture clone SF06E-BA10-B01 | 550548) |
| crenarchaeote enrichment culture clone SF06E-BA10-B02 | 550549) |
| crenarchaeote enrichment culture clone SF06E-BA10-B03 | 550550) |
| crenarchaeote enrichment culture clone SF06E-BA10-C01 | 550551) |
| crenarchaeote enrichment culture clone SF06E-BA10-C02 | 550552) |
| crenarchaeote enrichment culture clone SF06E-BA10-C03 | 550553) |
| crenarchaeote enrichment culture clone SF06E-BA10-D01 | 550554) |
| crenarchaeote enrichment culture clone SF06E-BA10-D02 | 550555) |
| crenarchaeote enrichment culture clone SF06E-BA10-D03 | 550556) |
| crenarchaeote enrichment culture clone SF06E-BA10-E01 | 550557) |
| crenarchaeote enrichment culture clone SF06E-BA10-E02 | 550558) |
| crenarchaeote enrichment culture clone SF06E-BA10-E03 | 550559) |
| crenarchaeote enrichment culture clone SF06E-BA10-F01 | 550560) |
| crenarchaeote enrichment culture clone SF06E-BA10-F02 | 550561) |
| crenarchaeote enrichment culture clone SF06E-BA10-F03 | 550562) |
| crenarchaeote enrichment culture clone SF06E-BA10-G01 | 550563) |
| crenarchaeote enrichment culture clone SF06E-BA10-G02 | 550564) |
| crenarchaeote enrichment culture clone SF06E-BA10-G03 | 550565) |
| crenarchaeote enrichment culture clone SF06E-BA10-H01 | 550566) |
| crenarchaeote enrichment culture clone SF06E-BA10-H02 | 550567) |
| crenarchaeote enrichment culture clone SF06E-BA10-H03 | 550568) |
| crenarchaeote enrichment culture clone SF06E-BA41-A01 | 550569) |
| crenarchaeote enrichment culture clone SF06E-BA41-A02 | 550570) |
| crenarchaeote enrichment culture clone SF06E-BA41-B01 | 550572) |
| crenarchaeote enrichment culture clone SF06E-BA41-B02 | 550573) |
| crenarchaeote enrichment culture clone SF06E-BA41-C01 | 550575) |
| crenarchaeote enrichment culture clone SF06E-BA41-C02 | 550576) |
| crenarchaeote enrichment culture clone SF06E-BA41-D01 | 550578) |
| crenarchaeote enrichment culture clone SF06E-BA41-D02 | 550579) |
| crenarchaeote enrichment culture clone SF06E-BA41-E01 | 550581) |
| crenarchaeote enrichment culture clone SF06E-BA41-F01 | 550584) |
| crenarchaeote enrichment culture clone SF06E-BA41-F02 | 550585) |
| crenarchaeote enrichment culture clone SF06E-BA41-G01 | 550587) |
| crenarchaeote enrichment culture clone SF06E-BA41-G02 | 550588) |
| crenarchaeote enrichment culture clone SF06E-BA41-H01 | 550590) |
| crenarchaeote enrichment culture clone SF06E-BA41-H02 | 550591) |
| crenarchaeote enrichment culture clone SF06E-BC11-A01 | 550593) |
| crenarchaeote enrichment culture clone SF06E-BC11-A02 | 550594) |
| crenarchaeote enrichment culture clone SF06E-BC11-B01 | 550596) |
| crenarchaeote enrichment culture clone SF06E-BC11-B02 | 550597) |
| crenarchaeote enrichment culture clone SF06E-BC11-C01 | 550599) |
| crenarchaeote enrichment culture clone SF06E-BC11-C02 | 550600) |
| crenarchaeote enrichment culture clone SF06E-BC11-D01 | 550601) |
| crenarchaeote enrichment culture clone SF06E-BC11-D02 | 550602) |
| crenarchaeote enrichment culture clone SF06E-BC11-E01 | 550603) |
| crenarchaeote enrichment culture clone SF06E-BC11-E02 | 550604) |
| crenarchaeote enrichment culture clone SF06E-BC11-F01 | 550606) |
| crenarchaeote enrichment culture clone SF06E-BC11-F02 | 550607) |
| crenarchaeote enrichment culture clone SF06E-BC11-G01 | 550609) |
| crenarchaeote enrichment culture clone SF06E-BC11-G02 | 550610) |
| crenarchaeote enrichment culture clone SF06E-BC11-H01 | 550612) |
| crenarchaeote enrichment culture clone SF06E-BC11-H02 | 550613) |
| crenarchaeote enrichment culture clone SF06E-BD31-A01 | 550615) |
| crenarchaeote enrichment culture clone SF06E-BD31-A02 | 550616) |
| crenarchaeote enrichment culture clone SF06E-BD31-B01 | 550618) |
| crenarchaeote enrichment culture clone SF06E-BD31-B02 | 550619) |
| crenarchaeote enrichment culture clone SF06E-BD31-C01 | 550621) |
| crenarchaeote enrichment culture clone SF06E-BD31-C02 | 550622) |
| crenarchaeote enrichment culture clone SF06E-BD31-D01 | 550624) |
| crenarchaeote enrichment culture clone SF06E-BD31-D02 | 550625) |
| crenarchaeote enrichment culture clone SF06E-BD31-E01 | 550627) |
| crenarchaeote enrichment culture clone SF06E-BD31-E02 | 550628) |
| crenarchaeote enrichment culture clone SF06E-BD31-F01 | 550630) |
| crenarchaeote enrichment culture clone SF06E-BD31-F02 | 550631) |
| crenarchaeote enrichment culture clone SF06E-BD31-G01 | 550633) |
| crenarchaeote enrichment culture clone SF06E-BD31-G02 | 550634) |
| crenarchaeote enrichment culture clone SF06E-BD31-H01 | 550636) |
| crenarchaeote enrichment culture clone SF06E-BD31-H02 | 550637) |
| crenarchaeote enrichment culture clone SF06E-BG30-A01 | 550639) |
| crenarchaeote enrichment culture clone SF06E-BG30-A02 | 550640 |
| crenarchaeote enrichment culture clone SF06E-BG30-A03 | 550641 |
| crenarchaeote enrichment culture clone SF06E-BG30-B01 | 550642 |
| crenarchaeote enrichment culture clone SF06E-BG30-B02 | 550643 |
| crenarchaeote enrichment culture clone SF06E-BG30-C01 | 550644 |
| crenarchaeote enrichment culture clone SF06E-BG30-C02 | 550645 |
| crenarchaeote enrichment culture clone SF06E-BG30-C03 | 550646 |
| crenarchaeote enrichment culture clone SF06E-BG30-D01 | 550647 |
| crenarchaeote enrichment culture clone SF06E-BG30-D02 | 550648 |
| crenarchaeote enrichment culture clone SF06E-BG30-E01 | 550649 |
| crenarchaeote enrichment culture clone SF06E-BG30-E02 | 550650 |
| crenarchaeote enrichment culture clone SF06E-BG30-F01 | 550651 |
| crenarchaeote enrichment culture clone SF06E-BG30-F02 | 550652 |
| crenarchaeote enrichment culture clone SF06E-BG30-F03 | 550653 |
| crenarchaeote enrichment culture clone SF06E-BG30-G01 | 550654 |
| crenarchaeote enrichment culture clone SF06E-BG30-G02 | 550655 |
| crenarchaeote enrichment culture clone SF06E-BG30-G03 | 550656 |
| crenarchaeote enrichment culture clone SF06E-BG30-H01 | 550657 |
| crenarchaeote enrichment culture clone SF06E-BG30-H02 | 550658 |
| crenarchaeote enrichment culture clone SF06E-BG30-H03 | 550659 |
| planktonic crenarchaeote | 110442 |
| unculturable Mariana archaeon no. 1 | 73126 |
| unculturable Mariana archaeon no. 11 | 73127 |
| unculturable Mariana archaeon no. 15 | 73128 |
| uncultured ammonia-oxidizinq crenarchaeote | 666997 |
| uncultured archaeon WCHA1-38 | 74272 |
| uncultured Crater Lake archaeon CL500-AR1 | 148262 |
| uncultured Crater Lake archaeon CL500-AR12 | 148263 |
| uncultured crenarchaeote | 29281 |
| uncultured crenarchaeote 10-H-08 | 311458 |
| uncultured crenarchaeote 29d5 | 684057 |
| uncultured crenarchaeote 57a5 | 684058 |
| uncultured crenarchaeote 76h13 | 684059 |
| uncultured crenarchaeote AM-20A_101 | 115035 |
| uncultured crenarchaeote AM-20A_102 | 115036 |
| uncultured crenarchaeote AM-20A_103 | 115037 |
| uncultured crenarchaeote AM-20A_104 | 115038 |
| uncultured crenarchaeote AM-20A_117 | 115039 |
| uncultured crenarchaeote AT-200_1 | 115040 |
| uncultured crenarchaeote AT-200_7 | 115041 |
| uncultured crenarchaeote AT-5_1 | 115042 |
| uncultured crenarchaeote CA-15_P18 | 115043 |
| uncultured crenarchaeote DeepAnt-EC39 | 247023 |
| uncultured crenarchaeote DN-200_1 | 115044 |
| uncultured crenarchaeote DN-5_1 | 115045 |
| uncultured crenarchaeote DS-5_1 | 115046 |
| uncultured crenarchaeote DS-5_P21 | 115047 |
| uncultured crenarchaeote FFSC1 | 78160 |
| uncultured crenarchaeote FFSC2 | 77776 |
| uncultured crenarchaeote FFSC3 | 78161 |
| uncultured crenarchaeote FFSC4 | 78162 |
| uncultured crenarchaeote FRD0 | 88890 |
| uncultured crenarchaeote KBSCul1 | 75613 |
| uncultured crenarchaeote KBSCul13 | 75618 |
| uncultured crenarchaeote KBSCul4 | 75614 |
| uncultured crenarchaeote KBSCul5 | 75615 |
| uncultured crenarchaeote KBSCul7 | 75616 |
| uncultured crenarchaeote KBSCul9 | 75617 |
| uncultured crenarchaeote KBSNatl | 75619 |
| uncultured crenarchaeote KBSNat11 | 75622 |
| uncultured crenarchaeote KBSNat12 | 75623 |
| uncultured crenarchaeote KBSNat2 | 75620 |
| uncultured crenarchaeote KBSNat20 | 75624 |
| uncultured crenarchaeote KBSNat4 | 75621 |
| uncultured crenarchaeote MCG | 529375 |
| uncultured crenarchaeote ME-450_20 | 115048 |
| uncultured crenarchaeote ME-450_5 | 115049 |
| uncultured crenarchaeote ME-450_9 | 115050 |
| uncultured crenarchaeote ME-450_P3 | 115051 |
| uncultured crenarchaeote ME-450_P5 | 115052 |
| uncultured crenarchaeote ODPB-A12 | 95929 |
| uncultured crenarchaeote ODPB-A18 | 95930 |
| uncultured crenarchaeote ODPB-A2 | 95924 |
| uncultured crenarchaeote ODPB-A3 | 95925 |
| uncultured crenarchaeote ODPB-A6 | 95926 |
| uncultured crenarchaeote ODPB-A7 | 95927 |
| uncultured crenarchaeote ODPB-A9 | 95928 |
| uncultured crenarchaeote pBRKC108 | 91318 |
| uncultured crenarchaeote pBRKC125 | 91319 |
| uncultured crenarchaeote pBRKC129 | 91315 |
| uncultured crenarchaeote pBRKC135 | 91314 |
| uncultured crenarchaeote pBRKC82 | 91316 |
| uncultured crenarchaeote pBRKC86 | 91313 |
| uncultured crenarchaeote pBRKC88 | 91317 |
| uncultured crenarchaeote SB95_1 | 115053 |
| uncultured crenarchaeote SB95_20 | 115054 |
| uncultured crenarchaeote TRC132-3 | 115020 |
| uncultured crenarchaeote TRC132-6 | 115021 |
| uncultured crenarchaeote TRC132-7 | 115022 |
| uncultured crenarchaeote TRC132-8 | 115023 |
| uncultured crenarchaeote TRC132-9 | 115024 |
| uncultured crenarchaeote TRC23-10 | 115025 |
| uncultured crenarchaeote TRC23-28 | 115026 |
| uncultured crenarchaeote TRC23-30 | 115027 |
| uncultured crenarchaeote TRC23-31 | 115028 |
| uncultured crenarchaeote TRC23-38 | 115029 |
| uncultured crenarchaeote TREC16-1 | 258888 |
| uncultured crenarchaeote TREC16-10 | 258884 |
| uncultured crenarchaeote TREC16-12 | 258883 |
| uncultured crenarchaeote TREC16-14 | 258882 |
| uncultured crenarchaeote TREC16-16 | 258881 |
| uncultured crenarchaeote TREC16-18 | 258880 |
| uncultured crenarchaeote TREC16-3 | 258887 |
| uncultured crenarchaeote TREC16-6 | 258886 |
| uncultured crenarchaeote TREC16-9 | 258885 |
| uncultured crenarchaeote TREC89-10 | 258879 |
| uncultured crenarchaeote TREC89-11 | 258878 |
| uncultured crenarchaeote TREC89-136 | 258870 |
| uncultured crenarchaeote TREC89-17 | 258874 |
| uncultured crenarchaeote TREC89-20 | 258877 |
| uncultured crenarchaeote TREC89-24 | 258876 |
| uncultured crenarchaeote TREC89-30 | 258875 |
| uncultured crenarchaeote TREC89-34 | 258873 |
| uncultured crenarchaeote TREC89-36 | 258872 |
| uncultured crenarchaeote TREC89-44 | 258871 |
| uncultured crenarchaeote VAL11 | 85495 |
| uncultured crenarchaeote VAL114 | 85494 |
| uncultured crenarchaeote VAL151 | 85499 |
| uncultured crenarchaeote VAL159 | 85496 |
| uncultured crenarchaeote VAL18 | 85504 |
| uncultured crenarchaeote VAL20 | 85500 |
| uncultured crenarchaeote VAL29 | 85503 |
| uncultured crenarchaeote VAL42 | 85501 |
| uncultured crenarchaeote VAL48 | 85502 |
| uncultured crenarchaeote VAL76 | 85505 |
| uncultured crenarchaeote VAL81 | 85498 |
| uncultured crenarchaeote VAL96 | 85497 |
| uncultured Front Ranqe soil crenarchaeote FRA0 | 147499 |
| uncultured Front Ranqe soil crenarchaeote FRA1 | 147500 |
| uncultured Front Ranqe soil crenarchaeote FRA27 | 147501 |
| uncultured Front Ranqe soil crenarchaeote FRA27x2 | 147502 |
| uncultured Front Ranqe soil crenarchaeote FRA31B | 147503 |
| uncultured Front Ranqe soil crenarchaeote FRA32 | 147504 |
| uncultured Front Ranqe soil crenarchaeote FRA33 | 147505 |
| uncultured Front Ranqe soil crenarchaeote FRA9 | 147506 |
| uncultured Front Ranqe soil crenarchaeote FRB1 | 147507 |
| uncultured Front Ranqe soil crenarchaeote FRB15 | 147508 |
| uncultured Front Ranqe soil crenarchaeote FRB25 | 147509 |
| uncultured Front Ranqe soil crenarchaeote FRB27 | 147510 |
| uncultured Front Ranqe soil crenarchaeote FRB31 | 147511 |
| uncultured Front Ranqe soil crenarchaeote FRB32B | 147512 |
| uncultured Front Ranqe soil crenarchaeote FRB32x2 | 147513 |
| uncultured Front Ranqe soil crenarchaeote FRB33 | 147514 |
| uncultured Front Ranqe soil crenarchaeote FRB38 | 147515 |
| uncultured Front Ranqe soil crenarchaeote FRB9A | 147516 |
| uncultured Front Ranqe soil crenarchaeote FRC0 | 147517 |
| uncultured Front Ranqe soil crenarchaeote FRC15 | 147518 |
| uncultured Front Ranqe soil crenarchaeote FRC1B | 147519 |
| uncultured Front Ranqe soil crenarchaeote FRC1x2 | 147520 |
| uncultured Front Ranqe soil crenarchaeote FRC27 | 147521 |
| uncultured Front Ranqe soil crenarchaeote FRC32 | 147522 |
| uncultured Front Ranqe soil crenarchaeote FRC33A | 147523 |
| uncultured Front Ranqe soil crenarchaeote FRC33B | 147524 |
| uncultured Front Ranqe soil crenarchaeote FRC38 | 147525 |
| uncultured Front Ranqe soil crenarchaeote FRC9 | 147526 |
| uncultured Front Ranqe soil crenarchaeote FRD0 | 147527 |
| uncultured Front Ranqe soil crenarchaeote FRD15 | 147528 |
| uncultured Front Ranqe soil crenarchaeote FRD25B | 147529 |
| uncultured Front Ranqe soil crenarchaeote FRD25x2 | 147530 |
| uncultured Front Ranqe soil crenarchaeote FRD31 | 147531 |
| uncultured Front Ranqe soil crenarchaeote FRD32 | 147532 |
| uncultured Front Ranqe soil crenarchaeote FRD33 | 147533 |
| uncultured Front Ranqe soil crenarchaeote FRD38 | 147534 |
| uncultured Front Ranqe soil crenarchaeote FRD9 | 147535 |
| uncultured Front Ranqe soil crenarchaeote FRD9x2 | 147536 |
| uncultured Green Bay ferromanganous micronodule archaeon ARA7 | 140618 |
| uncultured Green Bay ferromanganous micronodule archaeon ARC12 | 140619 |
| uncultured marine archaeon AEGEAN_56 | 147159 |
| uncultured marine archaeon AEGEAN_67 | 147162 |
| uncultured marine archaeon AEGEAN_69 | 147160 |
| uncultured marine archaeon AEGEAN_70 | 147161 |
| uncultured marine crenarchaeote | 115413 |
| uncultured marine group I crenarchaeote | 360837 |
| unidentified archaeon Antarctic12 | 33863 |
| unidentified archaeon Antarctic5 | 33864 |
| unidentified archaeon C11 | 52260 |
| unidentified archaeon C20 | 52261 |
| unidentified archaeon C33 | 52262 |
| unidentified archaeon C35 | 52263 |
| unidentified archaeon C46 | 52264 |
| unidentified archaeon C48 | 52265 |
| unidentified archaeon C6 | 52266 |
| unidentified archaeon ICHT | 43688 |
| unidentified archaeon LMA137 | 57672 |
| unidentified archaeon LMA226 | 57674 |
| unidentified archaeon LMA229 | 57673 |
| unidentified archaeon LMA238 | 57671 |
| unidentified archaeon OARB | 33862 |
| unidentified archaeon PM23 | 52267 |
| unidentified archaeon PM7 | 52268 |
| unidentified archaeon PM8 | 52269 |
| unidentified archaeon SCA11 | 50858 |
| unidentified archaeon SCA1145 | 50793 |
| unidentified archaeon SCA1150 | 50850 |
| unidentified archaeon SCA1151 | 50851 |
| unidentified archaeon SCA1154 | 50852 |
| unidentified archaeon SCA1158 | 50853 |
| unidentified archaeon SCA1166 | 50854 |
| unidentified archaeon SCA1170 | 50855 |
| unidentified archaeon SCA1173 | 50856 |
| unidentified archaeon SCA1175 | 50857 |
| unidentified hydrothermal vent archaeon PVA_OTU_2 | 45967 |
| unidentified hydrothermal vent archaeon PVA_OTU_4 | 45969 |

## Claims

1. A method for producing methane, the method comprising:
(a) supplying electricity to an anode (218) and a porous electrically conductive cathode (216) of a biological reactor (202), wherein the biological reactor (i) is coupled to a source of electricity (204) and a source of carbon dioxide (206), (ii) has at least a first chamber (212) containing at least the cathode (216), a culture comprising methanogenic microorganisms, and water, a second chamber (214) containing at least the anode (218), and a proton permeable, gas impermeable barrier (220) separating the anode (218) from the cathode (216), and (iii) has a current collector (230, 232) coupled to each of the anode (218) and the cathode (216),
wherein the cathode (216) is impregnated with the methanogenic microorganisms and with an aqueous electrolytic medium, wherein the methanogenic microorganisms are in a passage (238) formed between the barrier (220) and the current collector (230) coupled to the cathode (216) and located between an inlet for carbon dioxide (234) and an outlet for methane (236),
wherein the culture is maintained in the aqueous electrolytic medium in the passage (238) of the first chamber (212) at a temperature above 50 °C;
(b) supplying carbon dioxide to the first chamber (212) through the inlet (234) of the first chamber (212), whereupon the carbon dioxide is dissolved into the aqueous electrolytic medium;
(c) circulating the aqueous electrolytic medium comprising the dissolved carbon dioxide through the cathode (216); and
(d) collecting methane from the outlet (236) of the first chamber (212).

2. The method according to claim 1, wherein the culture is maintained at a temperature of about 55° C or higher, optionally, about 60 ° C or higher.

3. The method according to claim 1, wherein the culture comprises Archaea adapted to nearly stationary growth conditions.

4. The method according to claim 1, wherein the biological reactor (202) has an operating state and a dormant state, the reactor (202) changing from the dormant state to the operating state without addition of methanogenic microorganisms, optionally wherein the dormant state exists when the biological reactor (202) is decoupled from the source of electricity (204) or the source of carbon dioxide (206).

5. The method according to claim 1, wherein the barrier (220) comprises a solid polymer electrolyte membrane.

6. The method according to claim 1, wherein the porous electrically conductive cathode (216) comprises a reticulated carbon foam.

7. The method according to claim 1, wherein the culture comprises *Archaea* of the subkingdom *Euryarcheaota,* optionally wherein the culture is a monoculture of *Euryarcheaota.*

8. The method according to claim 7, wherein the *Archaea* consist of *Methanothermobacter thermautotrophicus.*

9. The method of claim 1, wherein the current collector coupled to the cathode (230) is a solid disc of material that maintains a sealed condition within the first chamber (212) between the inlet for the carbon dioxide (234) and the outlet for the methane (236).

10. The method according to claim 1, wherein the current collector for the anode (232) defines a porous gas diffusion layer on which an anode catalyst is disposed, wherein the porous gas diffusion layer permits gaseous byproducts to exit the second chamber (214).

11. The method according to claim 1, wherein the culture resides within the circulating aqueous electrolytic medium.

12. The method according to claim 1, wherein the culture is bound to the porous cathode (216).

13. The method according to claim 1, wherein water is a primary net electron donor for the methanogenic microorganisms.

14. The method according to claim 1, wherein an organic carbon source is absent in the medium.

## Patentansprüche

1. Verfahren zur Herstellung von Methan, wobei das Verfahren umfasst:
(a) Zuführen von Elektrizität zu einer Anode (218) und einer porösen, elektrisch leitenden Kathode (216) eines biologischen Reaktors (202), wobei der biologische Reaktor
(i) mit einer Elektrizitätsquelle (204) und einer Kohlendioxidquelle (206) gekoppelt ist,
(ii) mindestens eine erste Kammer (212) aufweist, die mindestens die Kathode (216), eine Kultur, die methanogene Mikroorganismen und Wasser umfasst, enthält, eine zweite Kammer (214), die mindestens die Anode (218), und eine protonendurchlässige, gasundurchlässige Barriere (220), die die Anode (218) von der Kathode (216) trennt, enthält und
(iii) einen Stromabnehmer (230, 232), der sowohl mit der Anode (218) als auch mit der Kathode (216) gekoppelt ist, aufweist,
wobei die Kathode (216) mit den methanogenen Mikroorganismen und mit einem wässrigen elektrolytischen Medium imprägniert ist, wobei sich die methanogenen Mikroorganismen in einem Durchgang (238) befinden, der zwischen der Barriere (220) und dem Stromabnehmer (230) gebildet ist, der mit der Kathode (216) gekoppelt ist und zwischen einem Einlass für Kohlendioxid (234) und einem Auslass für Methan (236) angeordnet ist,
wobei die Kultur in dem wässrigen elektrolytischen Medium in dem Durchgang (238) der ersten Kammer (212) bei einer Temperatur über 50 °C gehalten wird;
(b) Zuführen von Kohlendioxid zu der ersten Kammer (212) durch den Einlass (234) der ersten Kammer (212), woraufhin das Kohlendioxid in dem wässrigen elektrolytischen Medium gelöst wird;
(c) Zirkulieren des wässrigen elektrolytischen Mediums, das das gelöste Kohlendioxid umfasst, durch die Kathode (216); und
(d) Sammeln von Methan aus dem Auslass (236) der ersten Kammer (212).

2. Verfahren nach Anspruch 1, wobei die Kultur bei einer Temperatur von etwa 55° C oder höher, optional etwa 60° C oder höher, gehalten wird.

3. Verfahren nach Anspruch 1, wobei die Kultur *Archaea* umfasst, die an nahezu stationäre Wachstumsbedingungen angepasst sind.

4. Verfahren nach Anspruch 1, wobei der biologische Reaktor (202) einen Betriebszustand und einen Ruhezustand aufweist, wobei der Reaktor (202) ohne Zugabe von methanogenen Mikroorganismen vom Ruhezustand in den Betriebszustand wechselt, optional, wobei der Ruhezustand besteht, wenn der biologische Reaktor (202) von der Stromquelle (204) oder der Kohlendioxidquelle (206) abgekoppelt ist.

5. Verfahren nach Anspruch 1, wobei die Barriere (220) eine feste Polymerelektrolytmembran umfasst.

6. Verfahren nach Anspruch 1, wobei die poröse, elektrisch leitende Kathode (216) einen retikulierten Kohlenstoffschaum umfasst.

7. Verfahren nach Anspruch 1, wobei die Kultur *Archaea* des Unterreichs *Euryarchaeota* umfasst, optional wobei die Kultur eine Monokultur von *Euryarchaeota* ist.

8. Verfahren nach Anspruch 7, wobei die *Archaea* aus *Methanothermobacter thermautotrophicus* bestehen.

9. Verfahren nach Anspruch 1, wobei der an die Kathode (230) gekoppelte Stromabnehmer eine feste Scheibe aus einem Material ist, das innerhalb der ersten Kammer (212) zwischen dem Einlass für das Kohlendioxid (234) und dem Auslass für das Methan (236) einen abgedichteten Zustand aufrechterhält.

10. Verfahren nach Anspruch 1, wobei der Stromabnehmer für die Anode (232) eine poröse Gasdiffusionsschicht definiert, auf der ein Anodenkatalysator angeordnet ist, wobei die poröse Gasdiffusionsschicht gasförmige Nebenprodukte aus der zweiten Kammer (214) austreten lässt.

11. Verfahren nach Anspruch 1, wobei die Kultur in dem zirkulierenden wässrigen elektrolytischen Medium verbleibt.

12. Verfahren nach Anspruch 1, wobei die Kultur an die poröse Kathode (216) gebunden ist.

13. Verfahren nach Anspruch 1, wobei Wasser ein primärer Netto-Elektronendonator für die methanogenen Mikroorganismen ist.

14. Verfahren nach Anspruch 1, bei dem eine organische Kohlenstoffquelle in dem Medium fehlt.

## Revendications

1. Un procédé de production du méthane, le procédé comprenant :
(a) la délivrance d'électricité à une anode (218) et à une cathode poreuse électriquement conductrice (216) d'un réacteur biologique (202), dans lequel le réacteur biologique (i) est couplé à une source d'électricité (204) et à une source de dioxyde de carbone (206), (ii) possède au moins une première chambre (212) contenant au moins la cathode (216), une culture comprenant des microorganismes méthanogènes, et de l'eau, une seconde chambre (214) contenant au moins l'anode (218), et une barrière imperméable aux gaz et perméable aux protons (220) séparant l'anode (218) de la cathode (216), et (iii) possède un collecteur de courant (230, 232) couplé à chacune de l'anode (218) et de la cathode (216),
dans lequel la cathode (216) est imprégnée par les microorganismes méthanogènes et par un milieu électrolytique aqueux, dans lequel les microorganismes méthanogènes sont dans un passage (238) formé entre la barrière (220) et le collecteur de courant (230) couplé à la cathode (216) et situé entre une entrée du dioxyde de carbone (234) et une sortie du méthane (236),
dans lequel la culture est maintenue à une température supérieure à 50°C dans le milieu électrolytique aqueux dans le passage (238) de la première chambre (212) ;
(b) la délivrance du dioxyde de carbone à la première chambre (212) par l'intermédiaire de l'entrée (234) de la première chambre (212), le dioxyde de carbone y étant dissous dans le milieu électrolytique aqueux ;
(c) la mise en circulation, au travers de la cathode (216), du milieu électrolytique aqueux comprenant le dioxyde de carbone dissous ; et
(d) le recueil du méthane à partir de la sortie (236) de la première chambre (212).

2. Le procédé selon la revendication 1, dans lequel la culture est maintenue à une température d'environ 55°C ou plus, éventuellement d'environ 60°C ou plus.

3. Le procédé de la revendication 1, dans lequel la culture comprend des *Archaea* aptes à croître dans des conditions voisines de la stabilité.

4. Le procédé de la revendication 1, dans lequel le réacteur biologique (202) possède un état fonctionnel et un état dormant, le réacteur (202) passant de l'état dormant à l'état fonctionnel sans addition de microorganismes méthanogènes, éventuellement dans lequel l'état dormant existe lorsque le réacteur biologique (202) est découplé de la source d'électricité (204) ou de la source de dioxyde de carbone (206).

5. Le procédé selon la revendication 1, dans lequel la barrière (220) comprend une membrane électrolytique en polymère solide.

6. Le procédé selon la revendication 1, dans lequel la cathode poreuse électriquement conductrice (216) comprend une mousse de carbone réticulé.

7. Le procédé selon la revendication 1, dans lequel la culture comprend des *Archaea* du sous-domaine *Euryarcheaota,* éventuellement dans lequel la culture est une monoculture d'*Euryarcheaota.*

8. Le procédé selon la revendication 7, dans lequel les *Archaea* sont constituées de *Methanothermobacter thermautotrophicus.*

9. Le procédé de la revendication 1, dans lequel le collecteur de courant couplé à la cathode (230) est un disque solide de matière qui maintient une condition d'étanchéité au sein de la première chambre (212) entre l'entrée pour le dioxyde de carbone (234) et la sortie pour le méthane (236).

10. Le procédé de la revendication 1, dans lequel le collecteur de courant pour l'anode (232) définit une couche poreuse de diffusion gazeuse sur laquelle est disposé un catalyseur d'anode, la couche poreuse de diffusion gazeuse permettant aux sous-produits gazeux de sortir de la seconde chambre (214).

11. Le procédé de la revendication 1, dans lequel la culture réside au sein du milieu électrolytique aqueux circulant.

12. Le procédé de la revendication 1, dans lequel la culture est liée à la cathode poreuse (216).

13. Le procédé de la revendication 1, dans lequel l'eau est un donneur primaire net d'électrons pour les microorganismes méthanogènes.

14. Le procédé selon la revendication 1, dans lequel une source de carbone organique est absente du milieu.
